# EUROPEAN PATENT APPLICATION

(11) **EP 1 541 576 A1**
(43) Date of publication of application: **15.06.2005**
(21) Application number: 03741560.1
(22) Date of filing: 24.07.2003
(51) Int. Cl.: C07D 491/048

(54) **FUROISOQUINOLINE DERIVATIVE AND USE THEREOF**

(30) Priority: 26.07.2002 JP 2002217496
(71) Applicant: Takeda Pharmaceutical Company Limited, Osaka 541-0045 (JP)
(72) Inventor: INOUE, Yoshihisa c/o Takeda Pharmaceutical Co.Ltd., Osaka-shi, Osaka 532-8686 (JP); FUJII, Nobuhiro c/o Takeda Pharmaceutical Co.Ltd., Osaka-shi, Osaka 532-8686 (JP); GYOTEN, Michiyo c/o Takeda Pharmaceutical Co. Ltd., Osaka-shi, Osaka 532-8686 (JP); MATSUMOTO, Tatsumi c/oTakeda Pharmaceutical Co.Ltd, Osaka-shi, Osaka 532-8686 (JP)
(74) Representative: Lewin, John Harvey
(86) International application number: PCT/JP2003/009386
(87) International publication number: WO 2004/011470

(57) **Abstract**

The present invention provides a compound represented by the formula 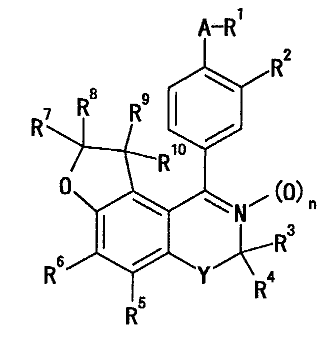 wherein A represents (1) a bond, (2) a group represented by the formula -CR^{a}=CR^{b}- (R^{a} and R^{b} each represent a hydrogen atom or C₁₋₆ alkyl) and the like; R¹ represents (1) cyano or (2) an optionally esterified or amidated carboxyl group; R² represents
(1) a hydrogen atom, (2) an optionally substituted hydroxy group,
(3) an optionally substituted amino group and the like; R³ and R⁴ each represent a hydrogen atom and the like; R⁵ represents a hydrogen atom and the like; R⁶ represents an optionally substituted hydroxy group and the like; R⁷and R⁸ each represent an optionally substituted hydrocarbon group and the like; R⁹ and R¹⁰ each represent (1) a hydrogen atom and the like; Y represents an optionally substituted methylene group; and n represents 0 or 1, or a salt thereof, which has an excellent phosphodiesterase IV inhibiting action.

## Description

### TECHNICAL FIELD

The present invention relates to a novel furoisoquinoline derivative which has a phosphodiesterase IV-inhibiting action, and is useful as a prophylactic and/or therapeutic agent against inflammatory diseases, atopic dermatitis, allergic rhinitis, asthma, chronic obstructive pulmonary diseases, chronic rheumatoid arthritis, autoimmune diseases, depression, Alzheimer's dementia, memory disorders, osteoporosis, diabetes, atherosclerosis and the like, and use thereof.

### BACKGROUND ART

These days, a large number of hormones and neurotransmitters function to increase or decrease the intracellular level of cyclic adenosine-3',5'-monophosphate (cAMP) which is an intracellular second messenger, whereby regulating the cellular functions. The intracellular cAMP level is regulated by a synthesizing enzyme and a degrading enzyme. Thus, cAMP is produced by adenyl cyclases and degraded by phosphodiesterase. This degrading enzyme also regulate the degradation of cyclic guanosine-3',5'-monophosphate (cGMP).

Eleven isozymes of the phosphodiesterase have been found so far [Proceedings of the National Academy of Sciences of the United States of America), Vol. 97, p. 3702 (2000)], and each functions to regulate the intracellular CAMP and cGMP levels in various cells such as those in central nervous system, circulatory organs, respiratory organs, digestive organs, genital organs, blood cells and tracheal smooth muscles, whereby controlling the cellular functions. It is also known that a phosphodiesterase isozyme referred to as phosphodiesterase IV exists predominantly in the inflammatory cells such as an eosinophil, neutrophil, monocyte, T-lymphocyte and macrophage [Clinical and Experimental Allergy, Vol.22, p. 337 (1992)].

Therapeutic agents against a bronchial asthma can be broadly classified into three groups. That is, the three groups include bronchodilators (e.g., β-adrenaline receptor agonists), the antiinflammatory agents (e.g., corticosteroids) and xanthine derivatives having both of the bronchodilating effect and the antiinflammatory effect (e.g., theophylline). Among these, theophylline has been used as a therapeutic agent against asthma for a long time. Theophylline has drawn attention recently since its bronchodilating effect has been found to be based on the phosphodiesterase-inhibiting effect. However, theophylline is a non-selective phosphodiesterase inhibitor and sometimes exhibits a cardiovascular side effect. Thus, its blood level should strictly be controlled to reduce the side effect. Accordingly, a medicament for treating an inflammatory disease such as asthma is desired to be one which inhibits the phosphodiesterase IV selectively and which has no effects on other isozymes of the phosphodiesterase.

A study result was reported to show a possibility that a phosphodiesterase IV-selective inhibitor is an effective therapeutic agent against an inflammatory disease such as asthma [Pulmonary Pharmacology, Vol. 7, p. 1 (1994)]. The compounds which are known to be evaluated clinically to have the phosphodiesterase IV-selective inhibiting action include Arofylline, Cilomilast, Roflumilast, V-11294A, CDC-801, BAY 19-8004, Cipamfylline, SCH-351591, PD 189659 and the like (Annual Reports in Medicinal Chemistry, Vol. 36, p41-56, (2001)).

Furthermore, as a compound having the phosphodiesterase IV-selective inhibiting action, a nicotinamide derivative is described in WO 01/57036, and a furoisoquinoline derivative in WO 01/70746 and WO 03/000695.

As a furoisoquinoline derivative, WO 02/04455 discloses a compound represented by the formula 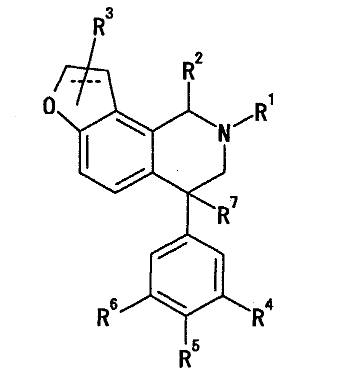 [wherein R¹ is C₁₋₆ alkyl, C₂₋₆ alkenyl, C₂₋₆ alkynyl, C₃₋₆ cycloalkyl, C₄₋₇ cycloalkylalkyl or benzyl (these may have a certain substituent),
R² is a hydrogen atom, C₁₋₆ alkyl, C₂₋₆ alkenyl, C₂₋₆ alkynyl, C₃₋₆ cycloalkyl, C₄₋₇ cycloalkylalkyl or C₁₋₆ haloalkyl,
R³ is a hydrogen atom, a halogen atom, C₁₋₆ alkyl, C₁₋₆ haloalkyl or C₃₋₆ cycloalkyl (the C₁₋₆ alkyl, C₁₋₆ haloalkyl or C₃₋₆ cycloalkyl may have a certain substituent),
R⁴, R⁵ and R⁶ are each independently a hydrogen atom, a halogen atom, nitro and the like, and
R⁷ is a hydrogen atom, a halogen atom, etc.] as a therapeutic agent for mental and/or neural deficiencies, based on an inhibitory action for reuptake of a norepinephrine transporter protein. Furthermore, WO 03/035650 discloses a compound represented by the formula 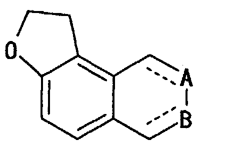 [wherein one of A and B is a nitrogen atom, and the other is a carbon atom, and 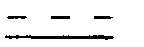 represents a single bond or a double bond] as an entry inhibitor.

A potent selective phosphodiesterase IV inhibitor is expected to have a sufficient prophylactic or therapeutic effect in a wide range of diseases accompanied with inflammations. The objective of the invention is to provide a novel heterocyclic compound which has selective phosphodiesterase IV-inhibiting effect and increases the intracellular cAMP level whereby exhibiting bronchodilating and antiinflammatory effects and which is also excellent in terms of safety, etc.

### DISCLOSURE OF INVENTION

The present inventors have made extensive studies, and firstly synthesized a novel furo[2,3-h]isoquinoline compound represented by having the formula 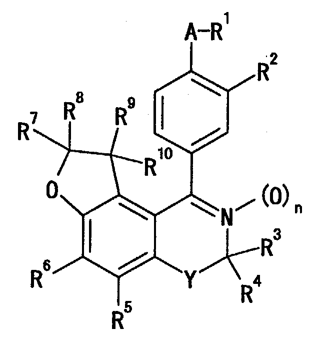 wherein A represents (1) a bond, (2) a group represented by the formula -CR^{a}=CR^{b}- (R^{a} and R^{b} each represent respectively a hydrogen atom or a C₁₋₆ alkyl group), (3) a group represented by the formula -(CONH)ₚ-(C(R^{c}) (R^{d}))_{q}- (R^{c} and R^{d} each represent a hydrogen atom or a C₁₋₆ alkyl group, p represents 0 or 1 and q represents 1 or 2), (4) a group represented by the formula-CH₂OCH₂- or (5) a group represented by the formula -OCH₂-;
R¹ represents (1) a cyano group or (2) an optionally esterified or amidated carboxyl group;
R² represents (1) a hydrogen atom, (2) an optionally substituted hydroxyl group, (3) an optionally substituted amino group, (4) an optionally substituted alkyl group, (5) an optionally esterified or amidated carboxyl group or (6) a nitro group, or R² and A or R¹ may be taken together with the adjacent carbon atom to form a ring;
R³ and R⁴ each represent (1) a hydrogen atom, (2) an optionally substituted hydrocarbon group or (3) an acyl group, or R³ and R⁴ may be taken together with the adjacent carbon atom to form an optionally substituted 3- to 8-membered ring;
R⁵ represents (1) a hydrogen atom, (2) a cyano group, (3) an optionally substituted hydrocarbon group, (4) an acyl group or (5) an optionally substituted hydroxyl group;
R⁶ represents (1) a hydrogen atom, (2) an optionally substituted hydrocarbon group, (3) an acyl group, (4) an optionally substituted heterocyclic group, (5) a halogen atom, (6) an optionally substituted hydroxyl group, (7) an optionally substituted thiol group, (8) a group represented by the formula-S(O)ᵣR¹¹ (R¹¹ represents an optionally substituted hydrocarbon group or an optionally substituted heterocyclic group, and r represents 1 or 2) or (9) an optionally substituted amino group;
R⁷ and R⁸ each represent (1) a hydrogen atom or (2) an optionally substituted hydrocarbon group, or R⁷ and R⁸ may be taken together with the adjacent carbon atom to form an optionally substituted 3- to 8-membered ring;
R⁹ and R¹⁰ are each (1) a hydrogen atom or (2) an optionally substituted hydrocarbon group;
Y represents an optionally substituted methylene group; and n represents 0 or 1,
provided that if A is a bond, R² is not a hydrogen atom, and if A is a group represented by the formula -(CONH)ₚ-(C(R^{c}) (R^{d}))_{q}- (R^{c} and R^{d} each represent a hydrogen atom or a C₁₋₆ alkyl group, p represents 0 or 1 and q represents 1 or 2), R⁶ is not methoxy, or a salt thereof, which is characterized by having (1) cyano or (2) an optionally esterified or amidated carboxyl group at the end of the substituent at the position 4 of the phenyl group which is a substituent at the position 1 of the furo[2,3-h]isoquinoline. Furthermore, the present inventors have found unexpectedly that such compound, based on the characteristic chemical structure, has an excellent phosphodiesterase IV-inhibiting action and is used as a prophylactic and/or therapeutic agent against inflammatory diseases, atopic dermatitis, asthma, chronic obstructive pulmonary diseases, chronic rheumatoid arthritis, autoimmune diseases, depression, Alzheimer's dementia, memory disorders, diabetes or atherosclerosis. The present inventors have made a further study based on these findings, and finally reached completion of the present invention.

That is, the present invention relates to:
[1] A compound represented by the formula 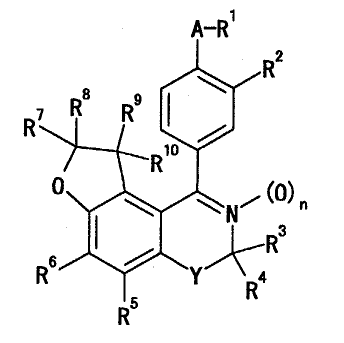 wherein A represents (1) a bond, (2) a group represented by the formula -CR^{a}=CR^{b}- (R^{a} and R^{b} each represent a hydrogen atom or a C₁₋₆ alkyl group), (3) a group represented by the formula - (CONH)ₚ-(C(R^{c}) (R^{d}))_{q}- (R^{c} and R^{d} each represent a hydrogen atom or a C₁₋₆ alkyl group, p represents 0 or 1 and q represents 1 or 2), (4) a group represented by the formula -CH₂OCH₂- or (5) a group represented by the formula -OCH₂-;
   R¹ represents (1) a cyano group or (2) an optionally esterified or amidated carboxyl group;
   R² represents (1) a hydrogen atom, (2) an optionally substituted hydroxyl group, (3) an optionally substituted amino group, (4) an optionally substituted alkyl group, (5) an optionally esterified or amidated carboxyl group or (6) a nitro group, or R² and A or R¹ may be taken together with the adjacent carbon atom to form a ring;
   R³ and R⁴ each represent (1) a hydrogen atom, (2) an optionally substituted hydrocarbon group or (3) an acyl group, or R³ and R⁴ may be taken together with the adjacent carbon atom to form an optionally substituted 3- to 8-membered ring;
   R⁵ represents (1) a hydrogen atom, (2) a cyano group, (3) an optionally substituted hydrocarbon group, (4) an acyl group or (5) an optionally substituted hydroxyl group;
   R⁶ represents (1) a hydrogen atom, (2) an optionally substituted hydrocarbon group, (3) an acyl group, (4) an optionally substituted heterocyclic group, (5) a halogen atom, (6) an optionally substituted hydroxyl group, (7) an optionally substituted thiol group, (8) a group represented by the formula-S(O)ᵣR¹¹ (R¹¹ represents an optionally substituted hydrocarbon group or an optionally substituted heterocyclic group, and r represents 1 or 2) or (9) an optionally substituted amino group;
   R⁷ and R⁸ each represent (1) a hydrogen atom or (2) an optionally substituted hydrocarbon group, or R⁷ and R⁸ may be taken together with the adjacent carbon atom to form an optionally substituted 3- to 8-membered ring;
   R⁹ and R¹⁰ each represent (1) a hydrogen atom or (2) an optionally substituted hydrocarbon group; Y represents an optionally substituted methylene group; and
   n represents 0 or 1,
   provided that if A is a bond, R² is not a hydrogen atom, and if A is a group represented by the formula -(CONH)ₚ-(C(R^{c}) (R^{d}))_{q}- (R^{c} and R^{d} each represent a hydrogen atom or a C₁₋₆ alkyl group, p represents 0 or 1 and q represents 1 or 2), R⁶ is not methoxy, or a salt thereof;
[2] the compound as described in the above-mentioned [1], wherein R¹ is (i) a cyano group, (ii) a carboxyl group, (iii) a C₁₋₆ alkoxy-carbonyl group which may have 1 to 5 substituents selected from a group consisting of (1) a halogen atom, (2) a C₁₋₃ alkylenedioxy group, (3) a nitro group, (4) a cyano group, (5) an optionally halogenated C₁₋₆ alkyl group, (6) an optionally halogenated C₂₋₆ alkenyl group, (7) an optionally halogenated C₂₋₆ alkynyl group, (8) a C₃₋₈ cycloalkyl group, (9) a C₆₋₁₄ aryl group, (10) an optionally halogenated C₁₋₆ alkoxy group, (11) an optionally halogenated C₁₋₆ alkylthio group, (12) a hydroxyl group, (13) an amino group, (14) a mono-C₁₋₆ alkylamino group, (15) a mono-C₆₋₁₄ arylamino group, (16) a di-C₁₋₆ alkylamino group, (17) a di-C₆₋₁₄ arylamino group, (18) an acyl group selected from formyl, carboxyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, sulfino, sulfo, C₁₋₆ alkoxysulfinyl, C₆₋₁₄ aryloxysulfinyl, C₁₋₆ alkoxysulfonyl and C₆₋₁₄ aryloxysulfonyl, (19) an acylamino group selected from formylamino, C₁₋₆ alkyl-carboxamide, C₆₋₁₄ aryl-carboxamide, C₁₋₆ alkoxy-carboxamide, C₁₋₆ alkylsulfonylamino and C₆₋₁₄ arylsulfonylamino, (20) an acyloxy group selected from C₁₋₆ alkyl-carbonyloxy, C₆₋₁₄ aryl-carbonyloxy, C₁₋₆ alkoxy-carbonyloxy, mono-C₁₋₆ alkyl-carbamoyloxy, di-C₁₋₆ alkyl-carbamoyloxy, mono-C₆₋₁₄ aryl-carbamoyloxy, di-C₆₋₁₄ aryl-carbamoyloxy and nicotinoyloxy, (21) a 5- to 14-membered heterocyclic group containing 1 to 4 hetero atoms selected from a nitrogen atom, a sulfur atom and an oxygen atom in addition to carbon atoms, (22) a phosphono group, (23) a C₆₋₁₄ aryloxy group, (24) a di-C₁₋₆ alkoxy-phosphoryl group, (25) a C₆₋₁₄ arylthio group, (26) a hydrazino group, (27) an imino group, (28) an oxo group, (29) a ureido group, (30) a C₁₋₆ alkyl-ureido group, (31) a di-C₁₋₆ alkyl-ureido group, (32) an oxide group and (33) a group formed by the binding of 2 or 3 groups selected from (1) to (32) listed above and the like (hereinafter, simply referred to as Substituent group A), (iv) a C₃₋₈ cycloalkyloxy-carbonyl group which may have 1 to 5 substituents selected from Substituent group A described above, (v) a C₇₋₁₆ aralkyloxy-carbonyl group which may have 1 to 5 substituents selected from Substituent group A described above, (vi) a C₆₋₁₄ aryloxy-carbonyl group which may have 1 to 5 substituents selected from Substituent group A described above, (vii) a carbamoyl group, (viii) a mono-C₁₋₆ alkyl-carbamoyl group which may have 1 to 5 substituents selected from Substituent group A described above, (ix) a di-C₁₋₆ alkyl-carbamoyl group which may have 1 to 5 substituents selected from Substituent group A described above, (x) a mono-C₆₋₁₄ aryl-carbamoyl group which may have 1 to 5 substituents selected from Substituent group A described above or (xi) a di-C₆₋₁₄ aryl-carbamoyl group which may have 1 to 5 substituents selected from Substituent group A described above.
   R² is (i) a hydrogen atom, (ii) a group represented by the formula -OR¹² (R¹² represents (a) a hydrogen atom, (b) a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A described above, or (c) an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above), (iii) a group represented by the formula-NR¹³R¹⁴ (R¹³ and R¹⁴ each represent (i') a hydrogen atom, (ii') a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A described above, (iii') an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above or (iv') a 5- to 14-membered heterocycle containing 1 to 4 hetero atoms selected from a nitrogen atom, a sulfur atom and an oxygen atom in addition to carbon atoms, which may have 1 to 5 substituents selected from Substituent group A described above, or R¹³ and R¹⁴ may be taken together with the adjacent a nitrogen atom to form a 5- to 14-membered ring), (iv) a C₁₋₆ alkylideneamino group which may have 1 to 5 substituents selected from Substituent group A described above, (v) a C₁₋₆ alkyl group which may have 1 to 5 substituents selected from Substituent group A described above, (vi) a carboxyl group, (vii) a C₁₋₆ alkoxy-carbonyl group which may have 1 to 5 substituents selected from Substituent group A described above, (viii) a C₃₋₈ cycloalkyloxy-carbonyl group which may have 1 to 5 substituents selected from Substituent group A described above, (ix) a C₇₋₁₆ aralkyloxy-carbonyl group which may have 1 to 5 substituents selected from Substituent group A described above, (x) a C₆₋₁₄ aryloxy-carbonyl group which may have 1 to 5 substituents selected from Substituent group A described above, (xi) a carbamoyl group, (xii) a mono-C₁₋₆ alkyl-carbamoyl group which may have 1 to 5 substituents selected from Substituent group A described above, (xiii) a di-C₁₋₆ alkyl-carbamoyl group which may have 1 to 5 substituents selected from Substituent group A described above, (xiv) a mono-C₆₋₁₄ aryl-carbamoyl group which may have 1 to 5 substituents selected from Substituent group A described above, (xv) a di-C₆₋₁₄ aryl-carbamoyl group which may have 1 to 5 substituents selected from Substituent group A described above or (xvi) a nitro group, or R² and A or R¹ may be taken together to form a 5- to 14-membered ring containing 1 to 4 hetero atoms selected from a nitrogen atom and an oxygen atom in addition to carbon atoms, which may have 1 to 5 substituents selected from Substituent group A described above;
   each of R³ and R⁴ is any of the following (i) to (iii):
   (i) a hydrogen atom,
   (ii) a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A described above,
   (iii) an acyl group selected from formyl, carboxyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, sulfino, sulfo, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above; or
      R³ and R⁴ may be taken together with the adjacent carbon atom to form C₃₋₈ cycloalkane or a 3- to 8-membered heterocycle, each of which may have 1 to 3 substituents selected from C₁₋₆ alkyl, C₆₋₁₄ aryl, C₇₋₁₆ aralkyl, amino, mono-C₁₋₆ alkylamino, mono-C₆₋₁₄ arylamino, di-C₁₋₆ alkylamino, di-C₆₋₁₄ arylamino and a 4- to 10-membered aromatic heterocyclic group,
      R⁵ is (i) a hydrogen atom, (ii) a cyano group, (iii) a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A described above, (iv) an acyl group selected from formyl, carboxyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, sulfino, sulfo, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above, or (v) a group represented by the formula -OR¹⁵ (R¹⁵ represents (a) a hydrogen atom, (b) a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A described above, or (c) an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above),
   R⁶ is any of the following (i) to (x):
   (i) a hydrogen atom,
   (ii) a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A described above,
   (iii) an acyl group selected from formyl, carboxyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, sulfino, sulfo, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above,
   (iv) a 5- to 14-membered heterocycle containing 1 to 4 hetero atoms selected from a nitrogen atom, a sulfur atom and an oxygen atom in addition to carbon atoms, which may have 1 to 5 substituents selected from Substituent group A described above,
   (v) a halogen atom,
   (vi) a group represented by the formula -OR¹⁶ (R¹⁶ represents (i') a hydrogen atom, (ii') a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A described above, (iii') an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above, or (iv') a 5- to 14-membered heterocycle containing 1 to 4 hetero atoms selected from a nitrogen atom, a sulfur atom and an oxygen atom in addition to carbon atoms, which may have 1 to 5 substituents selected from Substituent group A described above),
   (vii) a group represented by the formula -SR¹⁷ (R¹⁷ represents (i') a hydrogen atom, (ii') a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A described above, (iii') an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above or (iv') a 5- to 14-membered heterocycle containing 1 to 4 hetero atoms selected from a nitrogen atom, a sulfur atom and an oxygen atom in addition to carbon atoms, which may have 1 to 5 substituents selected from Substituent group A described above),
   (viii) a group represented by the formula -S(O)ᵣR¹¹ (R¹¹ represents (i') a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A described above or (ii') a 5- to 14-membered heterocycle containing 1 to 4 hetero atoms selected from a nitrogen atom, a sulfur atom and an oxygen atom in addition to carbon atoms, which may have 1 to 5 substituents selected from Substituent group A described above and r is 1 or 2) or
   (ix) a group represented by the formula =NR¹⁸R¹⁹ (R¹⁸ and R¹⁹ each represent (i') a hydrogen atom, (ii') a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A described above, (iii') an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above or (iv') a 5- to 14-membered heterocycle containing 1 to 4 hetero atoms selected from a nitrogen atom, a sulfur atom and an oxygen atom in addition to carbon atoms, which may have 1 to 5 substituents selected from Substituent group A described above),
   R⁷ and R⁸ are each (i) a hydrogen atom or (ii) a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A described above, or R⁷ and R⁸ may be taken together with the adjacent carbon atom to form C₃₋₈ cycloalkane or a 3- to 8-membered heterocycle, each of which may have 1 to 3 substituents selected from C₁₋₆ alkyl, C₆₋₁₄ aryl, C₇₋₁₆ aralkyl, amino, mono-C₁₋₆ alkylamino, mono-C₆₋₁₄ arylamino, di-C₁₋₆ alkylamino, di-C₆₋₁₄ arylamino and a 4- to 10-membered aromatic heterocyclic group;
   R⁹ and R¹⁰ are each (i) a hydrogen atom or (ii) a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A described above, and
   Y is a methylene group which may have 1 or 2 substituents selected from Substituent group A described above;
[3] the compound as described in the above-mentioned [1], wherein A is (1) a bond, (2) a group represented by the formula-CR^{a}=CR^{b}- (R^{a} and R^{b} each represent a hydrogen atom or a C₁₋₆ alkyl group), (3) a group represented by the formula -(CONH)ₚ-(C(R^{c}) (R^{d}))_{q}- (R^{c} and R^{d} each represent a hydrogen atom or a C₁₋₆ alkyl group, p represents 0 or 1 and q represents 1 or 2), (4) a group represented by the formula -CH₂OCH₂- or (5) a group represented by the formula -OCH₂-,
   R¹ is (1) a cyano group, (2) a carboxyl group, (3) a C₁₋₆ alkoxycarbonyl group, (4) a carbamoyl group or (5) an N-mono-C₁₋₆ alkylcarbamoyl group,
   R² is (1) a hydrogen atom, (2) a hydroxyl group, (3) a C₁₋₆ alkoxy group, (4) a C₇₋₁₆ aralkyloxy group, (5) an amino group, (6) a mono-C₁₋₆ alkylamino group which may have one substituent selected from carboxyl, carbamoyl, quinolyl, phenoxy and pyridyl, (7) a mono-C₇₋₁₆ aralkylamino group which may have one substituent selected from a halogen atom, cyano, C₁₋₆ alkoxy, carboxyl and C₁₋₆ alkoxycarbonyl, (8) a mono-C₆₋₁₄ arylamino group, (9) a mono-C₁₋₆ alkylcarbonylamino group which may have 1 to 3 substituents selected from a halogen atom, thienyl and C₁₋₆ alkoxycarbonyl-C₁₋₆ alkylthio, (10) a mono-C₁₋₆ alkylsulfonylamino group, (11) a mono-C₆₋₁₄ arylcarbonylamino group which may have one substituent selected from C₁₋₆ alkoxy and C₁₋₆ alkylcarbonylamino, (12) a quinolylcarbonylamino group, (13) a pyridylcarbonylamino group which may have 1 or 2 halogen atoms, (14) an indolylcarbonylamino group, (15) a N-C₁₋₆ alkyl-N-C₁₋₆ alkylcarbonylamino group which may have 1 to 4 substituents selected from a halogen atom, C₁₋₆ alkoxycarbonyl and quinolyl, (16) a N-C₁₋₆ alkylcarbonyl-N-C₇₋₁₆ aralkylamino group which may have 1 to 3 halogens, (17) a N-C₁₋₆ alkyl-N-pyridylcarbonylamino group, (18) a C₁₋₆ alkylideneamino group which may have one di-C₁₋₆ alkylamino, (19) a mono-C₁₋₆ alkylureido group which may have one C₁₋₆ alkoxycarbonyl, (20) a di-C₁₋₆ alkylureido, (21) a mono-C₆₋₁₄ arylureido group, (22) a 1-imidazolidinyl group which may have 1 to 3 substituents selected from C₁₋₆ alkyl and oxo, (23) a C₁₋₆ alkyl group, (24) a C₁₋₆ alkoxycarbonyl group, (25) a nitro group or (26) a 1-pyrrolidinyl group, or R² and A or R¹ may be taken together with the adjacent carbon atom to form a nitrogen-containing 5- to 7-membered ring which may have 1 to 3 substituents selected from (1) a hydroxyl group, (2) C₁₋₆ alkyl which may have one C₁₋₆ alkoxy-carbonyl, (3) C₇₋₁₆ aralkyl, (4) C₆₋₁₄ aryl and (5) oxo,
   R³ and R⁴ are each a C₁₋₆ alkyl group,
   R⁵ is a hydrogen atom,
   R⁶ is a C₁₋₆ alkoxy group,
   R⁷ and R⁸ are each a C₁₋₆ alkyl group,
   R⁹ and R¹⁰ are each a hydrogen atom,
   Y is a methylene group and
   n is 0;
[4] a compound represented by the formula 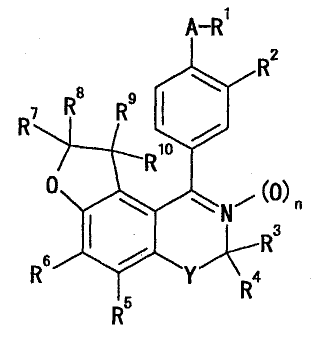 wherein A is (1) a bond, (2) a group represented by the formula -CH=CH-, (3) a group represented by the formula -CONH-C(R^{c}) (R^{d})- (R^{c} and R^{d} each represent a hydrogen atom or a C₁₋₆ alkyl group), or (4) a group represented by the formula -OCH₂-,
   R¹ is (1) a cyano group, (2) a carboxyl group, (3) a C₁₋₆ alkoxycarbonyl group, (4) a carbamoyl group or (5) an N-mono-C₁₋₆ alkylcarbamoyl group,
   R² is (1) a hydroxyl group, (2) a C₁₋₆ alkoxy group, (3) a C₇₋₁₆ aralkyloxy group, (4) an amino group, (5) a mono-C₁₋₆ alkylamino group which may have one substituent selected from carboxyl, carbamoyl, quinolyl, phenoxy and pyridyl, (6) a mono-C₇₋₁₆ aralkylamino group which may have one substituent selected from a halogen atom, cyano, C₁₋₆ alkoxy, carboxyl and C₁₋₆ alkoxycarbonyl, (7) a mono-C₆₋₁₄ arylamino group, (8) a mono-C₁₋₆ alkylcarbonylamino group which may have 1 to 3 substituents selected from a halogen atom, thienyl and C₁₋₆ alkoxycarbonyl-C₁₋₆ alkylthio, (9) a mono-C₁₋₆ alkylsulfonylamino group, (10) a mono-C₆₋₁₄ arylcarbonylamino group which may have one substituent selected from C₁₋₆ alkoxy and C₁₋₆ alkylcarbonylamino, (11) a quinolylcarbonylamino group, (12) a pyridylcarbonylamino group which may have 1 or 2 halogen atoms, (13) an indolylcarbonylamino group, (14) a N-C₁₋₆ alkyl-N-C₁₋₆ alkylcarbonylamino group which may have 1 to 4 substituents selected from a halogen atom, C₁₋₆ alkoxycarbonyl and quinolyl, (15) a N-C₁₋₆ alkylcarbonyl-N-C₇₋₁₆ aralkylamino group which may have 1 to 3 halogens, (16) a N-C₁₋₆ alkyl-N-pyridylcarbonylamino group, (17) a C₁₋₆ alkylideneamino group which may have one di-C₁₋₆ alkylamino, (18) a mono-C₁₋₆ alkylureido group which may have one C₁₋₆ alkoxycarbonyl, (19) a di-C₁₋₆ alkylureido group, (20) a mono-C₆₋₁₄ arylureido group, (21) a 1-imidazolidinyl group which may have 1 to 3 substituents selected from C₁₋₆ alkyl and oxo, (22) a C₁₋₆ alkyl group, (23) a C₁₋₆ alkoxycarbonyl group, (24) a nitro group or (25) a 1-pyrrolidinyl group, or
   R² and A or R¹ may be taken together with the adjacent carbon atom to form a nitrogen-containing 5- to 7-membered ring which may have 1 to 3 substituents selected from (1) a hydroxyl group, (2) a C₁₋₆ alkyl group which may have one C₁₋₆ alkoxy-carbonyl, (3) C₇₋₁₆ aralkyl group, (4) a C₆₋₁₄ aryl group and (5) an oxo group,
   R³ and R⁴ are each a C₁₋₆ alkyl group,
   R⁵ is a hydrogen atom,
   R⁶ is a C₂₋₆ alkoxy group,
   R⁷ and R⁸ are each a C₁₋₆ alkyl group,
   R⁹ and R¹⁰ are each a hydrogen atom,
   Y is a methylene group, and
   n is 0, or a salt thereof;
[5] a compound represented by the formula 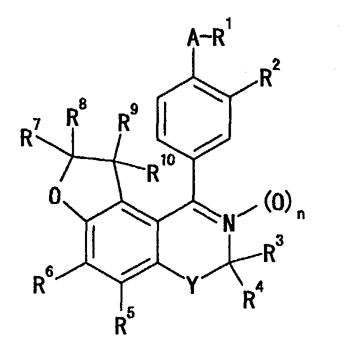 wherein A is (1) a group represented by the formula -CR^{a}=CR^{b}- (R^{a} and R^{b} each represent a hydrogen atom or a C₁₋₆ alkyl group), (2) a group represented by the formula -(CONH)ₚ-(C(R^{c})(R^{d}))_{q}- (R^{c} and R^{d} each represent a hydrogen atom or a C₁₋₆ alkyl group, p represents 0 or 1 and q represents 1 or 2), (3) a group represented by the formula -CH₂OCH₂- or (4) a group represented by the formula -OCH₂-,
   R¹ is (1) a carboxyl group, (2) a C₁₋₆ alkoxycarbonyl group, (3) an N-mono-C₁₋₆ alkylcarbamoyl group or (4) a carbamoyl group,
   R² is a hydrogen atom,
   R³ and R⁴ are each a C₁₋₆ alkyl group,
   R⁵ is a hydrogen atom,
   R⁶ is a C₂₋₆ alkoxy group,
   R⁷ and R⁸ are each a C₁₋₆ alkyl group,
   R⁹ and R¹⁰ are each a hydrogen atom,
   Y is a methylene group, and
   n is 0, or a salt thereof;
[6] the compound as described in the above-mentioned [4], wherein A is (1) a bond or (2) a group represented by the formula -CH=CH-;
[7] the compound as described in the above-mentioned [5], wherein A is (1) a group represented by the formula -CH=CH-, (2) a group represented by the formula -(C(R^{c}) (R^{d}))- (R^{c} and R^{d} each represent a hydrogen atom or a C₁₋₆ alkyl group) or (3) a group represented by the formula -CH₂OCH₂-;
[8] the compound as described in the above-mentioned [4], wherein R¹ is a carboxyl group or a carbamoyl group;
[9] the compound as described in the above-mentioned [5], wherein R¹ is a carboxyl group;
[10] the compound as described in the above-mentioned [4], wherein R² is (1) a C₁₋₆ alkoxy group, (2) a mono-C₁₋₆ alkylamino group, (3) a mono-C₇₋₁₆ aralkylamino group, (4) a quinolylcarbonylamino group or (5) a pyridylcarbonylamino group;
[11] the compound as described in the above-mentioned [4] or [5], wherein R³ and R⁴ are each methyl;
[12] the compound as described in the above-mentioned [4] or [5], wherein R⁶ is ethoxy;
[13] the compound as described in the above-mentioned [4] or [5], wherein R⁷ and R⁸ are each methyl;
[14] the compound as described in the above-mentioned [4], which is 4-(6-ethoxy-3,4,8,9-tetrahydro-3,3,8,8-tetramethylfuro[2,3-h]isoquinolin-1-yl)-2-[(phenylmethyl)amino]benzoic acid, 4-(6-ethoxy-3,4,8,9-tetrahydro-3,3,8,8-tetramethylfuro[2,3-h]isoquinolin-1-yl)-2-(ethylamino)benzoic acid, (E)-3-[4-(6-ethoxy-3,4,8,9-tetrahydro-3,3,8,8-tetramethylfuro[2,3-h]isoquinolin-1-yl)-2-methoxyphenyl]-2-propenoic acid, 4-(6-ethoxy-3,4,8,9-tetrahydro-3,3,8,8-tetramethylfuro[2,3-h]isoquinolin-1-yl)-2-[(2-quinolinylcarbonyl)amino]benzoic acid, 4-(6-ethoxy-3,4,8,9-tetrahydro-3,3,8,8-tetramethylfuro[2,3-h]isoquinolin-1-yl)-2-[(2-pyridinylcarbonyl)amino]benzene acetic acid, N-[2-(aminocarbonyl)-5-(6-ethoxy-3,4,8,9-tetrahydro-3,3,8,8-tetramethylfuro[2,3-h]isoquinolin-1-yl)phenyl]-2-pyridinecarboxamide or a salt thereof;
[15] the compound as described in the above-mentioned [5], which is [[4-(6-ethoxy-3,4,8,9-tetrahydro-3,3,8,8-tetramethylfuro[2,3-h]isoquinolin-1-yl)phenyl]methoxy]acetic acid, 4-(6-ethoxy-3,4,8,9-tetrahydro-3,3,8,8-tetramethylfuro[2,3-h]isoquinolin-1-yl)-α,α-dimethylbenzene acetic acid or a salt thereof;
[16] the pharmaceutical composition comprising the compound as described in the above-mentioned [1] or a prodrug thereof;
[17] the pharmaceutical composition as described in the above-mentioned [16], which is a phosphodiesterase IV inhibitor;
[18] the pharmaceutical composition as described in the above-mentioned [16], which is a prophylactic and/or therapeutic agent against inflammatory diseases, atopic dermatitis, allergic rhinitis, asthma, chronic obstructive pulmonary diseases, chronic rheumatoid arthritis, autoimmune diseases, depression, Alzheimer's dementia, memory disorders, osteoporosis, diabetes or atherosclerosis;
[19] a method of inhibiting phosphodiesterase IV which is characterized by administering to a mammal an effective amount of the compound as described in the above-mentioned [1] or a prodrug thereof;
[20] a method of preventing and/or treating inflammatory diseases, atopic dermatitis, allergic rhinitis, asthma, chronic obstructive pulmonary diseases, chronic rheumatoid arthritis, autoimmune diseases, depression, Alzheimer's dementia, memory disorders, osteoporosis, diabetes or atherosclerosis which is characterized by administering to a mammal an effective amount of the compound as described in the above-mentioned [1] or a prodrug thereof;
[21] use of the compound as described in the above-mentioned

[1] or a prodrug thereof for manufacturing a phosphodiesterase IV inhibitor;
[22] use of the compound as described in the above-mentioned [1] or a prodrug thereof for manufacturing a prophylactic and/or therapeutic agent against inflammatory diseases, atopic dermatitis, allergic rhinitis, asthma, chronic obstructive pulmonary diseases, chronic rheumatoid arthritis, autoimmune diseases, depression, Alzheimer's dementia, memory disorders, osteoporosis, diabetes or atherosclerosis; etc.

In the above formulae, A is (1) a bond, (2) a group represented by the formula -CR^{a}=CR^{b}- (R^{a} and R^{b} each represent a hydrogen atom or a C₁₋₆ alkyl group), (3) a group represented by the formula -(CONH)ₚ-(C(R^{c})(R^{d}))_{q}- (R^{c} and R^{d} each represent a hydrogen atom or a C₁₋₆ alkyl group, p represents 0 or 1 and q represents 1 or 2), (4) a group represented by the formula-CH₂OCH₂- or (5) a group represented by the formula -OCH₂-.

The C₁₋₆ alkyl group represented by R^{a}, R^{b}, R^{c} and R^{d} includes, for example, methyl, ethyl, propyl, isopropyl, butyl, isobutyl, sec-butyl, tert-butyl, pentyl, hexyl and the like.

The group represented by the "formula -CR^{a}=CR^{b}- (R^{a} and R^{b} each represent a hydrogen atom or a C₁₋₆ alkyl group)" includes, for example, a group represented by -CH=CH-, -C(C₁₋₆ alkyl)=CH-,-CH=C(C₁₋₆ alkyl) - or -C(C₁₋₆ alkyl)=C(C₁₋₆ alkyl)-, and among these, preferably -CH=CH-, -C(C₁₋₆ alkyl)=CH-, -CH=C(C₁₋₆ alkyl)-, particularly preferably a group represented by -CH=CH-,-C(methyl)=CH- or -CH=C (methyl)-.

The group represented by the "formula -(CONH)ₚ-(C(R^{c})(R^{d}))_{q}- (R^{c} and R^{d} each represent a hydrogen atom or a C₁₋₆ alkyl group, p is 0 or 1 and q is 1 or 2)" includes, for example, a group represented by -CH₂-, -CH(C₁₋₆ alkyl)-, -C(C₁₋₆ alkyl) (C₁₋₆ alkyl)-, -(CH₂)₂-, -(CH(C₁₋₆ alkyl))₂-, -(C(C₁₋₆ alkyl) (C₁₋₆ alkyl))₂-, -CONH-CH₂-, -CONH-CH(C₁₋₆ alkyl)-, -CONH-C(C₁₋₆ alkyl) (C₁₋₆ alkyl)-, -CONH-(CH₂)₂-, -CONH-(CH(C₁₋₆ alkyl))₂- or -CONH-(C(C₁₋₆ alkyl) (C₁₋₆ alkyl))₂-, and among these, preferably -CH₂-, -C(C₁₋₆ alkyl) (C₁₋₆ alkyl)-, -(CH₂)₂-, -CONH-CH₂-, -CONH-C(C₁₋₆ alkyl) (C₁₋₆ alkyl)-, particularly a group represented by -CH₂-, -C(methyl)₂-, -(CH₂)₂-, -CONH-CH₂- or -CONH-C (methyl)₂-.

A is preferably (1) a bond, (2) a group represented by the formula -CH=CH-, (3) a group represented by the formula-C(R^{c}) (R^{d})- (R^{c} and R^{d} each represent a hydrogen atom or a C₁₋₆ alkyl group) or (4) a group represented by the formula -CH₂OCH₂-, and among these, more preferably (1) a bond, (2) a group represented by the formula -CH=CH-, (3) a group represented by the formula-C(methyl)₂- or (4) a group represented by the formula -CH₂OCH₂-.

In the above formulae, R¹ is (1) a cyano group or (2) an optionally esterified or amidated carboxyl group.

The "optionally esterified or amidated carboxyl group" represented by R¹ includes, for example, (i) a carboxyl group, (ii) a C₁₋₆ alkoxy-carbonyl group which may have 1 to 5 substituents selected from (1) a halogen atom, (2) a C₁₋₃ alkylenedioxy group, (3) a nitro group, (4) a cyano group, (5) an optionally halogenated C₁₋₆ alkyl group, (6) an optionally halogenated C₂₋₆ alkenyl group, (7) an optionally halogenated C₂₋₆ alkynyl group, (8) a C₃₋₈ cycloalkyl group, (9) a C₆₋₁₄ aryl group, (10) an optionally halogenated C₁₋₆ alkoxy group, (11) an optionally halogenated C₁₋₆ alkylthio group, (12) a hydroxyl group, (13) an amino group, (14) a mono-C₁₋₆ alkylamino group, (15) a mono-C₆₋₁₄ arylamino group, (16) a di-C₁₋₆ alkylamino group, (17) a di-C₆₋₁₄ arylamino group, (18) an acyl group selected from formyl, carboxyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, sulfino, sulfo, C₁₋₆ alkoxysulfinyl, C₆₋₁₄ aryloxysulfinyl, C₁₋₆ alkoxysulfonyl and C₆₋₁₄ aryloxysulfonyl, (19) an acylamino group selected from formylamino, C₁₋₆ alkyl-carboxamide, C₆₋₁₄ aryl-carboxamide, C₁₋₆ alkoxy-carboxamide, C₁₋₆ alkylsulfonylamino and C₆₋₁₄ arylsulfonylamino,
(20) an acyloxy group selected from C₁₋₆ alkyl-carbonyloxy, C₆₋₁₄ aryl-carbonyloxy, C₁₋₆ alkoxy-carbonyloxy, mono-C₁₋₆ alkyl-carbamoyloxy, di-C₁₋₆ alkyl-carbamoyloxy, mono-C₆₋₁₄ aryl-carbamoyloxy, di-C₆₋₁₄ aryl-carbamoyloxy and nicotinoyloxy, (21) a 5- to 14-membered heterocyclic group containing 1 to 4 hetero atoms selected from a nitrogen atom, a sulfur atom and an oxygen atom in addition to carbon atoms, (22) a phosphono group, (23) a C₆₋₁₄ aryloxy group, (24) a di-C₁₋₆ alkoxy-phosphoryl group, (25) a C₆₋₁₄ arylthio group, (26) a hydrazino group, (27) an imino group, (28) an oxo group (except the case that it forms a ring together with a hydrocarbon group), (29) a ureido group, (30) a C₁₋₆ alkyl-ureido group, (31) a di-C₁₋₆ alkyl-ureido group, (32) an oxide group and (33) a group formed by binding of 2 or 3 groups selected from (1) to (32) listed above and the like a group consisting of (hereinafter, abbreviated as Substituent group A), (iii) a C₃₋₈ cycloalkyloxy-carbonyl group which may have 1 to 5 substituents selected from Substituent group A described above, (iv) a C₇₋₁₆ aralkyloxy-carbonyl group which may have 1 to 5 substituents selected from Substituent group A described above, (v) a C₆₋₁₄ aryloxy-carbonyl group which may have 1 to 5 substituents selected from Substituent group A described above, (vi) a carbamoyl group, (vii) a mono-C₁₋₆ alkyl-carbamoyl group which may have 1 to 5 substituents selected from Substituent group A described above, (viii) a di-C₁₋₆ alkyl-carbamoyl group which may have 1 to 5 substituents selected from Substituent group A described above, (ix) a mono-C₆₋₁₄ aryl-carbamoyl group which may have 1 to 5 substituents selected from Substituent group A described above or (x) a di-C₆₋₁₄ aryl-carbamoyl group which may have 1 to 5 substituents selected from Substituent group A described above and the like.

The "halogen atom" in the above-mentioned Substituent group A includes, for example, fluorine, chlorine, bromine and iodine.

The "C₁₋₃ alkylenedioxy group" in the above-mentioned Substituent group A includes, for example, methylenedioxy, ethylenedioxy and propylenedioxy.

The "optionally halogenated C₁₋₆ alkyl group" in the above-mentioned Substituent group A is, for example, a C₁₋₆ alkyl group (e.g., methyl, ethyl, propyl, isopropyl, butyl, isobutyl, sec-butyl, tert-butyl, pentyl, hexyl and the like) which may have 1 to 3 halogen atoms (e.g., fluorine, chlorine, bromine and iodine) and the like, and specifically methyl, chloromethyl, difluoromethyl, trichloromethyl, trifluoromethyl, ethyl, 2-bromoethyl, 2,2,2-trifluoroethyl, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl or the like.

The "optionally halogenated C₂₋₆ alkenyl group" in the above-mentioned Substituent group A includes, for example, a C₂₋₆ alkenyl group (e.g., vinyl, allyl, isopropenyl, 2-butenyl, 2-methyl-2-propenyl, 4-pentenyl, 5-hexenyl and the like) which may have 1 to 3 halogen atoms (e.g., fluorine, chlorine, bromine and iodine) and the like.

The "optionally halogenated C₂₋₆ alkynyl group" in the above-mentioned Substituent group A includes, for example, a C₂₋₆ alkynyl group (e.g., propargyl, ethynyl, 2-butynyl, 2-hexynyl and the like) which may have 1 to 3 halogen atoms (e.g., fluorine, chlorine, bromine and iodine) and the like.

The "C₃₋₈ cycloalkyl group" in the above-mentioned Substituent group A includes, for example, cyclopropyl, cyclobutyl, cyclopentyl, cyclohexyl, cycloheptyl, cyclooctyl and the like.

The "C₆₋₁₄ aryl group" in the above-mentioned Substituent group A includes, for example, phenyl, 1-naphthyl, 2-naphthyl, 1-anthryl, 2-anthryl, 9-anthryl, 1-phenanthryl, 2-phenanthryl, 3-phenanthryl, 4-phenanthryl, 9-phenanthryl and the like.

The "optionally halogenated C₁₋₆ alkoxy group" in the above-mentioned Substituent group A includes, for example, a C₁₋₆ alkoxy group (e.g., methoxy, ethoxy, propoxy, isopropoxy, n-butoxy, isobutoxy, sec-butoxy, tert-butoxy, pentyloxy, isopentyloxy, neopentyloxy and the like) which may have 1 to 3 halogen atoms (e.g., fluorine, chlorine, bromine and iodine) and the like, and specifically methoxy, ethoxy, propoxy, isopropoxy, butoxy, isobutoxy, sec-butoxy, tert-butoxy, trichloromethoxy, 3,3,3-trifluoropropoxy, 4,4,4-trifluorobutoxy, 5,5,5-trifluoropentyloxy, 6,6,6-trifluorohexyloxy and the like.

The "optionally halogenated C₁₋₆ alkylthio group" in the above-mentioned Substituent group A includes, for example, a C₁₋₆ alkylthio group (e.g., methylthio, ethylthio, propylthio, isopropylthio, butylthio, isobutylthio, sec-butylthio, tert-butylthio and the like) which may have 1 to 3 halogen atoms (e.g., fluorine, chlorine, bromine and iodine) and the like, and specifically methylthio, difluoromethylthio, trifluoromethylthio, ethylthio, propylthio, isopropylthio, butylthio, 4,4,4-trifluorobutylthio, pentylthio, hexylthio and the like.

The "mono-C₁₋₆ alkylamino group" in the above-mentioned Substituent group A includes, for example, methylamino, ethylamino, propylamino, isopropylamino, butylamino and the like.

The "mono-C₆₋₁₄ arylamino group" in the above-mentioned Substituent group A includes, for example, phenylamino, 1-naphthylamino, 2-naphthylamino and the like.

The "di-C₁₋₆ alkylamino group" in the above-mentioned Substituent group A includes, for example, dimethylamino, diethylamino, dipropylamino, diisopropylamino, dibutylamino, ethylmethylamino and the like.

The "di-C₆₋₁₄ arylamino group" in the above-mentioned Substituent group A includes, for example, diphenylamino, di(2-naphthyl)amino and the like.

The "C₁₋₆ alkyl-carbonyl" as the "acyl group" in the above-mentioned Substituent group A includes, for example, acetyl, propionyl, butyryl, isobutyryl, valeryl, isovaleryl, pivaloyl and the like.

The "C₃₋₈ cycloalkyl-carbonyl" as the "acyl group" in the above-mentioned Substituent group A includes, for example, cyclopropyl-carbonyl, cyclobutyl-carbonyl, cyclopentyl-carbonyl, cyclohexyl-carbonyl, cycloheptyl-carbonyl, cyclooctyl-carbonyl and the like.

The "C₁₋₆ alkoxy-carbonyl" as the "acyl group" in the above-mentioned Substituent group A includes, for example, methoxy-carbonyl, ethoxy-carbonyl, propoxy-carbonyl, isopropoxy-carbonyl, n-butoxy-carbonyl, isobutoxy-carbonyl, sec-butoxy-carbonyl, tert-butoxy-carbonyl, pentyloxy-carbonyl, isopentyloxy-carbonyl, neopentyloxy-carbonyl and the like.

The "C₆₋₁₄ aryl-carbonyl" as the "acyl group" in the above-mentioned Substituent group A includes, for example, benzoyl, 1-naphthoyl, 2-naphthoyl and the like.

The "C₇₋₁₆ aralkyl-carbonyl" as the "acyl group" in the above-mentioned Substituent group A includes, for example, phenylacetyl, 3-phenylpropionyl and the like.

The "C₆₋₁₄ aryloxy-carbonyl" as the "acyl group" in the above-mentioned Substituent group A includes, for example, phenoxycarbonyl, 2-naphthyloxycarbonyl and the like.

The "C₇₋₁₆ aralkyloxy-carbonyl" as the "acyl group" in the above-mentioned Substituent group A includes, for example, benzyloxycarbonyl, 2-naphthyloxycarbonyl and the like.

The "5- or 6-membered heterocyclic carbonyl containing 1 to 3 hetero atoms selected from a nitrogen atom, a sulfur atom and an oxygen atom in addition to carbon atoms" as the "acyl group" in the above-mentioned Substituent group A includes, for example, 1-pyrrolidinylcarbonyl, 4-piperidylcarbonyl, 1-piperazinylcarbonyl, 2-morpholinylcarbonyl, 4-pyridylcarbonyl, 3-thienylcarbonyl, 2-furylcarbonyl, 2-thiazolylcarbonyl and the like.

The "mono-C₁₋₆ alkyl-carbamoyl" as the "acyl group" in the above-mentioned Substituent group A includes, for example, methylcarbamoyl, ethylcarbamoyl and the like.

The "di-C₁₋₆ alkyl-carbamoyl" as the "acyl group" in the above-mentioned Substituent group A includes, for example, dimethylcarbamoyl, diethylcarbamoyl and the like.

The "mono-C₆₋₁₄ aryl-carbamoyl" as the "acyl group" in the above-mentioned Substituent group A includes, for example, phenylcarbamoyl, 2-naphthylcarbamoyl and the like.

The "di-C₆₋₁₄ aryl-carbamoyl" as the "acyl group" in the above-mentioned Substituent group A includes, for example, diphenylcarbamoyl and the like.

The "5- or 6-membered heterocyclic carbamoyl containing 1 to 3 hetero atoms selected from a nitrogen atom, a sulfur atom and an oxygen atom in addition to carbon atoms" as the "acyl group" in the above-mentioned Substituent group A includes, for example, 1-pyrrolidinylcarbamoyl, 4-piperidylcarbamoyl, 1-piperazinylcarbamoyl, 2-morpholinylcarbamoyl, 4-pyridylcarbamoyl, 3-thienylcarbamoyl, 2-furylcarbamoyl, 2-thiazolylcarbamoyl and the like.

The "C₁₋₆ alkyl-thiocarbonyl" as the "acyl group" in the above-mentioned Substituent group A includes, for example, methylthiocarbonyl, ethylthiocarbonyl and the like.

The "C₃₋₈ cycloalkyl-thiocarbonyl" as the "acyl group" in the above-mentioned Substituent group A includes, for example, cyclopentylthiocarbonyl, cyclohexylthiocarbonyl and the like.

The "C₁₋₆ alkoxy-thiocarbonyl" as the "acyl group" in the above-mentioned Substituent group A includes, for example, methoxythiocarbonyl, ethoxythiocarbonyl, propoxythiocarbonyl, butoxythiocarbonyl and the like.

The "C₆₋₁₄ aryl-thiocarbonyl" as the "acyl group" in the above-mentioned Substituent group A includes, for example, phenylthiocarbonyl, 2-naphthylthiocarbonyl and the like.

The "C₇₋₁₆ aralkyl-thiocarbonyl" as the "acyl group" in the above-mentioned Substituent group A includes, for example, benzylthiocarbonyl, phenethylthiocarbonyl and the like.

The "C₆₋₁₄ aryloxy-thiocarbonyl" as the "acyl group" in the above-mentioned Substituent group A includes, for example, phenoxythiocarbonyl, 2-naphthyloxythiocarbonyl and the like.

The "C₇₋₁₆ aralkyloxy-thiocarbonyl" as the "acyl group" in the above-mentioned Substituent group A includes, for example, benzyloxythiocarbonyl, 2-naphthylmethyloxythiocarbonyl and the like.

The "5- or 6-membered heterocyclic thiocarbonyl containing 1 to 3 hetero atoms selected from a nitrogen atom, a sulfur atom and an oxygen atom in addition to carbon atoms" as the "acyl group" in the above-mentioned Substituent group A includes, for example, 1-pyrrolidinylthiocarbonyl, 4-piperidylthiocarbonyl, 1-piperazinylthiocarbonyl, 2-morpholinylthiocarbonyl, 4-pyridylthiocarbonyl, 3-thienylthiocarbonyl, 2-furylthiocarbonyl, 2-thiazolylthiocarbonyl and the like.

The "mono-C₁₋₆ alkyl-thiocarbamoyl" as the "acyl group" in the above-mentioned Substituent group A includes, for example, methylthiocarbamoyl, ethylthiocarbamoyl and the like.

The "di-C₁₋₆ alkyl-thiocarbamoyl" as the "acyl group" in the above-mentioned Substituent group A includes, for example, dimethylthiocarbamoyl, diethylthiocarbamoyl and the like.

The "mono-C₆₋₁₄ aryl-thiocarbamoyl" as the "acyl group" in the above-mentioned Substituent group A includes, for example, phenylthiocarbamoyl, 2-naphthylthiocarbamoyl and the like.

The "di-C₆₋₁₄ aryl-thiocarbamoyl" as the "acyl group" in the above-mentioned Substituent group A includes, for example, diphenylthiocarbonyl and the like.

The "5- or 6-membered heterocyclic thiocarbamoyl containing 1 to 3 hetero atoms selected from a nitrogen atom, a sulfur atom and an oxygen atom in addition to carbon atoms" as the "acyl group" in the above-mentioned Substituent group A includes, for example, 1-pyrrolidinylthiocarbamoyl, 4-piperidylthiocarbamoyl, 1-piperazinylthiocarbamoyl, 2-morpholinylthiocarbamoyl, 4-pyridylthiocarbamoyl, 3-thienylthiocarbamoyl, 2-furylthiocarbamoyl, 2-thiazolylthiocarbamoyl and the like.

The "mono-C₁₋₆ alkylsulfamoyl" as the "acyl group" in the above-mentioned Substituent group A includes, for example, methylsulfamoyl, ethylsulfamoyl and the like.

The "di-C₁₋₆ alkylsulfamoyl" as the "acyl group" in the above-mentioned Substituent group A includes, for example, dimethylsulfamoyl, diethylsulfamoyl and the like.

The "C₆₋₁₄ arylsulfamoyl" as the "acyl group" in the above-mentioned Substituent group A includes, for example, phenylsulfamoyl and the like.

The "C₁₋₆ alkylsulfonyl" as the "acyl group" in the above-mentioned Substituent group A includes, for example, methylsulfonyl, ethylsulfonyl and the like.

The "C₆₋₁₄ arylsulfonyl" as the "acyl group" in the above-mentioned Substituent group A includes, for example, phenylsulfonyl, 2-naphthylsulfonyl and the like.

The "C₁₋₆ alkylsulfinyl" as the "acyl group" in the above-mentioned Substituent group A includes, for example, methylsulfinyl, ethylsulfinyl and the like.

The "C₆₋₁₄ arylsulfinyl" as the "acyl group" in the above-mentioned Substituent group A includes, for example, phenylsulfinyl, 2-naphthylsulfinyl and the like.

The "C₁₋₆ alkoxysulfinyl" as the "acyl group" in the above-mentioned Substituent group A includes, for example, methoxysulfinyl, ethoxysulfinyl and the like.

The "C₆₋₁₄ aryloxysulfinyl" as the "acyl group" in the above-mentioned Substituent group A includes, for example, phenoxysulfinyl and the like.

The "C₁₋₆ alkoxysulfonyl" as the "acyl group" in the above-mentioned Substituent group A includes, for example, methoxysulfonyl, ethoxysulfonyl and the like.

The "C₆₋₁₄ aryloxysulfonyl" as the "acyl group" in the above-mentioned Substituent group A includes, for example, phenoxysulfonyl and the like.

The "C₁₋₆ alkyl-carboxamide" as the "acylamino group" in the above-mentioned Substituent group A includes, for example, acetamide, propionamide and the like.

The "C₆₋₁₄ aryl-carboxamide" as the "acylamino group" in the above-mentioned Substituent group A includes, for example, benzamide, 2-naphthylcarboxamide and the like.

The "C₁₋₆ alkoxy-carboxamide" as the "acylamino group" in the above-mentioned Substituent group A includes, for example, methoxycarboxamide, ethoxycarboxamide, isopropoxycarboxamide, tert-butoxycarboxamide and the like.

The "C₁₋₆ alkylsulfonylamino" as the "acylamino group" in the above-mentioned Substituent group A includes, for example, methylsulfonylamino, ethylsulfonylamino and the like.

The "C₆₋₁₄ arylsulfonylamino" as the "acylamino group" in the above-mentioned Substituent group A includes, for example, phenylsulfonylamino, 2-naphthylsulfonylamino and the like.

The "C₁₋₆ alkyl-carbonyloxy" as the "acyloxy group" in the above-mentioned Substituent group A includes, for example, acetyloxy, propionyloxy and the like.

The "C₆₋₁₄ aryl-carbonyloxy" as the "acyloxy group" in the above-mentioned Substituent group A includes, for example, benzoyloxy, 2-naphthoyloxy and the like.

The "C₁₋₆ alkoxy-carbonyloxy" as the "acyloxy group" in the above-mentioned Substituent group A includes, for example, methoxycarbonyloxy, ethoxycarbonyloxy, isopropoxycarbonyloxy, tert-butoxycarbonyloxy and the like.

The "mono-C₁₋₆ alkyl-carbamoyloxy" as the "acyloxy group" in the above-mentioned Substituent group A includes, for example, methylcarbamoyloxy, ethylcarbamoyloxy and the like.

The "di-C₁₋₆ alkyl-carbamoyloxy" as the "acyloxy group" in the above-mentioned Substituent group A includes, for example, dimethylcarbamoyloxy, diethylcarbamoyloxy and the like.

The "mono-C₆₋₁₄ aryl-carbamoyloxy" as the "acyloxy group" in the above-mentioned Substituent group A includes, for example, phenylcarbamoyloxy, 2-naphthylcarbamoyloxy and the like.

The "di-C₆₋₁₄ aryl-carbamoyloxy" as the "acyloxy group" in the above-mentioned Substituent group A includes, for example, diphenylcarbamoyloxy and the like.

The "5- to 14-membered heterocyclic group containing 1 to 4 hetero atoms selected from a nitrogen atom, a sulfur atom and an oxygen atom in addition to carbon atoms" in the above-mentioned Substituent group A includes, for example, 4-pyridyl, 2-thienyl, 2-furyl, 2-thiazolyl, 3-indolyl, morpholino, piperazin-1-yl, piperidino, pyrrolidin-1-yl, 2-isoindolinyl and the like.

The "C₆₋₁₄ aryloxy group" in the above-mentioned Substituent group A includes, for example, phenoxy.

The "di-C₁₋₆ alkoxy-phosphoryl group" in the above-mentioned Substituent group A includes, for example, dimethoxyphosphoryl, diethoxyphosphoryl and the like.

The "C₆₋₁₄ arylthio group" in the above-mentioned Substituent group A includes, for example, phenylthio and the like.

The "C₁₋₆ alkyl-ureido group" in the above-mentioned Substituent group A includes, for example, methylureido, ethylureido and the like.

The "di-C₁₋₆ alkyl-ureido group" in the above-mentioned Substituent group A includes, for example, dimethylureido, diethylureido and the like.

The "C₁₋₆ alkoxy-carbonyl group" of the "C₁₋₆ alkoxy-carbonyl group which may have 1 to 5 substituents selected from the above-mentioned Substituent group A " as the "optionally esterified or amidated carboxyl group" represented by R¹ includes, for example, for example, methoxy-carbonyl, ethoxy-carbonyl, propoxy-carbonyl, isopropoxy-carbonyl, n-butoxy-carbonyl, isobutoxy-carbonyl, sec-butoxy-carbonyl, tert-butoxy-carbonyl, pentyloxy-carbonyl, isopentyloxy-carbonyl, neopentyloxy-carbonyl and the like.

The "C₃₋₈ cycloalkyloxy-carbonyl group" of the "C₃₋₈ cycloalkyloxy-carbonyl group which may have 1 to 5 substituents selected from Substituent group A described above" as the "optionally esterified or amidated carboxyl group" represented by R¹ includes, for example, cyclopropyloxy-carbonyl, cyclobutyloxy-carbonyl, cyclopentyloxy-carbonyl, cyclohexyloxy-carbonyl, cycloheptyloxy-carbonyl, cyclooctyloxy-carbonyl and the like.

The "C₇₋₁₆ aralkyloxy-carbonyl group" of the "C₇₋₁₆ aralkyloxy-carbonyl group which may have 1 to 5 substituents selected from Substituent group A described above" as the "optionally esterified or amidated carboxyl group" represented by R¹ includes, for example, benzyloxycarbonyl, 2-naphthyloxycarbonyl and the like.

The "C₆₋₁₄ aryloxy-carbonyl group" of the "C₆₋₁₄ aryloxy-carbonyl group which may have 1 to 5 substituents selected from Substituent group A described above" as the "optionally esterified or amidated carboxyl group" represented by R¹ includes, for example, phenoxycarbonyl, 2-naphthyloxycarbonyl and the like.

The "mono-C₁₋₆ alkyl-carbamoyl group" of the "mono-C₁₋₆ alkyl-carbamoyl group which may have 1 to 5 substituents selected from Substituent group A described above" as the "optionally esterified or amidated carboxyl group" represented by R¹ includes, for example, methylcarbamoyl, ethylcarbamoyl and the like.

The "di-C₁₋₆ alkyl-carbamoyl group" of the "di-C₁₋₆ alkyl-carbamoyl group which may have 1 to 5 substituents selected from Substituent group A described above" as the "optionally esterified or amidated carboxyl group" represented by R¹ includes, for example, dimethylcarbamoyl, diethylcarbamoyl and the like.

The "mono-C₆₋₁₄ aryl-carbamoyl group" of the "mono-C₆₋₁₄ aryl-carbamoyl group which may have 1 to 5 substituents selected from Substituent group A described above" as the "optionally esterified or amidated carboxyl group" represented by R¹ includes, for example, phenylcarbamoyl, 2-naphthylcarbamoyl and the like.

The "di-C₆₋₁₄ aryl-carbamoyl group" of the "di-C₆₋₁₄ aryl-carbamoyl group which may have 1 to 5 substituents selected from Substituent group A described above" as the "optionally esterified or amidated carboxyl group" represented by R¹ includes, for example, diphenylcarbamoyl and the like.

R¹ is preferably (1) a cyano group, (2) a carboxyl group, (3) a C₁₋₆ alkoxycarbonyl group, (4) a carbamoyl group, (5) an N-mono-C₁₋₆ alkylcarbamoyl group, and among these, R¹ is preferably carboxyl group or carbamoyl group.

In the above formulae, R² is (1) a hydrogen atom, (2) an optionally substituted hydroxyl group, (3) an optionally substituted amino group, (4) an optionally substituted alkyl group, (5) an optionally esterified or amidated carboxyl group or (6) a nitro group.

The "optionally substituted hydroxyl group" represented by R² includes, for example, a group represented by the formula -OR¹² (R¹² is (a) a hydrogen atom, (b) a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A, or (c) an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, each of which may have 1 to 5 substituents selected from Substituent group A described above).

The C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group of the "C₁₋₆ alkyl group, the C₂₋₆ alkenyl group, the C₂₋₆ alkynyl group, the C₃₋₈ cycloalkyl group, the C₃₋₈ cycloalkenyl group, the C₆₋₁₄ aryl group or the C₇₋₁₆ aralkyl group, each of which may have 1 to 5 substituents selected from Substituent group A" represented by R¹² includes, for example, the followings:
a C₁₋₆ alkyl group: methyl, ethyl, propyl, isopropyl, butyl, isobutyl, sec-butyl, tert-butyl, pentyl, hexyl and the like;
a C₂₋₆ alkenyl group: vinyl, allyl, isopropenyl, 2-butenyl, 2-methyl-2-propenyl, 4-pentenyl, 5-hexenyl and the like;
a C₂₋₆ alkynyl group: propargyl, ethynyl, 2-butynyl, 2-hexynyl and the like;
a C₃₋₈ cycloalkyl group: cyclopropyl, cyclobutyl, cyclopentyl, cyclohexyl, cycloheptyl, cyclooctyl and the like;
a C₃₋₈ cycloalkenyl group: 1-cyclopropenyl, 1-cyclobutenyl, 1-cyclopentenyl, 1-cyclohexenyl and the like;
a C₆₋₁₄ aryl group: phenyl, 1-naphthyl, 2-naphthyl, 1-anthryl, 2-anthryl, 9-anthryl, 1-phenanthryl, 2-phenanthryl, 3-phenanthryl, 4-phenanthryl, 9-phenanthryl and the like; and
a C₇₋₁₆ aralkyl group: benzyl, phenethyl, diphenylmethyl, 1-naphthylmethyl, 2-naphthylmethyl, 2-phenethyl, 2,2-diphenylethyl, 1-phenylpropyl, 2-phenylpropyl, 3-phenylpropyl, 4-phenylbutyl, 5-phenylpentyl, biphenylylmethyl, biphenylylethyl, biphenylylpropyl, biphenylylbutyl and the like.

The "C₁₋₆ alkyl-carbonyl", the "C₃₋₈ cycloalkyl-carbonyl", the "C₁₋₆ alkoxy-carbonyl", the "C₆₋₁₄ aryl-carbonyl", the "C₇₋₁₆ aralkyl-carbonyl", the "C₆₋₁₄ aryloxy-carbonyl", the "C₇₋₁₆ aralkyloxy-carbonyl", the "5- or 6-membered heterocyclic carbonyl containing 1 to 3 hetero atoms selected from a nitrogen atom, a sulfur atom and an oxygen atom in addition to carbon atoms", the "mono-C₁₋₆ alkyl-carbamoyl", the "di-C₁₋₆ alkyl-carbamoyl", the "mono-C₆₋₁₄ aryl-carbamoyl", the "di-C₆₋₁₄ aryl-carbamoyl", the "5- or 6-membered heterocyclic carbamoyl containing 1 to 3 hetero atoms selected from a nitrogen atom, a sulfur atom and an oxygen atom in addition to carbon atoms", the "C₁₋₆ alkyl-thiocarbonyl", the "C₃₋₈ cycloalkyl-thiocarbonyl", the "C₁₋₆ alkoxy-thiocarbonyl", the "C₆₋₁₄ aryl-thiocarbonyl", the "C₇₋₁₆ aralkyl-thiocarbonyl", the "C₆₋₁₄ aryloxy-thiocarbonyl", the "C₇₋₁₆ aralkyloxy-thiocarbonyl", the "5- or 6-membered heterocyclic thiocarbonyl containing 1 to 3 hetero atoms selected from a nitrogen atom, a sulfur atom and an oxygen atom in addition to carbon atoms", the "mono-C₁₋₆ alkyl-thiocarbamoyl", the "di-C₁₋₆ alkyl-thiocarbamoyl", the "mono-C₆₋₁₄ aryl-thiocarbamoyl", the "di-C₆₋₁₄ aryl-thiocarbamoyl", the "5- or 6-membered heterocyclic thiocarbamoyl containing 1 to 3 hetero atoms selected from a nitrogen atom, a sulfur atom and an oxygen atom in addition to carbon atoms", the "mono-C₁₋₆ alkylsulfamoyl", the "di-C₁₋₆ alkylsulfamoyl", the "C₆₋₁₄ arylsulfamoyl", the "C₁₋₆ alkylsulfonyl", the "C₆₋₁₄ arylsulfonyl", the "C₁₋₆ alkylsulfinyl", the "C₆₋₁₄ arylsulfinyl", the "C₁₋₆ alkoxysulfinyl", the "C₆₋₁₄ aryloxysulfinyl", the "C₁₋₆ alkoxysulfonyl" and the "C₆₋₁₄ aryloxysulfonyl" as the acyl group of the "acyl group which may have 1 to 5 substituents selected from Substituent group A described above" represented by R¹², includes those such as the "C₁₋₆ alkyl-carbonyl", the "C₃₋₈ cycloalkyl-carbonyl", the "C₁₋₆ alkoxy-carbonyl", the "C₆₋₁₄ aryl-carbonyl", the "C₇₋₁₆ aralkyl-carbonyl", the "C₆₋₁₄ aryloxy-carbonyl", the "C₇₋₁₆ aralkyloxy-carbonyl", the "5- or 6-membered heterocyclic carbonyl containing 1 to 3 hetero atoms selected from a nitrogen atom, a sulfur atom and an oxygen atom in addition to carbon atoms", the "mono-C₁₋₆ alkyl-carbamoyl", the "di-C₁₋₆ alkyl-carbamoyl", the "mono-C₆₋₁₄ aryl-carbamoyl", the "di-C₆₋₁₄ aryl-carbamoyl", the "5- or 6-membered heterocyclic carbamoyl containing 1 to 3 hetero atoms selected from a nitrogen atom, a sulfur atom and an oxygen atom in addition to carbon atoms", the "C₁₋₆ alkyl-thiocarbonyl", the "C₃₋₈ cycloalkyl-thiocarbonyl", the "C₁₋₆ alkoxy-thiocarbonyl", the "C₆₋₁₄ aryl-thiocarbonyl", the "C₇₋₁₆ aralkyl-thiocarbonyl", the "C₆₋₁₄ aryloxy-thiocarbonyl", the "C₇₋₁₆ aralkyloxy-thiocarbonyl", the "5- or 6-membered heterocyclic thiocarbonyl containing 1 to 3 hetero atoms selected from a nitrogen atom, a sulfur atom and an oxygen atom in addition to carbon atoms", the "mono-C₁₋₆ alkyl-thiocarbamoyl", the "di-C₁₋₆ alkyl-thiocarbamoyl", the "mono-C₆₋₁₄ aryl-thiocarbamoyl", the "di-C₆₋₁₄ aryl-thiocarbamoyl", the "5- or 6-membered heterocyclic thiocarbamoyl containing 1 to 3 hetero atoms selected from a nitrogen atom, a sulfur atom and an oxygen atom in addition to carbon atoms", the "mono-C₁₋₆ alkylsulfamoyl", the "di-C₁₋₆ alkylsulfamoyl", the "C₆₋₁₄ arylsulfamoyl", the "C₁₋₆ alkylsulfonyl", the "C₆₋₁₄ arylsulfonyl", the "C₁₋₆ alkylsulfinyl", the "C₆₋₁₄ arylsulfinyl", the "C₁₋₆ alkoxysulfinyl", the "C₆₋₁₄ aryloxysulfinyl", the "C₁₋₆ alkoxysulfonyl" and the "C₆₋₁₄ aryloxysulfonyl" in the acyl group of the above-mentioned Substituent group A.

The "optionally substituted amino group" represented by R² includes, for example, (1) a group represented by the formula-NR¹³R¹⁴ (R¹³ and R¹⁴ each represent (i') a hydrogen atom, (ii') a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A, (iii') an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, each of which may have 1 to 5 substituents selected from Substituent group A described above or (iv') a 5- to 14-membered heterocycle containing 1 to 4 hetero atoms selected from a nitrogen atom, a sulfur atom and an oxygen atom in addition to carbon atoms, each of which may have 1 to 5 substituents selected from Substituent group A described above, or R¹³ and R¹⁴ may be taken together with the adjacent a nitrogen atom to form a 5- to 14-membered ring), (2) a C₁₋₆ alkylideneamino group which may have 1 to 5 substituents selected from Substituent group A described above (e.g., methylideneamino, ethylideneamino and the like) and the like.

"The C₁₋₆ alkyl group, the C₂₋₆ alkenyl group, the C₂₋₆ alkynyl group, the C₃₋₈ cycloalkyl group, the C₃₋₈ cycloalkenyl group, the C₆₋₁₄ aryl group or the C₇₋₁₆ aralkyl group, each of which may have 1 to 5 substituents selected from Substituent group A" represented by R¹³ and R¹⁴ includes those such as the above-mentioned "C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A" represented by R¹².

The "acyl group which may have 1 to 5 substituents selected from Substituent group A described above" represented by R¹³ and R¹⁴ includes those such as the above-mentioned "acyl group which may have 1 to 5 substituents selected from Substituent group A described above" represented by R¹².

The "5- to 14-membered heterocycle containing 1 to 4 hetero atoms selected from a nitrogen atom, a sulfur atom and an oxygen atom in addition to carbon atoms " of the "5- to 14-membered heterocycle containing 1 to 4 hetero atoms selected from a nitrogen atom, a sulfur atom and an oxygen atom in addition to carbon atoms, each of which may have 1 to 5 substituents selected from Substituent group A described above" represented by R¹³ and R¹⁴ includes, for example, 4-pyridyl, 2-thienyl, 2-furyl, 2-thiazolyl, 3-indolyl, morpholino, piperazin-1-yl, piperidino, pyrrolidin-1-yl, 2-isoindolinyl and the like.

The 5- to 14-membered ring which R¹³ and R¹⁴ may form together with the adjacent nitrogen atom includes, for example, 1-pyrrolidinyl, 1-piperidyl, 1-piperazinyl, 4-morpholinyl, 4-thiomorpholinyl, 1,2-dihydropyridin-1-yl, 1-imidazolidinyl and the like.

The "optionally substituted alkyl group" represented by R² includes, for example, a C₁₋₆ alkyl group which may have 1 to 5 substituents selected from Substituent group A described above and the like. The C₁₋₆ alkyl group includes, for example, methyl, ethyl, propyl, isopropyl, butyl, isobutyl, sec-butyl, tert-butyl, pentyl, hexyl and the like.

The "optionally esterified or amidated carboxyl group" represented by R² includes, for example, those such as the "optionally esterified or amidated carboxyl group" represented by R¹.

R² is preferably (1) a hydrogen atom, (2) a hydroxyl group, (3) a C₁₋₆ alkoxy group, (4) a C₇₋₁₆ aralkyloxy group, (5) an amino group, (6) a mono-C₁₋₆ alkylamino group which may have one substituent selected from carboxyl, carbamoyl, quinolyl, phenoxy and pyridyl, (7) a mono-C₇₋₁₆ aralkylamino group which may have one substituent selected from a halogen atom, cyano, C₁₋₆ alkoxy, carboxyl and C₁₋₆ alkoxycarbonyl, (8) a mono-C₆₋₁₄ arylamino group, (9) a mono-C₁₋₆ alkylcarbonylamino group which may have 1 to 3 substituents selected from a halogen atom, thienyl and C₁₋₆ alkoxycarbonyl-C₁₋₆ alkylthio, (10) a mono-C₁₋₆ alkylsulfonylamino group, (11) a mono-C₆₋₁₄ arylcarbonylamino group which may have one substituent selected from C₁₋₆ alkoxy and C₁₋₆ alkylcarbonylamino, (12) a quinolylcarbonylamino group, (13) a pyridylcarbonylamino group which may have 1 or 2 halogen atoms, (14) an indolylcarbonylamino group, (15) a N-C₁₋₆ alkyl-N-C₁₋₆ alkylcarbonylamino group which may have 1 to 4 substituents selected from a halogen atom, C₁₋₆ alkoxycarbonyl and quinolyl, (16) a N-C₁₋₆ alkylcarbonyl-N-C₇₋₁₆ aralkylamino group which may have 1 to 3 halogens, (17) a N-C₁₋₆ alkyl-N-pyridylcarbonylamino group, (18) a C₁₋₆ alkylideneamino group which may have one di-C₁₋₆ alkylamino, (19) a mono-C₁₋₆ alkylureido group which may have one C₁₋₆ alkoxycarbonyl, (20) a di-C₁₋₆ alkylureido group, (21) a mono-C₆₋₁₄ arylureido group, (22) a 1-imidazolidinyl group which may have 1 to 3 substituents selected from C₁₋₆ alkyl and oxo, (23) a C₁₋₆ alkyl group, (24) a C₁₋₆ alkoxycarbonyl group, (25) a nitro group or (26) a 1-pyrrolidinyl group. Among these, R² is preferably (1) a hydrogen atom, (2) a C₁₋₆ alkoxy group, (3) a mono-C₁₋₆ alkylamino group, (4) a mono-C₇₋₁₆ aralkylamino group, (5) a quinolylcarbonylamino group or (6) a pyridylcarbonylamino group.

The ring which R² and A or R¹ may form together with the adjacent nitrogen atom includes, for example, a 5- to 8-membered heterocycle which may have 1 to 5 substituents selected from Substituent group A described above.

The "5- to 8-membered heterocycle" is a 5- to 8-membered heterocycle (preferably a 5- to 7-membered aliphatic heterocycle) containing one or more (e.g., 1 to 4, preferably 1 to 3) hetero atoms of one or two kinds selected from a nitrogen atom, a sulfur atom and an oxygen atom in addition to carbon atoms and the like.

Specifically, a partial structure represented by the formula 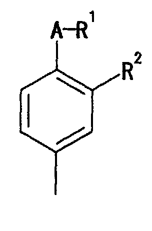 may have, for example, the structure represented by the formula 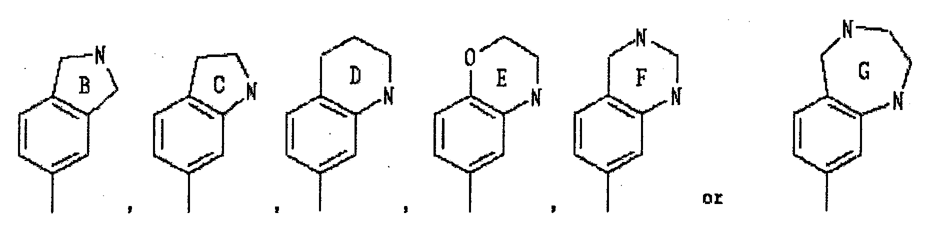 [wherein Rings B to G may have 1 to 5 substituents selected from the above-mentioned Substituent group A] and the like.

The substituent which the ring that R² and A or R¹ may form together with the adjacent nitrogen atom, may have is preferably, for example, (1) a hydroxyl group, (2) a C₁₋₆ alkyl group which may have one C₁₋₆ alkoxy-carbonyl (e.g., methyl, ethyl, propyl, butyl, isobutyl, sec-butyl, tert-butyl, pentyl, hexyl, methoxycarbonylmethyl, ethoxycarbonylmethyl and the like), (3) a C₇₋₁₆ aralkyl group (e.g., benzyl and the like), (4) a C₆₋₁₄ aryl group (e.g., phenyl and the like), (5) an oxo group and the like.

R³ and R⁴ each represent (1) a hydrogen atom, (2) an optionally substituted hydrocarbon group or (3) an acyl group, and R³ and R⁴ may be taken together with the adjacent carbon atom to form an optionally substituted 3- to 8-membered ring.

The "optionally substituted hydrocarbon group" represented by R³ and R⁴ includes, for example, a C₁₋₆ alkyl group (e.g., methyl, ethyl, propyl, isopropyl, butyl, isobutyl, sec-butyl, tert-butyl, pentyl, hexyl and the like), a C₂₋₆ alkenyl group (e.g., vinyl, allyl, isopropenyl, 2-butenyl, 2-methyl-2-propenyl, 4-pentenyl, 5-hexenyl and the like), a C₂₋₆ alkynyl group (e.g., propargyl, ethynyl, 2-butynyl, 2-hexynyl and the like), a C₃₋₈ cycloalkyl group (e.g., cyclopropyl, cyclobutyl, cyclopentyl, cyclohexyl, cycloheptyl, cyclooctyl and the like), a C₃₋₈ cycloalkenyl group (e.g., 1-cyclopropenyl, 1-cyclobutenyl, 1-cyclopentenyl, 1-cyclohexenyl and the like), a C₆₋₁₄ aryl group (e.g. , phenyl, 1-naphthyl, 2-naphthyl, 1-anthryl, 2-anthryl, 9-anthryl, 1-phenanthryl, 2-phenanthryl, 3-phenanthryl, 4-phenanthryl, 9-phenanthryl and the like) or a C₇₋₁₆ aralkyl group (e.g., benzyl, phenethyl, diphenylmethyl, 1-naphthylmethyl, 2-naphthylmethyl, 2-phenethyl, 2,2-diphenylethyl, 1-phenylpropyl, 2-phenylpropyl, 3-phenylpropyl, 4-phenylbutyl and 5-phenylpentyl), each of which may have 1 to 5 substituents selected from Substituent group A described above and the like.

The "acyl group" represented by R³ and R⁴ includes those such as the above-mentioned "acyl group" represented by Substituent group A.

The 3- to 8-membered ring which R³ and R⁴ form together with the adjacent carbon atom includes, for example, a 3- to 8-membered homocycle or heterocycle.

The 3- to 8-membered homocycle which R³ and R⁴ form together with the adjacent carbon atom includes 3- to 8-membered cyclic hydrocarbon comprising carbon atoms and hydrogen atoms, and specifically C₃₋₈ cycloalkane (e.g., cyclobutane, cyclopentane, cyclohexane, cycloheptane and cyclooctane), C₃₋₈ cycloalkene (e.g., cyclobutene, cyclopentene, cyclohexene, cycloheptene, cyclooctene) and the like. Among these, the 3- to 8-membered homocycle is preferably C₃₋₈ cycloalkane, particularly a 5- or 6-membered homocycle such as cyclopentane, cyclohexane and the like (particularly, cyclohexane).

The 3- to 8-membered heterocycle which R³ and R⁴ form together with the adjacent carbon atom includes a 5- to 8-membered aliphatic heterocycle (preferably a 5- or 6-membered aliphatic heterocycle) containing one or more (e.g., 1 to 4, preferably 1 to 3) hetero atoms of one or two kinds selected from a nitrogen atom, a sulfur atom and an oxygen atom in addition to carbon atoms and the like.

More specifically, it is, for example, a 5- to 8-membered (preferably 5- or 6-membered) aliphatic heterocycle containing 1 to 3 hetero atoms selected from a nitrogen atom, an oxygen atom and a sulfur atom such as piperidine, piperazine, morpholine, thiomorpholine, pyrrolidine, imidazolidine and the like such as in addition to carbon atoms and nitrogen atoms and the like.

R³ and R⁴ are preferably a C₁₋₆ alkyl group, particularly methyl, respectively.

In the above formulae, R⁵ is (1) a hydrogen atom, (2) a cyano group, (3) an optionally substituted hydrocarbon group, (4) an acyl group or (5) an optionally substituted hydroxyl group.

The "optionally substituted hydrocarbon group" represented by R⁵ includes those such as the above-mentioned "optionally substituted hydrocarbon group" represented by R³ and R⁴.

The "acyl group" represented by R⁵ includes those such as the above-mentioned "acyl group" represented by Substituent group A.

The "optionally substituted hydroxyl group" represented by R⁵ includes, for example, a group represented by the formula -OR¹⁵ (R¹⁵ is (a) a hydrogen atom, (b) a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A, or (c) an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, each of which may have 1 to 5 substituents selected from Substituent group A described above) and the like.

"The C₁₋₆ alkyl group, the C₂₋₆ alkenyl group, the C₂₋₆ alkynyl group, the C₃₋₈ cycloalkyl group, the C₃₋₈ cycloalkenyl group, the C₆₋₁₄ aryl group or the C₇₋₁₆ aralkyl group, each of which may have 1 to 5 substituents selected from Substituent group A" and the "acyl group which may have 1 to 5 substituents selected from Substituent group A described above" represented by R¹⁵ include those such as the above-mentioned "the C₁₋₆ alkyl group, the C₂₋₆ alkenyl group, the C₂₋₆ alkynyl group, the C₃₋₈ cycloalkyl group, the C₃₋₈ cycloalkenyl group, the C₆₋₁₄ aryl group or the C₇₋₁₆ aralkyl group, each of which may have 1 to 5 substituents selected from Substituent group A" and the "acyl group which may have 1 to 5 substituents selected from Substituent group A described above" represented by R¹².

R⁵ is preferably a hydrogen atom or a C₁₋₆ alkyl group optionally substituted with a cyano group, particularly preferably a hydrogen atom.

In the above formulae, R⁶ is (1) a hydrogen atom, (2) an optionally substituted hydrocarbon group, (3) an acyl group, (4) an optionally substituted heterocyclic group, (5) a halogen atom, (6) an optionally substituted hydroxyl group, (7) an optionally substituted thiol group, (8) a group represented by the formula-S(O)ᵣR¹¹ (R¹¹ represents an optionally substituted hydrocarbon group or an optionally substituted heterocyclic group, and r represents 1 or 2) or (9) an optionally substituted amino group.

The "optionally substituted hydrocarbon group" represented by R⁶ includes those such as the above-mentioned "optionally substituted hydrocarbon group" represented by R³ and R⁴.

The "acyl group" represented by R⁶ includes those such as the above-mentioned "acyl group" represented by Substituent group A.

The "heterocyclic group" of the "optionally substituted heterocyclic group" represented by R⁶ includes, for example, a 5- to 14-membered heterocyclic group containing 1 to 4 hetero atoms selected from a nitrogen atom, a sulfur atom and an oxygen atom in addition to carbon atoms (e.g., 4-pyridyl, 2-thienyl, 2-furyl, 2-thiazolyl, 3-indolyl, morpholino, piperazin-1-yl, piperidino, pyrrolidin-1-yl, 2-isoindolinyl and the like).

The "substituent" of the "optionally substituted heterocyclic group" represented by R⁶ includes 1 to 5 substituents selected from the above-mentioned Substituent group A.

The "halogen atom" represented by R⁶ includes, for example, fluorine, chlorine, bromine and iodine.

The "optionally substituted hydroxyl group" represented by R⁶ includes, for example, a group represented by the formula -OR¹⁶ (R¹⁶ is (i') a hydrogen atom, (ii') a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A, (iii') an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, each of which may have 1 to 5 substituents selected from Substituent group A described above or (iv') a 5- to 14-membered heterocycle containing 1 to 4 hetero atoms selected from a nitrogen atom, a sulfur atom and an oxygen atom in addition to carbon atoms, each of which may have 1 to 5 substituents selected from Substituent group A described above) and the like.

"The C₁₋₆ alkyl group, the C₂₋₆ alkenyl group, the C₂₋₆ alkynyl group, the C₃₋₈ cycloalkyl group, the C₃₋₈ cycloalkenyl group, the C₆₋₁₄ aryl group or the C₇₋₁₆ aralkyl group, each of which may have 1 to 5 substituents selected from Substituent group A" and the "acyl group which may have 1 to 5 substituents selected from Substituent group A described above" represented by R¹⁶ include those such as the above-mentioned "C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A" and the "acyl group which may have 1 to 5 substituents selected from Substituent group A described above" represented by R¹².

The "5- to 14-membered heterocycle containing 1 to 4 hetero atoms selected from a nitrogen atom, a sulfur atom and an oxygen atom in addition to carbon atoms, each of which may have 1 to 5 substituents selected from Substituent group A described above" represented by R¹⁶ includes, for example, 4-pyridyl, 2-thienyl, 2-furyl, 2-thiazolyl, 3-indolyl, morpholino, piperazin-1-yl, piperidino, pyrrolidin-1-yl, 2-isoindolinyl and the like, each of which may have 1 to 5 substituents selected from Substituent group A described above.

The "optionally substituted thiol group" represented by R⁶ includes, for example, a group represented by the formula -SR¹⁷ (R¹⁷ is (i') a hydrogen atom, (ii') a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A, (iii') an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, each of which may have 1 to 5 substituents selected from Substituent group A described above or (iv') a 5- to 14-membered heterocycle containing 1 to 4 hetero atoms selected from a nitrogen atom, a sulfur atom and an oxygen atom in addition to carbon atoms, each of which may have 1 to 5 substituents selected from Substituent group A described above).

"The C₁₋₆ alkyl group, the C₂₋₆ alkenyl group, the C₂₋₆ alkynyl group, the C₃₋₈ cycloalkyl group, the C₃₋₈ cycloalkenyl group, the C₆₋₁₄ aryl group or the C₇₋₁₆ aralkyl group, each of which may have 1 to 5 substituents selected from Substituent group A", the "acyl group which may have 1 to 5 substituents selected from Substituent group A described above" and the "5- to 14-membered heterocycle containing 1 to 4 hetero atoms selected from a nitrogen atom, a sulfur atom and an oxygen atom in addition to carbon atoms, each of which may have 1 to 5 substituents selected from Substituent group A described above" represented by R¹⁷ include those such as the above-mentioned "C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A", the "acyl group which may have 1 to 5 substituents selected from Substituent group A described above" and the "5- to 14-membered heterocycle containing 1 to 4 hetero atoms selected from a nitrogen atom, a sulfur atom and an oxygen atom in addition to carbon atoms, each of which may have 1 to 5 substituents selected from Substituent group A described above" represented by R¹⁶.

The "optionally substituted hydrocarbon group" represented by R¹¹ in the group represented by the "formula -S(O)ᵣR¹¹ (R¹¹ represents an optionally substituted hydrocarbon group or an optionally substituted heterocyclic group, and r represents 1 or 2)" represented by R⁶ includes, for example, a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A, and includes those such as the above-mentioned "C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A" represented by R¹⁶.

The "optionally substituted heterocyclic group" represented by R¹¹ in the group represented by the "formula -S(O)ᵣR¹¹ (R¹¹ represents an optionally substituted hydrocarbon group or an optionally substituted heterocyclic group, and r represents 1 or 2)" represented by R⁶ is a 5- to 14-membered heterocycle containing 1 to 4 hetero atoms selected from a nitrogen atom, a sulfur atom and an oxygen atom in addition to carbon atoms, each of which may have 1 to 5 substituents selected from Substituent group A described above and the like, and includes those such as the above-mentioned "5- to 14-membered heterocycle containing 1 to 4 hetero atoms selected from a nitrogen atom, a sulfur atom and an oxygen atom in addition to carbon atoms, each of which may have 1 to 5 substituents selected from Substituent group A described above" represented by R¹⁶.

The "optionally substituted amino group" represented by R⁶ includes, for example, a group represented by the formula -NR¹⁸R¹⁹ (R¹⁸ and R¹⁹ each represent (i') a hydrogen atom, (ii') a C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A, (iii') an acyl group selected from formyl , carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, each of which may have 1 to 5 substituents selected from Substituent group A described above or (iv') a 5- to 14-membered heterocycle containing 1 to 4 hetero atoms selected from a nitrogen atom, a sulfur atom and an oxygen atom in addition to carbon atoms, each of which may have 1 to 5 substituents selected from Substituent group A described above) and the like.

"The C₁₋₆ alkyl group, the C₂₋₆ alkenyl group, the C₂₋₆ alkynyl group, the C₃₋₈ cycloalkyl group, the C₃₋₈ cycloalkenyl group, the C₆₋₁₄ aryl group or the C₇₋₁₆ aralkyl group, each of which may have 1 to 5 substituents selected from Substituent group A", the "acyl group which may have 1 to 5 substituents selected from Substituent group A described above" and the "5- to 14-membered heterocycle containing 1 to 4 hetero atoms selected from a nitrogen atom, a sulfur atom and an oxygen atom in addition to carbon atoms, each of which may have 1 to 5 substituents selected from Substituent group A described above" represented by R¹⁸ and R¹⁹ include those such as the above-mentioned "C₁₋₆ alkyl group, a C₂₋₆ alkenyl group, a C₂₋₆ alkynyl group, a C₃₋₈ cycloalkyl group, a C₃₋₈ cycloalkenyl group, a C₆₋₁₄ aryl group or a C₇₋₁₆ aralkyl group, each of which may have 1 to 5 substituents selected from Substituent group A", the "acyl group which may have 1 to 5 substituents selected from Substituent group A described above" and the "5- to 14-membered heterocycle containing 1 to 4 hetero atoms selected from a nitrogen atom, a sulfur atom and an oxygen atom in addition to carbon atoms, each of which may have 1 to 5 substituents selected from Substituent group A described above" represented by R¹⁶.

R⁶ is preferably an optionally substituted hydroxyl group, a group represented by the formula -S(O)ᵣR¹¹ (R¹¹ represents an optionally substituted hydrocarbon group or an optionally substituted heterocyclic group, and r represents 1 or 2) or an optionally substituted amino group.

Among these, R⁶ is preferably (1) a group represented by the formula -OR¹⁶ wherein R¹⁶ is a C₁₋₆ alkyl group, (2) a group represented by the formula -S(O)ᵣR¹¹ wherein r is 1 and R¹¹ is a C₁₋₆ alkyl group or (3) a group represented by the formula -NR¹⁸R¹⁹ wherein R¹⁸ is C₁₋₆ alkyl-carbonyl and R¹⁹ is a hydrogen atom.

R⁶ is particularly preferably a group represented by the formula -OR¹⁶ wherein R¹⁶ is a C₁₋₆ alkyl group, particularly a C₂₋₆ alkyl group, and specifically ethyl.

Namely, R⁶ is preferably a C₁₋₆ alkoxy group, more preferably a C₂₋₆ alkoxy group, particularly ethoxy.

In the above formulae, R⁷ and R⁸ are each (1) a hydrogen atom or (2) an optionally substituted hydrocarbon group, or R⁷ and R⁸ may be taken together with the adjacent carbon atom to form an optionally substituted 3- to 8-membered ring.

The "optionally substituted hydrocarbon group" represented by R⁷ and R⁸ includes those such as the above-mentioned "optionally substituted hydrocarbon group" represented by R³ and R⁴.

The "optionally substituted 3- to 8-membered ring" which R⁷ and R⁸ form together with the adjacent carbon atom includes those such as the above-mentioned "optionally substituted 3- to 8-membered ring" which R³ and R⁴ form together with the adjacent carbon atom, preferably optionally substituted 3- to 8-membered homocycle, more preferably C₃₋₈ cycloalkane (e.g., cyclobutane, cyclopentane, cyclohexane, cycloheptane, cyclooctane), particularly preferably 5- or 6-membered homocycle such as cyclopentane, cyclohexane and the like (particularly, cyclopentane) .

R⁷ and R⁸ are each preferably a C₁₋₆ alkyl group (e.g., methyl, ethyl and the like) and the like, particularly preferably a methyl group.

In the above formulae, R⁹ and R¹⁰ are each (1) a hydrogen atom or (2) an optionally substituted hydrocarbon group.

The "optionally substituted hydrocarbon group" represented by R⁹ and R¹⁰ includes those such as the above-mentioned "optionally substituted hydrocarbon group" represented by R³ and R⁴.

R⁹ and R¹⁰ each represent preferably a hydrogen atom.

In the above formulae, Y is an optionally substituted methylene group. The substituent of the methylene group is a group selected from the above-mentioned Substituent group A.

Among these, Y is preferably methylene.

In the above formulae, n is 0 or 1. Among these, n is preferably 0.

The preferable compound of the present invention is as follows:
a compound represented by the formula 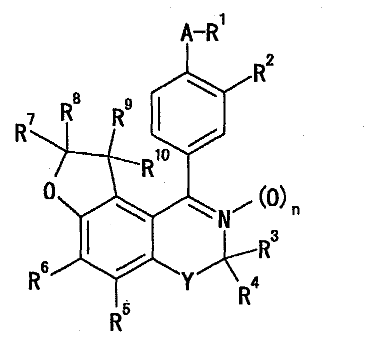
wherein A is (1) a bond, (2) a group represented by the formula -CH=CH-, (3) a group represented by the formula -CONH-C(R^{c}) (R^{d})- (R^{c} and R^{d} each represent a hydrogen atom or C₁₋₆ alkyl), or (4) a group represented by the formula -OCH₂-,
R¹ is (1) cyano, (2) carboxyl, (3) C₁₋₆ alkoxycarbonyl, (4) carbamoyl, or (5) N-mono-C₁₋₆ alkylcarbamoyl,

R² is (1) a hydroxyl group, (2) a C₁₋₆ alkoxy group, (3) a C₇₋₁₆ aralkyloxy group, (4) an amino group, (5) a mono-C₁₋₆ alkylamino group which may have one substituent selected from carboxyl, carbamoyl, quinolyl, phenoxy and pyridyl, (6) a mono-C₇₋₁₆ aralkylamino group which may have one substituent selected from a halogen atom, cyano, C₁₋₆ alkoxy, carboxyl and C₁₋₆ alkoxycarbonyl, (7) a mono-C₆₋₁₄ arylamino group, (8) a mono-C₁₋₆ alkylcarbonylamino group which may have 1 to 3 substituents selected from a halogen atom, thienyl and C₁₋₆ alkoxycarbonyl-C₁₋₆ alkylthio, (9) a mono-C₁₋₆ alkylsulfonylamino group, (10) a mono-C₆₋₁₄ arylcarbonylamino group which may have one substituent selected from C₁₋₆ alkoxy and C₁₋₆ alkylcarbonylamino, (11) a quinolylcarbonylamino group, (12) a pyridylcarbonylamino group which may have 1 or 2 halogen atoms, (13) an indolylcarbonylamino group, (14) a N-C₁₋₆ alkyl-N-C₁₋₆ alkylcarbonylamino group which may have 1 to 4 substituents selected from a halogen atom, C₁₋₆ alkoxycarbonyl and quinolyl, (15) a N-C₁₋₆ alkylcarbonyl-N-C₇₋₁₆ aralkylamino group which may have 1 to 3 halogens, (16) a N-C₁₋₆ alkyl-N-pyridylcarbonylamino group, (17) a C₁₋₆ alkylideneamino group which may have one di-C₁₋₆ alkylamino, (18) a mono-C₁₋₆ alkylureido group which may have one C₁₋₆ alkoxycarbonyl, (19) a di-C₁₋₆ alkylureido, (20) a mono-C₆₋₁₄ arylureido group, (21) a 1-imidazolidinyl group which may have 1 to 3 substituents selected from C₁₋₆ alkyl and oxo, (22) a C₁₋₆ alkyl group, (23) a C₁₋₆ alkoxycarbonyl group, (24) a nitro group or (25) a 1-pyrrolidinyl group, or R² and A or R¹ may be taken together with the adjacent carbon atom to form a nitrogen-containing 5- to 7-membered ring which may have 1 to 3 substituents selected from (1) a hydroxyl group, (2) C₁₋₆ alkyl which may have one C₁₋₆ alkoxy-carbonyl, (3) C₇₋₁₆ aralkyl, (4) C₆₋₁₄ aryl and (5) oxo,
R³ and R⁴ each represent a C₁₋₆ alkyl group,
R⁵ is a hydrogen atom,
R⁶ is a C₂₋₆ alkoxy group,
R⁷ and R⁸ are each a C₁₋₆ alkyl group,
R⁹ and R¹⁰ are each a hydrogen atom,
Y is a methylene group, and
n is 0, or a salt thereof.

Among these, the compound of the present invention is preferably a compound represented in Examples 1 to 32, 35 to 92, 98, 99, 102, 103, 111 to 113 or 121 to 168 or a salt thereof as described below, particularly 4-(6-ethoxy-3,4,8,9-tetrahydro-3,3,8,8-tetramethylfuro[2,3-h]isoquinolin-1-yl)-2-[(phenylmethyl)amino]benzoic acid, 4-(6-ethoxy-3,4,8,9-tetrahydro-3,3,8,8-tetramethylfuro[2,3-h]isoquinolin-1-yl)-2-(ethylamino)benzoic acid, (E)-3-[4-(6-ethoxy-3,4,8,9-tetrahydro-3,3,8,8-tetramethylfuro[2,3-h]isoquinolin-1-yl)-2-methoxyphenyl]-2-propenoic acid, 4-(6-ethoxy-3,4,8,9-tetrahydro-3,3,8,8-tetramethylfuro[2,3-h]isoquinolin-1-yl)-2-[(2-quinolinylcarbonyl)amino]benzoic acid, 4-(6-ethoxy-3,4,8,9-tetrahydro-3,3,8,8-tetramethylfuro[2,3-h]isoquinolin-1-yl)-2-[(2-pyridinylcarbonyl)amino]benzene acetic acid, N-[2-(aminocarbonyl)-5-(6-ethoxy-3,4,8,9-tetrahydro-3,3,8,8-tetramethylfuro[2,3-h]isoquinolin-1-yl)phenyl]-2-pyridinecarboxamide or a salt thereof.

Furthermore, in the same manner, the compound of the present invention is preferably a compound represented by the formula 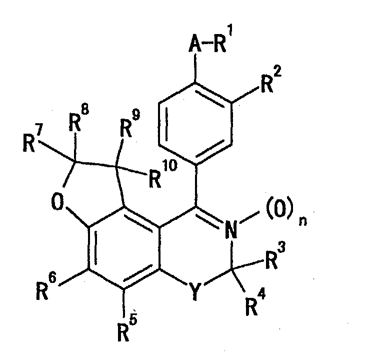 wherein A is (1) a group represented by the formula -CR^{a}=CR^{b}- (R^{a} and R^{b} each represent a hydrogen atom or C₁₋₆ alkyl), (2) a group represented by the formula -(CONH)ₚ-(C(R^{c})(R^{d}))_{q}- (R^{c} and R^{d} each represent a hydrogen atom or C₁₋₆ alkyl, p is 0 or 1 and q is 1 or 2), (3) a group represented by the formula -CH₂OCH₂- or (4) a group represented by the formula -OCH₂-, R¹ is (1) a carboxyl group, (2) a C₁₋₆ alkoxycarbonyl group, (3) an N-mono-C₁₋₆ alkylcarbamoyl group or (4) a carbamoyl group, R² is a hydrogen atom, R³ and R⁴ are each a C₁₋₆ alkyl group, R⁵ is a hydrogen atom, R⁶ is C₂₋₆ alkoxy group, R⁷ and R⁸ are each a C₁₋₆ alkyl group, R⁹ and R¹⁰ are each a hydrogen atom, Y is a methylene group and n is 0, or a salt thereof.

Among these, the compound of the present invention is preferably a compound represented in below-described Examples 33, 34, 93 to 97, 100, 101, 104 to 110 or 114 to 120 or a salt thereof, particularly [[4-(6-ethoxy-3,4,8,9-tetrahydro-3,3,8,8-tetramethylfuro[2,3-h]isoquinolin-1-yl)phenyl]methoxy]acetic acid, 4-(6-ethoxy-3,4,8,9-tetrahydro-3,3,8,8-tetramethylfuro[2,3-h]isoquinolin-1-yl)-α,α-dimethylbenzene acetic acid or a salt thereof, particularly [[4-(6-ethoxy-3,4,8,9-tetrahydro-3,3,8,8-tetramethylfuro[2,3-h]isoquinolin-1-yl)phenyl]methoxy]acetic acid hydrochloride or 4-(6-ethoxy-3,4,8,9-tetrahydro-3,3,8,8-tetramethylfuro[2,3-h]isoquinolin-1-yl)-α,α-dimethylbenzene acetic acid hydrochloride hydrate.

Salts of the compounds of the present invention include a pharmacologically acceptable salt, etc., for example, a salt with an inorganic base, an ammonium salt, a salt with an organic base, a salt with an inorganic acid, a salt with an organic acid, a salt with a basic or acidic amino acid, etc. Suitable examples of the salt with an inorganic base include an alkali metal salt such as a sodium salt, potassium salt, etc.; an alkaline earth metal salt such as a calcium salt, a magnesium salt, etc.; an aluminum salt; etc. Suitable examples of the salt with an organic base include a salt with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc. Suitable examples of the salt with an inorganic acid include a salt with hydrochloric acid, hydrobromic acid, nitric acid, sulfuric acid, phosphoric acid, etc. Suitable examples of the salt with an organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, etc. Suitable examples of the salt with a basic amino acid include a salt with arginine, lysine, ornithine, etc. Suitable examples of the salt with an acidic amino acid include a salt with aspartic acid, glutamic acid, etc.

Among those, a pharmacologically acceptable salt is preferred, and the examples thereof include, in the case of having a basic functional group, a salt with an inorganic acid such as hydrochloric acid, hydrobromic acid, nitric acid, sulfuric acid and phosphoric acid, etc., a salt with an organic acid such as acetic acid, phthalic acid, fumaric acid, oxalic acid, tartaric acid, maleic acid, citric acid, succinic acid, methanesulfonic acid and p-toluenesulfonic acid, etc. and, in the case of having an acidic functional group, an alkaline metal salt such as a sodium salt, a potassium salt, etc., an alkaline earth metal salt such as a calcium salt, magnesium salt, etc., and an ammonium salt.

The prodrug of the compound of the present invention or a salt thereof means a compound which is converted to the compound of the present invention under the physiological condition or with reaction in vivo by an enzyme, a gastric acid or the like, that is, a compound which is converted to the compound of the present invention by enzymatic oxidation, reduction, hydrolysis, etc.; a compound which is converted to the compound of the present invention with hydrolysis by gastric acid, etc.; etc. Examples of the prodrug of the compound of the present invention include a compound wherein an amino group of the compound of the present invention is substituted with acyl, alkyl or phosphoric acid (e.g. a compound wherein an amino group of the compound of the present invention is substituted with eicosanyl, alanyl, pentylaminocarbonyl, (5-methyl-2-oxo-1,3-dioxolen-4-yl)methoxycarbonyl, tetrahydrofuranyl, pyrrolidylmethyl, pivaloyloxymethyl or tert-butyl, etc.); a compound wherein a hydroxyl group of the compound of the present invention is substituted with acyl, alkyl, phosphoric acid or boric acid (e.g. a compound wherein a hydroxyl group of the compound of the present invention is substituted with acetyl, palmitoyl, propanoyl, pivaloyl, succinyl, fumaryl, alanyl or dimethylaminomethylcarbonyl, etc.); a compound wherein a carboxyl group of the compound of the present invention is substituted with ester or amide (e.g. a compound wherein a carboxyl group of the compound of the present invention is substituted with ethyl ester, phenyl ester, carboxymethyl ester, dimethylaminomethyl ester, pivaloyloxymethyl ester, ethoxycarbonyloxyethyl ester, phthalidyl ester, (5-methyl-2-oxo-1,3-dioxolen-4-yl)methyl ester, cyclohexyloxycarbonylethyl ester or methyl amide, etc.); etc. These prodrugs can be manufactured by a per se known method from the compound of the present invention.

In addition, the prodrug of the compound of the present invention may be a compound which is converted into the compound of the present invention under the physiological conditions as described in "Pharmaceutical Research and Development", Vol.7 (Molecular Design), pp. 163-198, published in 1990 by Hirokawa Publishing Co.

Furthermore, the compound in Reference Examples 27 to 115 as described below has also a phosphodiesterase IV inhibiting action in the similar manner to the compound of the present invention.

The compound of the present invention or a salt thereof can be prepared based on Reaction Formula 1 to Reaction Formula 4 as described below and modifications thereof.

Symbols of the compounds in the following Reaction Formula schemes are as described above unless otherwise stated. Furthermore, when Compounds (III), (VI) and (VIII) which are included in the compound of the present invention, and Compounds (I), (II), (IV), (V) and (VII) which are corresponding to intermediates thereof in the Reaction Formulae, form salts thereof, and the salts include, for example, those such as the above-mentioned salts of the compound of the present invention.

Compounds (I), (IV) and (V) can be prepared by, for example, the method described in WO 01/70746 or JP-A-2001-335579, or a modification thereof.

Compound (II) is commercially available, or can be prepared according to a per se known method or a modification thereof.

For the general solvent used in the following reactions, alcohols represent methanol, ethanol, 1-propanol, 2-propanol, tert-butyl alcohol and the like; ethers represent diethyl ether, diisopropyl ether, diphenyl ether, tetrahydrofuran, 1,4-dioxane, 1,2-dimethoxyethane and the like; hydrocarbons represent benzene, toluene, cyclohexane, hexane and the like; amides represent N,N-dimethylformamide, N,N-dimethylacetamide, hexamethylphosphoric triamide and the like; halogenated hydrocarbons represent dichloromethane, chloroform, carbon tetrachloride, 1,2-dichloroethane and the like; organic acids represent formic acid, acetic acid, propionic acid, trifluoroacetic acid, methanesulfonic acid and the like; esters represent methyl acetate, ethyl acetate, propyl acetate, isopropyl acetate, butyl acetate, isobutyl acetate, tert-butyl acetate, pentyl acetate, hexyl acetate and the like unless otherwise described.

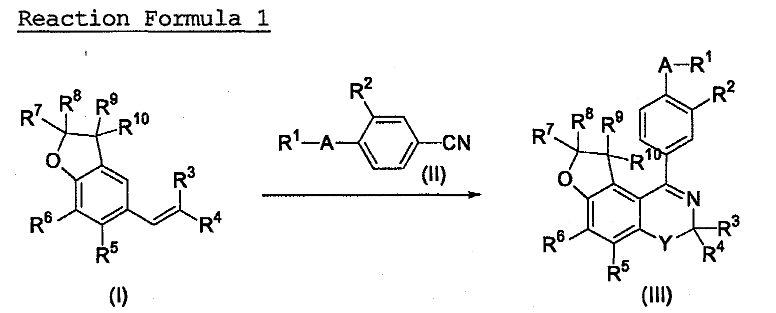

Compound (III) [wherein Y represents CH₂ or CH(OH)] is prepared by reacting Compound (I) with Compound (II) under the presence of an acid or a halogenating reagent.

The amount of Compound (II) to be used is about 0.5 to about 5 moles, preferably about 0.5 to about 2 moles, relative to 1 mole of Compound (I).

The "acid" includes mineral acids such as sulfuric acid, hydrogen chloride, hydrogen bromide, hydrogen iodide, perchloric acid, etc., Lewis acids such as a boron trifluoride diethyl ether complex, zinc chloride, aluminum chloride and the like organic acids such as methanesulfonic acid, trifluoromethanesulfonic acid, ethanesulfonic acid, benzenesulfonic acid, toluenesulfonic acid and the like and the like. The amount of the acid to be used is about 1 to about 10 moles, preferably about 1 to about 6 moles, relative to 1 mole of Compound (I).

The "halogenating reagent" includes halogens such as bromine, chlorine, iodine, etc., imides such as N-bromosuccinimide, etc., halogen adducts such as benzyltrimethylammonium dichloroiodate, benzyltrimethylammonium tribromide, etc. and the like. The amount of the halogenating reagent to be used is about 1 to about 5 moles, preferably about 1 to about 2 moles, relative to 1 mole of Compound (I).

This reaction is conducted advantageously using a solvent which is inert to the reaction. Such solvent is not particularly limited as long as the reaction proceeds, but preferably, for example, hydrocarbons, organic acids, halogenated hydrocarbons, esters or a mixture thereof and the like.

The reaction time is usually about 10 minutes to about 48 hours, preferably about 15 minutes to about 24 hours. The reaction temperature is usually about -20 to about 150°C, preferably about 0 to about 100°C.

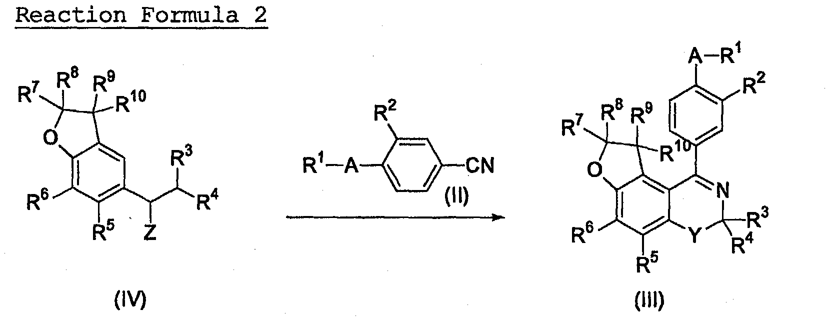

Compound (III) [wherein Y represents CH₂ or CH(OH)] is prepared by reacting Compound (IV) [wherein Z is an optionally substituted hydroxyl group or a halogen atom] with Compound (II) under the presence of an acid or a halogenating reagent.

The "optionally substituted hydroxyl group" represented by Z includes, for example, (1) a hydroxyl group, (2) an optionally halogenated C₁₋₆ alkylcarbonyloxy (e.g., acetyloxy, trifluoroacetyloxy, propionyloxy and the like), (3) an optionally halogenated C₁₋₆ alkylsulfonyloxy (e.g., methanesulfonyloxy, trifluoromethanesulfonyloxy, ethanesulfonyloxy and the like) and (4) C₆₋₁₀ arylsulfonyloxy which may have 1 to 3 substituents selected from halogen, C₁₋₆ alkyl, C₁₋₆ alkoxy and nitro (e.g., phenylsulfonyloxy, naphthylsulfonyloxy, p-chlorophenylsulfonyloxy, m-nitrophenylsulfonyloxy, p-toluenesulfonyloxy and the like).

The "halogen atom" represented by Z includes, for example, fluorine, chlorine, bromine and iodine.

The amount of Compound (II) to be used is about 0.5 to about 5 moles, preferably about 0.5 to about 2 moles, relative to 1 mole of Compound (I).

The "acid" includes mineral acids such as sulfuric acid, hydrogen chloride, hydrogen bromide, hydrogen iodide, perchloric acid, etc., Lewis acids such as a boron trifluoride diethyl ether complex, zinc chloride, aluminum chloride, etc., organic acids such as methanesulfonic acid, trifluoromethanesulfonic acid, ethanesulfonic acid, benzenesulfonic acid, toluenesulfonic acid, etc. and the like. The amount of the acid to be used is about 1 to about 10 moles, preferably about 1 to about 6 moles, relative to 1 mole of Compound (I).

The "halogenating reagent" includes halogens such as bromine, chlorine, iodine, etc., imides such as N-bromosuccinimide, etc., halogen adducts such as benzyltrimethylammonium dichloroiodate, benzyltrimethylammonium tribromide, etc. and the like. The amount of the halogenating reagent to be used is about 1 to about 5 moles, preferably about 1 to about 2 moles, relative to 1 mole of Compound (I).

This reaction is conducted advantageously using a solvent which is inert to the reaction. Such solvent is not particularly limited as long as the reaction proceeds, but preferably, for example, hydrocarbons, organic acids, halogenated hydrocarbons, esters or a mixture thereof and the like.

The reaction time is usually about 10 minutes to about 48 hours, preferably about 15 minutes to about 24 hours. The reaction temperature is usually about -20 to about 150°C, preferably about 0 to about 100°C.

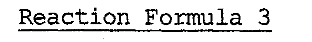

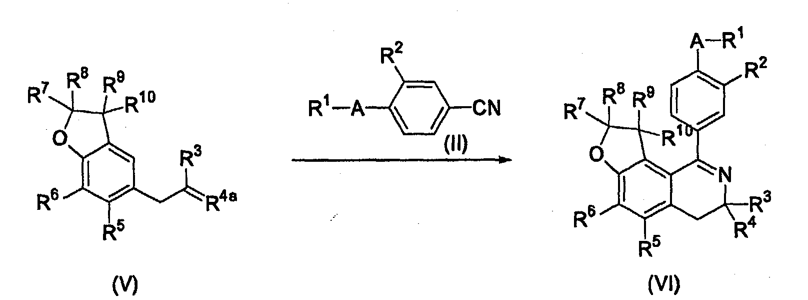

Compound (VI) is prepared by reacting Compound (V) [wherein R^{4a} is a divalent group formed by removing one hydrogen atom from R⁴] with Compound (II) under the presence of an acid or a halogenating reagent.

The amount of Compound (II) to be used is about 0.5 to about 5 moles, preferably about 0.5 to about 2 moles, relative to 1 mole of Compound (I).

The "acid" includes mineral acids such as sulfuric acid, hydrogen chloride, hydrogen bromide, hydrogen iodide, perchloric acid, etc., Lewis acids such as boron trifluoride diethyl ether complex, zinc chloride, aluminum chloride, etc., organic acids such as methanesulfonic acid, trifluoromethanesulfonic acid, ethanesulfonic acid, benzenesulfonic acid, toluenesulfonic acid, etc. and the like. The amount of the acid to be used is about 1 to about 10 moles, preferably about 1 to about 6 moles, relative to 1 mole of Compound (I).

The "halogenating reagent" includes halogens such as bromine, chlorine, iodine, etc., imides such as N-bromosuccinimide, etc., halogen adducts such as benzyltrimethylammonium dichloroiodate, benzyltrimethylammonium tribromide, etc. and the like. The amount of the halogenating reagent to be used is about 1 to about 5 moles, preferably about 1 to about 2 moles, relative to 1 mole of Compound (I).

This reaction is conducted advantageously using a solvent which is inert to the reaction. Such solvent is not particularly limited as long as the reaction proceeds, but preferably, for example, hydrocarbons, organic acids, halogenated hydrocarbons, esters or a mixture thereof and the like.

The reaction time is usually about 10 minutes to about 48 hours, preferably about 15 minutes to about 24 hours. The reaction temperature is usually about -20 to about 150°C, preferably about 0 to about 100°C.

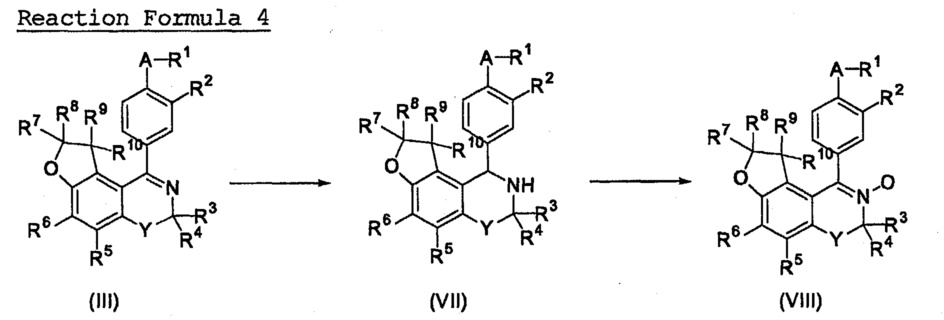

Compound (VII) is prepared by reducing Compound (III).

The reducing method includes, for example, reduction with a metal hydrogen complex such as sodium borohydride, etc., reduction with borane complexes such as a borane tetrahydrofuran complex, a borane dimethylsulfide complex, etc., reduction with alkylboranes such as thexyl borane, disiamyl borane, etc., reduction with diborane, reduction with metals such as zinc, aluminum, tin, iron, etc., reduction with alkali metals such as sodium, lithium, etc./liquid ammonia (Birch reduction), reduction by hydrogenation reaction and the like.

In the case of reduction with the metal hydrogen complex, the amount is about 1 to about 10 moles, preferably about 1 to about 3 moles, relative to 1 mole of Compound (III).

In the case of reduction with the borane complexes, alkylboranes or diborane, the amount is about 1 to about 10 moles, preferably about 1 to about 5 moles, relative to 1 mole of Compound (III).

In the case of reduction with metals or alkali metals, the amount is about 1 to about 20 equivalents, preferably about 1 to about 5 equivalents to 1 mole of Compound (III).

In this reaction, if desired, Lewis acid may be used. The "Lewis acid" includes, for example, aluminum chloride, aluminum bromide, titanium (IV) chloride, tin (II) chloride, zinc chloride, boron trichloride, boron tribromide, boron trifluoride and the like. The amount of the Lewis acid to be used is about 1 to about 5 moles, preferably about 1 to about 2 moles, relative to Compound (III) .

A hydrogenation reaction may also be used for the reduction, and in such a case, the catalyst includes palladium carbon, platinum (IV) oxide, Raney nickel, Raney cobalt and the like. The amount of the catalyst to be used is about 5 to about 1000% by weight, preferably about 10 to about 300% by weight to Compound (III).

This reaction is conducted advantageously using a solvent which is inert to the reaction. Such solvent is not particularly limited as long as the reaction proceeds, but preferably, for example, a solvent such as alcohols, ethers, hydrocarbons, amides, organic acids and the like or a mixed solvent thereof and the like.

The reaction time varies depending on kinds and amount of a reducing agent to be used or activity or amount of a catalyst, but usually about 1 hour to about 100 hours, preferably about 1 hour to about 50 hours. The reaction temperature is usually about -20 to about 120°C, preferably about 0 to about 80°C. When a hydrogenation catalyst is used, the hydrogen pressure is usually about 1 to about 100 atm.

Compound (VIII) is prepared by oxidizing Compound (VII).

The oxidizing agent used in oxidation includes, for example, hydrogen peroxide and the like. The amount of the oxidizing agent to be used is about 1 to about 20 moles, preferably about 1 to about 5 moles, relative to 1 mole of Compound (VII).

In this reaction, it is preferable to use a catalyst such as sodium tungstate (VI) and the like. The amount of the catalyst to be used is about 0.05 to about 1 mole, preferably about 0.05 to about 0.5 mole, relative to 1 mole of Compound (VII).

This reaction is conducted advantageously using a solvent which is inert to the reaction. Such a solvent is not particularly limited as long as the reaction proceeds, but preferably, for example, a solvent such as alcohols, hydrocarbons, amides, halogenated hydrocarbons, water and the like or a mixed solvent thereof and the like.

The reaction time is usually about 30 minutes to about 48 hours, preferably about 1 hour to about 24 hours. The reaction temperature is usually about -20 to about 150°C, preferably about 0 to about 100°C.

In each of the reactions described above, a starting compound having an amino, carboxy or hydroxy as its substituent may be present as a compound in which a protective group used ordinarily in a peptide chemistry has been introduced into such a substituent, and an objective compound can be obtained by deprotection if necessary after the reaction.

A protective group for amino includes, for example, formyl, or C₁₋₆ alkyl-carbonyl (e.g., acetyl, propionyl and the like), benzoyl, C₁₋₆ alkoxy-carbonyl (e.g., methoxycarbonyl, ethoxycarbonyl and the like), phenyloxycarbonyl, C₇₋₁₀ aralkyloxy-carbonyl (e.g., benzyloxycarbonyl and the like), trityl and phthaloyl, each of which may have a substituent, etc. The substituent includes a halogen atom (e.g., fluorine, chlorine, bromine and iodine and the like), C₁₋₆ alkyl-carbonyl (e.g., acetyl, propionyl, valeryl and the like), nitro and the like. The number of substituents is 1 to 3 or so.

A protective group for carboxyl includes, for example, C₁₋₆ alkyl (e.g., methyl, ethyl, propyl, isopropyl, butyl, tert-butyl and the like), phenyl, trityl and silyl, each of which may have a substituent, etc. The substituent includes a halogen atom (e.g., fluorine, chlorine, bromine and iodine and the like), formyl, C₁₋₆ alkyl-carbonyl (e.g., acetyl, propionyl, butylcarbonyl and the like), nitro, C₁₋₆ alkyl (e.g., methyl, ethyl, tert-butyl and the like), C₆₋₁₀ aryl (e.g., phenyl, naphthyl and the like) and the like. The number of substituents is 1 to 3 or so.

A protective group for hydroxy includes, for example, formyl, or C₁₋₆ alkyl (e.g., methyl, ethyl, propyl, isopropyl, butyl, tert-butyl and the like), phenyl, C₇₋₁₁ aralkyl (e.g., benzyl and the like), C₁₋₆ alkyl-carbonyl (e.g., acetyl, propionyl and the like), phenyloxycarbonyl, C₇₋₁₁ aralkyloxy-carbonyl (e.g., benzyloxycarbonyl and the like), tetrahydropyranyl, tetrahydrofuranyl and silyl, each of which may have a substituent, etc. The substituent includes a halogen atom (e.g., fluorine, chlorine, bromine and iodine and the like), C₁₋₆ alkyl (e.g., methyl, ethyl, tert-butyl and the like), C₇₋₁₁ aralkyl (e.g., benzyl and the like), C₆₋₁₀ aryl (e.g., phenyl, naphthyl and the like), nitro and the like. The number of substituents is 1 to 4 or so.

A deprotection method may be a per se known method or a modification thereof such as a treatment with an acid, base, UV, hydrazine, phenylhydrazine, sodium N-methyldithiocarbamate, tetrabutylammonium fluoride, palladium (II) acetate and the like, as well as a reduction.

In any case, deprotection, acylation, alkylation, hydrogenation, oxidation, reduction, carbon chain elongation and substituent exchange reaction are further used if necessary, alone or in combination thereof to synthesize the compound of the present invention. These reactions may employ the methods described for example in New Course of Experimental Chemistry, Vols. 14 and 15, 1977 (MARUZEN) and the like.

When an objective product is obtained in a free form by a reaction described above, and it may be then converted into a salt in accordance with an ordinary method, and when it is obtained as a salt then it may be converted into a free form or another salt in accordance with an ordinary method. The compound of the present invention thus obtained can be isolated and purified from a reaction solution by a known method such as solvent conversion, concentration, solvent extraction, fraction distillation, crystallization, recrystallization, chromatography and the like.

When the compound of the present invention is present as a configuration isomer, a diastereomer, a conformer and the like, and it can be then isolated, if desired, by a separation or purification procedure described above. When the compound of the present invention is present as a racemate, it can be resolved into an S form and an R form by an ordinary optical resolution method.

When the compound of the present invention has its stereoisomers, then individual isomers or a mixture thereof may also be encompassed in the invention.

The compound of the present invention may be hydrated or non-hydrated.

The compound of the present invention may be labeled with an isotope (e.g., ³H, ¹⁴C and ³⁵S) and the like.

Since the compound of the present invention or a salt thereof has an excellent phosphodiesterase inhibiting effect, particularly a phosphodiesterase IV-inhibiting effect and a low toxicity and also is safe, it can be used as a prophylactic or therapeutic agent in mammals (for example, human, mouse, dog, rat, cattle, etc.) against inflammatory diseases, autoimmune disease, diabetes, atherosclerosis, graft versus host disease, multiple sclerosis, sepsis, psoriasis, osteoporosis, depression, central dysfunction after cerebrovascular occlusion, cerebrovascular dementia, Alzheimer's dementia, memory disorders, obesity, cardiac insufficiency, pulmonary fibrosis, allergic diseases (e.g., atopic dermatitis, allergic rhinitis, urticaria), angina pectoris, myocardial infarction, hypertension, pulmonary hypertension, renal diseases, brain function diseases, immunity deficiency, ophthalmological diseases, male or female sexual disorders and the like, as well as a phosphodiesterase IV inhibitor. Especially, the compound of the present invention or a salt thereof can be used as a prophylactic or therapeutic agent against inflammatory diseases, atopic dermatitis, allergic rhinitis, asthma, chronic obstructive pulmonary diseases, chronic rheumatoid arthritis, autoimmune diseases, depression, Alzheimer's dementia, memory disorders, osteoporosis, diabetes, atherosclerosis and the like, as well as a phosphodiesterase IV inhibitor. The administration route may be oral or parenteral.

Particularly, the compound of the present invention can be used, by inhibiting phosphodiesterase IV A, as a prophylactic or therapeutic agent against asthma, atopic dermatitis, allergic rhinitis, chronic obstructive pulmonary diseases, rheumatoid arthritis, depression, Alzheimer's dementia, memory disorders, multiple sclerosis or osteoporosis, by inhibiting phosphodiesterase IV B, as a prophylactic or therapeutic agent against asthma, atopic dermatitis, allergic rhinitis, chronic obstructive pulmonary diseases, rheumatoid arthritis, depression, Alzheimer's dementia, memory disorders, multiple sclerosis or osteoporosis, by inhibiting phosphodiesterase IV C, as a prophylactic or therapeutic agent against asthma, atopic dermatitis, allergic rhinitis, chronic obstructive pulmonary diseases, rheumatoid arthritis, multiple sclerosis or osteoporosis, and by inhibiting phosphodiesterase IV D, as a prophylactic or therapeutic agent against asthma, atopic dermatitis, allergic rhinitis, chronic obstructive pulmonary diseases, rheumatoid arthritis, depression, Alzheimer's dementia, memory disorders, multiple sclerosis or osteoporosis.

Specific examples of formulation include a tablet (including a sugar-coated tablet and a film-coated tablet), pills, capsules (including microcapsules), granules, fine powders, powders, syrups, emulsions, suspensions, injectable preparations, inhalation preparations, ointments, eye drops, aerosols, ophthalmic ointments, hard ointments, suppositories, troches, poultices, liniments and the like. Any of these formulations can be prepared in accordance with an ordinary method (for example a method described in Japanese Pharmacopoeia).

The amount of the compound of the present invention in a formulation according to the invention may vary depending on the formulation, and it is usually 0.01 to 100% by weight, preferably 0.1 to 50% by weight, more preferably 0.5 to 20% by weight based on the whole formulation.

Specifically, a tablet is produced by mixing a medicament as it is with an excipient, binder, disintegrating agent or other suitable additives to form a homogenous mass, granulating by a suitable method, combining with a lubricant and the like, and then compression-molding it, or by mixing a medicament as it is with an excipient, a binder, a disintegrating agent or other suitable additives to form a homogenous mass and then compression-molding it, or by preparing a granule first and then compression-molding it directly or after mixing it with suitable additives to form a homogenous mass. The formulation can further contain a coloring agent, a flavoring agent and the like, if necessary. The formulation can further be film-coated by a suitable coating agent.

In a method for producing an injection formulation, a certain amount of a medicament is dissolved, suspended or emulsified in an injection solvent, physiological saline, Ringer's solution and the like when the medicament is water-soluble, or usually in vegetable oils and the like when the medicament is water-insoluble, whereby obtaining a certain quantity, or a certain amount of the medicament is sealed in a vial for an injection formulation.

A carrier for an oral formulation includes a material customarily used in the pharmaceutical field, such as starch, mannitol, crystalline cellulose, sodium carboxymethylcellulose and the like. A carrier for the injectable preparation, for example, distilled water, physiological saline, a glucose solution, an infusion solution and the like. Other additives generally used in a formulation may also be added properly.

The dose of such a formulation may vary depending on the age, the body weight, the condition, the administration route, the administration frequency and the like, but for example, a daily dose in an adult suffering from asthma is generally 0.01 to 100 mg/kg, preferably 0.1 to 50 mg/kg, more preferably 0.5 to 10 mg/kg as an active ingredient (the compound of the present invention), which is given orally once or twice divided a day.

While the compound of the invention can exhibit an excellent phosphodiesterase IV-inhibiting activity even when being given alone, it can also be used in combination (multi-medicament combination) with other pharmaceutical components other than the compound of the present invention (hereinafter abbreviated as a combination drug).

Such a combination drug includes, for example, an anti-chronic obstructive pulmonary disease agent (e.g., a β stimulant: fenoterol, salbutamol, terbutaline, formoterol, salmeterol, a mucolytic agent: ambroxol, erdosteine, carbocisteine, an expectorant: fudosteine, an antioxidant: N-acetyl cysteine and the like), an anti-asthma agent (for example, fluticasone propionate, beclomethasone propionate, theophylline, aminophylline, procaterol, ketotifen, azelastine, seratrodast, etc.), an anti-allergic agent (for example, fexofenadine, epinastine, ebastine, etc.), an anticholinergic agent (for example, thiotropium bromide, ipratropium bromide, flutropium bromide, oxitropium bromide, etc.), an anti-inflammatory agent (for example, diclofenac sodium, ibuprofen, indomethacin, loxoprofen sodium, etc.), an antibacterial agent (for example, cefixime, cefdinir, ofloxacin, tosufloxacin tosylate, levofloxacin, etc.), an antifungal agent (for example, fluconazole, itraconazole, etc.), a diabetes-treating agent (for example, pioglitazone, nateglinide, voglibose, acarbose, etc.) and the like.

In combination of the compound of the present invention and the combination drug, the administration time of the compound of the present invention and the combination drug is not restricted, and the compound of the present invention or the combination drug can be administered to the subject simultaneously, or may be administered at different times. The dosage of the combination drug may be determined according to the administration amount clinically used, and can be appropriately selected depending on the subject to be administered, the administration route, the disease, the combination and the like.

The administration mode of the compound of the present invention and the combination drug is not particularly limited, and it may be sufficient that the compound of the present invention and the combination drug are combined in administration. Examples of such administration mode include the following methods:
(1) a method wherein the compound of the present invention and the combination drug are simultaneously produced to give a single preparation which is administered;
(2) a method wherein the compound of the present invention and the combination drug are separately produced to give two kinds of preparations which are administered simultaneously through the same administration route;
(3) a method wherein the compound of the present invention and the combination drug are separately produced to give two kinds of preparations which are administered through the same administration route at different times;
(4) a method wherein the compound of the present invention and the combination drug are separately produced to give two kinds of preparations which are administered simultaneously by different administration routes;
(5) a method wherein the compound of the present invention and the combination drug are separately produced to give two kinds of preparations which are administered by different administration routes at different times (for example, the compound of the present invention and the combination drug are administered in this order, or in the reverse order) and the like. Hereinafter, these administration modes are referred to as the combination preparation of the present invention.

The combination preparation of the present invention has low toxicity, and for example, the compound of the present invention and/or the above-mentioned combination drug can be mixed with a pharmacologically acceptable carrier according to a per se known method to give a pharmaceutical composition, for example, a tablet (including a sugar-coated tablet and a film-coated tablet), powders, granules, capsules (including a soft capsule), solutions, injections, suppositories, sustained release agents and the like, each of which can be safely administered orally or parenterally (e.g., locally, rectally, intravenously and the like). The injection can be administered by intravenous, intramuscular, subcutaneous, intra-organ, intranasal, intradermal, instillation, intracerebral, intrarectal, intravaginal, intraperitoneal, intratumoral, juxtaposition of tumor and administration directly to the lesion.

The pharmacologically acceptable carrier which may be used in production of the combination preparation includes those used for the above-mentioned pharmaceutical composition of the present invention.

The compounding ratio of the compound of the present invention to the combination drug in the combination preparation of the present invention can be appropriately selected depending on the administration subject, the administration route, the diseases and the like.

For example, the content of the compound of the present invention in the combination preparation varies depending on the form of preparation, and is usually from about 0.01% by weight to about 100% by weight, preferably from about 0.1% by weight to about 50% by weight, more preferably from about 0.5% by weight to about 20% by weight, relative to the total of the preparation.

The content of the combination drug in the combination preparation of the present invention varies depending on the form of preparation, and is usually from about 0.01% by weight to about 100% by weight, preferably from about 0.1% by weight to about 50% by weight, more preferably from about 0.5% by weight to about 20% by weight, to the total of the preparation.

The content of additives such as a carrier and the like in the combination preparation of the present invention varies depending on the form of preparation, and is usually from about 1% by weight to about 99.99% by weight, preferably from about 10% by weight to about 90% by weight, to the total of the preparation.

When the compound of the present invention and the combination drug are formulated separately, the same contents may be adopted.

These preparations can be manufactured by a per se known method commonly used in the pharmaceutical manufacturing process.

For example, the compound of the present invention and the combination drug can be provided as an injectable preparation such as an aqueous injection together with a dispersing agent (e.g., Tween 80 (manufactured by Atlas Powder, US), HCO 60 (manufactured by Nikko Chemicals), polyethylene glycol, carboxymethyl cellulose, sodium alginate, hydroxypropylmethyl cellulose, dextrin and the like), a stabilizer (e.g., ascorbic acid, sodium pyrosulfite and the like), a surfactant (e.g., Polysorbate 80, Macrogol and the like), a solubilizer (e.g., glycerin, ethanol and the like), a buffer (e.g., phosphoric acid and an alkali metal salt thereof, citric acid and an alkali metal salt thereof and the like), an isotonizing agent (e.g., sodium chloride, potassium chloride, mannitol, sorbitol, glucose and the like), a pH regulator (e.g., hydrochloric acid, sodium hydroxide and the like), a preservative (e.g.', ethyl p-oxybenzoate, benzoic acid, methylparaben, propylparaben, benzyl alcohol and the like), a dissolving agent (e.g., cone. glycerin, meglumine and the like), a solubilizing agent (e.g., propylene glycol, sucrose and the like), a soothing agent (e.g., glucose, benzyl alcohol and the like) and the like, or an oily injection by dissolving, suspending or emulsifying them in a vegetable oil such as olive oil, sesame oil, cotton seed oil, corn oil and the like or a solubilizing agent such as propylene glycol.

In the case of a preparation for oral administration, the compound of the present invention and the combination drug can be provided as a preparation for oral administration according to a per se known method by adding an excipient (e.g., lactose, sucrose, starch and the like), a disintegrating agent (e.g., starch, calcium carbonate and the like), a binder (e.g., starch, arabic gum, carboxymethyl cellulose, polyvinylpyrrolidone, hydroxypropyl cellulose and the like), a lubricant (e.g., talc, magnesium stearate, polyethylene glycol 6000 and the like) and the like, to the compound of the present invention or the combination drug, and compression-molding the mixture, then if desired, coating the molded product by a per se known method for the purpose of masking of taste, enteric property or sustained release. The coating agent includes, for example, hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (methacrylic acid/acrylic acid copolymer, manufactured by Rohm, Germany), pigment (e.g., iron oxide red, titanium dioxide and the like) and the like. The preparation for oral administration may be either a rapid release preparation or a sustained release preparation.

For example, in the case of a suppository, the compound of the present invention and the combination drug can be provided as an oil or aqueous solid, semisolid or liquid suppository according to a per se known method. The oily substrate used in the above-mentioned composition includes, for example, glycerides of higher fatty acids [e.g., cacao butter, Witepsol (manufactured by Dynamit Nobel, Germany) and the like], intermediate grade fatty acids [e.g., Myglyol (manufactured by Dynamit Nobel, Germany) and the like], or vegetable oils (e.g., sesame oil, soy bean oil, cotton seed oil and the like) and the like. Further, the aqueous base includes, for example, polyethylene glycols and propylene glycol, and the aqueous gel base includes, for example, natural gums, cellulose derivatives, vinyl polymers, acrylic acid polymers and the like.

The above-mentioned sustained release agent includes sustained release microcapsules and the like. For obtaining a sustained release microcapsule, a per se known method can be adopted. For example, it is preferable to mold into a sustained release preparation shown in [2] below.

A compound of the present invention is preferably molded into an oral administration preparation such as a solid preparation (e.g., powder, granule, tablet, capsule and the like) and the like, or molded into a rectal administration preparation such as a suppository. Particularly, an oral administration preparation is preferable.

The combination drug can be provided as the above-mentioned drug form depending on the kind of the drug.

In the following, there will be shown specifically [1] an injection of the compound of the present invention or the combination drug and a production method thereof, [2] a rapid release preparation or sustained release preparation of the compound of the present invention or the combination drug and a production method thereof and [3] a sublingual tablet, a buccal or an intraoral rapid integrating agent of the compound of the present invention or the combination drug or a production method thereof.

### [1] Injectable preparation and a production method thereof

It is preferred that an injectable preparation is prepared by dissolving the compound of the present invention or the combination drug in water. This injectable preparation may be allowed to contain a benzoate and/or a salicylate.

The injection is obtained by dissolving the compound of the present invention or the combination drug, and if desired, a benzoate and/or a salicylate, into water.

The above-mentioned salts of benzoic acid and salicylic acid include, for example, salts of alkali metals such as sodium, potassium and the like, salts of alkaline earth metals such as calcium, magnesium and the like, ammonium salts, meglumine salts, organic acid salts such as tromethamol and the like.

The concentration of the compound of the present invention or the combination drug in the injection is from 0.5 w/v% to 50 w/v%, preferably from about 3 w/v% to about 20 w/v%. The concentration of a salt of benzoic acid or/and a salt of salicylic acid is from 0.5 w/v% to 50 w/v%, preferably from 3 w/v% to 20 w/v%.

Conventional additives to be used in an injection may be appropriately added in a preparation of the present invention. Examples of the additives include a stabilizer (e.g. ascorbic acid, sodium pyrosulfite and the like), a surfactant (e.g., Polysorbate 80, macrogol and the like), a solubilizer (e.g., glycerin, ethanol and the like), a buffer (e. g., phosphoric acid and an alkali metal salt thereof, citric acid and an alkali metal salt thereof and the like), an isotonizing agent (e.g., sodium chloride, potassium chloride and the like), a dispersing agent (e.g., hydroxypropylmethyl cellulose, dextrin and the like), a pH regulator (e.g., hydrochloric acid, sodium hydroxide and the like), a preservative (e.g., ethyl p-oxybenzoate, benzoic acid and the like), a dissolving agent (e.g., conc. glycerin, meglumine and the like), a solubilizing agent (e.g., propylene glycol, sucrose and the like), a soothing agent (e.g., glucose, benzyl alcohol and the like) and the like. These additives are blended in a usual proportion generally used in an injection.

It is advantageous that the pH of the injection is controlled from 2 to 12, preferably from 2.5 to 8.0 by addition of a pH regulator.

An injectable preparation is obtained by dissolving the compound of the present invention or the combination drug and if desired, a salt of benzoic acid and/or a salt of salicylic acid, and if necessary, the above-mentioned additives into water. These may be dissolved in any order, and can be appropriately dissolved by the method similar to that in a conventional method of producing an injection.

An aqueous solution for injection may be advantageously heated, alternatively, for example, filter sterilization, high pressure heat sterilization and the like can be conducted in the same manner as those for a usual injection, to provide an injection.

It may be advantageous that an aqueous solution for injection is subjected to high pressure heat sterilization at 100°C to 121°C for 5 minutes to 30 minutes.

Further, a preparation endowed with the antibacterial property of a solution may also be produced so that it can be used as a preparation which is divided and administered multiple-times.

### [2] A sustained release preparation or a rapid release preparation, and a production method thereof

Preferred is a sustained release preparation which is obtained, by coating a core containing the compound of the present invention or the combination drug with a film forming agent such as a water-insoluble substance, swellable polymer and the like, if desired. For example, a sustained release preparation for oral administration of once administration a day type is preferable.

The water insoluble substance used in film forming agent includes, for example, cellulose ethers such as ethyl cellulose, butyl cellulose and the like; cellulose esters such as cellulose acetate, cellulose propionate and the like; polyvinyl esters such as polyvinyl acetate, polyvinyl butyrate and the like; an acrylic acid/methacrylic acid copolymer, a methyl methacrylate copolymer, an ethoxyethyl methacrylate/cinnamoethyl methacrylate/aminoalkyl methacrylate copolymer, polyacrylic acid, polymethacrylic acid, methacrylic acid alkyl amide copolymer, poly(methyl methacrylate), polymethacrylate, polymethacryl amide, amino alkyl methacrylate copolymer, poly(methacrylic acid anhydride), glycidyl methacrylate copolymer, and specially an Eudragits (manufactured by Rohm Pharma) such as Eudragit RS-100, RL-100, RS-30D, RL-30D, RL-PO, RS-PO (a copolymer of ethyl acrylate/methyl methacrylate/chlorotrimethylmethacrylate/ethyl ammonium), Eudragit NE-30D (a copolymer of methyl methacrylate/ethyl acrylate) and the like, a hydrogenated oil such as hardened caster oil (e.g., Lubri wax (Freund Corporation) and the like) and the like; a wax such as carnauba wax, fatty acid glycerin ester, paraffin and the like; polyglycerin fatty acid ester and the like.

The swellable polymer is preferably a polymer having acidic dissociating group and pH-dependent swelling property, and a polymer having an acidic dissociating group which swells little in an area such as the stomach and swells in a neutral area such as the small intestine or the large intestine.

The polymer having the acidic dissociating group and pH-dependent swelling property includes, for example, crosslinkable polyacrylic polymer such as Carbomer 934P, 940, 941, 974P, 980, 1342 and the like, polycarbophil, calcium polycarbophil (all manufactured by BF Goodrich.), Hibiswako 103, 104, 105, 304 (all manufactured by Wako Pure Chemical Co., Ltd.) and the like.

The film forming agent used in a sustained release preparation may further contain a hydrophilic substance.

The hydrophilic substance includes, for example, a polysaccharide optionally having sulfuric acid group such as pullulans, dextrin, alginic acid alkali metal salt and the like; a polysaccharide having a hydroxyalkyl group or a carboxyalkyl group such as hydroxypropyl cellulose, hydroxypropyl methyl cellulose, sodium carboxymethyl cellulose and the like; methyl cellulose; polyvinyl pyrrolidone; polyvinyl alcohol; polyethylene glycol; and the like.

The content of the water-insoluble substance in the film forming agent of the sustained release preparation is about 30%(w/w) to about 90%(w/w), preferably about 35%(w/w) to about 80%(w/w), and more preferably about 40%(w/w) to about 75%(w/w). The content of the swellable polymer is about 3%(w/w) to about 30%(w/w), preferably about 3%(w/w) to about 15%(w/w). The film forming agent may further contain a hydrophilic substance, in this case, the content of the hydrophilic substance in the film forming agent is about 50%(w/w) or less, preferably about 5%(w/w) to about 40%(w/w), and more preferably about 5%(w/w) to about 35%(w/w). This %(w/w) indicates % by weight based on a film forming agent composition which is obtained by removing a solvent (e.g., water, lower alcohols such as methanol, ethanol, etc. and the like) from a solution of the film forming agent.

The sustained release preparation is manufactured by preparing a core containing drugs as exemplified below, then, coating the resulted core with a liquid of the film forming agent prepared by heating and dissolving a water-insoluble substance, a swellable polymer and the like or by dissolving or dispersing it in a solvent.

### I. Preparation of a core containing a drug

The form of a core containing a drug to be coated with a film forming agent (hereinafter, sometimes simply referred to as a core) is not particularly limited, and preferably the core is formed into particles such as granules or fine particles.

When the core is composed of granules or fine particles, the average particle size thereof is preferably from about 150 to about 2,000 µm, further preferably, from about 500 µm to about 1,400 µm.

Preparation of the core can be conducted by a usual production method. For example, it can be prepared by mixing a suitable excipient, a binder, a disintegrating agent, a lubricant, a stabilizer and the like with a drug, and subjecting the mixture to wet-extrusion granulation, fluidized-bed granulation or the like.

The content of the drugs in the core is from about 0.5%(w/w) to about 95%(w/w), preferably from about 5%(w/w) to about 80%(w/w), further preferably from about 30%(w/w) to about 70%(w/w).

The excipient contained in the core includes, for example, saccharides such as sucrose, lactose, mannitol, glucose and the like, starch, crystalline cellulose, calcium phosphate, corn starch and the like Among them, crystalline cellulose and corn starch are preferable.

The binders include, for example, polyvinyl alcohol, hydroxypropyl cellulose, polyethylene glycol, polyvinyl pyrrolidone, Pluronic F68, arabic gum, gelatin, starch and the like. The disintegrating agents include, for example, carboxymethyl cellulose calcium (ECG505), croscarmellose sodium (Ac-Di-Sol), crosslinkable polyvinyl pyrrolidone (crospovidone), low-substituted hydroxypropyl cellulose (L-HPC) and the like.
Among these, hydroxypropyl cellulose, polyvinyl pyrrolidone and low-substituted hydroxypropyl cellulose are preferable. The lubricant or the aggregation inhibitor includes, for example, talc, magnesium stearate and an inorganic salt thereof. The lubricant includes a polyethylene glycol and the like. The stabilizing agent includes an acid such as tartaric acid, citric acid, succinic acid, fumaric acid, maleic acid and the like.

In addition to the above-mentioned preparation methods, the core can also be prepared by, for example, a rolling granulation method in which a drug or a mixture of the drug with an excipient, a lubricant and the like is added portionwise to an inert carrier particle which is the center of the core while spraying a binder dissolved in a suitable solvent such as water, lower alcohols (e.g., methanol, ethanol, etc.) and the like, a pan coating method, a fluidized bed coating method or a melt granulating method. The inert carrier particle includes, for example, those made of sucrose, lactose, starch, crystalline cellulose or waxes, and the average particle size thereof is preferably from about 100 µm to about 1,500 µm.

For the purpose of separating the drug contained in the core from the film forming agent, the surface of the core may be coated with a protective agent. The protective agent includes, for example, the above-mentioned hydrophilic substances, water-insoluble substances and the like. The protective agent is preferably polyethylene glycol, and polysaccharides having a hydroxyalkyl group or carboxyalkyl group, more preferably hydroxypropylmethyl cellulose and hydroxypropyl cellulose. The protective agent may contain a stabilizer such as acids such as tartaric acid, citric acid, succinic acid, fumaric acid, maleic acid and the like, and a lubricant such as talc and the like.
When the protective agent is used, the coating amount is from about 1%(w/w) to about 15% (w/w), preferably from about 1%(w/w) to about 10%(w/w), further preferably from about 2%(w/w) to about 8%(w/w), based on the core.

The coating of the protective agent can be carried out by a usual coating method, and specifically the coating can be carried out by spraying the protective agent by a fluidized bed coating method, a pan coating method and the like.

### II. Coating of a core with a film forming agent

A core obtained in the above-mentioned step I is coated with a solution of the film forming agent obtained by heating and dissolving the above-mentioned water-insoluble substance and a pH-dependent swellable polymer and a hydrophilic substance, or by dissolving or dispersing them in a solvent, to give a sustained release preparation.

The method for coating a core with a solution of the film forming agent includes, for example, a spray coating method and the like.

The composition ratio of a water-insoluble substance, a swellable polymer and a hydrophilic substance in a solution of the film forming agent is appropriately selected so that the contents of these components in a coated film are those as described above, respectively.

The coating amount of the film forming agent is from about 1%(w/w) to about 90%(w/w), preferably from about 5%(w/w) to about 50%(w/w), further preferably from about 5%(w/w) to about 35%(w/w), based on the core (not including coating amount of the protective agent).

The solvent in the solution of the film forming agent includes water or an organic solvent alone or in admixture thereof. In the case of use in admixture, the mixing ratio of water to the organic solvent (water/organic solvent: ratio by weight) can be varied in the range from 1 to 100%, and preferably from about 1% to about 30%. The organic solvent is not particularly limited as long as it dissolves a water-insoluble substance, and for example, it includes lower alcohols such as methanol, ethanol, 2-propanol, 1-butanol and the like, lower alkanones such as acetone and the like, acetonitrile, chloroform, dichloromethane and the like. Among them, lower alcohols are preferable, and ethanol and 2-propanol are particularly preferable. Water, and a mixture of water with an organic solvent are preferably used as a solvent for a film forming agent. In this case, if necessary, an acid such as tartaric acid, citric acid, succinic acid, fumaric acid, maleic acid and the like may also be added into a solution of the film forming agent for stabilizing the solution of the film forming agent.

The operation in the case of coating by a spray coating method can be conducted by a usual coating method, and specifically it can be conducted by spray-coating a solution of the film forming agent onto a core, for example, by a fluidized bed coating method, a pan coating method and the like. In this case, if necessary, talc, titanium oxide, magnesium stearate, calcium stearate, light anhydrous silicic acid and the like may also be added as a lubricant, and glycerin fatty esters, hardened castor oils, triethyl citrate, cetyl alcohol, stearyl alcohol and the like may also be added as a plasticizer.

After coating with a film forming agent, if necessary, an antistatic agent such as talc and the like may be mixed.

The rapid release preparation may be liquid (a solution, a suspension, an emulsion and the like) or solid (particles, a pill, a tablet and the like). It may be an oral preparation or a parenteral preparation such as an injectable preparation and the like, and preferably an oral preparation.

The rapid release preparation may usually also contain a carrier, an additive and an excipient customarily used in the field of formulation (hereinafter, sometimes abbreviated as the excipient) in addition to the drug as an active component. The preparation excipient used is not particularly limited as long as it is an excipient ordinarily used as a preparation excipient.
For example, the excipient for an oral solid preparation includes lactose, starch, corn starch, crystalline cellulose (Avicel PH101, manufactured by Asahi Chemical Industry Co., Ltd. and the like), powdered sugar, granulated sugar, mannitol, light anhydrous silicic acid, magnesium carbonate, calcium carbonate, L-cysteine and the like, and preferably corn starch, mannitol and the like. These excipients can be used alone or in combinations of two or more. The content of the excipient is, for example, from about 4.5 w/w% to about 99.4 w/w%, preferably from about 20 w/w% to about 98.5 w/w%, further preferably from about 30 w/w% to about 97 w/w%, based on the total amount of the rapid release preparation.

The content of a drug in the rapid release preparation can be appropriately selected in the range from about 0.5% to about 95%, preferably from about 1% to about 60% based on the total amount of the rapid release preparation.

When the rapid release preparation is an oral solid preparation, it usually contains a disintegrating agent in addition to the above-mentioned components. The disintegrating agent includes, for example, carboxymethyl cellulose calcium (ECG-505, manufactured by Gotoku Yakuhin), croscarmellose sodium (for example, Actisol, manufactured by Asahi Chemical Industry Co., Ltd.), crospovidone (for example, Kollidon CL, manufactured by BASF), low-substituted hydroxypropyl cellulose (manufactured by Shin-Etsu Chemical Co., Ltd.), carboxymethyl starch (manufactured by Matsutani Kagaku K.K.), carboxymethyl starch sodium (Exprotab, manufactured by Kimura Sangyo), partially pregelatinized starch (PCS, manufactured by Asahi Chemical Industry Co., Ltd.) and the like, and for example, includes those which disintegrate a granule by contacting with water and absorbing water, causing swelling, or making a channel between an effective ingredient constituting the core and an excipient. These disintegrating agents can be used alone or in combinations of two or more. The amount of the disintegrating agent used is appropriately selected depending on the kind and the compounding amount of a drug used, formulation design for release property and the like, and for example, from about 0.05 w/w% to about 30 w/w%, preferably from about 0.5 w/w% to about 15 w/w%, based on the total amount of the rapid releasing agent.

When the rapid release preparation is an oral solid preparation, it may further contain if desired, additives conventional in solid preparations in addition to the above-mentioned composition. Such an additive includes, for example, a binder (e.g., sucrose, gelatin, arabic gum powder, methyl cellulose, hydroxypropyl cellulose, hydroxypropylmethyl cellulose, carboxylmethyl cellulose, polyvinylpyrrolidone, pullulans, dextrin and the like), a lubricant (e.g., polyethylene glycol, magnesium stearate, talc, light anhydrous silicic acid (for example, Aerosil (Nippon Aerosil Co., Ltd.)), a surfactant (e.g., anionic surfactants such as sodium alkylsulfate and the like, nonionic surfactants such as polyoxyethylene fatty acid ester and polyoxyethylene sorbitan fatty acid ester, polyoxyethylene castor oil derivatives and the like), a coloring agent (e.g., a tar-based pigment, caramel, iron oxide red, titanium oxide and riboflavins), and if necessary, an appetizing agent (e.g., a sweetening agent, a flavoring agent and the like), an adsorbent, a preservative, a wetting agent, an antistatic agent and the like. Further, a stabilizer such as an organic acid such as tartaric acid, citric acid, succinic acid, fumaric acid and the like may also be added.

The above-mentioned binder includes preferably hydroxypropyl cellulose, polyethylene glycol and polyvinylpyrrolidone and the like.

The rapid releasing preparation can be prepared by mixing the above-mentioned components, and if necessary, further kneading the mixture, and molding it based on a usual technology of producing preparations. The above-mentioned mixing is conducted by generally used methods, for example, mixing, kneading and the like. Specifically, when a rapid release preparation is formed, for example, into a particle, it can be prepared, according to the same means as in the above-mentioned method for preparing a core of a sustained release preparation, by mixing the components using a vertical granulator, a universal kneader (manufactured by Hata Tekkosho), a fluidized bed granulator FD-5S (manufactured by Pulek) and the like, and then subjecting the mixture to wet extrusion granulation, fluidized bed granulation and the like.

Thus obtained rapid releasing preparation and sustained releasing preparation may be themselves provided as the products, or provided appropriately together with preparation excipients and the like, separately, by an ordinary method to a preparation, and then they may be administered simultaneously or may be administered in combination at any administration interval, or they may be themselves provided as one oral preparation (e.g., granules, fine particles, tablets, capsules and the like) or provided as one oral preparation together with preparation excipients and the like. It may also be permissible that they are provided as granules or fine particles, and filled in the same capsule to be used as a preparation for oral administration.

### [3] Sublingual, buccal or intraoral rapid disintegrating agent and a production method thereof

Sublingual, buccal or intraoral rapid disintegrating agents may be a solid preparation such as a tablet and the like, or may be an oral mucosa membrane patch (film).

The sublingual, buccal or intraoral rapid disintegrating agent is preferably a preparation containing the compound of the present invention or the combination drug and an excipient. It may contain also auxiliary agents such as a lubricant, an isotonizing agent, a hydrophilic carrier, a water-dispersible polymer, a stabilizer and the like. Further, for easy absorption and increased bioavailability, β-cyclodextrin or β-cyclodextrin derivatives (e.g., hydroxypropyl-β-cyclodextrin and the like) and the like may also be contained.

The above-mentioned excipient includes lactose, sucrose, D-mannitol, starch, crystalline cellulose, light anhydrous silicic acid and the like. The lubricant includes magnesium stearate, calcium stearate, talc, colloidal silica and the like, and particularly preferably magnesium stearate and colloidal silica. The isotonizing agent includes sodium chloride, glucose, fructose, mannitol, sorbitol, lactose, saccharose, glycerin, urea and the like, and particularly preferably mannitol. The hydrophilic carrier includes a swellable hydrophilic carrier such as crystalline cellulose, ethyl cellulose, crosslinkable polyvinylpyrrolidone, light anhydrous silicic acid, silicic acid, dicalcium phosphate, calcium carbonate and the like, and particularly preferably crystalline cellulose (e.g., fine crystalline cellulose and the like). The water-dispersible polymer includes gums (e.g., gum tragacanth, acacia gum and cyamoposis gum), alginates (e.g., sodium alginate), cellulose derivatives (e.g., methyl cellulose, carboxymethyl cellulose, hydroxymethyl cellulose, hydroxypropyl cellulose and hydroxypropylmethyl cellulose), gelatin, water-soluble starch, polyacrylic acids (e.g., Carbomer), polymethacrylic acid, polyvinyl alcohol, polyethylene glycol, polyvinylpyrrolidone, polycarbophil, ascorbate, palmitates and the like, and preferably hydroxypropylmethyl cellulose, polyacrylic acid, alginate, gelatin, carboxymethyl cellulose, polyvinylpyrrolidone, polyethylene glycol and the like, particularly preferably hydroxypropylmethyl cellulose. The stabilizer includes cysteine, thiosorbitol, tartaric acid, citric acid, sodium carbonate, ascorbic acid, glycine, sodium sulfite and the like, and particularly preferably citric acid and ascorbic acid.

The sublingual, buccal or intraoral rapid disintegrating agent can be manufactured by mixing the compound of the present invention or the combination drug and an excipient by a per se known method. Further, if desired, an auxiliary agent such as a lubricant, an isotonizing agent, a hydrophilic carrier, a water-dispersible polymer, a stabilizer, a coloring agent, a sweetening agent, a preservative and the like may be mixed. The sublingual, buccal or intraoral rapid disintegrating agent is obtained by mixing the above-mentioned components simultaneously or at time intervals, and then subjecting the mixture to tablet-making molding under pressure. For obtaining suitable hardness, it may also be permissible that the materials are moistened by using a solvent such as water, alcohol and the like if desired before and after the tablet making process, and after the molding, the materials are dried, to obtain a product.

In the case of molding into a mucosa membrane patch (film), the compound of the present invention or the combination drug and the above-mentioned water-dispersible polymer (preferably, hydroxypropyl cellulose and hydroxypropylmethyl cellulose), an excipient and the like are dissolved in a solvent such as water and the like, and the resulted solution is cast to give a film. Further, additives such as a plasticizer, a stabilizer, an antioxidant, a preservative, a coloring agent, a buffer, a sweetening agent and the like may also be added. For imparting suitable elasticity to the film, glycols such as polyethylene glycol, propylene glycol and the like may be contained, or for enhancing adhesion of the film to an intraoral mucosa membrane lining, a bio-adhesive polymer (e.g., polycarbophil, carbopol) may also be contained. In the casting, a solution is poured on the non-adhesive surface, spread to uniform thickness (preferably, about 10 micron to about 1,000 micron) by an application tool such as a doctor blade and the like, and the solution is then dried to form a film. It may be advantageous that thus formed film is dried at room temperature or under heat, and cut into desired surface area.

The intraoral rapid disintegrating preparation is preferably solid rapid diffuse preparation composed of a network body comprising the compound of the present invention or the combination drug, and a water-soluble or water-diffusible carrier which is inert to the compound of the present invention or the combination drug. This network body is obtained by sublimating a solvent from the solid composition constituted of a solution prepared by dissolving the compound of the present invention or the combination drug in a suitable solvent.

The composition of an intraoral rapid disintegrating agent preferably contains a matrix forming agent and a secondary component in addition to the compound of the present invention or the combination drug.

The matrix forming agent includes animal proteins or vegetable proteins such as gelatins, dextrins, soybean, wheat, psyllium seed protein and the like; rubber substances such as arabic gum, guar gum, agar, xanthan gum and the like; polysaccharides; alginic acids; carboxymethyl celluloses; carrageenans; dextrans; pectins; synthetic polymers such as polyvinylpyrrolidone and the like; substances derived from a gelatin-arabic gum complex and the like. Further, it includes saccharides such as mannitol, dextrose, lactose, galactose, trehalose and the like; cyclic saccharides such as cyclodextrin and the like; inorganic salts such as sodium phosphate, sodium chloride, aluminum silicate and the like; amino acids having 2 to 12 carbon atoms such as glycine, L-alanine, L-aspartic acid, L-glutamic acid, L-hydroxyproline, L-isoleucine, L-leucine, L-phenylalanine and the like.

One or more of the matrix forming agent(s) can be introduced in a solution or a suspension before solidification. Such matrix forming agent may be present in addition to a surfactant, or may be present with the surfactant excluded. The matrix forming agents may help to keep the compound of the present invention or the combination drug diffused in the solution or the suspension, in addition to formation of the matrix.

The composition may contain secondary components such as a preservative, an antioxidant, a surfactant, a thickening agent, a coloring agent, a pH controlling agent, a flavoring agent, a sweetening agent, a food taste masking agent and the like. The coloring agent includes red, black and yellow iron oxides, and FD & C dyes such as FD & C Blue 2, FD & C Red 40 and the like manufactured by Elis and Eberald. Examples of the suitable flavoring agent include mint, raspberry, licorice, orange, lemon, grapefruit, caramel, vanilla, cherry, grape flavor and combinations thereof. Examples of the suitable pH controlling agent include citric acid, tartaric acid, phosphoric acid, hydrochloric acid and maleic acid. Examples of the suitable sweetening agent include aspartame, acesulfame K and thaumatine and the like. Examples of the suitable food taste-masking agent include sodium bicarbonate, ion exchange resin, cyclodextrin-inclusion compounds, adsorbent substances and microcapsulated apomorphine.

The preparation contains the compound of the present invention or the combination drug in an amount of usually from about 0.1% by weight to about 50% by weight, preferably from about 0.1% by weight to about 30% by weight, and is preferably a preparation (such as the above-mentioned sublingual agent, buccal and the like) which can dissolve 90% or more of the compound of the present invention or the combination drug (into water) within the time range of about 1 minute to about 60 minutes, preferably of about 1 minute to about 15 minutes, more preferably of about 2 minutes to about 5 minutes, and intraoral rapid disintegrating preparations which are disintegrated within the range of 1 second to 60 seconds, preferably of 1 to 30 seconds, further preferably of 1 to 10 seconds after being placed in the oral cavity.

The content of the above-mentioned excipient in the whole preparation is from about 10% by weight to about 99% by weight, preferably from about 30% by weight to about 90% by weight. The content of the β-cyclodextrin or β-cyclodextrin derivative in the whole preparation is from 0 to about 30% by weight. The content of the lubricant in the whole preparation is from about 0.01% by weight to about 10% by weight, preferably from about 1% by weight to about 5% by weight. The content of the isotonizing agent in the whole preparation is from about 0.1% by weight to about 90% by weight, preferably from about 10% by weight to about 70% by weight. The content of the hydrophilic carrier agent in the whole preparation is from about 0.1% by weight to about 50% by weight, preferably from about 10% by weight to about 30% by weight. The content of the water-dispersible polymer in the whole preparation is from about 0.1 to about 30% by weight, preferably from about 10% by weight to about 25% by weight. The content of the stabilizer in the whole preparation is from about 0.1% by weight to about 10% by weight, preferably from about 1% by weight to about 5% by weight. The above-mentioned preparation may further contain additives such as a coloring agent, a sweetening agent, a preservative and the like, if necessary.

The dose of a combination preparation of the present invention varies depending on the kind of the compound (I) of the present invention, the age, the body weight, the symptoms, the drug form, the administration method, the administration time and the like, and for example, to a patient (adult, body weight: about 60 kg), the combination preparation is administered intravenously at a dose of about 0.01 to about 1,000 mg/kg/day, preferably about 0.01 to about 100 mg/kg/day, more preferably about 0.1 to about 100 mg/kg/day, particularly about 0.1 to about 50 mg/kg/day, particularly about 1.5 to about 30 mg/kg/day in terms of the compound of the present invention or the combination drug, respectively, once or several times divided a day. Of course, since the dose as described above varies depending on various conditions, it may be sometimes sufficient to administer smaller amounts than the above-mentioned dosage, and further it may be sometimes necessary to administer greater amounts than that.

The amount of the combination drug can be set at any value unless side effects are problematical. The daily dosage in terms of the combination drug varies depending on the severity of symptoms, the age, the sex, the body weight, the sensitivity difference of the subject, the administration time and interval, the property, formulation and kind of the pharmaceutical preparation, the kind of effective ingredient and the like, but not particularly limited thereto; for example, in the case of oral administration, the dose of the drug is usually from about 0.001 mg to 2,000 mg, preferably from about 0.01 mg to 500 mg, further preferably from about 0.1 mg to 100 mg, per 1 kg body weight of a mammal, which is usually administered once to four times divided a day.

In administration of the combination preparation, the compound of the present invention may be administered after administration of the combination drug or the combination drug may be administered after administration of the compound of the present invention, though they may be administered simultaneously. When administered at a time interval, the interval varies depending on the effective ingredient, the drug form and the administration method. For example, when the combination drug is administered first, the compound of the present invention is administered within time range of from 1 minute to 3 days, preferably from 10 minutes to 1 day, more preferably from 15 minutes to 1 hour after administration of the combination drug. When the compound of the present invention is administered first, the combination drug is administered within time range of from 1 minute to 1 day, preferably from 10 minutes to 6 hours, more preferably from 15 minutes to 1 hour after administration of the compound of the present invention.

### BEST MODE FOR CARRYING OUT THE INVENTION

The present invention is further detailed in the following Reference Examples, Examples, Formulation Examples and Experimental Examples, any of which are for illustrative purpose only and is not intended to restrict the invention and can be modified without departing from the scope of the invention.

In the following Reference Examples and Examples, the term "room temperature" usually means a temperature from about 10 to about 35°C. % means a mol/mol% when used for a yield and otherwise it means % by weight. A basic silica gel used was NH-DM1020 manufactured by FUJI SILYSIA CHEMICAL LTD. Any unidentifiable broad peak such as those of OH and NH protons in each proton NMR spectrum are not included in the data.

Corn starch, hydroxypropylcellulose, magnesium stearate, lactose, croscarmellose sodium, hydroxypropylmethyl cellulose, iron (III) oxide and titanium oxide to be used in the following Formulation Examples were appropriate products for Japanese Pharmacopoeia revision 14th.

Abbreviations shown below are defined as follows:
s: Singlet
d: Doublet
t: Triplet
q: Quartet
m: Multiplet
J: Coupling constant
Hz: Hertz
CDCl₃: chloroform-d
DMSO-d₆: dimethylsulfoxide-d6
¹H NMR: Proton nuclear magnetic resonance

### Reference Example 1

### Methyl 2-amino-4-(aminocarbonyl)benzoate

To a solution of 1-methyl 2-aminoterephthalate (8.0 g, 41.0 mmol) in N,N-dimethylformamide (40 ml) were added 1-hydroxy-1H-benzotriazole·ammonium salt (6.86 g, 45.1 mmol) and 1-ethyl-3-(3-dimethylaminopropyl)carbodiimide hydrochloride (8.64 g, 45.1 mmol) under ice-cooling, and the mixture was stirred at the same temperature for 1.5 hours and at room temperature for 30 minutes. The reaction mixture was combined with ice water, and the precipitated crystals were taken by filtration, and washed with water and diethyl ether. The filtrate was neutralized with sodium hydrogen carbonate, and extracted four times with a mixed solution of tetrahydrofuran-ethyl acetate. The combined organic layer was dried over sodium sulfate, filtered, and concentrated under reduced pressure. The residue was crystallized from water-ethyl acetate to give the title compound (7.32 g, yield 92%).
¹H NMR (DMSO-d₆) δ 3.80 (3H, s), 6.74 (2H, s), 6.94 (1H, dd, J = 8.4, 1.8 Hz), 7.24 (1H, d, J = 1.8 Hz), 7.37 (1H, br s), 7.72 (1H, d, J = 8.4 Hz), 7.91 (1H, br s).

### Reference Example 2

### Methyl 4-cyano-2-[(trifluoroacetyl)amino]benzoate

To a solution of methyl 2-amino-4-(aminocarbonyl)benzoate (4.02 g, 20.7 mmol) in tetrahydrofuran (40 ml) were added triethylamine (6.43 ml, 45.5 mmol) and trifluoroacetic anhydride (6.34 ml, 45.5 mmol) under ice-cooling, and the mixture was stirred at the same temperature for 30 minutes. The reaction mixture was combined with ice water, and extracted twice with ethyl acetate. The combined organic layer was washed with a saturated aqueous solution of sodium chloride, dried over magnesium sulfate, filtered, and concentrated under reduced pressure. The residue was subjected to a silica gel column chromatography (hexane/ethyl acetate, 10:1) to give the title compound (5.65 g, quantitative).
¹H NMR (CDCl₃) δ 4.03 (3H, s), 7.53 (1H, dd, J = 8.2, 1.4 Hz), 8.22 (1H, d, J = 8.2 Hz), 9.04 (1H, d, J = 1.4 Hz), 12.30 (1H, br s).

### Reference Example 3

### Methyl 2-amino-4-cyanobenzoate

To a suspension of methyl 4-cyano-2-[(trifluoroacetyl)amino]benzoate (108 g, 397 mmol) in methanol (850 ml) was added potassium carbonate (60.3 g, 436 mmol), and the mixture was stirred for 2 hours at 50°C. After cooling, methanol was distilled off under reduced pressure, the residue was combined with water, and extracted twice with ethyl acetate. The combined organic layer was washed with 0.5 M hydrochloric acid, and a saturated aqueous solution of sodium chloride, dried over sodium sulfate, filtered, and concentrated under reduced pressure. The residue was crystallized from ethyl acetate-diisopropyl ether to give the title compound (53.9 g, yield 76%).
¹H NMR (CDCl₃) δ 3.90 (3H, s), 5.93 (2H, br s), 6.87 (1H, dd, J = 8.4, 1.8 Hz), 6.94 (1H, d, J = 1.8 Hz), 7.93 (1H, d, J = 8.4 Hz).

### Reference Example 4

### Methyl 4-cyano-2-(ethylamino)benzoate

To a solution of methyl 2-amino-4-cyanobenzoate (3.04 g, 17.3 mmol) in acetic acid (10 ml) were added acetaldehyde (90%) (10 ml) and sodium triacetoxy borohydride (7.71 g, 36.4 mmol) under ice-cooling, and the mixture was stirred at the same temperature for 1 hour and at room temperature for 2 hours. The reaction mixture was combined with ice water, neutralized with sodium hydrogen carbonate, and extracted with ethyl acetate. The combined organic layer was washed with water and a saturated aqueous solution of sodium chloride, dried over sodium sulfate, filtered, and concentrated under reduced pressure. The resultant crystals were washed with diisopropyl ether-hexane to give the title compound (800 mg, yield 23%). The mother liquor was concentrated under reduced pressure, the residue was subjected to a silica gel column chromatography (hexane/ethyl acetate, 50:1 followed by 30:1), and the obtained crystals were washed with hexane to give the title compound (920 mg, yield 26%).
¹H NMR (CDCl₃) δ 1.34 (3H, t, J = 7.0 Hz), 3.16-3.29 (2H, m), 3.88 (3H, s), 6.77-6.82 (1H, m), 6.90 (1H, s), 7.77 (1H, br s), 7.95 (1H, d, J = 7.6 Hz).

### Reference Example 5

### Methyl [(4-cyanophenyl)methoxy]acetate

To a solution of 4-cyanobenzenemethanol (5.02 g, 37.7 mmol) in tetrahydrofuran (30 ml) was added sodium hydride (66% dispersion in oil) (1.51 g, 41.5 mmol), and the mixture was stirred for 1.5 hours at 60°C. Under ice-cooling, methyl bromoacetate (3.93 ml, 41.5 mmol) was added thereto, and the mixture was stirred for 30 minutes at 60°C. After cooling, methyl bromoacetate (1.07 ml, 11.3 mmol) was further added thereto, and the mixture was stirred for 1 hour at 60°C. The reaction mixture was poured into a saturated aqueous solution of ammonium chloride under ice-cooling, and extracted twice with ethyl acetate. The combined organic layer was washed with a saturated aqueous solution of sodium chloride, dried over magnesium sulfate, filtered, and concentrated under reduced pressure. The residue was subjected to a silica gel column chromatography (hexane/ethyl acetate 5:1) to give the title compound (4.42 g, yield 57%).
¹H NMR (CDCl₃) δ 3.78 (3H, s), 4.17 (2H, s), 4.69 (2H, s), 7.49 (2H, d, J = 8.1 Hz), 7.65 (2H, d, J = 8.1 Hz).

### Reference Example 6

### 4-Iodo-3-methoxybenzonitrile

To a solution of 3-hydroxy-4-iodobenzonitrile (946 mg, 3.86 mmol) in N,N-dimethylformamide (6 ml) were added iodomethane (0.26 ml, 4.25 mmol) and sodium hydride (66% dispersion in oil) (155 mg, 4.25 mmol) under ice-cooling, and the mixture was stirred at room temperature for 2 hours. The reaction mixture was combined with ice water, and extracted twice with ethyl acetate. The combined organic layer was washed with water and a saturated aqueous solution of sodium chloride, dried over magnesium sulfate, and concentrated under reduced pressure to give the title compound (790 mg, yield 79%).
¹H NMR (CDCl₃) δ 3.93 (3H, s), 6.97-7.02 (2H, m), 7.90 (1H, d, J = 8.4 Hz).

### Reference Example 7

### 4-Cyano-2-methoxyphenyl trifluoromethanesulfonate

To a solution of 4-hydroxy-3-methoxybenzonitrile (10.9 g, 73.1 mmol) in ethyl acetate (75 ml) were added pyridine (13.0 ml, 161 mmol) and trifluoromethanesulfonic anhydride (22.7 g, 80.4 mmol) under ice-cooling, and the mixture was stirred for 1 hour at the same temperature. The reaction mixture was combined with ice water, and extracted twice with ethyl acetate. The combined organic layer was washed with 1 M hydrochloric acid, 1 M aqueous solution of sodium hydroxide, water and a saturated aqueous solution of sodium chloride, dried over magnesium sulfate, concentrated under reduced pressure, and crystallized from ethyl acetate-hexane to give the title compound (15.7 g, yield 76%).
¹H NMR (CDCl₃) δ 3.98 (3H, s), 7.30-7.35 (3H, m).

### Reference Example 8

### Methyl (E)-3-(4-cyano-2-methoxyphenyl)-2-propenoate

To a solution of 4-iodo-3-methoxybenzonitrile (790 mg, 3.05 mmol) in N,N-dimethylformamide (7 ml) were added methyl acrylate (0.57 ml, 6.30 mmol) dicyclohexylmethylamine (1.67 ml, 6.33 mmol), tris(2-methylphenyl)phosphine (118 mg, 0.338 mmol) and palladium (II) acetate (22 mg, 0.0980 mmol), and the mixture was stirred for 13 hours at 90°C. After cooling, the reaction mixture was combined with water, and extracted twice with ethyl acetate. The combined organic layer was washed with water and a saturated aqueous solution of sodium chloride, dried over magnesium sulfate, filtered, and concentrated under reduced pressure. The resultant crystals were washed with hexane to give the title compound (550 mg, yield 83%).

### Alternative synthetic method

To a suspension of 4-cyano-2-methoxyphenyl trifluoromethanesulfonate (5.03 g, 17.9 mmol), palladium (II) acetate (40 mg, 0.179 mmol) and sodium carbonate (2.28 g, 21.5 mmol) in N,N-dimethylacetamide (30 ml) were added triethyl phosphite (0.31 ml, 1.79 mmol) and methyl acrylate (3.22 ml, 35.8 mmol), and the mixture was stirred for 20 hours under nitrogen atmosphere at 100°C. After cooling, the reaction mixture was combined with water, and extracted with ethyl acetate. The organic layer was washed with water and a saturated aqueous solution of sodium chloride, dried over magnesium sulfate, filtered, and concentrated under reduced pressure. The resultant crystals were washed with diisopropyl ether to give the title compound (610 mg, yield 16%). The mother liquor was concentrated under reduced pressure, the residue was subjected to a silica gel column chromatography (hexane/ethyl acetate, 10:1 followed by 3:1) to give the title compound (560 mg, yield 14%).
¹H NMR (CDCl₃) δ 3.82 (3H, s), 3.93 (3H, s), 6.58 (1H, d, J = 16.1 Hz), 7.14 (1H, d, J = 1.5 Hz), 7.27 (1H, dd, J = 8.2, 1.5 Hz), 7.57 (1H, d, J = 8.2 Hz), 7.94 (1H, d, J = 16.1 Hz).

### Reference Example 9

### 4-Iodo-3-(phenylmethoxy)benzonitrile

With benzyl bromide, the title compound was obtained by the method similar to that in Reference Example 6. Yield 77%.
¹H NMR (CDCl₃) δ 5.19 (2H, s), 6.98-7.04 (2H, m), 7.35-7.46 (5H, m), 7.90 (1H, d, J = 7.8 Hz).

### Reference Example 10

### Methyl (E)-3-[4-cyano-2-(phenylmethoxy)phenyl]-2-propenoate

From 4-iodo-3-(phenylmethoxy)benzonitrile, the title compound was obtained by the method similar to that in Reference Example 8. Yield 74%.
¹H NMR (CDCl₃) δ 3.81 (3H, s), 5.19 (2H, s), 6.60 (1H, d, J = 16.3 Hz), 7.19 (1H, s), 7.27 (1H, d, J = 8.0 Hz), 7.60 (1H, d, J = 8.0 Hz), 8.01 (1H, d, J = 16.3 Hz).

### Reference Example 11

### Methyl 5-cyano-2-[(E)-3-methoxy-3-oxo-1-propenyl]benzoate

From methyl 5-cyano-2-iodobenzoate, the title compound was obtained by the method similar to that in Reference Example 8. Yield 73%.
¹H NMR (CDCl₃) d 3.84 (3H, s), 3.97 (3H, s), 6.36 (1H, d, J = 16.0 Hz), 7.69 (1H, d, J = 8.0 Hz), 7.81 (1H, dd, J = 8.0, 1.4 Hz), 8.28 (1H, d, J = 1.4 Hz), 8.43 (1H, d, J = 16.0 Hz).

### Reference Example 12

### Methyl 4-cyano-α,α-dimethylbenzeneacetate

To a solution of methyl 4-cyanobenzeneacetate (2.646 g, 15.1 mmol) and iodomethane (2.4 ml) in N,N-dimethylformamide (50 ml) was slowly added 60% oily sodium hydride (1.55 g, 38.8 mmol) under ice-cooling. The reaction mixture was stirred for 2 hours at room temperature. The reaction solution was slowly added to ice water, and extracted with ethyl acetate. The extracts were washed with a saturated aqueous solution of sodium chloride, dried, and concentrated under reduced pressure. The residue was subjected to a silica gel column chromatography (ethyl acetate/hexane 3:1) to give the title compound (2.996 g, yield 98%).
¹H NMR (CDCl₃) δ 1.59 (6H, s), 3.67 (3H, s), 7.42-7.47 (2H, m), 7.61-7.65 (2H, m).

### Reference Example 13

### Methyl 4-cyano-2-nitrobenzeneacetate

To a solution of 4-cyano-2-nitrobenzeneacetic acid (4.236 g, 20.5 mmol) in methanol (80 ml) was added dropwise thionyl chloride (1.5 ml) under ice-cooling. The reaction mixture was stirred at room temperature for 2 hours, and concentrated under reduced pressure. The residue was combined with a saturated aqueous solution of sodium hydrogen carbonate, and extracted with ethyl acetate. The extracts were washed with a saturated aqueous solution of sodium chloride, dried, and concentrated under reduced pressure to give the title compound (4.40 g, yield 97%).
¹H NMR (CDCl₃) δ 3.74 (3H, s), 4.11 (2H, s), 7.53 (1H, d), 7.88 (1H, dd), 8.42 (1H, d).

### Reference Example 14

### Methyl 2-amino-4-cyanobenzeneacetate

To a solution of methyl 4-cyano-2-nitrobenzeneacetate (2.793 g, 12.7 mmol) in toluene (40 ml) were added magnesium sulfate (770 mg) and 10% palladium/carbon (50% water-containing product) (268 mg), and the mixture was subjected to catalytic reduction at room temperature for 6 hours under hydrogen atmosphere. The catalyst and magnesium sulfate were separated by filtration, and the filtrate was concentrated under reduced pressure to give the title compound (2.293 g, yield 95%).
¹H NMR (CDCl₃) δ 3.60 (2H, s), 3.71 (3H, s), 4.28 (2H, br s), 6.94-7.04 (2H, m), 7.16 (1H, d).

### Reference Example 15

### Methyl 4-cyano-2-(trifluoroacetylamino)benzeneacetate

To a solution of methyl 4-cyano-2-nitrobenzeneacetate (2.241 g, 10.2 mmol) in toluene (30 ml) were added magnesium sulfate (636 mg) and 10% palladium/carbon (50% water-containing product) (110 mg), and the mixture was subjected to catalytic reduction under hydrogen atmosphere at room temperature for 5 hours. The catalyst and magnesium sulfate were separated by filtration, and the filtrate was concentrated under reduced pressure. To a solution of the residue and triethylamine (1.7 ml) in toluene (10 ml) was added dropwise a solution of trifluoroacetic anhydride (1.6 ml) in toluene (5 ml) under ice-cooling, and the reaction mixture was stirred at 0°C for 30 minutes. The reaction solution was combined with water, and extracted with ethyl acetate. The extracts were washed with a saturated aqueous solution of sodium chloride, dried, and concentrated under reduced pressure to give the title compound (2.288 g, yield 79%).
¹H NMR (CDCl₃) δ 3.75 (2H, s), 3.80 (3H, s), 7.38 (1H, d), 7.51 (1H, dd), 8.28 (1H, d), 10.32 (1H, br s).

### Reference Example 16

### Methyl 4-cyano-2-[(2-pyridinylcarbonyl)amino]benzeneacetate

To a solution of methyl 2-amino-4-cyanobenzeneacetate (1.155 g, 6.07 mmol) and 4-(dimethylamino)pyridine (2.234 g, 18.2 mmol) in N,N-dimethylformamide (50 ml) was added picolinic acid chloride hydrochloride (1.628 g, 9.15 mmol) and the mixture was stirred for 2 hours at room temperature. Picolinic acid chloride hydrochloride (0.527 g, 2.96 mmol) was further was added to the reaction mixture, and the mixture was stirred at room temperature for 1 hour. The obtained mixture was combined with water, and alkalified with an aqueous solution of 10% potassium carbonate, and extracted with ethyl acetate. The organic layer was washed with a saturated aqueous solution of sodium chloride, dried, and concentrated under reduced pressure. The resultant crude crystals were recrystallized from ethyl acetate-hexane to give the title compound (1.034 g, yield 58%).
¹H NMR (CDCl₃) δ 3.78 (3H, s), 3.79 (2H, s), 7.36-7.56 (3H, m), 7.94 (1H, dt), 8.27-8.34 (1H, m), 8.64-8.69 (1H, m), 10.70 (1H, br s) .

### Reference Example 17

### Methyl 4-cyano-α,α-dimethyl-2-nitrobenzeneacetate

To a solution of methyl 4-cyano-2-nitrobenzeneacetate (1.69 g, 7.68 mmol) and iodomethane (1.9 ml) in N,N-dimethylformamide (30 ml) was slowly added 60% oily sodium hydride (937 mg, 23.4 mmol) under ice-cooling. The reaction mixture was stirred at room temperature for 3 hours. The reaction solution was slowly added to ice water, and extracted with ethyl acetate. The extracts were washed with a saturated aqueous solution of sodium chloride, dried, and concentrated under reduced pressure. The residue was subjected to a silica gel column chromatography (ethyl acetate/hexane 3:1) to give the title compound (1.864 g, yield 98%).
¹H NMR (CDCl₃) δ 1.70 (6H, s), 3.66 (3H, s), 7.76 (1H, d), 7.89 (1H, dd), 8.21 (1H, d).

### Reference Example 18

### 6-Cyano-1,3-dihydro-3,3-dimethyl-2H-indol-2-one

To a solution of methyl 4-cyano-α,α-dimethyl-2-nitrobenzeneacetate (1.237 g, 4.98 mmol) in methanol (20 ml) was added 10% palladium/carbon (50% water-containing product) (252 mg) under hydrogen atmosphere at room temperature for 14 hours, and the mixture was subjected to catalytic reduction. The catalyst was separated by filtration, and the filtrate was concentrated under reduced pressure to give the title compound (915 mg, yield 99%).
¹H NMR (CDCl₃) δ 1.34 (6H, s), 7.24-7.32 (2H, m), 7.41 (1H, dd).

### Reference Example 19

### Methyl (E)-3-(4-cyano-2-nitrophenyl)-2-propenoate

To a solution of methyl diethylphosphonoacetate (4.2 ml) in tetrahydrofuran (10 ml) was added 60% oily sodium hydride (633 mg, 15.8 mmol) at 0°C, and the mixture was stirred at room temperature for 30 minutes. To the reaction mixture solution was added 4-formyl-3-nitrobenzonitrile (2685 mg, 15.2 mmol) at room temperature, and the mixture was further stirred at room temperature for 2.5 hours. The reaction mixture was combined with 20% ammonia water, and extracted with ethyl acetate. The extracts were washed with a saturated aqueous solution of sodium chloride, dried, and concentrated under reduced pressure. The resultant crude crystals were recrystallized from ethyl acetate-hexane to give the title compound (2695 mg, yield 76%).
¹H NMR (CDCl₃) δ 3.86 (3H, s), 6.45 (1H, d), 7.77 (1H, d), 7.89-7.96 (1H, m), 8.10 (1H, d), 8.35 (1H, d).

### Reference Example 20

### Ethyl (E)-3-(4-cyano-2-nitrophenyl)-2-propenoate

With ethyl diethylphosphonoacetate, the title compound was obtained by the method similar to that in Reference Example 19. Yield 40%.
¹H NMR (CDCl₃) δ 1.36 (3H, t, J = 7.2 Hz), 4.31 (2H, q, J = 7.2 Hz), 6.45 (1H, d, J = 15.4 Hz), 7.78 (1H, d, J = 7.8 Hz), 7.93 (1H, d, J = 7.8 Hz), 8.09 (1H, d, J = 15.4 Hz), 8.35 (1H, s).

### Reference Example 21

### Methyl (E)-3-(2-amino-4-cyanophenyl)-2-propenoate

A mixed solution of methyl (E)-3-(4-cyano-2-nitrophenyl)-2-propenoate (2.695 g, 11.6 mmol) and tin (II) chloride (11.01 g, 58.1 mmol) in ethanol (50 ml) and ethyl acetate (50 ml) was stirred for 2 hours at 70°C. The reaction mixture was cooled, and neutralized by adding a saturated aqueous solution of sodium hydrogen carbonate, and the insolubles were separated by filtration. The organic layer was separated, washed with a saturated aqueous solution of sodium chloride, dried, and concentrated under reduced pressure to give the title compound (1.771 g, yield 76%).
¹H NMR (CDCl₃) δ 3.82 (3H, s), 4.14 (2H, br s), 6.42 (1H, d), 6.94-7.05 (2H, m), 7.42 (1H, d), 7.74 (1H, d).

### Reference Example 22

### Ethyl (E)-3-(2-amino-4-cyanophenyl)-2-propenoate

From ethyl (E)-3-(4-cyano-2-nitrophenyl)-2-propenoate, the title compound was obtained by the method similar to that in Reference Example 21. Yield 79%.
¹H NMR (CDCl₃) δ 1.35 (3H, t, J = 7.2 Hz), 4.18 (2H, br s), 4.28 (2H, q, J = 7.2 Hz), 6.42 (1H, d, J = 16.1 Hz), 6.97 (1H, s), 7.02 (1H, d, J = 8.0 Hz), 7.42 (1H, d, J = 8.0 Hz), 7.74 (1H, d, J = 16.1 Hz).

### Reference Example 23

### Methyl (4-cyano-2-nitrophenoxy)acetate

To a solution of 4-cyano-2-nitrophenol (8.21 g, 50.0 mmol) and methyl bromoacetate (5.2 ml, 55 mmol) in N,N-dimethylformamide (50 ml) was added potassium carbonate (8.30 g, 60.1 mmol), and the mixture was stirred for 2 hours at room temperature. The reaction mixture was combined with water, and extracted twice with ethyl acetate. The combined organic layer was washed twice with water, 0.5 M hydrochloric acid and water, and concentrated under reduced pressure. The residue was washed with a mixed solution of ethyl acetate-diisopropyl ether to give the title compound (6.41 g, yield 54%).
¹H NMR (CDCl₃) δ 3.83 (3H, s), 4.89 (2H, s), 7.06 (1H, d, J = 8.9 Hz), 7.81 (1H, dd, J = 8.9, 2.1 Hz), 8.19 (1H, d, J = 2.1 Hz).

### Reference Example 24

### Methyl (2-amino-4-cyanophenoxy)acetate

To a solution of methyl (4-cyano-2-nitrophenoxy)acetate (1.00 g, 3.80 mmol) in tetrahydrofuran (10 ml) was added 10% palladium/carbon (50% water-containing product) (0.10 g), and the mixture was stirred at 0°C for 2 hours and at room temperature for 1 hour under hydrogen atmosphere. The catalyst was filtered off, and the filtrate was concentrated under reduced pressure. The residue was washed with diisopropyl ether to give the title compound (731 mg, yield 93%).
¹H NMR (CDCl₃) δ 3.82 (3H, s), 4.14 (2H, br s), 4.71 (2H, s), 6.69 (1H, d, J = 8.3 Hz), 6.95 (1H, d, J = 1.9 Hz), 7.01 (1H, dd, J = 8.3, 1.9 Hz).

### Reference Example 25

### Methyl (E)-3-(4-cyano-2-methylphenyl)-2-propenoate

A suspension of 4-bromo-3-methylbenzonitrile (1.96 g, 10.0 mmol), methyl acrylate (1.1 ml, 12 mmol), palladium (II) acetate (90 mg, 0.40 mmol) and tris(2-methylphenyl)phosphine (488 mg, 1.60 mmol) in triethylamine (10 ml) was stirred under nitrogen atmosphere at 100°C for 14 hours. The reaction mixture was combined with ethyl acetate, and the mixture was filtered through Hyflo Super-Cel (trade name), and the filtrate was concentrated under reduced pressure. The residue was subjected to a silica gel column chromatography (hexane/ethyl acetate, 100:1, 10:1 followed by 5:1), and recrystallized from ethyl acetate-hexane to give the title compound (423 mg, yield 21%).
¹H NMR (CDCl₃) δ 2.47 (3H, s), 3.84 (3H, s), 6.43 (1H, d, J = 15.9 Hz), 7.48-7.53 (2H, m), 7.61 (1H, d, J = 8.4 Hz), 7.91 (1H, d, J = 15.9 Hz).

### Reference Example 26

### Ethyl (E)-3-(4-cyanophenyl)-2-butenoate and ethyl (Z)-3-(4-cyanophenyl)-2-butenoate

To a solution of ethyl diethylphosphonoacetate (2.71 g, 12.1 mmol) in tetrahydrofuran (20 ml) was added sodium hydride (66% dispersion in oil) (0.44 g, 12 mmol) under ice-cooling, and the mixture was stirred at the same temperature for 10 minutes. To the obtained mixture was added portionwise 4-acetylbenzonitrile, and the mixture was stirred at room temperature 24 hours. The reaction mixture was poured into a saturated aqueous solution of ammonium chloride, and the mixture was extracted twice with ethyl acetate. The combined organic layer was washed twice with water, and concentrated under reduced pressure. The residue was subjected to a silica gel column chromatography (hexane/ethyl acetate, 15:1 followed by 5:1) to give (E)-form (748 mg, yield 34%, crystallized from diisopropyl ether-hexane) and (Z)-form (341 mg, yield 16%, oil).
(E)-form: ¹H NMR (CDCl₃) δ 1.33 (3H, t, J = 7.1 Hz), 2.57 (3H, d, J = 1.5 Hz), 4.23 (2H, q, J = 7.1 Hz), 6.15 (1H, q, J = 1.5 Hz), 7.56 (2H, d, J = 8.7 Hz), 7.68 (2H, d, J = 8.7 Hz).
(Z)-form: ¹H NMR (CDCl₃) δ 1.11 (3H, t, J = 7.2 Hz), 2.17 (3H, d, J = 1.7 Hz), 4.01 (2H, q, J = 7.2 Hz), 5.97 (1H, q, J = 1.7 Hz), 7.30 (2H, d, J = 8.4 Hz), 7.65 (2H, d, J = 8.4 Hz).

### Reference Example 27

### [4-(6-Ethoxy-3,4,8,9-tetrahydro-3,3,8,8-tetramethylfuro[2,3-h]isoquinolin-1-yl)phenyl]methyl acetate

To a solution of 7-ethoxy-2,3-dihydro-2,2-dimethyl-α-(1-methylethyl)-5-benzofuranmethanol (1.26 g, 4.78 mmol) and 4-cyanobenzenemethanol (530 mg, 3.98 mmol) in toluene (3.5 ml) and acetic acid (2 ml) was added conc. sulfuric acid (0.53 ml, 9.95 mmol), and the mixture was stirred for 1 hour at 80°C. Ethanol (35.9 ml) was added dropwise thereto at the same temperature and the mixture was stirred for 30 minutes. After cooling, the reaction mixture was poured into ice water, and washed with diisopropyl ether. The aqueous layer was neutralized with sodium hydrogen carbonate, and extracted three times with ethyl acetate. The combined organic layer was washed with a saturated aqueous solution of sodium chloride, dried over sodium sulfate, filtered, and concentrated under reduced pressure. The residue was subjected to a basic silica gel column chromatography (hexane/ethyl acetate, 10:1 followed by 5:1), and crystallized from diisopropyl ether-hexane to give the title compound (614 mg, yield 37%).
¹H NMR (CDCl₃) δ 1.23 (6H, s), 1.28 (6H, s), 1.46 (3H, t, J = 7.0 Hz), 2.11 (3H, s), 2.20 (2H, s), 2.66 (2H, s), 4.18 (2H, q, J = 7.0 Hz), 5.15 (2H, s), 6.60 (1H, s), 7.34-7.42 (4H, m).

### Reference Example 28

### 4-(6-Ethoxy-3,4,8,9-tetrahydro-3,3,8,8-tetramethylfuro[2,3-h]isoquinolin-1-yl)benzenemethanol

To a solution of [4-(6-ethoxy-3,4,8,9-tetrahydro-3,3,8,8-tetramethylfuro[2,3-h]isoquinolin-1-yl)phenyl]methyl acetate (881 mg, 2.09 mmol) in methanol (4 ml) was added 5 M aqueous solution (1 ml) of sodium hydroxide, and the mixture was stirred for 1 hour at room temperature. Methanol was distilled off under reduced pressure, the residue was combined with water and diisopropyl ether, and neutralized with 5 M hydrochloric acid. The precipitated crystals were taken by filtration, and washed with water and diisopropyl ether to give the title compound (666 mg, yield 84%).
¹H NMR (CDCl₃) δ 1.24 (6H, s), 1.31 (6H, s), 1.46 (3H, t, J = 7.0 Hz), 2.21 (2H, s), 2.66 (2H, s), 4.18 (2H, q, J = 7.0 Hz), 4.72 (2H, s), 6.60 (1H, s), 7.33 (2H, d, J = 8.6 Hz), 7.38 (2H, d, J = 8.6 Hz).

### Reference Example 29

### 4-(6-Ethoxy-3,4,8,9-tetrahydro-3,3,8,8-tetramethylfuro[2,3-h]isoquinolin-1-yl)phenol

To a solution of 4-cyanophenol (3.00 g, 25.2 mmol) and 7-ethoxy-2,3-dihydro-2,2-dimethyl-α-(1-methylethyl)-5-benzofuranmethanol (8.66 g, 32.8 mmol) in acetic acid (30 ml)-toluene (40 ml) was added conc. sulfuric acid (3.49 ml, 65.5 mmol), and the mixture was stirred at 80°C for 1 hour. The reaction solution was cooled with ice, water was added thereto, and the mixture was washed with diisopropyl ether. The aqueous layer was again ice-cooled and alkalified with conc. ammonia water, and extracted with ethyl acetate. The extracts were washed with water, and concentrated under reduced pressure. The residue was purified with a silica gel column chromatography (ethyl acetate/methanol 19:1), and crystallized from ethyl acetate-hexane to give the title compound (1.47 g, yield 16%).
¹H NMR (CDCl₃) δ 1.28 (6H, s), 1.30 (6H, s), 1.46 (3H, t, J = 7.0 Hz), 2.24 (2H, s), 2.69 (2H, s), 4.18 (2H, q, J = 7.0 Hz), 6.45 (2H, d, J = 8.6 Hz), 6.59 (1H, s), 7.03 (2H, d, J = 8.6 Hz), 8.01 (1H, br s).

### Reference Example 30

### 3-[6-(Ethylthio)-3,4,8,9-tetrahydro-3,3,8,8-tetramethylfuro[2,3-h]isoquinolin-1-yl]benzoic acid

To a solution of 1-methylethyl 3-[6-(ethylthio)-3,4,8,9-tetrahydro-3,3,8,8-tetramethylfuro[2,3-h]isoquinolin-1-yl]benzoate (2.28 g, 5.05 mmol) in methanol (10 ml) was added 5 M aqueous solution (5 ml) of sodium hydroxide, and the mixture was stirred at room temperature for 15 hours. The reaction solution was adjusted at pH 4.5 with 5 M hydrochloric acid, methanol was added thereto, and the solvent was distilled off under reduced pressure. The residue was dissolved in water, and extracted three times with ethyl acetate. The combined organic layer was concentrated under reduced pressure, the residue was recrystallized from ethyl acetate-diisopropyl ether to give the title compound (1.50 g, yield 73%).
¹H NMR (CDCl₃) δ 1.28 (6H, s), 1.39 (3H, t, J = 7.2 Hz), 1.52 (6H, s), 2.17 (2H, s), 2.92 (2H, s), 3.04 (2H, q, J = 7.2 Hz), 6.95 (1H, s), 7.46 (1H, t, J = 7.4 Hz), 7.61 (1H, d, J = 7.4 Hz), 8.03 (1H, d, J = 7.4 Hz), 8.13 (1H, s).

### Reference Example 31

### N-(2-Amino-1,1-dimethyl-2-oxoethyl)-3-[6-(ethylthio)-3,4,8,9-tetrahydro-3,3,8,8-tetramethylfuro[2,3-h]isoquinolin-1-yl]benzamide

To a solution of 3-[6-(ethylthio)-3,4,8,9-tetrahydro-3,3,8,8-tetramethylfuro[2,3-h]isoquinolin-1-yl]benzaic acid (1.76 g, 4.30 mmol), α,α-dimethylglycine amide hydrochloride (655 mg, 4.73 mmol), 1-hydroxy-1H-benzotriazole monohydrate (724 mg, 4.73 mmol) and triethylamine (1.50 ml, 10.8 mmol) in N,N-dimethylformamide (8 ml) was added 1-ethyl-3-(3-dimethylaminopropyl)carbodiimide hydrochloride (1.07 g, 5.59 mmol) under ice-cooling, and the mixture was stirred at room temperature for 15 hours. To the reaction mixture was poured ice water, and the mixture was extracted three times with ethyl acetate. The combined organic layer was washed with an aqueous solution of sodium chloride and a saturated aqueous solution of sodium chloride, dried over sodium sulfate, filtered, and concentrated under reduced pressure. The residue was crystallized from ethyl acetate-diisopropyl ether to give the title compound (2.01 g, yield 95%).
¹H NMR (CDCl₃) δ 1.25 (6H, s), 1.30 (6H, s), 1.34 (3H, t, J = 7.4 Hz), 1.71 (6H, s), 2.17 (2H, s), 2.68 (2H, s), 3.00 (2H, q, J = 7.4 Hz), 5.51 (1H, br s), 6.45 (1H, br s), 6.93 (1H, s), 7.02 (1H, s), 7.42-7.52 (2H, m), 7.85-7.89 (2H, m).

### Reference Example 32

### N-(2-Amino-1,1-dimethyl-2-oxoethyl)-3-[6-(ethylsulfinyl)-3,4,8,9-tetrahydro-3,3,8,8-tetramethylfuro[2,3-h]isoquinolin-1-yl]benzamide

To a suspension of N-(2-amino-1,1-dimethyl-2-oxoethyl)-3-[6-(ethylthio)-3,4,8,9-tetrahydro-3,3,8,8-tetramethylfuro[2,3-h]isoquinolin-1-yl]benzamide (826 mg, 1.67 mmol) in methanol (7 ml) was added a solution of sodium periodate (895 mg, 4.18 mmol) in water (5 ml) under ice-cooling, and the mixture was stirred at room temperature for 40 hours. The solvent was distilled off under reduced pressure, and the residue was combined with an aqueous solution of sodium chloride, and extracted three times with tetrahydrofuran. The combined organic layer was dried over sodium sulfate, and concentrated under reduced pressure. The residue was subjected to a basic silica gel column chromatography (hexane/ethyl acetate 1:3 followed by ethyl acetate) to give the title compound (85 mg), and a mixture of the starting materials and the title compound (528 mg). The mixture was dissolved in methanol (6 ml), a solution of sodium periodate (450 mg, 2.10 mmol) in water (2.5 ml) was added thereto, and the mixture was stirred at room temperature for 18 hours. The solvent was distilled off under reduced pressure, and the residue was combined with an aqueous solution of sodium chloride, and extracted twice with ethyl acetate-tetrahydrofuran. The combined organic layer was dried over sodium sulfate, and concentrated under reduced pressure. The residue was subjected to a basic silica gel column chromatography (hexane/ethyl acetate 1:3 followed by ethyl acetate), collected with those previously obtained, and recrystallized from ethyl acetate-diisopropyl ether to give the title compound (292 mg, yield 34%).
¹H NMR (CDCl₃) δ 1.20-1.31 (15H, m), 1.73 (6H, s), 2.18 (2H, s), 2.77 (2H, s), 2.83-3.18 (2H, m), 5.48 (1H, br s), 6.37 (1H, br s), 7.05 (1H, s), 7.44 (1H, s), 7.48-7.56 (2H, m), 7.83-7.88 (2H, m).

### Reference Example 33

### N-(2-Amino-1,1-dimethyl-2-oxoethyl)-3-[6-(ethylsulfonyl)-3,4,8,9-tetrahydro-3,3,8,8-tetramethylfuro[2,3-h]isoquinolin-1-yl]benzamide

To a suspension of N-(2-amino-1,1-dimethyl-2-oxoethyl)-3-[6-(ethylsulfinyl)-3,4,8,9-tetrahydro-3,3,8,8-tetramethylfuro[2,3-h]isoquinolin-1-yl]benzamide (588 mg, 1.19 mmol) in methanol (6 ml) was added a solution of sodium periodate (1.27 g, 5.96 mmol) in water (5 ml) under ice-cooling, and the mixture was stirred at 60°C for 18 hours. After cooling, solvent was distilled off under reduced pressure, to the residue were added an aqueous solution of sodium chloride, sodium hydrogen carbonate and ethyl acetate, and the precipitated crystals were taken by filtration to give the title compound (265 mg, yield 50%). The filtrate was extracted twice with a mixed solution of ethyl acetate-tetrahydrofuran, the combined organic layer was dried over sodium sulfate, and concentrated under reduced pressure. The residue was subjected to a basic silica gel column chromatography (hexane/ethyl acetate 1:3 followed by ethyl acetate), and recrystallized from ethyl acetate to give the title compound (185 mg, yield 30%).
¹H NMR (CDCl₃+DMSO-d₆ 5 drops) δ 1.26 (6H, s), 1.30 (3H, t, J = 7.5 Hz), 1.30 (6H, s), 1.72 (6H, s), 2.24 (2H, s), 2.75 (2H, s), 3.34 (2H, q, J = 7.5 Hz), 6.11 (1H, br s), 6.89 (1H, br s), 7.49-7.52 (3H, m), 7.75 (1H, s), 7.90-7.94 (2H, m).

### Reference Example 34

### Ethyl 3-(3,4,8,9-tetrahydro-6-methoxy-3,3,8,8-tetramethylfuro[2,3-h]isoquinolin-1-yl)benzoate

To a solution of 2,3-dihydro-7-methoxy-2,2-dimethyl-a-(1-methylethyl)-5-benzofuranmethanol (16.0 g, 63.9 mmol) and 3-cyanobenzoic acid (7.84 g, 53.3 mmol) in toluene (60 ml) and acetic acid (35 ml) was added conc. sulfuric acid (7.10 ml, 133 mmol), and the mixture was stirred for 1 hour at 80°C. Ethanol (35.9 ml) was added dropwise thereto at the same temperature and the mixture was stirred for 30 minutes. After cooling, the reaction mixture was poured into ice water, and washed with diethyl ether. The aqueous layer was neutralized with conc. ammonia water, and extracted twice with diethyl ether. The combined organic layer was washed with a saturated aqueous solution of sodium chloride, dried over sodium sulfate, filtered, and concentrated under reduced pressure. The residue was subjected to a basic silica gel column chromatography (hexane/ethyl acetate 20:1), and the obtained crystals were washed with pentane to give the title compound (2.11 g, yield 10%).
¹H NMR (CDCl₃) δ 1.26 (6H, s), 1.30 (6H, s), 1.49 (3H, t, J = 7.2 Hz), 2.17 (2H, s), 2.70 (2H, s), 3.93 (3H, s), 4.38 (2H, q, J = 7.2 Hz), 6.63 (1H, s), 7.43-7.64 (2H, m), 8.06-8.11 (2H, m).

### Reference Example 35

### 3-(3,4,8,9-Tetrahydro-6-hydroxy-3,3,8,8-tetramethylfuro[2,3-h]isoquinolin-1-yl)benzoic acid hydrobromide

A solution of 3-(3,4,8,9-tetrahydro-6-methoxy-3,3,8,8-tetramethylfuro[2,3-h]isoquinolin-1-yl)benzoic acid (840 mg, 2.21 mmol) in 48% hydrobromic acid (6 ml) was stirred at 110°C for 36 hours. After ice-cooling, the precipitated crystals were taken by filtration, and washed with water and diethyl ether to give the title compound (676 mg, yield 69%).
¹H NMR (DMSO-d₆) δ 1.22 (6H, s), 1.43 (6H, s), 2.07 (1H, d, J = 14.6 Hz), 2.18 (1H, d, J = 14.6 Hz), 3.09 (2H, s), 6.78 (1H, s), 7.73-7.87 (2H, m), 8.14-8.28 (2H, m), 11.29 (1H, br s), 12.32 (1H, br s).

### Reference Example 36

### 3,4,8,9-Tetrahydro-6-methoxy-α,α,3,3,8,8-hexamethyl-1-phenylfuro[2,3-h]isoquinoline-5-acetonitrile

To a solution of 3,4,8,9-tetrahydro-6-methoxy-3,3,8,8-tetramethyl-1-phenylfuro[2,3-h]isoquinoline-5-acetonitrile (215 mg, 0.574 mmol) in N,N-dimethylformamide (2 ml) iodomethane (0.079 ml, 1.26 mmol) was added sodium hydride (66% dispersion in oil) (46 mg, 1.26 mmol) under ice-cooling, and the mixture was stirred at the same temperature for 30 minutes and at room temperature for 20 hours. The reaction mixture was combined with ice water, and extracted twice with ethyl acetate. The combined organic layer was washed with water and a saturated aqueous solution of sodium chloride, dried over sodium sulfate, and concentrated under reduced pressure. The residue was subjected to a basic silica gel column chromatography (hexane/ethyl acetate 20:1), and recrystallized from hexane to give the title compound (77 mg, yield 33%).
¹H NMR (CDCl₃+DMSO-d₆ 5 drops) δ 1.20 (6H, s), 1.29 (6H, s), 1.94 (6H, s), 2.15 (2H, s), 2.88 (2H, s), 3.95 (3H, s), 7.39-7.45 (5H, m) .

### Reference Example 37

### 1-Methylethyl 3-[3,4,8,9-tetrahydro-3,3,8,8-tetramethyl-6-(propylthio)furo[2,3-h]isoquinolin-1-yl]benzoate

To a solution of 1.57 M n-butyllithium/hexane (42.3 ml, 66.4 mmol) were added dropwise a solution of N,N,N',N'-tetramethylethylenediamine (10.0 ml, 66.4 mmol) in tetrahydrofuran (15 ml), a solution of 7-bromo-2,3-dihydro-2,2-dimethyl-5-(2-methyl-1-propenyl)benzofuran (4.68 g, 16.6 mmol) in tetrahydrofuran (15 ml) and a solution of n-propyl disulfate (20 g, 133 mmol) in tetrahydrofuran (15 ml) at -78°C in this order, and the mixture was stirred for 15 hours with elevating the temperature naturally to room temperature. The reaction mixture was poured into a saturated aqueous solution of ammonium chloride, and the mixture was extracted twice with ethyl acetate. The combined organic layer was washed with a saturated aqueous solution of sodium chloride, dried over magnesium sulfate, filtered, and concentrated under reduced pressure. The residue was subjected to a silica gel column chromatography (hexane followed by hexane/ethyl acetate 50:1) to give a mixture of 2,3-dihydro-2,2-dimethyl-5-(2-methyl-1-propenyl)-7-(propylthio)benzofuran and 2,3-dihydro-2,2-dimethyl-5-(2-methyl-1-propenyl)benzofuran at about 15:2 (4.11 g).

To a suspension of the obtained mixture (3.10 g) and 3-cyanobenzoate 1-methylethyl (1.83 g, 9.65 mmol) in acetic acid (6 ml) and toluene (13 ml) was added dropwise conc. sulfuric acid (1.29 ml, 24.1 mmol) under ice-cooling, and the mixture was stirred for 1.5 hours at 60°C. Conc. sulfuric acid (0.51 ml, 9.65 mmol) and 2-propanol (11.7 ml) were further added dropwise thereto, and the mixture was heated under reflux for 5 hours. To the reaction mixture was poured ice water, the mixture was neutralized with sodium hydrogen carbonate, and extracted twice with ethyl acetate. The combined organic layer was washed with a saturated aqueous solution of sodium chloride, dried over sodium sulfate, filtered, and concentrated under reduced pressure. The residue was dissolved in N,N-dimethylformamide (20 ml). 2-iodopropane (0.48 ml, 4.83 mmol) and potassium carbonate (668 mg, 4.83 mmol) were added thereto, and the mixture was stirred for 15 hours at room temperature. To the reaction mixture was poured ice water, and the mixture was extracted twice with ethyl acetate. The combined organic layer was washed with a saturated aqueous solution of sodium chloride, dried over sodium sulfate, filtered, and concentrated under reduced pressure. The residue was subjected to a silica gel column chromatography (hexane/ethyl acetate, 10:1 followed by 5:1) to give the title compound (1.00 g, yield 22%) .
Oily matter.

### Reference Example 38

### 3-[3,4,8,9-Tetrahydro-3,3,8,8-tetramethyl-6-(propylthio)furo[2,3-h]isoquinolin-1-yl]benzoic acid

From 1-methylethyl 3-[3,4,8,9-tetrahydro-3,3,8,8-tetramethyl-6-(propylthio)furo[2,3-h]isoquinolin-1-yl]benzoate, the title compound was obtained by the method similar to that in Reference Example 30. Yield 17%.
Melting point: 206-208°C (ethyl acetate-diisopropyl ether).
¹H NMR (CDCl₃) δ 1.11 (3H, t, J = 7.5 Hz), 1.25 (6H, s), 1.51 (3H, s), 1.74-1.86 (2H, m), 1.91 (3H, s), 2.10 (2H, s), 2.92 (1H, d, J = 13.0 Hz), 3.04 (2H, t, J = 7.2 Hz), 3.41 (1H, d, J = 13.0 Hz), 6.97 (1H, s), 7.66 (1H, dd, J = 7.8, 7.5 Hz), 7.68 (1H, s), 8.00 (1H, d, J = 7.5 Hz), 8.12 (1H, d, J = 7.8 Hz).

### Reference Example 39

### N-(2-Amino-1,1-dimethyl-2-oxoethyl)-3-[3,4,8,9-tetrahydro-3,3,8,8-tetramethyl-6-(propylthio)furo[2,3-h]isoquinolin-1-yl]benzamide

From 3-[3,4,8,9-tetrahydro-3,3,8,8-tetramethyl-6-(propylthio)furo[2,3-h]isoquinolin-1-yl]benzoic acid, the title compound was obtained by the method similar to that in Reference Example 31. Yield 57%.
Melting point:195-197°C (diisopropyl ether).
¹H NMR (CDCl₃) d 1.05 (3H, t, J = 7.2 Hz), 1.25 (6H, s), 1.30 (6H, s), 1.62-1.78 (2H, m), 1.71 (6H, s), 2.17 (2H, s), 2.68 (2H, s), 2.95 (2H, t, J = 7.3 Hz), 5.49 (1H, br s), 6.43 (1H, br s), 6.92 (1H, s), 6.96 (1H, s), 7.43-7.52 (2H, m), 7.85-7.89 (2H, m).

### Reference Example 40

### N-(2-Amino-1,1-dimethyl-2-oxoethyl)-3-[3,4,8,9-tetrahydro-3,3,8,8-tetramethyl-6-(propylsulfinyl)furo[2,3-h]isoquinolin-1-yl]benzamide

From N-(2-amino-1,1-dimethyl-2-oxoethyl)-3-[3,4,8,9-tetrahydro-3,3,8,8-tetramethyl-6-(propylthio)furo[2,3-h]isoquinolin-1-yl]benzamide, the title compound was obtained by the method similar to that in Reference Example 32. Yield 87%.
¹H NMR (CDCl₃) δ 1.07 (3H, t, J = 7.5 Hz), 1.23-1.35 (12H, m), 1.60-2.05 (2H, m), 1.72 (6H, s), 2.19 (2H, s), 2.75-3.05 (2H, m), 2.80 (2H, s), 5.51 (1H, br s), 6.39 (1H, br s), 7.12 (1H, br s), 7.44-7.55 (2H, m), 7.85-7.92 (2H, m).

### Reference Example 41

### α-Fluoro-3,4,8,9-tetrahydro-6-methoxy-3,3,8,8-tetramethyl-1-phenylfuro[2,3-h]isoquinoline-5-acetonitrile

To a solution of 3,4,8,9-tetrahydro-6-methoxy-3,3,8,8-tetramethyl-1-phenylfuro[2,3-h]isoquinoline-5-acetonitrile (301 mg, 0.804 mmol) in tetrahydrofuran (5 ml) was added dropwise a solution of 1.54 M tert-butyllithium/pentane (1.15 ml, 1.77 mmol) at -78°C, and the mixture was stirred for 1 hour at the same temperature. A solution of N-fluorobenzenesulfonimide (634 mg, 2.01 mmol) in tetrahydrofuran (5 ml) was added dropwise at -78°C thereto, and the mixture was stirred with elevating the temperature naturally to room temperature for 3 hours. The reaction mixture was combined with ice water, and extracted twice with ethyl acetate. The combined organic layer was washed with a saturated aqueous solution of sodium chloride, dried over sodium sulfate, filtered, and concentrated under reduced pressure. The residue was subjected to a basic silica gel column chromatography (hexane/ethyl acetate, 30:1 followed by 10:1), and crystallized from pentane to give the title compound (198 mg, yield 63%).
¹H NMR (CDCl₃) δ 1.27 (6H, s), 1.30 (6H, s), 2.13 (1H, d, J = 14.0 Hz), 2.22 (1H, d, J = 14.0 Hz), 2.82 (1H, d, J = 15.6 Hz), 2.98 (1H, dd, J = 15.6, 3.6 Hz), 4.04 (3H, s), 6.66 (1H, d, J = 45.2 Hz), 7.39 (5H, s).

### Reference Example 42

### α,α-Difluoro-3,4,8,9-tetrahydro-6-methoxy-3,3,8,8-tetramethyl-1-phenylfuro[2,3-h]isoquinoline-5-acetonitrile

To a solution of α-fluoro-3,4,8,9-tetrahydro-6-methoxy-3,3,8,8-tetramethyl-1-phenylfuro[2,3-h]isoquinoline-5-acetonitrile (89 mg, 0.227 mmol) in tetrahydrofuran (1 ml) was added dropwise a solution of 1.54 M tert-butyllithium/pentane (0.16 ml, 0.249 mmol) at -78°C, and the mixture was stirred for 1 hour at the same temperature. A solution of N-fluorobenzenesulfonimide (93 mg, 0.295 mmol) in tetrahydrofuran (5 ml) was added dropwise at -78°C thereto, and the mixture was stirred for 15 hours with elevating the temperature naturally to room temperature. The reaction mixture was combined with ice water, and extracted twice with ethyl acetate. The combined organic layer was washed with a saturated aqueous solution of sodium chloride, dried over sodium sulfate, filtered, and concentrated under reduced pressure. The residue was subjected to a silica gel column chromatography (hexane/ethyl acetate, 10:1 followed by 5:1), and crystallized from diisopropyl ether-hexane to give the title compound (14 mg, yield 15%).
¹H NMR (CDCl₃) δ 1.23 (6H, s), 1.30 (6H, s), 2.19 (2H, t, J = 1.8 Hz), 2.85 (2H, t, J = 2.8 Hz), 4.08 (3H, s), 7.40 (5H, s).

### Reference Example 43

### Ethyl 3-(3,4,8,9-tetrahydro-6-hydroxy-3,3,8,8-tetramethylfuro[2,3-h]isoquinolin-1-yl)benzoate hydrochloride

To a solution of ethyl 3-(6-ethoxy-3,4,8,9-tetrahydro-3,3,8,8-tetramethylfuro[2,3-h]isoquinolin-1-yl)benzoate (117 mg, 0.278 mmol) in 1,2-dichloroethane (2 ml) were added trichloroacetyl chloride (0.037 ml, 0.333 mmol) and aluminum chloride (44 mg, 0.333 mmol) under ice-cooling, and the mixture was stirred at room temperature for 6 hours. The reaction mixture was poured into ice water, neutralized with 5 M aqueous solution of sodium hydroxide, and extracted twice with ethyl acetate. The combined organic layer was washed with water and a saturated aqueous solution of sodium chloride, dried over sodium sulfate, and concentrated under reduced pressure. The residue was subjected to a basic silica gel column chromatography (ethyl acetate) to give the title compound as free base. This was dissolved in ethyl acetate, a solution of 4 M hydrogen chloride/ethyl acetate was added thereto, and the mixture was concentrated under reduced pressure, and the residue was crystallized from ethyl acetate to give the title compound (43 mg, yield 36%).
¹H NMR (DMSO-d₆) δ 1.22 (6H, s), 1.33 (3H, t, J = 6.9 Hz), 1.43 (6H, s), 2.05-2.20 (2H, m), 3.07 (2H, s), 4.37 (2H, q, J = 6.9 Hz), 6.84 (1H, s), 7.78 (1H, t, J = 7.8 Hz), 7.88 (1H, d, J = 7.8 Hz), 8.15 (1H, d, J = 1.2 Hz), 8.27 (1H, dd, J = 7.8, 1.2 Hz), 11.43 (1H, br s).

### Reference Example 44

### 1-Methylethyl 3-[3,4,8,9-tetrahydro-3,3,8,8-tetramethyl-6-(methylthio)furo[2,3-h]isoquinolin-1-yl]benzoate hydrochloride

From 7-bromo-2,3-dihydro-2,2-dimethyl-5-(2-methyl-1-propenyl)benzofuran, the title compound was obtained as free base by the method similar to that in Reference Example 37. This was dissolved in ethyl acetate, a solution of 4 M hydrogen chloride/ethyl acetate was added thereto, and the mixture was concentrated under reduced pressure, and the residue was crystallized from hexane to give the title compound. Yield 8.9%.
¹H NMR (DMSO-d₆) δ 1.26 (6H, s), 1.30 (6H, s), 1.36 (6H, d, J = 6.3 Hz), 2.17 (2H, s), 2.51 (3H, s),2.70 (2H, s), 5.20-5.33 (1H, m), 7.47 (1H, t, J = 7.7 Hz), 7.61 (1H, dt, J = 7.7, 1.5 Hz), 8.04 (1H, t, J = 1.5 Hz), 8.08 (1H, dt, J = 7.7, 1.5 Hz).

### Reference Example 45

### 3-[3,4,8,9-Tetrahydro-3,3,8,8-tetramethyl-6-(methylthio)furo[2,3-h]isoquinolin-1-yl]benzoic acid hydrochloride

From 1-methylethyl 3-[3,4,8,9-tetrahydro-3,3,8,8-tetramethyl-6-(methylthio)furo[2,3-h]isoquinolin-1-yl]benzoate, the title compound was obtained as free base by the method similar to that in Reference Example 30. This was dissolved in ethyl acetate, and a solution of 4 M hydrogen chloride/ethyl acetate was added thereto. The mixture was concentrated under reduced pressure, and the residue was crystallized from ethanol-ethyl acetate-diisopropyl ether to give the title compound. Yield 95%.
¹H NMR (DMSO-d₆) δ 1.23 (6H, s), 1.45 (6H, s), 2.10-2.25 (2H, m), 2.57 (3H, s), 3.17 (2H, s), 7.19 (1H, s), 7.77 (1H, t, J = 7.5 Hz), 7.87 (1H, d, J = 7.5 Hz), 8.19 (1H, s), 8.27 (1H, d, J = 7.5 Hz).

### Reference Example 46

### N-(2-Amino-1,1-dimethyl-2-oxoethyl)-3-[3,4,8,9-tetrahydro-3,3,8,8-tetramethyl-6-(methylthio)furo[2,3-h]isoquinolin-1-yl]benzamide

From 3-[3,4,8,9-tetrahydro-3,3,8,8-tetramethyl-6-(methylthio)furo[2,3-h]isoquinolin-1-yl]benzoic acid hydrochloride, the title compound was obtained by the method similar to that in Reference Example 31. Yield 71%.
¹H NMR (CDCl₃) δ 1.26 (6H, s), 1.30 (6H, s), 1.71 (6H, s), 2.17 (2H, s), 2.50 (3H, s), 2.70 (2H, s), 5.41 (1H, br s), 6.43 (1H, br s), 6.86 (1H, s), 6.97 (1H, s), 7.43-7.49 (2H, m), 7.85-7.89 (2H, m).

### Reference Example 47

### N-(2-Amino-1,1-dimethyl-2-oxoethyl)-3-[3,4,8,9-tetrahydro-3,3,8,8-tetramethyl-6-(methylsulfinyl)furo[2,3-h]isoquinolin-1-yl]benzamide

From N-(2-amino-1,1-dimethyl-2-oxoethyl)-3-[3,4,8,9-tetrahydro-3,3,8,8-tetramethyl-6-(methylthio)furo[2,3-h]isoquinolin-1-yl]benzamide, the title compound was obtained by the method similar to that in Reference Example 32. Yield 59%.
¹H NMR (CDCl₃) δ 1.20-1.43 (12H, m), 1.69 (6H, s), 2.19 (2H, s), 2.78 (2H, s), 2.83 (3H, s), 5.44 (1H, br s), 6.36 (1H, br s), 7.06 (1H, s), 7.48-7.57 (3H, m), 7.80-7.90 (2H, m).

### Reference Example 48

### 2-[3-(3,4,8,9-Tetrahydro-6-hydroxy-3,3,8,8-tetramethylfuro[2,3-h]isoquinolin-1-yl)phenyl]-5,5-dimethyl-1,5-dihydro-4H-imidazol-4-one

To a solution of N-(2-amino-1,1-dimethyl-2-oxoethyl)-3-(3,4,8,9-tetrahydro-6-methoxy-3,3,8,8-tetramethylfuro[2,3-h]isoquinolin-1-yl)benzamide (546 mg, 1.18 mmol) in 1,2-dichloroethane (3 ml) were added trichloroacetyl chloride (0.21 ml, 2.94 mmol) and aluminum chloride (392 mg, 2.94 mmol) under ice-cooling, and the mixture was stirred at room temperature for 60 hours. Trichloroacetyl chloride (0.20 ml, 2.83 mmol) and aluminum chloride (472 mg, 3.54 mmol) were added thereto under ice-cooling, and the mixture was stirred at room temperature for 87 hours. The reaction mixture was poured into ice water, neutralized with 5 M aqueous solution of sodium hydroxide, and the precipitate was taken by filtration. The filtrate was extracted with a mixed solution of tetrahydrofuran-ethyl acetate, the combined organic layer was washed with a saturated aqueous solution of sodium chloride, dried over sodium sulfate, and concentrated under reduced pressure. The residue was subjected to a basic silica gel column chromatography (ethyl acetate followed by ethyl acetate/methanol 10:1), and the obtained crystals were washed with diisopropyl ether to give the title compound (163 mg, yield 32%).
¹H NMR (CDCl₃+ DMSO-d₆ 5 drops) δ 1.24 (6H, s), 1.29 (6H, s), 1.80 (6H, s), 2.16 (2H, s), 2.63 (2H, s), 6.60 (1H, s), 7.45-7.55 (2H, m), 7.80 (1H, br s), 7.91-7.98 (2H, m).

### Reference Example 49

### N-(2-Amino-1,1-dimethyl-2-oxoethyl)-3-(3,4,8,9-tetrahydro-6-hydroxy-3,3,8,8-tetramethylfuro[2,3-h]isoquinolin-1-yl)benzamide

To a solution of N-(2-amino-1,1-dimethyl-2-oxoethyl)-3-(3,4,8,9-tetrahydro-6-methoxy-3,3,8,8-tetramethylfuro[2,3-h]isoquinolin-1-yl)benzamide (10.1 g, 21.8 mmol) in 1,2-dichloroethane (60 ml) were added trichloroacetyl chloride (1.55 ml, 21.8 mmol) and aluminum chloride (10.2 g, 76.3 mmol) under ice-cooling, and the mixture was stirred at room temperature for 60 hours. Trichloroacetyl chloride (1.55 ml, 21.8 mmol) and aluminum chloride (4.36 g, 32.7 mmol) were added thereto under ice-cooling, and the mixture was stirred for 15 hours at room temperature. The reaction mixture was combined with ice water, ethyl acetate and tetrahydrofuran, and neutralized with 5 M aqueous solution of sodium hydroxide. The aqueous layer was separated, and extracted with a mixed solution of tetrahydrofuran-ethyl acetate. The combined organic layer was washed with a saturated aqueous solution of sodium chloride, dried over sodium sulfate, and concentrated under reduced pressure. The residue was subjected to a basic silica gel column chromatography (ethyl acetate/methanol 10:1), and the obtained crystals were washed with diisopropyl ether to give the title compound (8.17 g, yield 83%).
¹H NMR (CDCl₃) δ 1.24 (6H, s), 1.28 (6H, s), 1.69 (6H, s), 2.15 (2H, s), 2.63 (2H, s), 5.97 (1H, br s), 6.59 (1H, s), 6.83 (1H, br s), 7.44-7.52 (2H, m),7.60 (1H, s), 7.87-7.91 (2H, m).

### Reference Example 50

### N-(2-Amino-1,1-dimethyl-2-oxoethyl)-3-[6-(2-fluoroethoxy)-3,4,8,9-tetrahydro-3,3,8,8-tetramethylfuro[2,3-h]isoquinolin-1-yl]benzamide

To a solution of N-(2-amino-1,1-dimethyl-2-oxoethyl)-3-(3,4,8,9-tetrahydro-6-hydroxy-3,3,8,8-tetramethylfuro[2,3-h]isoquinolin-1-yl)benzamide hydrochloride (148 mg, 0.305 mmol) in N,N-dimethylformamide (2 ml) were added potassium carbonate (89 mg, 0.641 mmol) and 1-bromo-2-fluoroethane (0.025 ml, 0.335 mmol), and the mixture was stirred at room temperature for 1 hour and at 60°C for 2 hours. After cooling, the reaction mixture was poured into ice water, and extracted twice with ethyl acetate. The combined organic layer was washed with an aqueous solution of sodium chloride and a saturated aqueous solution of sodium chloride, dried over sodium sulfate, and concentrated under reduced pressure. The residue was subjected to a basic silica gel column chromatography (ethyl acetate), and the obtained crystals were washed with diisopropyl ether to give the title compound (64 mg, yield 42%).
¹H NMR (CDCl₃) δ 1.26 (6H, s), 1.30 (6H, s), 1.72 (6H, s), 2.17 (2H, s), 2.69 (2H, s), 4.39 (2H, dt, J = 27.8, 4.4 Hz), 4.78 (2H, dt, J = 46.4, 4.4 Hz), 5.30 (1H, br s), 6.43 (1H, br s), 6.67 (1H, s), 6.93 (1H, br s), 7.46-7.50 (2H, m), 7.85-7.90 (2H, m).

### Reference Example 51

### N-(2-Amino-1,1-dimethyl-2-oxoethyl)-3-[3,4,8,9-tetrahydro-3,3,8,8-tetramethyl-6-(methylsulfonyl)furo[2,3-h]isoquinolin-1-yl]benzamide

From N-(2-amino-1,1-dimethyl-2-oxoethyl)-3-[3,4,8,9-tetrahydro-3,3,8,8-tetramethyl-6-(methylsulfinyl)furo[2,3-h]isoquinolin-1-yl]benzamide, the title compound was obtained by the method similar to that in Reference Example 33. Yield 65%.
¹H NMR (CDCl₃) δ 1.27 (6H, s), 1.36 (6H, s), 1.74 (6H, s), 2.23 (2H, s), 2.75 (2H, s), 3.21 (3H, s), 5.43 (1H, br s), 6.30 (1H, br s), 7.05 (1H, s), 7.48-7.53 (2H, m), 7.57 (1H, s), 7.85-7.92 (2H, m).

### Reference Example 52

### N-(2,Amino-1,1-dimethyl-2-oxoethyl)-3-[3,4,8,9-tetrahydro-3,3,8,8-tetramethyl-6-(2,2,2-trifluoroethoxy)furo[2,3-h]isoquinolin-1-yl]benzamide

To a solution of N-(2-amino-1,1-dimethyl-2-oxoethyl)-3-(3,4,8,9-tetrahydro-6-hydroxy-3,3,8,8-tetramethylfuro[2,3-h]isoquinolin-1-yl)benzamide (359 mg, 0.799 mmol) in N,N-dimethylformamide (3 ml) were added potassium carbonate (121 mg, 0.878 mmol) and 1,1,1-trifluoro-2-iodoethane (0.087 ml, 0.878 mmol), and the mixture was stirred at room temperature for 2 hours and at 50°C for 4 hours. Potassium carbonate (121 mg, 0.878 mmol) and 1,1,1-trifluoro-2-iodoethane (0.17 ml, 1.76 mmol) were added thereto, and the mixture was stirred at room temperature for 60 hours and at 50°C for 4 hours. 1,1,1-trifluoro-2-iodoethane (0.087 ml, 0.878 mmol) was added thereto, and the mixture was stirred at 50°C for 4 hours. 1,1,1-trifluoro-2-iodoethane (0.17 ml, 1.76 mmol) was added thereto, and the mixture was stirred at 50°C for 18 hours. After cooling, the reaction mixture was combined with ice water, and extracted twice with ethyl acetate. The combined organic layer was washed with a saturated aqueous solution of sodium chloride, dried over sodium sulfate, and concentrated under reduced pressure. The residue was subjected to a basic silica gel column chromatography (hexane/ethyl acetate 1:2 followed by ethyl acetate), and crystallized with diethyl ether-diisopropyl ether to give the title compound (102 mg, yield 24%).
¹H NMR (CDCl₃) δ 1.25 (6H, s), 1.30 (6H, s), 1. 72 (6H, s), 2.17 (2H, s), 2.67 (2H, s), 4.54 (2H, q, J = 8.4 Hz), 5.38 (1H, br s), 6.43 (1H, br s), 6.71 (1H, s), 6.97 (1H, s), 7.44-7.49 (2H, m), 7.84-7.88 (2H, m).

### Reference Example 53

### 1-(3-Bromophenyl)-3,4,8,9-tetrahydro-N,3,3,8,8-pentamethylfuro[2,3-h]isoquinolin-6-amine dihydrochloride

To a solution of [1-(3-bromophenyl)-3,4,8,9-tetrahydro-3,3,8,8-tetramethylfuro[2,3-h]isoquinolin-6-yl] trifluoromethanesulfonate (183 mg, 0.345 mmol) in 40% methylamine/methanol was added a solution (3 ml) of ammonium chloride (37 mg, 0.690 mmol), and the mixture was stirred under nitrogen atmosphere in a sealed tube at 150°C for 18 hours. After cooling, the solvent was distilled off under reduced pressure, and the residue was dissolved by adding water and ethyl acetate. The aqueous layer was separated, and extracted with ethyl acetate.
The combined organic layer was washed with a saturated aqueous solution of sodium chloride, dried over sodium sulfate, and concentrated under reduced pressure. The residue was subjected to a basic silica gel column chromatography (hexane/ethyl acetate, 10:1) to give the title compound as free base. This was dissolved in ethyl acetate, a solution of 4 M hydrogen chloride/ethyl acetate was added thereto, and the mixture was concentrated under reduced pressure to give the title compound (34 mg, yield 20%). Amorphous.
¹H NMR (DMSO-d₆) δ 1.27 (6H, s), 1.37 (3H, s), 1. 42 (3H, s), 2.08 (1H, d, J = 15.5 Hz), 2.20 (1H, d, J = 15.5 Hz), 2.88 (3H, d, J = 4.8 Hz), 2.98 (1H, d, J = 16.2 Hz), 3.18 (1H, d, J = 16.2 Hz), 6.53 (1H, s), 7.15-7.25 (1H, m), 7.45-7.60 (2H, m), 7.84 (1H, s), 7.85-7.95 (1H, m), 11.44 (1H, s).

### Reference Example 54

### 2-[3-(6-Ethoxy-3,4,8,9-tetrahydro-3,3,8,8-tetramethylfuro[2,3-h]isoquinolin-1-yl)phenyl]-5,5-dimethyl-1,5-dihydro-4H-imidazol-4-one

From 2-[3-(3,4,8,9-tetrahydro-6-hydroxy-3,3,8,8-tetramethylfuro[2,3-h]isoquinolin-1-yl)phenyl]-5,5-dimethyl-1,5-dihydro-4H-imidazol-4-one and iodoethane, the title compound was obtained by the method similar to that in Reference Example 50. Yield 83%.
¹H NMR (CDCl₃) δ 1.25 (6H, s), 1.31 (6H, s), 1.47 (3H, t, J = 7.0 Hz), 1.80 (6H, s), 2.16 (2H, s), 2.68 (2H, s), 4.19 (2H, q, J = 7.0 Hz), 6.60-6.62 (2H, m), 7.48-7.50 (2H, m), 7.85 (1H, s), 7.92-7.96 (1H, m).

### Reference Example 55

### 5-(Bromomethyl)-1-(3-bromophenyl)-3,4,8,9-tetrahydro-6-methoxy-3,3,8,8-tetramethylfuro[2,3-h]isoquinoline

To a suspension of 1-(3-bromophenyl)-3,4,8,9-tetrahydro-6-methoxy-3,3,8,8-tetramethyl-1-phenylfuro[2,3-h]isoquinoline (1.47 g, 3.55 mmol), paraformaldehyde (94%) (170 mg, 5.32 mmol) and sodium bromide (603 mg, 5.86 mmol) in acetic acid (1.02 ml, 17.8 mmol) was added conc. sulfuric acid (0.57 ml, 10.7 mmol), and the mixture was stirred for 20 hours at 100°C. After cooling, the reaction mixture was combined with ice water, neutralized with conc. ammonia water, and extracted twice with ethyl acetate. The combined organic layer was washed with a saturated aqueous solution of sodium chloride, dried over sodium sulfate, filtered, and concentrated under reduced pressure. The residue was subjected to a silica gel column chromatography (hexane/ethyl acetate, 10:1) to give the title compound (673 mg, yield 37%). Amorphous.
¹H NMR (CDCl₃) δ 1.27 (6H, s), 1.32 (6H, s), 2.19 (2H, s), 2.70 (2H, s), 4.05 (3H, s), 4.63 (2H, s), 7.26-7.35 (2H, m), 7.50-7.56 (2H, m).

### Reference Example 56

### 1-(3-Bromophenyl)-3,4,8,9-tetrahydro-6-methoxy-3,3,8,8-tetramethylfuro[2,3-h]isoquinoline-5-acetonitrile

To a solution of 5-(bromomethyl)-1-(3-bromophenyl)-3,4,8,9-tetrahydro-6-methoxy-3,3,8,8-tetramethylfuro[2,3-h]isoquinoline (670 mg, 1.32 mmol) in N,N-dimethylformamide (7 ml) was added a solution of sodium cyanate (65 mg, 1.32 mmol) in water (2 ml), and the mixture was stirred at room temperature for 2 hours. The reaction mixture was combined with water, and extracted twice with ethyl acetate. The combined organic layer was washed with water and a saturated aqueous solution of sodium chloride, dried over sodium sulfate, filtered, and concentrated under reduced pressure. The residue was subjected to a basic silica gel column chromatography (hexane/ethyl acetate, 10:1), and crystallized from hexane to give the title compound (131 mg, yield 49%).
¹H NMR (CDCl₃) δ 1.28 (6H, s), 1.32 (6H, s), 2.20 (2H, s), 2.68 (2H, s), 3.73 (2H, s), 4.05 (3H, s), 7.25-7.36 (2H, m), 7.50-7.57 (2H, m).

### Reference Example 57

### 1-(3-Aminophenyl)-3,4,8,9-tetrahydro-6-methoxy-3,3,8,8-tetramethylfuro[2,3-h]isoquinoline-5-acetonitrile

To a solution of 1-(3-bromophenyl)-3,4,8,9-tetrahydro-6-methoxy-3,3,8,8-tetramethylfuro[2,3-h]isoquinoline-5-acetonitrile (170 mg, 0.375 mmol) in toluene (1.5 ml) were added benzophenoneimine (0.075 ml, 0.450 mmol) 2,2'-bis(diphenylphosphino)-1,1'-binaphthyl (17.5 mg, 0.0281 mmol), sodium tert-butoxide (50.4 mg, 0.525 mmol) and tris(dibenzylideneacetone)dipalladium(0) (8.6 mg, 0.00938 mmol), and the mixture was stirred for 15 hours under nitrogen atmosphere at 80°C. After cooling, the reaction mixture was combined with ice water, and extracted twice with ethyl acetate. The combined organic layer was washed with a saturated aqueous solution of sodium chloride, dried over sodium sulfate, filtered, and concentrated under reduced pressure. The residue was dissolved in tetrahydrofuran (3 ml), 1 M hydrochloric acid (1 ml) was added thereto, and the mixture was stirred for 30 minutes at room temperature. The reaction mixture was combined with ice water, and extracted twice with ethyl acetate. The combined organic layer was washed with a saturated aqueous solution of sodium chloride, dried over sodium sulfate, filtered, and concentrated under reduced pressure. The residue was subjected to a basic silica gel column chromatography (hexane/ethyl acetate 2:1) to give the title compound (94 mg, yield 64%).
Oily matter.
¹H NMR (CDCl₃) δ 1.26 (6H, s), 1.31 (6H, s), 2.30 (2H, s), 2.66 (2H, s), 3.73 (2H, s), 4.02 (3H, s), 6.67 (3H, m), 7.10-7.18 (1H, m).

### Reference Example 58

### N-(2-Amino-1,1-dimethyl-2-oxoethyl)-3-(3,4,8,9-tetrahydro-3,3,8,8-tetramethyl-6-propoxyfuro[2,3-h]isoquinolin-1-yl)benzamide

With 1-iodopropane, the title compound was obtained by the method similar to that in Reference Example 50. Yield 62%.
¹H NMR (CDCl₃) δ 1.04 (3H, t, J = 7.5 Hz), 1.26 (6H, s), 1.30 (6H, s), 1.75-1.91 (2H, m), 2.17 (2H, s), 2.69 (2H, s), 4.07 (2H, q, J = 6.9 Hz), 5.40 (1H, br s), 5.47 (1H, br s), 6.62 (1H, s), 7.02 (1H, s), 7.45-7.50 (2H, m), 7.86-7.89 (2H, m).

### Reference Example 59

### N-(2-Amino-1,1-dimethyl-2-oxoethyl)-3-[6-(2-amino-2-oxoethoxy)-3,4,8,9-tetrahydro-3,3,8,8-tetramethylfuro[2,3-h]isoquinolin-1-yl]benzamide hydrochloride

With 2-iodoacetamide, the title compound was obtained as free base by the method similar to that in Reference Example 50. This was dissolved in ethyl acetate, a solution of 4 M hydrogen chloride/ethyl acetate was added thereto, and the mixture was concentrated under reduced pressure. The resultant crystals were washed with ethyl acetate to give the title compound was obtained. Yield 68%.
¹H NMR (DMSO-d₆) δ 1.25 (6H, s), 1.48 (12H, s), 2.20-2.30 (2H, m), 3.05-3.20 (2H, m), 4.70 (2H, s), 6.91 (1H, s), 6.91 (1H, s), 6.94 (1H, s), 7.20 (1H, s), 7.41 (1H, s), 7.54 (1H, s), 7.70-7.74 (2H, m), 8.15-8.30 (2H, m), 8.47 (1H, s).

### Reference Example 60

### 3-[5-(Cyanomethyl)-3,4,8,9-tetrahydro-6-hydroxy-3,3,8,8-tetramethylfuro[2,3-h]isoquinolin-1-yl]-N-methylbenzamide

To a solution of 3-[5-(cyanomethyl)-3,4,8,9-tetrahydro-6-methoxy-3,3,8,8-tetramethylfuro[2,3-h]isoquinolin-1-yl]-N-methylbenzamide hydrochloride (529 mg, 1.13 mmol) in 1,2-dichloroethane (3 ml) was added aluminum chloride (452 mg, 3.39 mmol) under ice-cooling, and the mixture was stirred at room temperature for 4.5 hours. Aluminum chloride (301 mg, 2.26 mmol) was further added thereto, and the mixture was stirred at room temperature for 3 hours. The reaction mixture was combined with ice water, ethyl acetate and tetrahydrofuran, and alkalified with 5 M aqueous solution of sodium hydroxide, and the precipitate was taken by filtration. The aqueous layer was separated, and extracted twice with a mixed solution of ethyl acetate-tetrahydrofuran. The combined organic layer was washed with a saturated aqueous solution of sodium chloride, dried over sodium sulfate, and concentrated under reduced pressure. The resultant crystals were washed with diisopropyl ether to give the title compound (280 mg, yield 59%).
¹H NMR (CDCl₃) δ 1.25 (6H, s), 1.35 (6H, s), 2.14 (2H, s), 2.76 (2H, s), 2.97 (3H, d, J = 4.8 Hz), 3.72 (2H, s), 6.80-6.85 (1H, m), 7.47-7.50 (2H, m), 7.85-7.93 (2H, m).

### Reference Example 61

### 3-[5-(Cyanomethyl)-6-ethoxy-3,4,8,9-tetrahydro-3,3,8,8-tetramethylfuro[2,3-h]isoquinolin-1-yl]-N-methylbenzamide

From 3-[5-(cyanomethyl)-3,4,8,9-tetrahydro-6-hydroxy-3,3,8,8-tetramethylfuro[2,3-h]isoquinolin-1-yl]-N-methylbenzamide and iodoethane, the title compound was obtained by the method similar to that in Reference Example 50. Yield 79%.
¹H NMR (CDCl₃) δ 1.26 (12H, s), 1.39 (3H, t, J = 7.0 Hz), 2.10 (2H, s), 2.68 (2H, s), 3.00 (3H, d, J = 4.8 Hz), 3.76 (2H, s), 4.34 (2H, q, J = 7.0 Hz), 6.40-6.53 (1H, br), 7.44-7.50 (2H, m), 7.75 (1H, s), 7.83-7.89 (1H, m).

### Reference Example 62

### N-(2-Amino-1,1-dimethyl-2-oxoethyl)-3-[3,4,8,9-tetrahydro-6-(2-hydroxyethoxy)-3,3,8,8-tetramethylfuro[2,3-h]isoquinolin-1-yl]benzamide

To a solution of N-(2-amino-1,1-dimethyl-2-oxoethyl)-3-(3,4,8,9-tetrahydro-6-hydroxy-3,3,8,8-tetramethylfuro[2,3-h]isoquinolin-1-yl)benzamide (438 mg, 0.974 mmol) in N,N-dimethylformamide (4 ml) were added potassium carbonate (148 mg, 1.07 mmol), potassium iodide (16 mg, 0.0974 mmol) and 2-bromoethanol (0.076 ml, 1.07 mmol), and the mixture was stirred at 60°C for 5 hours. 2-bromoethanol (0.035 ml, 0.487 mmol) was further added thereto, and the mixture was stirred for 15 hours at 60°C. After cooling, the reaction mixture was combined with ice water, and extracted twice with ethyl acetate. The combined organic layer was washed with an aqueous solution of sodium chloride and a saturated aqueous solution of sodium chloride, dried over sodium sulfate, and concentrated under reduced pressure. The residue was subjected to a basic silica gel column chromatography (hexane/ethyl acetate, 50:1 followed by 20:1), and crystallized from ethyl acetate to give the title compound (209 mg, yield 44%).
¹H NMR (CDCl₃) δ 1.25 (6H, s), 1.30 (6H, s), 1.71 (6H, s), 2.18 (2H, s), 2.68 (2H, s), 3.95 (2H, t, J = 4.4 Hz), 4.21 (2H, t, J = 4.4 Hz), 5.43 (1H, br s), 6.45 (1H, br s), 6.66 (1H, s), 7.01 (1H, s), 7.42-7.49 (2H, m), 7.84-7.90 (2H, m).

### Reference Example 63

### N-(2-Amino-1,1-dimethyl-2-oxoethyl)-3-[3,4,8,9-tetrahydro-3,3,8,8-tetramethyl-6-(2-propynyloxy)furo[2,3-h]isoquinolin-1-yl]benzamide

With 3-bromo-1-propyne, the title compound was obtained by the method similar to that in Reference Example 50. Yield 76%.
¹H NMR (CDCl₃) δ 1.25 (6H, s), 1.30 (6H, s), 1.71 (6H, s), 2.18 (2H, s), 2.59 (1H, t, J = 2.2 Hz), 2.70 (2H, s), 4.83 (2H, d, J = 2.2 Hz), 5.81 (1H, br s), 6.78 (1H, br s), 7.41-7.49 (2H, m), 7.86-7.91 (2H, m).

### Reference Example 64

### N-(2-Amino-1,1-dimethyl-2-oxoethyl)-3-[6-(difluoromethoxy)-3,4,8,9-tetrahydro-3,3,8,8-tetramethylfuro[2,3-h]isoquinolin-1-yl]benzamide

To a suspension of N-(2-amino-1,1-dimethyl-2-oxoethyl)-3-(3,4,8,9-tetrahydro-6-hydroxy-3,3,8,8-tetramethylfuro[2,3-h]isoquinolin-1-yl)benzamide (451 mg, 1.00 mmol) in 1,4-dioxane (4 ml) were added chlorobenzyltriethyl ammonium (11 mg, 0.050 mmol) and a solution of sodium hydroxide (120 mg, 3.01 mmol) in water (0.12 ml). Chlorodifluoromethane was bubbled under ice-cooling for 10 minutes and the mixture was stirred at room temperature for 5 hours. Chlorodifluoromethane was bubbled again, and the mixture was stirred for 15 hours at room temperature. The reaction mixture was combined with ice water, and extracted twice with ethyl acetate. The combined organic layer was washed with a saturated aqueous solution of sodium chloride, dried over sodium sulfate, and concentrated under reduced pressure. The residue was subjected to a silica gel column chromatography (ethyl acetate/methanol, 50:1 followed by 10:1), and crystallized from diethyl ether-hexane to give the title compound (276 mg, yield 55%).
¹H NMR (CDCl₃) δ 1.26 (6H, s), 1.30 (6H, s), 1.72 (6H, s), 2.20 (2H, s), 2.69 (2H, s), 5.41 (1H, br s), 6.40 (1H, br s), 6.57 (1H, d, J = 74.2 Hz), 6.87 (1H, s), 7.01 (1H, s), 7.47-7.50 (2H, m), 7.85-7.90 (2H, m).

### Reference Example 65

### Methyl 3-[5-(cyanomethyl)-3,4,8,9-tetrahydro-6-hydroxy-3,3,8,8-tetramethylfuro[2,3-h]isoquinolin-1-yl]benzoate

From methyl 3-[5-(cyanomethyl)-3,4,8,9-tetrahydro-6-methoxy-3,3,8,8-tetramethylfuro[2,3-h]isoquinolin-1-yl]benzoate, the title compound was obtained by the method similar to that in Reference Example 60. Yield 40%.
¹H NMR (CDCl₃) δ 1.27 (12H, s), 2.16 (2H, s), 2.72 (2H, s), 3.76 (2H, s), 3.93 (3H, s), 5,75 (1H, br s), 7.47-7.51 (1H, m), 7.58-7.61 (1H, m),8.05 (1H, s), 8.15-8.12 (1H, m).

### Reference Example 66

### Methyl 3-[5-(cyanomethyl)-6-ethoxy-3,4,8,9-tetrahydro-3,3,8,8-tetramethylfuro[2,3-h]isoquinolin-1-yl]benzoate

From methyl 3-[5-(cyanomethyl)-3,4,8,9-tetrahydro-6-hydroxy-3,3,8,8-tetramethylfuro[2,3-h]isoquinolin-1-yl]benzoate and iodoethane, the title compound was obtained by the method similar to that in Reference Example 50. Yield 64%.
¹H NMR (CDCl₃) δ 1.27 (6H, s), 1.28 (6H, s), 1.39 (3H, t, J = 7.2 Hz), 2.11 (2H, s), 2.69 (2H, s), 3.76 (2H, s), 3.93 (3H, s), 4.34 (2H, q, J = 7.2 Hz), 7.49 (1H, t, J = 7.5 Hz), 7.59-7.62 (1H, m), 8.06-8.11 (2H, m).

### Reference Example 67

### N-(2-Amino-1,1-dimethyl-2-oxoethyl)-3-[5-(cyanomethyl)-6-ethoxy-3,4,8,9-tetrahydro-3,3,8,8-tetramethylfuro[2,3-h]isoquinolin-1-yl]benzamide

From methyl 3-[5-(cyanomethyl)-6-ethoxy-3,4,8,9-tetrahydro-3,3,8,8-tetramethylfuro[2,3-h]isoquinolin-1-yl]benzoate, 3-[5-(cyanomethyl)-6-ethoxy-3,4,8,9-tetrahydro-3,3,8,8-tetramethylfuro[2,3-h]isoquinolin-1-yl]benzoic acid was obtained by the method similar to that in Reference Example 30. From the resultant benzoic acid derivative, the title compound was obtained by the method similar to that in Reference Example 31. Yield 72%.
¹H NMR (CDCl₃) δ 1.32-1.50 (15H, m), 1.73 (6H, s), 2.28 (2H, s), 2.92 (2H, s), 3.77 (3H, s), 4.52 (2H, q, J = 7.0 Hz), 5.35 (1H, br s), 6.50 (1H, br s), 7.41 (1H, d, J = 8.0 Hz), 7.55 (1H, t, J = 8.0 Hz), 8.09 (1H, d, J = 8.0 Hz), 8.34 (1H, br s), 8.44 (1H, s).

### Reference Example 68

### 4-(6-Ethoxy-3,4,8,9-tetrahydro-3,3,8,8-tetramethylfuro[2,3-h]isoquinolin-1-yl)-N-methylbenzamide

From 7-ethoxy-2,3-dihydro-2,2-dimethyl-a-(1-methylethyl)-5-benzofuranmethanol and 4-cyano-N-methylbenzamide, the title compound was obtained by the method similar to that in Reference Example 34. Yield 50%.
¹H NMR (CDCl₃) δ 1.25 (6H, s), 1.29 (6H, s), 1.46 (3H, t, J = 7.0 Hz), 2.16 (2H, s), 2.67 (2H, s), 3.04 (3H, d, J = 5.0 Hz), 4.18 (2H, q, J = 7.0 Hz), 6.29 (1H, t, J = 5.0 Hz), 6.61 (1H, s), 7.45 (2H, dd, J = 6.8, 1.8 Hz), 7.78 (2H, dd, J = 6.8, 1.8 Hz).

### Reference Example 69

### 4-(6-Ethoxy-3,4,8,9-tetrahydro-3,3,8,8-tetramethylfuro[2,3-h]isoquinolin-1-yl)benzenamine dihydrochloride

From 7-ethoxy-2,3-dihydro-2,2-dimethyl-a-(1-methylethyl)-5-benzofuranmethanol and 4-aminobenzonitrile, the title compound was obtained as free base by the method similar to that in Reference Example 34. This was dissolved in ethyl acetate, and a solution of 4 M hydrogen chloride/ethyl acetate was added thereto. The mixture was concentrated under reduced pressure, and the residue was crystallized from ethanol-diisopropyl ether to give the title compound. Yield 31%.
¹H NMR (DMSO-d₆) δ 1.29 (6H, s), 1.36 (6H, s), 1.36 (3H, t, J = 7.0 Hz), 2.50 (2H, s), 3.04 (2H, s), 4.21 (2H, q, J = 7.0 Hz), 6.76 (2H, d, J = 8.4 Hz), 7.02 (1H, s), 7.08 (2H, br s), 7.36 (2H, d, J = 8.4 Hz), 11.66 (1H, br s).

### Reference Example 70

### N-[4-(6-Ethoxy-3,4,8,9-tetrahydro-3,3,8,8-tetramethylfuro[2,3-h]isoquinolin-1-yl)phenyl]acetamide

To a solution of 4-(6-ethoxy-3,4,8,9-tetrahydro-3,3,8,8-tetramethylfuro[2,3-h]isoquinolin-1-yl)benzenamine dihydrochloride (323 mg, 0.738 mmol) in tetrahydrofuran (3 ml) were added triethylamine (0.34 ml, 2.44 mmol) and acetyl chloride (0.058 ml, 0.812 mmol) under ice-cooling, and the mixture was stirred at room temperature for 2 hours. The reaction mixture was combined with ice water, and extracted twice with ethyl acetate. The combined organic layer was washed with water and a saturated aqueous solution of sodium chloride, dried over sodium sulfate, filtered, and concentrated under reduced pressure. The residue was subjected to a basic silica gel column chromatography (hexane/ethyl acetate, 2:1 followed by 1:1), and crystallized from diethyl ether-hexane to give the title compound (191 mg, yield 64%).
¹H NMR (CDCl₃) δ 1.22 (6H, s), 1.31 (6H, s), 1.45 (3H, t, J = 7.0 Hz), 2.18 (3H, s), 2.25 (2H, s), 2.65 (2H, s), 4.17 (2H, q, J = 7.0 Hz), 6.58 (1H, s), 7.34 (2H, d, J = 8.0 Hz), 7.40 (1H, s), 7.52 (2H, d, J = 8.0 Hz).

### Reference Example 71

### 3,4,8,9-Tetrahydro-N,3,8,8-tetramethyl-6-(methylamino)-1-phenylfuro[2,3-h]isoquinoline-3-methanamine

A mixture of 3-(bromomethyl)-6-ethoxy-3,4,8,9-tetrahydro-3,8,8-trimethyl-1-phenylfuro[2,3-h]isoquinoline hydrochloride (0.50 g, 1.08 mmol) and 40% methylamine/methanol a solution of (4 ml) was stirred for 15 hours at 160°C under nitrogen atmosphere in a sealed tube. The reaction solution was concentrated under reduced pressure, and the residue was alkalified with an aqueous solution of sodium hydrogen carbonate, and extracted with ethyl acetate. The extracts were washed with water, and concentrated under reduced pressure. The residue was subjected to a silica gel column chromatography (from ethyl acetate/methanol 19:1 to ethyl acetate/methanol/triethylamine 92:5:3), then purified with a basic silica gel column chromatography (hexane/ethyl acetate 3:1), and recrystallized from ethyl acetate-diisopropyl ether to give the title compound (0.18 g, yield 46%).
Melting point: 126 to 128°C.
¹H NMR (CDCl₃) δ 1.08 (3H, s), 1.22 (3H, s), 1.28 (3H, s), 2.10 (1H, d, J = 15.8 Hz), 2.19 (1H, d, J = 15.8 Hz), 2.46 (3H, s), 2.47 (1H, d, J = 15.2 Hz), 2.67 (1H, d, J = 10.8 Hz), 2.81 (1H, d, J = 10.8 Hz), 2.91 (3H, d, J = 5.0 Hz), 3.05 (2H, d, J = 15.2 Hz), 3.98 (1H, br d, J = 5.0 Hz), 6.31 (1H, s), 7.37 (5H, s).

### Reference Example 72

### 2-[(6-Ethoxy-3,4,8,9-tetrahydro-3,8,8-trimethyl-1-phenylfuro[2,3-h]isoquinolin-3-yl)methyl]-1H-isoindol-1,3(2H)-dione

A solution of 3-(bromomethyl)-6-ethoxy-3,4,8,9-tetrahydro-3,8,8-trimethyl-1-phenylfuro[2,3-h]isoquinoline hydrochloride (0.80 g, 1.72 mmol) in N,N-dimethylformamide (15 ml) was cooled with ice, and to this solution were added sodium hydride (66% dispersion in oil) (62.6 mg, 1.72 mmol) and potassium phthalimide (0.414 g, 2.24 mmol). The mixture was stirred under nitrogen atmosphere at 170°C for 3 hours and cooled to room temperature.
The reaction solution was combined with water, and extracted with ethyl acetate. The extracts were washed with water, and concentrated under reduced pressure. The residue was purified with a silica gel column chromatography (from hexane/ethyl acetate to 3:2 ethyl acetate), and recrystallized from ethyl acetate-hexane to give the title compound (0.53g, yield 62%).
Melting point: 187 to 189°C.
¹H NMR (CDCl₃) δ 1.18 (3H, s), 1.21 (3H, s), 1.37 (3H, s), 1.44 (3H, t, J = 7.0 Hz), 2.04 (1H, d, J = 16.4 Hz), 2.11 (1H, d, J = 16.4 Hz), 2.79 (1H, d, J = 15.8 Hz), 3.00 (1H, d, J = 15.8 Hz), 3.84 (1H, d, J = 13.6 Hz), 3.95 (1H, d, J = 13.6 Hz), 4.13 (2H, q, J = 7.0 Hz), 6.53 (1H, s), 7.36-7.44 (5H, m), 7.63-7.78 (4H, m).

### Reference Example 73

### 6-Ethoxy-3,4,8,9-tetrahydro-3,8,8-trimethyl-1-phenylfuro[2,3-h]isoquinoline-3-acetonitrile

To a solution of 3-(bromomethyl)-6-ethoxy-3,4,8,9-tetrahydro-3,8,8-trimethyl-1-phenylfuro[2,3-h]isoquinoline (3.00 g, 7.00 mmol) in dimethylsulfoxide (24 ml) was added sodium cyanate (0.378 g, 7.70 mmol), and the mixture was stirred for 15 hours under nitrogen atmosphere at 80°C. The reaction solution was combined with water, and extracted with ethyl acetate. The extracts were washed with water, and concentrated under reduced pressure. The residue was purified with a silica gel column chromatography (from hexane/ethyl acetate 4:1 to 7:3) to give the title compound (0.51 g, yield 19%).
Amorphous.
¹H NMR (CDCl₃) δ 1.30 (3H, s), 1.33 (3H, s), 1.36 (3H, s), 1.48 (3H, t, J = 6.9 Hz), 2.21 (2H, s), 2.58 (1H, d, J = 16.5 Hz), 2.71 (1H, d, J = 16.5 Hz), 2.81 (1H, d, J = 15.6 Hz), 2.91 (1H, d, J = 15.6 Hz), 4.20 (2H, q, J = 6.9 Hz), 6.66 (1H, s), 7.40 (5H, s).

### Reference Example 74

### 6-Ethoxy-3,4,8,9-tetrahydro-3,8,8-trimethyl-1-phenylfuro[2,3-h]isoquinolin-3-acetamide

To a solution of ice-cooled 6-ethoxy-3,4,8,9-tetrahydro-3,8,8-trimethyl-1-phenylfuro[2,3-h]isoquinoline-3-acetonitrile (0.41 g, 1.09 mmol) in methanol (5 ml) were added 1 M aqueous solution of sodium hydroxide (1.64 ml, 1.64 mmol) and 30% hydrogen peroxide water (0.186g, 1.64 mmol), and the mixture was stirred at room temperature for 24 hours. The reaction solution was combined with water, and extracted with ethyl acetate. The extracts were washed with water, and concentrated under reduced pressure. The residue was purified with a silica gel column chromatography (ethyl acetate/methanol 19:1) to give the title compound (0.31 g, yield 72%).
Amorphous.
¹H NMR (CDCl₃) δ 1.13 (3H, s), 1.28 (3H, s), 1.36 (3H, s), 1.47 (3H, t, J = 7.0 Hz), 2.15 (1H, d, J = 16.2 Hz), 2.31 (1H, d, J = 16.2 Hz), 2.53 (1H, d, J = 14.4 Hz), 2.56 (1H, d, J = 15.4 Hz), 2.68 (1H, d, J = 14.4 Hz), 2.88 (1H, d, J = 15.4 Hz), 4.19 (2H, q, J = 7.0 Hz), 5.48-5.50 (1H, m), 6.63 (1H, s), 7.41 (5H, s), 7.90 (1H, br d, J = 4.0 Hz).

### Reference Example 75

### 6-Ethoxy-3,4,8,9-tetrahydro-3,8,8-trimethyl-1-phenylfuro[2,3-h]isoquinoline-3-methanamine

To a suspension of 2-[(6-ethoxy-3,4,8,9-tetrahydro-3,8,8-trimethyl-1-phenylfuro[2,3-h]isoquinolin-3-yl)methyl]-1H-isoindol-1,3(2H)-dione (2.44 g, 4.93 mmol) in ethanol (20 ml) was added hydrazine monohydrate (0.550 ml, 11.3 mmol), and the mixture was heated under reflux for 3 hours. The reaction solution was cooled to room temperature, alkalified with 1 M aqueous solution of sodium hydroxide, and extracted with ethyl acetate. The extracts were washed with 1 M aqueous solution of sodium hydroxide and water, and concentrated under reduced pressure. The residue was purified with a basic silica gel column chromatography (ethyl acetate/methanol 97:3) to give the title compound (1.71 g, yield 95%).
Amorphous.
¹H NMR (CDCl₃) δ 1.06 (3H, s), 1.28 (3H, s), 1.33 (3H, s), 1.46 (3H, t, J = 7.0 Hz), 2.14 (1H, d, J = 16.2 Hz), 2.24 (1H, d, J = 16.2 Hz), 2.47 (1H, d, J = 15.6 Hz), 2.79 (1H, d, J = 12.8 Hz), 2.88 (1H, d, J = 12.8 Hz), 2.91 (1H, d, J = 15.6 Hz), 4.19 (2H, q, J = 7.0 Hz), 6.62 (1H, s), 7.39 (5H, s).

### Reference Example 76

### N-[(6-Ethoxy-3,4,8,9-tetrahydro-3,8,8-trimethyl-1-phenylfuro[2,3-h]isoquinolin-3-yl)methyl]benzamide

To a solution of ice-cooled 6-ethoxy-3,4,8,9-tetrahydro-3,8,8-trimethyl-1-phenylfuro[2,3-h]isoquinoline-3-methanamine (0.20 g, 0.55 mmol) in tetrahydrofuran (3 ml) was added 1 M aqueous solution of sodium hydroxide (1.4 ml, 1.4 mmol), and further benzoyl chloride (0.096 ml, 0.82 mmol) was added dropwise thereto. The mixture was stirred under ice-cooling for 30 minutes, combined with water, and extracted with ethyl acetate. The extracts were washed with water, and concentrated under reduced pressure to give the title compound (0.24 g, yield 93%).
Amorphous.
¹H NMR (CDCl₃) δ 1. 07 (3H, s), 1.27 (3H, s), 1.33 (3H, s), 1.46 (3H, t, J = 7.0 Hz), 2.12 (1H, d, J = 16.2 Hz), 2.24 (1H, d, J = 16.2 Hz), 2.57 (1H, d, J = 15.6 Hz), 2.94 (1H, d, J = 15.6 Hz), 3.6₁₋₃.70 (1H, m), 3.78-3.88 (1H, m), 4.18 (2H, q, J = 7.0 Hz), 6.64 (1H, s), 6.97-7.02 (1H, m), 7.41-7.51 (8H, m), 7.75-7.79 (2H, m).

### Reference Example 77

### 2-Chloro-N-[(6-ethoxy-3,4,8,9-tetrahydro-3,8,8-trimethyl-1-phenylfuro[2,3-h]isoquinolin-3-yl)methyl]acetamide

To a solution of ice-cooled 6-ethoxy-3,4,8,9-tetrahydro-3,8,8-trimethyl-1-phenylfuro[2,3-h]isoquinoline-3-methanamine (0.540 g, 1.48 mmol) in tetrahydrofuran (7 ml) was added 1 M aqueous solution of sodium hydroxide (3.7 ml, 3.7 mmol), and further chloroacetyl chloride (0.177 ml, 2.22 mmol) was added dropwise thereto. The mixture was stirred under ice-cooling for 15 minutes, combined with water, and extracted with ethyl acetate. The extracts were washed with water, and concentrated under reduced pressure to give the title compound (0.64 g, yield 98%). Amorphous.
¹H NMR (CDCl₃) δ 1.04 (3H, s), 1.28 (3H, s), 1.35 (3H, s), 1.47 (3H, t, J = 6.9 Hz), 2.15 (1H, d, J = 16.2 Hz), 2.27 (1H, d, J = 16.2 Hz), 2.54 (1H, d, J = 12.0 Hz), 2.85 (1H, d, J = 12.0 Hz), 3.39-3.46 (1H, m), 3.63-3.70 (1H, m), 4.07 (2H, s), 4.18 (2H, q, J = 6.9 Hz), 6.63 (1H, s), 7.32-7.42 (6H, m).

### Reference Example 78

### N-[(6-Ethoxy-3,4,8,9-tetrahydro-3,8,8-trimethyl-1-phenylfuro[2,3-h]isoquinolin-3-yl)methyl]-1H-imidazole-1-acetamide

To a solution of 2-chloro-N-[(6-ethoxy-3,4,8,9-tetrahydro-3,8,8-trimethyl-1-phenylfuro[2,3-h]isoquinolin-3-yl)methyl]acetamide (0.340 g, 0.771 mmol) in N,N-dimethylformamide (5 ml) were added sodium hydride (66% dispersion in oil) (33.6 mg, 0.925 mmol) and imidazole (0.157 g, 2.31 mmol), and the mixture was stirred at room temperature for 30 minutes. The reaction solution was combined with water, and extracted with ethyl acetate. The extracts were washed with water, and concentrated under reduced pressure. The residue was purified with a basic silica gel column chromatography (ethyl acetate/methanol 19:1) to give the title compound (0.262 g, yield 72%).
Amorphous.
¹H NMR (CDCl₃) δ 0.91 (3H, s), 1.26 (3H, s), 1.36 (3H, s), 1.46 (3H, t, J = 7.0 Hz), 2.13 (1H, d, J = 16.2 Hz), 2.31 (1H, d, J = 16.2 Hz), 2.46 (1H, d, J = 15.2 Hz), 2.75 (1H, d, J = 15.2 Hz), 3.23-3.33 (1H, m), 3.59-3.70 (1H, m), 4.18 (2H, q, J = 7.0 Hz), 4.67 (2H, s), 6.50-6.58 (1H, m), 6.61 (1H, s), 6.93 (1H, s), 7.08-7.10 (2H, m), 7.28-7.31 (2H, m), 7.41-7.44 (2H, m), 7.52 (1H, s).

### Reference Example 79

### N-[(6-Ethoxy-3,4,8,9-tetrahydro-3,8,8-trimethyl-1-phenylfuro[2,3-h]isoquinolin-3-yl)methyl]methanesulfonamide

To a solution of ice-cooled 6-ethoxy-3,4,8,9-tetrahydro-3,8,8-trimethyl-1-phenylfuro[2,3-h]isoquinoline-3-methanamine (0.200 g, 0.549 mmol) in tetrahydrofuran (3 ml) was added 1 M aqueous solution of sodium hydroxide (1.4 ml, 1.4 mmol), and further methanesulfonyl chloride (0.064 ml, 0.82 mmol) was added dropwise thereto. The mixture was stirred under ice-cooling for 30 minutes, combined with water, and extracted with ethyl acetate. The extracts were washed with water, concentrated under reduced pressure, and further the residue was washed with hexane and dried to give the title compound (0.17 g, yield 70%).
Amorphous.
¹H NMR (CDCl₃) δ 1.05 (3H, s), 1.27 (3H, s), 1.35 (3H, s), 1.47 (3H, t, J = 7.0 Hz), 2.13 (1H, d, J = 16.2 Hz), 2.28 (1H, d, J = 16.2 Hz), 2.46 (1H, d, J = 15.4 Hz), 2.96 (3H, s), 3.05 (1H, d, J = 15.4 Hz), 3.18-3.26 (1H, m), 3.37-3.44 (1H, m), 4.19 (2H, q, J = 7.0 Hz), 5.04-5.10 (1H, m), 6.63 (1H, s), 7.40 (5H, s).

### Reference Example 80

### N-[(6-Ethoxy-3,4,8,9-tetrahydro-3,8,8-trimethyl-1-phenylfuro[2,3-h]isoquinolin-3-yl)methyl]-2-(dimethylamino)acetamide

To a solution of 2-chloro-N-[(6-ethoxy-3,4,8,9-tetrahydro-3,8,8-trimethyl-1-phenylfuro[2,3-h]isoquinolin-3-yl)methyl]acetamide (0.300 g, 0.680 mmol) in tetrahydrofuran (4 ml) was added an aqueous solution of 40% dimethylamine (1 ml), and the mixture was stirred at room temperature for 1 hour. The reaction solution was combined with water, and extracted with ethyl acetate. The extracts were washed with water, and concentrated under reduced pressure to give the title compound (0.230 g, yield 75%).
Amorphous.
¹H NMR (CDCl₃) δ 1.05 (3H, s), 1.27 (3H, s), 1.33 (3H, s), 1.46 (3H, t, J = 7.0 Hz), 2.12 (1H, d, J = 16.4 Hz), 2.23 (1H, d, J = 16.4 Hz), 2.26 (6H, s), 2.53 (1H, d, J = 15.8 Hz), 2.84 (1H, d, J = 15.8 Hz), 2.97 (2H, s), 3.43-3.62 (2H, m), 4.18 (2H, q, J = 7.0 Hz), 6.62 (1H, s), 7.38-7.44 (5H, m), 7.67-7.75 (1H, m).

### Reference Example 81

### N-[(6-Ethoxy-3,4,8,9-tetrahydro-3,8,8-trimethyl-1-phenylfuro[2,3-h]isoquinolin-3-yl)methyl]acetamide

From 6-ethoxy-3,4,8,9-tetrahydro-3,8,8-trimethyl-1-phenylfuro[2,3-h]isoquinoline-3-methanamine and acetic anhydride, the title compound was obtained by the method similar to that in Reference Example 79. Yield 63%.
Amorphous.
¹H NMR (CDCl₃) δ 1.01 (3H, s), 1.27 (3H, s), 1.34 (3H, s), 1.46 (3H, t, J = 7.0 Hz), 2.00 (3H, s), 2.12 (1H, d, J = 16.2 Hz), 2.24 (1H, d, J = 16.2 Hz), 2.49 (1H, d, J = 15.6 Hz), 2.86 (1H, d, J = 15.6 Hz), 3.40-3.51 (1H, m), 3.53-3.63 (1H, m), 4.18 (2H, q, J = 7.0 Hz), 6.17-6.24 (1H, m), 6.62 (1H, s), 7.41 (5H, s).

### Reference Example 82

### 3-(6-Ethoxy-3,4,8,9-tetrahydro-4-hydroxy-3,3,8,8-tetramethylfuro[2,3-h]isoquinolin-1-yl)-N-methylbenzamide

A mixture of 3-(6-ethoxy-3,4,8,9-tetrahydro-3,3,8,8-tetramethylfuro[2,3-h]isoquinolin-1-yl)-N-methylbenzamide (2.78 g, 6.84 mmol), N-bromosuccinimide (1.46 g, 8.21 mmol) and 2,2'-azobis(isobutyronitrile) (0.112 g, 0.684 mmol) was heated under reflux for 1 hour. The reaction solution was cooled to room temperature, and concentrated under reduced pressure. The residue was combined with an aqueous solution of sodium hydrogen carbonate and extracted with ethyl acetate. The extracts were washed with water, and concentrated under reduced pressure. The residue was purified with a basic silica gel column chromatography (ethyl acetate), and recrystallized from ethyl acetate-hexane to give the title compound (0.400 g, yield 14%).
Melting point: 186 to 188°C.
¹H NMR (CDCl₃) δ 1.18 (3H, s), 1.26 (3H, s), 1.30 (6H, s), 1.47 (3H, t, J = 6.9 Hz), 2.14 (2H, s), 2.94 (3H, d, J = 4.8 Hz), 4.12 (2H, q, J = 6.9 Hz), 4.38 (1H, br s), 6.90 (1H, s), 6.96 (1H, br s), 7.41-7.46 (2H, m), 7.71 (1H, s), 7.84-7.88 (1H, m).

### Reference Example 83

### 3-(6-Ethoxy-4-fluoro-3,4,8,9-tetrahydro-3,3,8,8-tetramethylfuro[2,3-h]isoquinolin-1-yl)-N-methylbenzamide

A solution of 3-(6-ethoxy-3,4,8,9-tetrahydro-4-hydroxy-3,3,8,8-tetramethylfuro[2,3-h]isoquinolin-1-yl)-N-methylbenzamide (0.100 g, 0.237 mmol) in dichloromethane (3 ml) was cooled to-78°C, to this solution was added diethylaminosulfur trifluoride (41.7 µl, 0.284 mmol), and the mixture was slowly cooled to room temperature with stirring the mixture. The reaction solution was combined with an aqueous solution of sodium hydrogen carbonate, and extracted with ethyl acetate. The extracts were washed with water, and concentrated under reduced pressure. The residue was purified with a silica gel column chromatography (ethyl acetate), and recrystallized from ethyl acetate-hexane to give the title compound (79.0 mg, yield 79%).
Melting point: 172 to 173°C.
¹H NMR (CDCl₃) δ 1.12 (3H, s), 1.28 (3H, s), 1.33 (3H, s), 1.47 (3H, t, J = 7.0 Hz), 1.48 (3H, s), 2.13 (1H, d, J = 16.2 Hz), 2.22 (1H, d, J = 16.2 Hz), 2.97 (3H, d, J = 4.6 Hz), 4.22 (2H, q, J = 7.0 Hz), 5.10 (1H, d, J = 50.8 Hz), 6.4₃₋₆.53 (1H, m), 6.87-6.89 (1H, m), 7.42-7.50 (2H, m), 7.79 (1H, s), 7.85-7.93 (1H, m).

### Reference Example 84

### 3-(6-Ethoxy-3,4,8,9-tetrahydro-3,3,8,8-tetramethyl-4-oxofuro[2,3-h]isoquinolin-1-yl)-N-methylbenzamide

To a solution of 3-(6-ethoxy-3,4,8,9-tetrahydro-4-hydroxy-3,3,8,8-tetramethylfuro[2,3-h]isoquinolin-1-yl)-N-methylbenzamide (0.450 g, 1.07 mmol) in chloroform (5 ml) was added manganese dioxide (1.5 g), and the mixture was stirred for 15 hours at room temperature. The reaction solution was filtered, and the filtrate was concentrated under reduced pressure. The residue was purified with a silica gel column chromatography (ethyl acetate) and recrystallized from ethyl acetate-hexane to give the title compound (0.289 g, yield 64%).
Melting point: 233 to 235°C.
¹H NMR (CDCl₃) δ 1.33 (6H, s), 1.49 (3H, t, J = 7.0 Hz), 1.50 (6H, s), 2.16 (2H, s), 2.98 (3H, d, J = 4.4 Hz), 4.25 (2H, q, J = 7.0 Hz), 6.47 (1H, br d, J = 4.4 Hz), 7.42-7.52 (2H, m), 7.56 (1H, s), 7.75-7.79 (1H, m), 7.86-7.92 (1H, m).

### Reference Example 85

### 3-(6-Ethoxy-3,4,8,9-tetrahydro-3,3,8,8-tetramethylfuro[2,3-h]isoquinolin-1-yl)-N,N-dimethylbenzamide

To a solution of 3-(6-ethoxy-3,4,8,9-tetrahydro-3,3,8,8-tetramethylfuro[2,3-h]isoquinolin-1-yl)benzoic acid hydrochloride (0.200 g, 0.465 mmol) and 1-hydroxy-1H-benzotriazole monohydrate (78.4 mg, 0.512 mmol) in N,N-dimethylformamide (2 ml) was added 1-ethyl-3-(3-dimethylaminopropyl)carbodiimide hydrochloride (0.116 g, 0.605 mmol) under ice-cooling, and the mixture was stirred under ice-cooling for 30 minutes. To this mixture was added an aqueous solution of 50% dimethylamine (1 ml), and the mixture was further stirred for 1 hour under ice-cooling. The reaction solution was combined with water, and extracted with ethyl acetate. The extracts were washed with water and concentrated under reduced pressure to give the title compound (0.188 g, yield 96%).
Oily matter.
¹H NMR (CDCl₃) δ 1.24 (6H, s), 1.31 (6H, s), 1.46 (3H, t, J = 7.0 Hz), 2.23 (2H, s), 2.67 (2H, s), 3.00-3.08 (6H, m), 4.18 (2H, q, J = 7.0 Hz), 6.60 (1H, s), 7.42-7.49 (4H, m).

### Reference Example 86

### 1-[3-(6-Ethoxy-3,4,8,9-tetrahydro-3,3,8,8-tetramethylfuro[2,3-h]isoquinolin-1-yl)benzoyl]pyrrolidine

To a suspension of 3-(6-ethoxy-3,4,8,9-tetrahydro-3,3,8,8-tetramethylfuro[2,3-h]isoquinolin-1-yl)benzoic acid hydrochloride (0.20 g, 0.465 mmol), 1-hydroxy-1H-benzotriazole monohydrate (78.4 mg, 0.512 mmol) and pyrrolidine (58.2 µl, 0.698 mmol) in N,N-dimethylformamide (2 ml) were added 1-ethyl-3-(3-dimethylaminopropyl)carbodiimide hydrochloride (0.116 g, 0.605 mmol) and triethylamine (0.195 ml, 1.40 mmol), and the mixture was stirred at room temperature for 1 hour. The reaction solution was combined with water, and extracted with ethyl acetate. The extracts were washed with water and concentrated under reduced pressure to give the title compound (0.175 g, yield 84%).
Oily matter.
¹H NMR (CDCl₃) δ 1.23 (6H, s), 1.31 (6H, s), 1.46 (3H, t, J = 7.0 Hz), 1.82-1.99 (4H, m), 2.22 (2H, s), 2.67 (2H, s), 3.44 (2H, t, J = 6.2 Hz), 3.63 (2H, t, J = 6.4 Hz), 4.18 (2H, q, J = 7.0 Hz), 6.60 (1H, s), 7.38-7.60 (4H, m).

### Reference Example 87

### 1-[3-(6-Ethoxy-3,4,8,9-tetrahydro-3,3,8,8-tetramethylfuro[2,3-h]isoquinolin-1-yl)benzoyl]piperidine

From 3-(6-ethoxy-3,4,8,9-tetrahydro-3,3,8,8-tetramethylfuro[2,3-h]isoquinolin-1-yl)benzoic acid hydrochloride and piperidine, the title compound was obtained by the method similar to that in Reference Example 86. Yield 79%.
Oily matter.
¹H NMR (CDCl₃) δ 1.23 (6H, s), 1.31 (6H, s), 1.46 (3H, t, J = 7.0 Hz), 1.48-1.71 (6H, m), 2.22 (2H, s), 2.67 (2H, s), 3.39 (2H, br s), 3.68 (2H, br s), 4.18 (2H, q, J = 7.0 Hz), 6.60 (1H, s), 7.38-7.50 (4H, m).

### Reference Example 88

### 1,1-Dimethylethyl 3-[[[3-(6-Ethoxy-3,4,8,9-tetrahydro-3,3,8,8-tetramethylfuro[2,3-h]isoquinolin-1-yl)phenyl]amino]carbonyl]-1-piperidinecarboxylate

To a solution of 3-(6-ethoxy-3,4,8,9-tetrahydro-3,3,8,8-tetramethylfuro[2,3-h]isoquinolin-1-yl)benzenamine (3.50 g, 9.60 mmol), 1-hydroxy-1H-benzotriazole monohydrate (1.62 g, 10.6 mmol) and 1-[[(1,1-dimethylethyl)oxy]carbonyl]-3-piperidinecarboxylic acid (2.20 g, 9.60 mmol) in N,N-dimethylformamide (30 ml) were added 1-ethyl-3-(3-dimethylaminopropyl)carbodiimide hydrochloride (2.39 g, 12.5 mmol) and triethylamine (4.02 ml, 28.8 mmol), and the mixture was stirred at 50°C for 2 hours. The reaction solution was combined with water, and extracted with ethyl acetate. The extracts were washed with water, and concentrated under reduced pressure. The residue was purified with a silica gel column chromatography (from hexane/ethyl acetate 1:4 to ethyl acetate) and recrystallized from ethyl acetate-hexane to give the title compound (3.04 g, yield 55%).
Melting point: 202 to 203°C.
¹H NMR (CDCl₃) δ 1.19 (3H, s), 1.24 (3H, s), 1.31 (6H, s), 1.38-1.72 (2H, m), 1.45 (9H, s), 1.46 (3H, t, J = 7.0 Hz), 1.91 (2H, br s), 2.26 (2H, s), 2.45 (1H, br s), 2.65 (2H, s), 3.09 (1H, br s), 3.38 (1H, br s), 3.72 (1H, br s), 3.90-3.98 (1H, m), 4.18 (2H, q, J = 7.0 Hz), 6.58 (1H, s), 7.03 (1H, d, J = 7.8 Hz), 7.22-7.30 (1H, m), 7.48 (1H, s), 7.75 (1H, d, J = 8.2 Hz), 8.52 (1H, br s).

### Reference Example 89

### N-[3-(6-Ethoxy-3,4,8,9-tetrahydro-3,3,8,8-tetramethylfuro[2,3-h]isoquinolin-1-yl)phenyl]-3-piperidinecarboxamide dihydrochloride

To a solution of 1,1-dimethylethyl 3-[[[3-(6-ethoxy-3,4,8,9-tetrahydro-3,3,8,8-tetramethylfuro[2,3-h]isoquinolin-1-yl)phenyl]amino]carbonyl]-1-piperidinecarboxylate (2.48 g, 4.31 mmol) in ethanol (30 ml) was added a solution of 4 M hydrogen chloride/ethyl acetate (2.5 ml), and the mixture was stirred for 1 hour at 60°C. The reaction solution was cooled to room temperature and concentrated under reduced pressure. The residue was crystallized from ethyl acetate to give the title compound (2.18 g, yield 92%).
Melting point: 256 to 257°C (Decomposed).
¹H NMR (DMSO-d₆) δ 1.18 (3H, t, J = 7.1 Hz), 1.24 (6H, s), 1.46 (6H, br s), 1.59-1.80 (3H, m), 2.06-2.10 (1H, m), 2.22-2.43 (2H, m), 2.86-3.48 (7H, m), 4.24 (2H, q, J = 7.1 Hz), 7.08 (1H, s), 7.32 (1H, d, J = 7.8 Hz), 7.59 (1H, t, J = 7.8 Hz), 7.88 (1H, br s), 7.99 (1H, s), 9.18-9.29 (1H, m), 9.33-9.48 (1H, m).

### Reference Example 90

### N-[3-(6-Ethoxy-3,4,8,9-tetrahydro-3,3,8,8-tetramethylfuro[2,3-h]isoquinolin-1-yl)phenyl]urea

To a suspension of 3-(6-ethoxy-3,4,8,9-tetrahydro-3,3,8,8-tetramethylfuro[2,3-h]isoquinolin-1-yl)benzenamine dihydrochloride (0.800 g, 1.83 mmol) in tetrahydrofuran (10 ml) was added triethylamine (0.510 ml, 3.66 mmol), and the mixture was stirred until the red color was dissipated. To this mixture was added sodium cyanate (0.238 g, 3.66 mmol) and the mixture was cooled with ice, and trifluoroacetic acid (0.705 ml, 9.15 mmol) was added dropwise thereto. The mixture was stirred for 2 hours at room temperature, combined with an aqueous solution of sodium hydrogen carbonate, and extracted with ethyl acetate. The extracts were washed with water, and concentrated under reduced pressure. The residue was recrystallized from ethyl acetate-hexane to give the title compound (0.470 g, yield 63%).
Melting point: 202 to 205°C.
¹H NMR (CDCl₃) δ 1.24 (6H, s), 1.30 (6H, s), 1.45 (3H, t, J = 7.0 Hz), 2.27 (2H, s), 2.67 (2H, s), 4.17 (2H, q, J = 7.0 Hz), 4.92 (2H, br s), 6.58 (1H, s), 6.94 (1H, d, J = 7.2 Hz), 7.19 (1H, d, J = 8.2 Hz), 7.25-7.28 (1H, m), 7.36 (1H, d, J = 7.2 Hz), 7.75 (1H, br s).

### Reference Example 91

### 2-Methyl-N-[3-(6-ethoxy-3,4,8,9-tetrahydro-3,3,8,8-tetramethylfuro[2,3-h]isoquinolin-1-yl)phenyl]-2-[(trifluoroacetyl)amino]propanamide

To a suspension of 3-(6-ethoxy-3,4,8,9-tetrahydro-3,3,8,8-tetramethylfuro[2,3-h]isoquinolin-1-yl)benzenamine dihydrochloride (1.50 g, 3.43 mmol), 2-methyl-2-[(trifluoroacetyl)amino]propionic acid (0.683 g, 3.43 mmol) and 1-hydroxy-1H-benzotriazole monohydrate (0.525 g, 3.43 mmol) in N,N-dimethylformamide (10 ml) were added 1-ethyl-3-(3-dimethylaminopropyl)carbodiimide hydrochloride (0.855 g, 4.46 mmol) and triethylamine (1.67 ml, 12.0 mmol), and the mixture was stirred at room temperature for 15 hours. The reaction solution was combined with water, and extracted with ethyl acetate. The extracts were washed with water, and concentrated under reduced pressure. The residue was purified with a silica gel column chromatography (ethyl acetate/methanol 97:3) to give the title compound (1.32 g, yield 71%).
Amorphous.
¹H NMR (CDCl₃) δ 1.24 (6H, s), 1.32 (6H, s), 1.47 (3H, t, J = 7.0 Hz), 1.76 (6H, s), 2.29 (2H, s), 2.66 (2H, s), 4.18 (2H, q, J = 7.0 Hz), 6.60 (1H, s), 7.15 (1H, d, J = 7.8 Hz), 7.37 (1H, t, J = 7.8 Hz), 7.45-7.49 (1H, m), 7.64-7.71 (1H, m), 7.80 (2H, s).

### Reference Example 92

### N-[3-(6-Ethoxy-3,4,8,9-tetrahydro-3,3,8,8-tetramethylfuro[2,3-h]isoquinolin-1-yl)phenyl]-5-oxo-2-pyrrolidinecarboxamide

From 3-(6-ethoxy-3,4,8,9-tetrahydro-3,3,8,8-tetramethylfuro[2,3-h]isoquinolin-1-yl)benzenamine dihydrochloride and 5-oxo-2-pyrrolidinecarboxylic acid, the title compound was obtained by the method similar to that in Reference Example 91. Yield 78%.
Amorphous.
¹H NMR (CDCl₃) δ 1.22 (6H, br s), 1.30 (6H, s), 1.46 (3H, t, J = 7.0 Hz), 2.00 (2H, br s), 2.21 (2H, s), 2.28-2.55 (4H, m), 4.08-4.23 (3H, m), 6.58 (1H, s), 7.07 (1H, d, J = 8.0 Hz), 7.15-7.30 (2H, m), 7.54 (1H, s), 7.71 (1H, d, J = 8.0 Hz), 8.71 (1H, br s).

### Reference Example 93

### 2-Amino-2-methyl-N-[3-(6-ethoxy-3,4,8,9-tetrahydro-3,3,8,8-tetramethylfuro[2,3-h]isoquinolin-1-yl)phenyl]propanamide

To a solution of 2-methyl-N-[3-(6-ethoxy-3,4,8,9-tetrahydro-3,3,8,8-tetramethylfuro[2,3-h]isoquinolin-1-yl)phenyl]-2-[(trifluoroacetyl)amino]propanamide (1.25 g, 2.29 mmol) in tetrahydrofuran (10 ml) was added 2 M aqueous solution (5 ml) of sodium hydroxide, and the mixture was stirred at 90°C for 24 hours. The reaction solution was combined with water, and extracted with ethyl acetate. The extracts were washed with water and concentrated under reduced pressure to give the title compound (0.98 g, yield 95%).
Amorphous.
¹H NMR (CDCl₃) δ 1.23 (6H, s), 1.31 (6H, s), 1.43-1.48 (9H, m), 2.28 (2H, s), 2.65 (2H, s), 4.18 (2H, q, J = 7.8 Hz), 6.59 (1H, s), 7.07 (1H, d, J = 7.8 Hz), 7.33 (1H, t, J = 7.8 Hz), 7.54 (1H, s), 7.82 (1H, d, J = 7.8 Hz), 9.97 (1H, s).

### Reference Example 94

### 5,5-Dimethyl-3-[3-(6-ethoxy-3,4,8,9-tetrahydro-3,3,8,8-tetramethylfuro[2,3-h]isoquinolin-1-yl)phenyl]-2,4-imidazolidinedione

To a solution of 2-amino-2-methyl-N-[3-(6-ethoxy-3,4,8,9-tetrahydro-3,3,8,8-tetramethylfuro[2,3-h]isoquinolin-1-yl)phenyl]propanamide (0.890 g, 1.98 mmol) in N,N-dimethylformamide (10 ml) was added N,N'-carbonyldiimidazole (0.685 g, 4.22 mmol), and the mixture was stirred for 20 hours at room temperature. The reaction solution was combined with a saturated aqueous solution of sodium hydrogen carbonate, and extracted with ethyl acetate. The extracts were washed with water, and concentrated under reduced pressure. The residue was purified with a silica gel column chromatography (from hexane/ethyl acetate 1:1 to ethyl acetate), and recrystallized from ethyl acetate-hexane to give the title compound (0.370 g, yield 39%).
Melting point: 237 to 238°C.
¹H NMR (CDCl₃) δ 1.25-1.32 (18H, m), 1.47 (3H, t, J = 6.9 Hz), 2.37 (2H, br s), 2.69 (2H, s), 4.19 (2H, q, J = 6.9 Hz), 6.60 (1H, s), 7.28-7.34 (1H, m), 7.42-7.48 (2H, m), 7.55 (1H, br s), 7.62 (1H, s).

### Reference Example 95

### 3-[3-(6-Ethoxy-3,4,8,9-tetrahydro-3,3,8,8-tetramethylfuro[2,3-h]isoquinolin-1-yl)phenyl]-2,4-imidazolidinedione

To a suspension of 3-(6-ethoxy-3,4,8,9-tetrahydro-3,3,8,8-tetramethylfuro[2,3-h]isoquinolin-1-yl)benzenamine dihydrochloride (0.800 g, 1.83 mmol) in tetrahydrofuran (12 ml) was added triethylamine (0.561 ml, 4.03 mmol), and the mixture was stirred until the red color was dissipated. To this mixture was added ethyl isocyanatoacetate (0.215 ml, 1.92 mmol), and the mixture was stirred at 60°C for 1.5 hours. The reaction solution was concentrated under reduced pressure, and the residue was combined with 5 M hydrochloric acid (14 ml), and stirred for 2 hours at 85°C. The reaction solution was combined with an aqueous solution of sodium hydrogen carbonate, and extracted with ethyl acetate. The extracts were washed with water, and concentrated under reduced pressure. The residue was purified with a basic silica gel column chromatography (from hexane/ethyl acetate 1:4 to ethyl acetate/methanol 97:3), and recrystallized from ethyl acetate-hexane to give the title compound (0.695 g, yield 85%).
Melting point: 202 to 204°C.
¹H NMR (CDCl₃) δ 1.25 (6H, br s), 1.33 (6H, s), 1.46 (3H, t, J = 7.0 Hz), 2.36 (2H, br s), 2.66 (2H, s), 3.98 (2H, s), 4.18 (2H, q, J = 7.0 Hz), 6.59 (1H, s), 7.41-7.52 (4H, m).

### Reference Example 96

### 3-[3-(6-Ethoxy-3,4,8,9-tetrahydro-3,3,8,8-tetramethylfuro[2,3-h]isoquinolin-1-yl)phenyl]-1-methyl-2,4-imidazolidinedione

To a solution of ice-cooled 3-[3-(6-ethoxy-3,4,8,9-tetrahydro-3,3,8,8-tetramethylfuro[2,3-h]isoquinolin-1-yl)phenyl]-2,4-imidazolidinedione (0.567 g, 1.27 mmol) in tetrahydrofuran (5 ml) was added sodium hydride (66% dispersion in oil) (55.3 mg, 1.52 mmol), and the mixture was stirred under ice-cooling for 20 minutes. To this mixture was added dropwise iodomethane (94.6 µl, 1.52 mmol), and the mixture was further stirred for 1 hour under ice-cooling. The reaction solution was combined with sodium hydrogen carbonate water, and extracted with ethyl acetate. The extracts were washed with water, and concentrated under reduced pressure. The residue was purified with a basic silica gel column chromatography (hexane/ethyl acetate 1:4) to give the title compound (0.375 g, yield 64%).
Amorphous.
¹H NMR (CDCl₃) δ 1.23 (6H, s), 1.33 (6H, s), 1.45 (3H, t, J = 7.0 Hz), 2.36 (2H, br s), 2.65 (2H, s), 3.06 (3H, s), 4.00 (2H, s), 4.18 (2H, q, J = 7.0 Hz), 6.58 (1H, s), 7.48-7.52 (4H, m).

### Reference Example 97

### 1-Acetyl-N-[3-(6-ethoxy-3,4,8,9-tetrahydro-3,3,8,8-tetramethylfuro[2,3-h]isoquinolin-1-yl)phenyl]-3-piperidinecarboxamide

A suspension of N-[3-(6-ethoxy-3,4,8,9-tetrahydro-3,3,8,8-tetramethylfuro[2,3-h]isoquinolin-1-yl)phenyl]-3-piperidinecarboxamide dihydrochloride (0.400 g, 0.729 mmol) in tetrahydrofuran (3 ml) was cooled with ice, 2 M aqueous solution of sodium hydroxide (1.5 ml, 3.0 mmol) was added thereto, and the mixture was stirred. To this mixture was added dropwise acetic anhydride (0.103 ml, 1.09 mmol), and the mixture was stirred at room temperature for 1 hour. The reaction solution was combined with water, and extracted with ethyl acetate. The extracts were washed with water and concentrated under reduced pressure to give the title compound (0.38 g, quantitative).
Amorphous.
¹H NMR (CDCl₃) δ 1.16 (3H, s), 1.23 (3H, s), 1.30 (6H, s), 1.45 (3H, t, J = 7.0 Hz), 1.50-1.55 (1H, m), 1.62-1.81 (1H, m), 1.99 (2H, s), 2.13 (3H, s), 2.25 (2H, s), 2.62-2.75 (3H, m), 3.28-3.47 (2H, m), 3.59-3.66 (0.7H, m), 3.87-3.93 (0.3H, m), 4.11-4.21 (2.7H, m), 4.47-4.55 (0.3H, m), 6.56-6.60 (1H, m), 6.98-7.04 (1H, m), 7.21-7.25 (1H, m), 7.35 (0.3H, s), 7.53 (0.7H, s), 7.64-7.68 (0.7H, m), 7.87-7.92 (0.3H, m), 8.70 (0.3H, br s), 8.98 (0.7H, br s).

### Reference Example 98

### N3-[3-(6-Ethoxy-3,4,8,9-tetrahydro-3,3,8,8-tetramethylfuro[2,3-h]isoquinolin-1-yl)phenyl]-1,3-piperidinedicarboxamide

From N-[3-(6-ethoxy-3,4,8,9-tetrahydro-3,3,8,8-tetramethylfuro[2,3-h]isoquinolin-1-yl)phenyl]-3-piperidinecarboxamide dihydrochloride and sodium cyanate, the title compound was obtained by the method similar to that in Reference Example 90. Yield 75%.
Melting point: 119 to 121°C (recrystallized from ethyl acetate).
¹H NMR (DMSO-d₆) δ 1.13 (6H, s), 1.22 (6H, s), 1.32 (3H, t, J = 7.0 Hz), 1.50-1.66 (2H, m), 1.81-2.01 (1H, m), 2.28 (2H, s), 2.31-2.48 (1H, m), 2.53-2.66 (4H, m), 2.70-2.81 (1H, m), 3.83-3.95 (1H, m), 4.00-4.13 (3H, m), 5.96 (2H, s), 6.75 (1H, s), 7.03 (1H, d, J = 7.5 Hz), 7.34 (1H, t, J = 7.5 Hz), 7.60-7.71 (2H, m), 10.07 (1H, s).

### Reference Example 99

### N3-[3-(6-Ethoxy-3,4,8,9-tetrahydro-3,3,8,8-tetramethylfuro[2,3-h]isoquinolin-1-yl)phenyl]-N1-methyl-1,3-piperidinedicarboxamide

To a suspension of N-[3-(6-ethoxy-3,4,8,9-tetrahydro-3,3,8,8-tetramethylfuro[2,3-h]isoquinolin-1-yl)phenyl]-3-piperidinecarboxamide dihydrochloride (0.500 g, 0.912 mmol) in tetrahydrofuran (10 ml) was added triethylamine (0.280 ml, 2.01 mmol), and the mixture was stirred at room temperature for 15 minutes. To this mixture was added dropwise methyl isocyanate (64.5 µl, 1.09 mmol), and the mixture was stirred at room temperature for 1.5 hours. The reaction solution was combined with water, and extracted with ethyl acetate. The extracts were washed with water and concentrated under reduced pressure to give the title compound (0.30 g, yield 62%).
Amorphous.
¹H NMR (DMSO-d₆) δ 1.12 (6H, s), 1.22 (6H, s), 1.33 (3H, t, J = 7.0 Hz), 1.56-1.64 (2H, m), 1.91 (1H, br d, J = 10.4 Hz), 2.28 (2H, s), 2.33-2.45 (1H, m), 2.50-2.67 (7H, m), 2.76 (1H, t, J = 12.6 Hz), 3.90 (1H, br d, J = 12.6 Hz), 4.01-4.13 (3H, m), 6.37-6.47 (1H, m), 6.77 (1H, s), 7.01 (1H, d, J = 7.6 Hz), 7.32 (1H, t, J = 7.6 Hz), 7.60-7.65 (2H, m), 10.01 (1H, s).

### Reference Example 100

### N-[3-(6-Ethoxy-3,4,8,9-tetrahydro-3,3,8,8-tetramethylfuro[2,3-h]isoquinolin-1-yl)phenyl]-2,2,2-trifluoroacetamide

To a suspension of 3-(6-ethoxy-3,4,8,9-tetrahydro-3,3,8,8-tetramethylfuro[2,3-h]isoquinolin-1-yl)benzenamine dihydrochloride (0.800 g, 1.83 mmol) in tetrahydrofuran (10 ml) was added triethylamine (1.27 ml, 9.15 mmol), and the mixture was stirred until the red color was dissipated. This was cooled with ice, trifluoroacetic anhydride (0.387 ml, 2.75 mmol) was added dropwise thereto, and the mixture was stirred for 1 hour. The reaction solution was combined with water, and extracted with ethyl acetate. The extracts were washed with water, and concentrated under reduced pressure. The residue was and recrystallized from ethyl acetate-hexane to give the title compound (0.65 g, yield 77%).
¹H NMR (CDCl₃) δ 1.29 (12H, s), 1.46 (3H, t, J = 6.9 Hz), 2.21 (2H, br s), 2.69 (2H, s), 4.18 (2H, q, J = 6.9 Hz), 6.59 (1H, s), 7.10-7.12 (2H, m), 7.24-7.29 (1H, m), 7.35 (1H, d, J = 8.1 Hz), 10.06 (1H, br s).

### Reference Example 101

### N-[3-(6-Ethoxy-3,4,8,9-tetrahydro-3,3,8,8-tetramethylfuro[2,3-h]isoquinolin-1-yl)phenyl]benzenemethanamine

To a solution of ice-cooled N-[3-(6-ethoxy-3,4,8,9-tetrahydro-3,3,8,8-tetramethylfuro[2,3-h]isoquinolin-1-yl)phenyl]-2,2,2-trifluoroacetamide (0.500 g, 1.09 mmol) and benzyl bromide (0.155 ml, 1.31 mmol) in tetrahydrofuran (5 ml) was added sodium hydride (66% dispersion in oil) (47.4 mg, 1.31 mmol), and the mixture was stirred at 60°C for 15 hours. The reaction solution was poured into 0.2 M hydrochloric acid, and washed with diisopropyl ether. The aqueous layer was alkalified with an aqueous solution of sodium hydrogen carbonate, and extracted with ethyl acetate. The extracts were washed with water, and concentrated under reduced pressure. The residue was purified with a basic silica gel column chromatography (hexane/ethyl acetate 9:1 from 17:3) to give the title compound (0.310 g, yield 63%).
Amorphous.
¹H NMR (CDCl₃) δ 1.23 (6H, s), 1.32 (6H, s), 1.45 (3H, t, J = 7.0 Hz), 2.31 (2H, s), 2.65 (2H, s), 4.02 (1H, br s), 4.17 (2H, q, J = 7.0 Hz), 4.34 (2H, s), 6.57-6.71 (4H, m), 7.15 (1H, t, J = 8.0 Hz), 7.29-7.35 (5H, m).

### Reference Example 102

### 4-Oxo-4-[[3-(3,4,8,9-tetrahydro-6-methoxy-3,3,8,8-tetramethylfuro[2,3-h]isoquinolin-1-yl)phenyl]amino]butyric acid

To a solution of succinic anhydride (503 mg, 5.03 mmol) in tetrahydrofuran (5 ml) was added a solution of 3-(3,4,8,9-tetrahydro-6-methoxy-3,3,8,8-tetramethylfuro[2,3-h]isoquinolin-1-yl)benzenamine (1.76 g, 5.02 mmol) in tetrahydrofuran (5 ml), and the mixture was stirred at room temperature for 1.5 hours. The reaction mixture was combined with diethyl ether, and the crystals were taken by filtration to give the title compound (2.09 g, yield 92%).
¹H NMR (CDCl₃) δ 1.30 (6H, s), 1.41 (6H, br s), 2.30 (2H, br s), 2.52 (4H, s), 2.84 (2H, s), 3.94 (3H, s), 6.64 (1H, s), 7.03 (1H, d, J = 7.5 Hz), 7.21-7.28 (1H, m), 7.58 (1H, d, J = 7.5 Hz), 7.71 (1H, s), 9.83 (1H, s).

### Reference Example 103

### N-Methyl-N'-[3-(3,4,8,9-tetrahydro-6-methoxy-3,3,8,8-tetramethylfuro[2,3-h]isoquinolin-1-yl)phenyl]butanediamide

To a solution of 4-oxo-4-[[3-(3,4,8,9-tetrahydro-6-methoxy-3,3,8,8-tetramethylfuro[2,3-h]isoquinolin-1-yl)phenyl]amino]butyric acid (226 mg, 0.502 mmol) and 1-hydroxy-1H-benzotriazole·ammonium salt (92 mg, 0.60 mmol) in N,N-dimethylformamide (1 ml) was added 1-ethyl-3-(3-dimethylaminopropyl)carbodiimide hydrochloride (125 mg, 0.652 mmol), and the mixture was stirred at room temperature for 15 hours. The reaction mixture was combined with water and a saturated aqueous solution of sodium hydrogen carbonate, and extracted three times with chloroform. The combined organic layer was washed with water and a saturated aqueous solution of sodium chloride, dried over sodium sulfate, filtered, and concentrated under reduced pressure. The residue was crystallized from ethyl acetate-diethyl ether to give the title compound (142 mg, yield 63%).
¹H NMR (CDCl₃) δ 1.23 (6H, br s), 1.31 (6H, s), 2.26 (2H, s), 2.60-2.75 (6H, m), 3.92 (3H, s), 5.58 (1H, br s), 5.91 (1H, br s), 6.59 (1H, s), 7.02 (1H, d, J = 7.7 Hz), 7.26 (1H, t, J = 7.7 Hz), 7.44 (1H, t, J = 1.7 Hz), 7.65-7.70 (1H, m), 8.64 (1H, br s).

### Reference Example 104

### N-[3-(3,4,8,9-Tetrahydro-6-methoxy-3,3,8,8-tetramethylfuro[2,3-h]isoquinolin-1-yl)phenyl]butanediamide

To 4-oxo-4-[[3-(3,4,8,9-tetrahydro-6-methoxy-3,3,8,8-tetramethylfuro[2,3-h]isoquinolin-1-yl)phenyl]amino]butyric acid (226 mg, 0.502 mmol), a solution of 40% methylamine/methanol (55 mg, 0.71 mmol) and a solution of 1-hydroxy-1H-benzotriazole monohydrate (85 mg, 0.56 mmol) in N,N-dimethylformamide (1 ml) was added 1-ethyl-3-(3-dimethylaminopropyl)carbodiimide hydrochloride (125 mg, 0.652 mmol), and the mixture was stirred at room temperature for 15 hours. The reaction mixture was combined with water and a saturated aqueous solution of sodium hydrogen carbonate, and extracted twice with ethyl acetate. The combined organic layer was washed with water and a saturated aqueous solution of sodium chloride, dried over sodium sulfate, filtered, and concentrated under reduced pressure. The residue was crystallized from ethyl acetate-diethyl ether to give the title compound (187 mg, yield 80%).
¹H NMR (CDCl₃) δ 1.23 (6H, br s), 1.31 (6H, s), 2.28 (2H, s), 2.53-2.60 (2H, m), 2.64-2.72 (4H, m), 2.77-2.82 (3H, m), 3.92 (3H, s), 5.95-6.08 (1H, m), 6.60 (1H, s), 7.04 (1H, d, J = 7.2 Hz), 7.25-7.34 (1H, m), 7.48 (1H, s), 7.64-7.70 (1H, m), 8.48-8.68 (1H, m).

### Reference Example 105

### 1,1-Dimethylethyl [3-oxo-3-[[3-(3,4,8,9-tetrahydro-6-methoxy-3,3,8,8-tetramethylfuro[2,3-h]isoquinolin-1-yl)phenyl]amino]propyl]carbamate

A solution of 3-(3,4,8,9-tetrahydro-6-methoxy-3,3,8,8-tetramethylfuro[2,3-h]isoquinolin-1-yl)benzenamine (701 mg, 2.00 mmol), 3-(tert-butoxycarbonylamino)propionic acid (417 mg, 2.20 mmol), 1-hydroxy-1H-benzotriazole monohydrate (337 mg, 2.20 mmol) and 1-ethyl-3-(3-dimethylaminopropyl)carbodiimide hydrochloride (499 mg, 2.60 mmol) in N,N-dimethylformamide (4 ml) was stirred at room temperature for 24 hours. The reaction mixture was combined with water and a saturated aqueous solution of sodium hydrogen carbonate, and extracted twice with ethyl acetate. The combined organic layer was washed twice with water, and concentrated under reduced pressure. The residue was purified with a basic silica gel column chromatography (ethyl acetate) to give the title compound (1.0 g, yield 96%).
Amorphous.
¹H NMR (CDCl₃) δ 1.23 (6H, br s), 1.32 (6H, s), 1.43 (9H, s), 2.29 (2H, s), 2.56 (2H, t, J = 5.9 Hz), 2.68 (2H, s), 3.47 (2H, q, J = 5.9 Hz), 3.92 (3H, s), 5.12-5.24 (1H, m), 6.60 (1H, s), 7.04-7.10 (1H, m), 7.30 (1H, t, J = 8.0 Hz), 7.46-7.51 (1H, m), 7.63-7.68 (1H, m), 7.96-8.05 (1H, m).

### Reference Example 106

### 3-Amino-N-[3-(3,4,8,9-tetrahydro-6-methoxy-3,3,8,8-tetramethylfuro[2,3-h]isoquinolin-1-yl)phenyl]propanamide

To a solution of 1,1-dimethylethyl [3-oxo-3-[[3-(3,4,8,9-tetrahydro-6-methoxy-3,3,8,8-tetramethylfuro[2,3-h]isoquinolin-1-yl)phenyl]amino]propyl]carbamate (930 mg, 1.84 mmol) in ethanol (5 ml) was added a solution of conc. hydrochloric acid (0.50 ml)/ethanol (1 ml), and the mixture was stirred at 80°C for 5 hours. The reaction mixture was concentrated under reduced pressure. The residue was combined with water and ethyl acetate, neutralized with a saturated aqueous solution of sodium hydrogen carbonate, and extracted twice with ethyl acetate. The combined organic layer was washed with a saturated aqueous solution of sodium chloride, dried through sodium sulfate-basic silica gel (eluting with ethyl acetate followed by chloroform/methanol 5:1), and concentrated under reduced pressure to give the title compound (280 mg, yield 36%).
¹H NMR (CDCl₃) δ 1.24 (6H, br s), 1.32 (6H, s), 2.30 (2H, s), 2.40-2.49 (2H, m), 2.68 (2H, s), 3.08-3.14 (2H, m), 3.92 (3H, s), 6.60 (1H, s), 7.06 (1H, dt, J = 7.9, 1.4 Hz), 7.32 (1H, t, J = 7.9 Hz), 7.39 (1H, t, J = 1.8 Hz), 7.82 (1H, ddd, J = 7.9, 2.2, 0.8 Hz), 10.14 (1H, br s).

### Reference Example 107

### 3-(Acetylamino)-N-[3-(3,4,8,9-tetrahydro-6-methoxy-3,3,8,8-tetramethylfuro[2,3-h]isoquinolin-1-yl)phenyl]propanamide

To a solution of 3-amino-N-[3-(3,4,8,9-tetrahydro-6-methoxy-3,3,8,8-tetramethylfuro[2,3-h]isoquinolin-1-yl)phenyl]propanamide (191 mg, 0.453 mmol) and triethylamine (82 µl, 0.59 mmol) in tetrahydrofuran (3 ml) was added dropwise acetyl chloride (38 µl, 0.54 mmol) under ice-cooling, and the mixture was stirred at the same temperature for 10 minutes. The reaction mixture was combined with water and a saturated aqueous solution of sodium hydrogen carbonate, and extracted twice with ethyl acetate. The combined organic layer was washed twice with water, and concentrated under reduced pressure. The residue was crystallized from ethyl acetate-hexane to give the title compound (188 mg, yield 90%).
¹H NMR (CDCl₃) δ 1.24 (6H, br s), 1.32 (6H, s), 1.97 (3H, s), 2.29 (2H, s), 2.54 (2H, m), 2.69 (2H, s), 3.53-3.62 (2H, m), 3.92 (3H, s), 6.35-6.42 (1H, m), 6.60 (1H, s), 7.08 (1H, d, J = 8.0 Hz), 7.31 (1H, t, J = 8.0 Hz), 7.56 (1H, s), 7.60 (1H, d, J = 8.0 Hz), 8.07 (1H, br s).

### Reference Example 108

### N-[2-(Acetylamino)ethyl]-3-(3,4,8,9-tetrahydro-6-methoxy-3,3,8,8-tetramethylfuro[2,3-h]isoquinolin-1-yl)benzamide

To a solution of 3-(3,4,8,9-tetrahydro-6-methoxy-3,3,8,8-tetramethylfuro[2,3-h]isoquinolin-1-yl)benzoic acid hydrochloride (208 mg, 0.500 mmol), N-acetylethylenediamine (61 mg, 0.60 mmol) and 1-hydroxy-1H-benzotriazole monohydrate (92 mg, 0.60 mmol) in N,N-dimethylformamide (1 ml) were added triethylamine (0.21 ml, 1.5 mmol) and 1-ethyl-3-(3-dimethylaminopropyl)carbodiimide hydrochloride (125 mg, 0.652 mmol), and the mixture was stirred at room temperature for 15 hours. The reaction mixture was combined with water and a saturated aqueous solution of sodium hydrogen carbonate, and extracted twice with ethyl acetate. The combined organic layer was washed twice with water, and concentrated under reduced pressure. The residue was crystallized from ethyl acetate-diethyl ether to give the title compound (169 mg, yield 73%).
¹H NMR (CDCl₃) δ 1.25 (6H, br s), 1.30 (6H, s), 2.00 (3H, s), 2.18 (2H, s), 2.69 (2H, s), 3.43-3. 59 (4H, m), 3.93 (3H, s), 6.4₂₋₆.50 (1H, m), 6.62 (1H, s), 7.30-7.36 (1H, m), 7.41-7.50 (2H, m), 7.83 (1H, dt, J = 6.5, 2.2 Hz), 7.88-7.90 (1H, m).

### Reference Example 109

### 3-(Acetylamino)-N-[3-(3,4,8,9-tetrahydro-6-methoxy-3,3,8,8-tetramethylfuro[2,3-h]isoquinolin-1-yl)phenyl]benzamide

To a solution of 3-(3,4,8,9-tetrahydro-6-methoxy-3,3,8,8-tetramethylfuro[2,3-h]isoquinolin-1-yl)benzenamine (351 mg, 1.00 mmol), 3-(acetamido)benzoic acid (198 mg, 1.11 mmol) and 1-hydroxy-1H-benzotriazole monohydrate (169 mg, 1.10 mmol) in N,N-dimethylformamide (1 ml) was added 1-ethyl-3-(3-dimethylaminopropyl)carbodiimide hydrochloride (250 mg, 1.30 mmol), and the mixture was stirred at room temperature for 14 hours. The reaction mixture was combined with water and a saturated aqueous solution of sodium hydrogen carbonate, and extracted twice with ethyl acetate. The combined organic layer was washed with water and a saturated aqueous solution of sodium chloride, dried through sodium sulfate-basic silica gel (eluting with ethyl acetate followed by ethyl acetate/methanol 5:1), and concentrated under reduced pressure. The residue was purified with a basic silica gel column chromatography (hexane/ethyl acetate 1:1, ethyl acetate followed by ethyl acetate/methanol 5:1) to give the title compound (505 mg, yield 99%).
Amorphous.
¹H NMR (CDCl₃) δ 1.25 (6H, br s), 1.32 (6H, s), 2.09 (3H, s), 2.33 (2H, s), 2.69 (2H, br s), 3.92 (3H, s), 6.60 (1H, s), 7.07 (1H, d, J = 7.8 Hz), 7.28-7.38 (2H, m), 7.50 (1H, d, J = 7.8 Hz), 7.62 (1H, s), 7.70-7.77 (2H, m), 7.89 (1H, s), 8.30 (1H, br s), 8.43 (1H, br s).

### Reference Example 110

### 3-(Acetylamino)-N-[3-(6-ethoxy-3,4,8,9-tetrahydro-3,3,8,8-tetramethylfuro[2,3-h]isoquinolin-1-yl)phenyl]benzamide

To a solution of 3-(6-ethoxy-3,4,8,9-tetrahydro-3,3,8,8-tetramethylfuro[2,3-h]isoquinolin-1-yl)benzenamine dihydrochloride (438 mg, 1.00 mmol), 3-(acetamido)benzoic acid (198 mg, 1.11 mmol) and 1-hydroxy-1H-benzotriazole monohydrate (169 mg, 1.10 mmol) in N,N-dimethylformamide (1.5 ml) were added triethylamine (0.51 ml, 3.7 mmol) and 1-ethyl-3-(3-dimethylaminopropyl)carbodiimide hydrochloride (250 mg, 1.30 mmol), and the mixture was stirred at room temperature for 40 hours. The reaction mixture was combined with water and a saturated aqueous solution of sodium hydrogen carbonate, and extracted twice with ethyl acetate. The combined organic layer was washed with water and a saturated aqueous solution of sodium chloride, dried through sodium sulfate-basic silica gel (eluting with ethyl acetate), and concentrated under reduced pressure. The residue was crystallized from ethyl acetate-diisopropyl ether to give the title compound (331 mg, yield 63%).
¹H NMR (CDCl₃) δ 1.25 (6H, br s), 1.32 (6H, s), 1.46 (3H, t, J = 7.0 Hz), 2.12 (3H, s), 2.31 (2H, s), 2.67 (2H, br s), 4.18 (2H, q, J = 7.0 Hz), 6.59 (1H, s), 7.06-7.11 (1H, m), 7.33 (1H, t, J = 8.0 Hz), 7.37 (1H, t, J = 8.0 Hz), 7.53 (1H, d, J = 8.1 Hz), 7.61 (1H, t, J = 1.8 Hz), 7.70-7.80 (2H, m), 7.92 (1H, s), 8.21 (1H, br s), 8.24 (1H, s).

### Reference Example 111

### Methyl [3-(3,4,8,9-Tetrahydro-6-methoxy-3,3,8,8-tetramethylfuro[2,3-h]isoquinolin-1-yl)phenoxy]acetate

To a solution of 3-(3,4,8,9-tetrahydro-6-methoxy-3,3,8,8-tetramethylfuro[2,3-h]isoquinolin-1-yl)phenol (479 mg, 1.36 mmol) and methyl bromoacetate (0.15 ml, 1.6 mmol) in N,N-dimethylformamide (3 ml) was added potassium carbonate (245 mg, 1.77 mmol), and the mixture was stirred at room temperature for 23 hours. The reaction mixture was combined with water, and extracted twice with ethyl acetate. The combined organic layer was washed twice with water, and concentrated under reduced pressure. The residue was purified with a silica gel column chromatography (hexane/ethyl acetate 2:1) to give the title compound (550 mg, yield 95%).
Amorphous.
¹H NMR (CDCl₃) δ 1.24 (6H, s), 1.32 (6H, s), 2.23 (2H, s), 2.69 (2H, s), 3.79 (3H, s), 3.92 (3H, s), 4.66 (2H, s), 6.60 (1H, s), 6.92 (1H, dd, J = 2.3, 1.4 Hz), 6.96 (1H, ddd, J = 8.2, 2.6, 1.0 Hz), 7.01 (1H, dt, J = 7.8, 1.2 Hz), 7.27-7.34 (1H, m).

### Reference Example 112

### [3-(3,4,8,9-Tetrahydro-6-methoxy-3,3,8,8-tetramethylfuro[2,3-h]isoquinolin-1-yl)phenoxy]acetic acid

To a solution of methyl [3-(3,4,8,9-tetrahydro-6-methoxy-3,3,8,8-tetramethylfuro[2,3-h]isoquinolin-1-yl)phenoxy]acetate (545 mg, 1.29 mmol) in methanol (3 ml) was added 5 M aqueous solution of sodium hydroxide (0.64 ml, 3.2 mmol), and the mixture was heated under reflux for 15 minutes. The reaction mixture was combined with 1 M hydrochloric acid (3.2 ml, 3.2 mmol) under ice-cooling, and the mixture was twice extracted with chloroform. The combined organic layer was washed with a saturated aqueous solution of sodium chloride, dried over sodium sulfate, filtered, concentrated under reduced pressure to give the title compound (0.52 g, yield 98%).
Amorphous.
¹H NMR (DMSO-d₆) δ 1.15 (6H, s), 1.22 (6H, s), 2.23 (2H, s), 2.66 (2H, s), 3.81 (3H, s), 4.72 (2H, s), 6.79-6.83 (2H, m), 6.93 (1H, d, J = 8.0 Hz), 7.00 (1H, dd, J = 8.0, 2.4 Hz), 7.33 (1H, t, J = 8.0 Hz).

### Reference Example 113

### N-Methyl-2-[3-(3,4,8,9-tetrahydro-6-methoxy-3,3,8,8-tetramethylfuro[2,3-h]isoquinolin-1-yl)phenoxy]acetamide

To a solution of [3-(3,4,8,9-tetrahydro-6-methoxy-3,3,8,8-tetramethylfuro[2,3-h]isoquinolin-1-yl)phenoxy]acetic acid (544 mg, 1.33 mmol), 40% methylamine/methanol a solution of (0.23 g, 3.0 mmol) and 1-hydroxy-1H-benzotriazole monohydrate (244 mg, 1.46 mmol) in N,N-dimethylformamide (4 ml) was added 1-ethyl-3-(3-dimethylaminopropyl)carbodiimide hydrochloride (331 mg, 1.73 mmol), and the mixture was stirred at room temperature for 44 hours. The reaction mixture was combined with water and a saturated aqueous solution of sodium hydrogen carbonate, and extracted twice with ethyl acetate. The combined organic layer was washed twice with water, and concentrated under reduced pressure. The residue was crystallized from ethyl acetate-diisopropyl ether to give the title compound (412 mg, yield 73%).
¹H NMR (CDCl₃) δ 1.24 (6H, br s), 1.32 (6H, s), 2.23 (2H, s), 2.69 (2H, s), 2.91 (3H, d, J = 5.1 Hz), 3.93 (3H, s), 4.53 (2H, s), 6.55-6.68 (1H, m), 6.61 (1H, s), 6.92 (1H, ddd, J = 8.2, 2.7, 0.9 Hz), 6.99-7.04 (2H, m), 7.32 (1H, t, J = 8.2 Hz).

### Reference Example 114

### Methyl 2-bromo-4-(6-ethoxy-3,4,8,9-tetrahydro-3,3,8,8-tetramethylfuro[2,3-h]isoquinolin-1-yl)benzoate

With methyl 2-bromo-4-cyanobenzoate, the title compound was obtained by the method similar to that in Reference Example 29. Yield 37%.
Amorphous.
¹H NMR (CDCl₃) δ 1.24 (6H, s), 1.34 (6H, s), 1.46 (3H, t, J = 7.0 Hz), 2.24 (2H, s), 2.66 (2H, s), 3.95 (3H, s), 4.18 (2H, q, J = 7.0 Hz), 6.61 (1H, s), 7.41 (1H, dd, J = 1.2, 8.0 Hz), 7.73 (1H, d, J = 1.2 Hz), 7.84 (1H, d, J = 8.0 Hz).

### Reference Example 115

### Methyl 2-bromo-4-(3,4,8,9-tetrahydro-6-methoxy-3,3,8,8-tetramethylfuro[2,3-h]isoquinolin-1-yl)benzoate

From 2,3-dihydro-7-methoxy-2,2-dimethyl-a-(1-methylethyl)-5-benzofuranmethanol and methyl 2-bromo-4-cyanobenzoate, the title compound was obtained by the method similar to that in Reference Example 29. Yield 31%.
Amorphous.
¹H NMR (CDCl₃) δ 1.24 (6H, s), 1.35 (6H, s), 2.26 (2H, s), 2.68 (2H, s), 3.93 (3H, s), 3.96 (3H, s), 6.62 (1H, s), 7.41 (1H, d, J = 8.1 Hz), 7.74 (1H, d, J = 1.2 Hz), 7.85 (1H, dd, J = 1.2, 8.1 Hz).

### Reference Example 116

### Methyl 4-(aminocarbonyl)-2-nitrobenzoate

A solution of methyl 2-nitroterephthalate (50 g) in 1-methyl-2-pyrrolidone (150 ml) was cooled to 10°C, and thionyl chloride (39.6 g) was added dropwise thereto at 10°C. The mixture was stirred at 10°C for 1 hour to prepare a solution of acid chloride. Separately, a mixed solution of 25% ammonia water (350 ml) and toluene (250 ml) was cooled to -10°C. The previously obtained solution of acid chloride was added dropwise thereto -at 10°C.
The solution of acid chloride was washed well with 1-methyl-2-pyrrolidone (50 ml), while keeping the temperature at -10°C. The mixture was stirred at -10°C for 30 minutes, and the temperature was elevated to 0°C. Then, water (250 ml) was added dropwise thereto at 0 to 10°C. The mixture was stirred at 0 to 10°C for 2 hours, and the precipitated crystals were taken by filtration, and washed with water (500 ml) to give the title compound (45.16 g, yield 91%).
¹H NMR (DMSO-d₆) δ 3.86 (3H, s), 7.81 (1H, s), 7.94 (1H, d, J = 7.9 Hz), 8.26 (1H, d, J = 7.9 Hz), 8.35 (1H, s), 8.47 (1H, s).

### Reference Example 117

### Methyl 4-(aminocarbonyl)-2-(ethylamino)benzoate

To a solution of methyl 4-(aminocarbonyl)-2-nitrobenzoate methyl (20 g) in tetrahydrofuran (200 ml), methanol (200 ml) and acetic acid (100 ml) was added 90% acetaldehyde (13.1 g). Then, 5% palladium carbon (hydrate, 2 g) was added thereto. The reaction was conducted under 0.8 MPa of hydrogen pressure at 45 to 55°C for 1 hour. The catalyst was filtered off while keeping the temperature of the reaction solution at 45 to 55°C. The crystals were washed with tetrahydrofuran/methanol=1/1 (100 ml). The mixture was concentrated under reduced pressure to the volume of 200 ml. Water (200 ml) was added dropwise thereto at 20 to 30°C. The mixture was stirred at 20 to 30°C for 30 minutes, cooled, and stirred for 1 hour at 0 to 10°C. The precipitated crystals were taken by filtration, and washed with water (200 ml) to give the title compound (18.58 g, yield 94%).
¹H NMR (CDCl₃) δ 1.32 (3H, t, J = 7.2 Hz), 3.25-3.34 (2H, m), 3.88 (3H, s), 5.75 (1H, br), 6.08 (1H, br), 6.83 (1H, dd, J = 1.6, 8.3 Hz), 7.17 (1H, d, J = 1.6 Hz), 7.68 (1H, br), 7.94 (1H, d, J = 8.3 Hz).

### Reference Example 118

### Methyl 4-cyano-2-(ethylamino)benzoate

Methyl 4-(aminocarbonyl)-2-(ethylamino)benzoate (71.82 g) was suspended in pyridine (718 ml). Phosphorus oxychloride (54.5 g) was added dropwise thereto while keeping the temperature at 20 to 30°C. The mixture was stirred for 1 hour at 20 to 30°C. The mixture was cooled to 0 to 10°C, and water (1436 ml) which was cooled to 0 to 10°C, was added dropwise thereto while keeping the temperature at 0 to 10°C. The mixture was stirred at 0 to 10°C for 2 hours, the crystals were taken by filtration, and washed with water (719 ml) to give the title compound (61.5 g, yield 93%).
¹H NMR (CDCl₃) δ 1.34 (3H, t, J = 7.1 Hz), 3.18-3.27 (2H, m), 3.89 (3H, s), 6.79 (1H, dd, J = 1.4, 8.2 Hz), 6.91 (1H, s), 7.76 (1H, br), 7.94 (1H, d, J = 8.2 Hz).

### Reference Example 119

### Methyl 4-cyano-2-pyrrolidinylbenzeneacetate

To a mixed solution of methyl 2-amino-4-cyanobenzeneacetate (478 mg, 2.51 mmol), 2,5-dimethoxytetrahydrofuran (468 mg, 3.54 mmol) and sulfuric acid (2.8 ml) in methanol (3 ml) and tetrahydrofuran (7 ml) was added portionwise sodium borohydride (485 mg, 12.8 mmol). The reaction mixture solution was stirred at room temperature for 14 hours. The reaction mixture was combined with water, and extracted with ethyl acetate. The extracts were washed with a saturated aqueous solution of sodium chloride, dried, and concentrated under reduced pressure. The residue was purified with a silica gel column chromatography (ethyl acetate/hexane 3:1) to give the title compound (279 mg, yield 45%).
¹H NMR (CDCl₃) δ 1.90-1.98 (4H, m), 3.12-3.18 (4H, m), 3.70 (3H, s), 3.73 (2H, s), 7.14-7.24 (3H, m).

### Example 1

### Methyl 2-amino-4-(6-ethoxy-3,4,8,9-tetrahydro-3,3,8,8-tetramethylfuro[2,3-h]isoquinolin-1-yl)benzoate

To a solution of 7-ethoxy-2,3-dihydro-2,2-dimethyl-a-(1-methylethyl)-5-benzofuranmethanol (11.3 g, 42.9 mmol) and methyl 2-amino-4-cyanobenzoate (6.30 g, 35.8 mmol) in toluene (40 ml) and acetic acid (25 ml) was added conc. sulfuric acid (5.92 ml, 111 mmol) under ice-cooling, and the mixture was stirred for 2 hours at 80°C. Methanol (18 ml) was added dropwise thereto, and the mixture was stirred for 30 minutes at 65°C. After cooling, the reaction mixture was combined with ice water, neutralized with sodium hydrogen carbonate, and extracted three times with ethyl acetate. The combined organic layer was extracted three times with 1 M hydrochloric acid, and the combined aqueous layer was neutralized with sodium hydrogen carbonate, and extracted twice with ethyl acetate. The combined organic layer was washed with a saturated aqueous solution of sodium chloride, dried over sodium sulfate, filtered, and concentrated under reduced pressure. To the residue was added ethyl acetate, and the precipitated crystals were removed. The mother liquor was concentrated under reduced pressure, and the residue was purified with a basic silica gel column chromatography (hexane/ethyl acetate, 5:1 followed by 3:1) to give the title compound (7.33 g, yield 49%).
Amorphous.
¹H NMR (CDCl₃) δ 1.24 (6H, s), 1.33 (6H, s), 1.46 (3H, t, J = 7.0 Hz), 2.36 (2H, s), 2.66 (2H, s), 3.89 (3H, s), 4.18 (2H, q, J = 7.0 Hz), 5.76 (2H, s), 6.59 (1H, s), 6.62 (1H, dd, J = 7.8, 1.5 Hz), 6.73 (1H, d, J = 1.5 Hz), 7.85 (1H, d, J = 7.8 Hz).

### Example 2

### Methyl 2-amino-4-(6-ethoxy-3,4,8,9-tetrahydro-3,3,8,8-tetramethylfuro[2,3-h]isoquinolin-1-yl)benzoate hydrochloride

Methyl 2-amino-4-(6-ethoxy-3,4,8,9-tetrahydro-3,3,8,8-tetramethylfuro[2,3-h]isoquinolin-1-yl)benzoate (7.33 g, 17.3 mmol) was dissolved in ethyl acetate, and a solution of 4 M hydrogen chloride/ethyl acetate (8.7 ml) was added thereto. The mixture was concentrated under reduced pressure, and the residue was crystallized from ethanol-ethyl acetate to give the title compound (7.16 g, yield 90%).
¹H NMR (DMSO-d₆) δ 1.27 (6H, s), 1.37 (3H, t, J = 7.0 Hz), 1.43 (6H, s), 2.39 (2H, s), 3.15 (2H, s), 3.85 (3H, s), 4.24 (2H, q, J = 7.0 Hz), 6.90 (1H, d, J = 8.2, 1.4 Hz), 6.97 (1H, d, J = 1.4 Hz), 7.08 (1H, s), 7.91 (1H, d, J = 8.2 Hz).

### Example 3

### Methyl 4-(6-ethoxy-3,4,8,9-tetrahydro-3,3,8,8-tetramethylfuro[2,3-h]isoquinolin-1-yl)-2-[(trifluoroacetyl)amino]benzoate

To a solution of methyl 2-amino-4-(6-ethoxy-3,4,8,9-tetrahydro-3,3,8,8-tetramethylfuro[2,3-h]isoquinolin-1-yl)benzoate (5.54 g, 13.1 mmol) in tetrahydrofuran (50 ml) were added triethylamine (2.01 ml, 14.4 mmol) and trifluoroacetic anhydride (1.94 ml, 13.8 mmol) under ice-cooling, and the mixture was stirred for 1 hour at room temperature. The reaction mixture was combined with ice water, and extracted twice with ethyl acetate. The combined organic layer was washed with a saturated aqueous solution of sodium chloride, dried over sodium sulfate, filtered, and concentrated under reduced pressure. The residue was crystallized from diisopropyl ether to give the title compound (4.14 g, yield 61%). The mother liquor was concentrated under reduced pressure, and again crystallized from diisopropyl ether to give the title compound (590 mg, yield 8.7%). Further, the mother liquor was concentrated under reduced pressure, and crystallized from hexane to give the title compound (1.08 g, yield 16%).
¹H NMR (CDCl₃) δ 1.32 (12H, s), 1.47 (3H, t, J = 7.2 Hz), 2.29 (2H, s), 2.74 (2H, s), 4.01 (3H, s), 4.21 (2H, q, J = 7.2 Hz), 6.63 (1H, s), 7.36 (1H, dd, J = 8.0, 1.4 Hz), 8.15 (1H, d, J = 8.0 Hz), 8.70 (1H, d, J = 1.4 Hz), 12.28 (1H, s).

### Example 4

### Methyl 4-(6-ethoxy-3,4,8,9-tetrahydro-3,3,8,8-tetramethylfuro[2,3-h]isoquinolin-1-yl)-2-[(phenylmethyl)(trifluoroacetyl)amino]benzoate hydrochloride

To a solution of methyl 4-(6-ethoxy-3,4,8,9-tetrahydro-3,3,8,8-tetramethylfuro[2,3-h]isoquinolin-1-yl)-2-[(trifluoroacetyl)amino]benzoate (104 mg, 0.201 mmol) in N,N-dimethylformamide (1 ml) were added potassium tert-butoxide (25 mg, 0.221 mmol) and benzyl bromide (0.026 ml, 0.221 mmol) under ice-cooling, and the mixture was stirred at room temperature for 3 hours. The reaction mixture was combined with ice water, and extracted twice with ethyl acetate. The combined organic layer was washed with water and a saturated aqueous solution of sodium chloride, dried over sodium sulfate, filtered, and concentrated under reduced pressure. The residue was purified with a silica gel column chromatography (hexane/ethyl acetate, 5:1 followed by 3:1) to give the title compound as free base. This was dissolved in ethyl acetate and a solution of 4 M hydrogen chloride/ethyl acetate was added thereto. The mixture was concentrated under reduced pressure, and the residue was crystallized from ethyl acetate-diisopropyl ether to give the title compound (73 mg, yield 56%).
¹H NMR (DMSO-d₆) δ 1.20 (6H, s), 1.37 (3H, t, J = 6.8 Hz), 1.44 (3H, s), 1.54 (3H, s), 2.13 (2H, s), 3.07 (1H, d, J = 19.5 Hz), 3.23 (1H, d, J = 19.5 Hz), 3.51 (3H, s), 4.24 (2H, q, J = 6.8 Hz), 4.61-4.75 (1H, br), 5.15-5.30 (1H, br), 7.08 (1H, s), 7.10-7.20 (2H, m), 7.27-7.29 (3H, m), 7.78-8.07 (3H, m).

### Example 5

### Methyl 4-(6-ethoxy-3,4,8,9-tetrahydro-3,3,8,8-tetramethylfuro[2,3-h]isoquinolin-1-yl)-2-[(phenylmethyl)amino]benzoate

To a solution of methyl 4-(6-ethoxy-3,4,8,9-tetrahydro-3,3,8,8-tetramethylfuro[2,3-h]isoquinolin-1-yl)-2-[(phenylmethyl)(trifluoroacetyl)amino]benzoate (1.11 g, 1.82 mmol) in methanol (6 ml) was added potassium carbonate (504 mg, 3.65 mmol), and the mixture was stirred for 1 hour at 65°C. After cooling, methanol was distilled off under reduced pressure, and the residue was combined with water, and extracted twice with ethyl acetate. The combined organic layer was washed with water and a saturated aqueous solution of sodium chloride, dried over sodium sulfate, filtered, and concentrated under reduced pressure. The residue was purified with a basic silica gel column chromatography (hexane/ethyl acetate, 10:1 followed by 5:1) to give the title compound (697 mg, yield 75%).
Amorphous.
¹H NMR (CDCl₃) δ 1.23 (6H, s), 1.33 (6H, s), 1.46 (3H, t, J = 7.0 Hz), 2.25 (2H, s), 2.65 (2H, s), 3.87 (3H, s), 4.17 (2H, q, J = 7.0 Hz), 4.44 (2H, d, J = 5.4 Hz), 6.57-6.60 (2H, m), 6.72 (1H, s), 7.25-7.34 (5H, m), 7.92 (1H, d, J = 8.1 Hz), 8.13 (1H, t, J = 5.4 Hz).

### Example 6

### 4-(6-Ethoxy-3,4,8,9-tetrahydro-3,3,8,8-tetramethylfuro[2,3-h]isoquinolin-1-yl)-2-[(phenylmethyl)amino]benzoic acid

To a solution of methyl 4-(6-ethoxy-3,4,8,9-tetrahydro-3,3,8,8-tetramethylfuro[2,3-h]isoquinolin-1-yl)-2-[(phenylmethyl)amino]benzoate (586 mg, 1.14 mmol) in methanol (5 ml) was added 5 M aqueous solution (1 ml) of sodium hydroxide, and the mixture was stirred at room temperature for 1.5 hours and at 60°C for 2.5 hours. Methanol was distilled off under reduced pressure, and the residue was neutralized with 5 M hydrochloric acid, and extracted twice with ethyl acetate. The combined organic layer was washed with a saturated aqueous solution of sodium chloride, dried over sodium sulfate, and concentrated under reduced pressure. The residue was purified with a silica gel column chromatography (hexane/ethyl acetate 1:2 followed by ethyl acetate), and recrystallized from ethyl acetate-diethyl ether-diisopropyl ether to give the title compound (159 mg, yield 28%).
¹H NMR (CDCl₃) δ 1.25 (6H, s), 1.47 (6H, s), 1.47 (3H, t, J = 7.0 Hz), 2.20 (2H, br s), 2.83 (2H, s), 4.20 (2H, q, J = 7.0 Hz), 4.29 (2H, s), 6.39 (1H, d, J = 8.0 Hz), 6.50 (1H, s), 6.60 (1H, s), 7.15-7.25 (5H, m), 7.79 (1H, d, J = 8.0 Hz), 8.40-9.00 (1H, br).

### Example 7

### Ethyl 4-(6-ethoxy-3,4,8,9-tetrahydro-3,3,8,8-tetramethylfuro[2,3-h]isoquinolin-1-yl)-2-[(phenylmethyl)amino]benzoate

To ethanol (2 ml) was added dropwise thionyl chloride (0.095 ml, 1.30 mmol) under ice-cooling, and the mixture was stirred at the same temperature for 10 minutes. 4-(6-ethoxy-3,4,8,9-tetrahydro-3,3,8,8-tetramethylfuro[2,3-h]isoquinolin-1-yl)-2-[(phenylmethyl)amino]benzoic acid (180 mg, 0.361 mmol) was added thereto, and the mixture was stirred at 80°C for 36 hours. After cooling, ethanol was distilled off under reduced pressure, and the residue was combined with water, and neutralized with 1 M aqueous solution of sodium hydroxide. The mixture was extracted twice with ethyl acetate, the combined organic layer was washed with a saturated aqueous solution of sodium chloride, dried over sodium sulfate, filtered, and concentrated under reduced pressure. The residue was purified with a basic silica gel column chromatography (hexane/ethyl acetate, 10:1), and crystallized from diisopropyl ether-hexane to give the title compound (84 mg, yield 44%).
¹H NMR (CDCl₃) δ 1.23 (6H, s), 1.30 (6H, s), 1.36-1.49 (6H, m), 2.26 (2H, s), 2.66 (2H, s), 4.17 (2H, q, J = 7.0 Hz), 4.32 (2H, q, J = 7.0 Hz), 4.43 (2H, d, J = 5.4 Hz), 6.56-6.59 (2H, m), 6.71 (1H, s), 7.25-7.35 (5H, m), 7.93 (1H, d, J = 8.2 Hz), 8.14 (1H, t, J = 5.4 Hz).

### Example 8

### Ethyl 2-[acetyl(phenylmethyl)amino]-4-(6-ethoxy-3,4,8,9-tetrahydro-3,3,8,8-tetramethylfuro[2,3-h]isoquinolin-1-yl)benzoate

To a solution of ethyl 4-(6-ethoxy-3,3,8,8-tetramethyl-3,4,8,9-tetrahydrofuro[2,3-h]isoquinolin-1-yl)-2-[(phenylmethyl)amino]benzoate (610 mg, 1.16 mmol) in tetrahydrofuran (6 ml) were added triethylamine (0.18 ml, 1.27 mmol) and acetyl chloride (0.091 ml, 1.27 mmol) under ice-cooling, and the mixture was stirred at room temperature for 2 hours and at 60°C for 2 hours. After cooling, sodium hydride (66% dispersion in oil) (46 mg, 1.27 mmol) and acetyl chloride (0.091 ml, 1.27 mmol) were added thereto, and the mixture was stirred at room temperature for 15 hours. The reaction mixture was combined with ice water, and extracted twice with ethyl acetate. The combined organic layer was washed with a saturated aqueous solution of sodium chloride, dried over sodium sulfate, filtered, and concentrated under reduced pressure. The residue was purified with a silica gel column chromatography (hexane/ethyl acetate, 5:1 followed by 3:1), and crystallized from diisopropyl ether-hexane to give the title compound (283 mg, yield 43%).
¹H NMR (CDCl₃) δ 1.17 (3H, s), 1.25 (3H, s), 1.30 (6H, s), 1.30 (3H, t, J = 7.4 Hz), 1.46 (3H, t, J = 7.0 Hz), 1.89 (3H, s), 2.15 (2H, s), 2.65 (2H, s), 4.10 (2H, q, J = 7.0 Hz), 4.19 (2H, q, J = 7.4 Hz), 4.64 (1H, d, J = 14.2 Hz), 4.96 (1H, d, J = 14.2 Hz), 6.60 (1H, s), 7.04 (1H, d, J = 1.6 Hz), 7.20-7.30 (5H, m), 7.45 (1H, dd, J = 8.0, 1.6 Hz), 7.96 (1H, d, J = 8.0 Hz).

### Example 9

### 2-[Acetyl(phenylmethyl)amino]-4-(6-ethoxy-3,4,8,9-tetrahydro-3,3,8,8-tetramethylfuro[2,3-h]isoquinolin-1-yl)benzoic acid

To a solution of ethyl 2-[acetyl(phenylmethyl)amino]-4-(6-ethoxy-3,4,8,9-tetrahydro-3,3,8,8-tetramethylfuro[2,3-h]isoquinolin-1-yl)benzoate (369 mg, 0.649 mmol) in methanol (3 ml) was added 5 M aqueous solution (1 ml) of sodium hydroxide, and the mixture was stirred at room temperature for 3 hours. Methanol was distilled off under reduced pressure, and the residue was neutralized with 5 M hydrochloric acid. The precipitated crystals were taken by filtration, and washed with water and diethyl ether to give the title compound (183 mg, yield 52%). The filtrate was concentrated under reduced pressure, and to the residue water were added ethyl acetate and tetrahydrofuran. The aqueous layer was separated, and extracted three times with tetrahydrofuran-ethyl acetate. The combined organic layer was washed with a saturated aqueous solution of sodium chloride, dried over sodium sulfate, filtered, and concentrated under reduced pressure to give the title compound (148 mg, yield 42%).
¹H NMR (CDCl₃) δ 1.22 (3H, s), 1.26 (6H, s), 1.41 (3H, s), 1.46 (3H, t, J = 7.0 Hz), 1.70 (2H, s), 2.63-2.90 (5H, m), 4.04 (1H, d, J = 13.8 Hz), 4.19 (2H, q, J = 7.0 Hz), 5.58 (1H, d, J = 13.8 Hz), 6.59 (1H, s), 6.80 (1H, br s), 7.19 (5H, s), 7.43 (1H, d, J = 7.9 Hz), 7.99 (1H, d, J = 7.9 Hz).

### Example 10

### 7-(6-Ethoxy-3,4,8,9-tetrahydro-3,3,8,8-tetramethylfuro[2,3-h]isoquinolin-1-yl)-2-methyl-1-(phenylmethyl)-4(1H)-quinazolinone

To a solution of 2-[acetyl(phenylmethyl)amino]-4-(6-ethoxy-3,4,8,9-tetrahydro-3,3,8,8-tetramethylfuro[2,3-h]isoquinolin-1-yl)benzoic acid (221 mg, 0.409 mmol), 1-hydroxy-1H-benzotriazole·ammonium salt (68 mg, 0.450 mmol) and triethylamine (0.063 ml, 0.450 mmol) in N,N-dimethylformamide (2 ml) was added 1-ethyl-3-(3-dimethylaminopropyl)carbodiimide hydrochloride (86 mg, 0.450 mmol), and the mixture was stirred at room temperature for 15 hours. The reaction mixture was combined with ice water, and extracted twice with ethyl acetate. The combined organic layer was washed with water and a saturated aqueous solution of sodium chloride, dried over sodium sulfate, filtered, and concentrated under reduced pressure. The residue was purified with a basic silica gel column chromatography (hexane/ethyl acetate 1:2 followed by ethyl acetate), and crystallized from ethyl acetate-diisopropyl ether to give the title compound (132 mg, yield 62%).
¹H NMR (CDCl₃) δ 1.22 (12H, s), 1.44 (3H, t, J = 7.0 Hz), 1.85 (2H, br s), 2.65 (5H, s), 4.16 (2H, q, J = 7.0 Hz), 5.42 (2H, s), 6.57 (1H, s), 7.05 (2H, dd, J = 8.0, 2.8 Hz), 7.30-7.35 (4H, m), 7.42 (1H, dd, J = 8.0, 1.0 Hz), 8.41 (1H, d, J = 8.0 Hz).

### Example 11

### Methyl 2-(benzoylamino)-4-(6-ethoxy-3,4,8,9-tetrahydro-3,3,8,8-tetramethylfuro[2,3-h]isoquinolin-1-yl)benzoate

To a suspension of methyl 2-amino-4-(6-ethoxy-3,4,8,9-tetrahydro-3,3,8,8-tetramethylfuro[2,3-h]isoquinolin-1-yl)benzoate hydrochloride (978 mg, 2.13 mmol) in tetrahydrofuran (10 ml) were added triethylamine (0.88 ml, 6.30 mmol) and benzoyl chloride (0.25 ml, 2.17 mmol), and the mixture was stirred for 1 hour at 70°C. After cooling, the reaction mixture was combined with ice water, neutralized with sodium hydrogen carbonate, and extracted twice with ethyl acetate. The combined organic layer was washed with a saturated aqueous solution of sodium chloride, dried over sodium sulfate, filtered, and concentrated under reduced pressure. The residue was crystallized from ethyl acetate-diisopropyl ether to give the title compound (593 mg, yield 53%).
¹H NMR (CDCl₃) δ 1.32 (6H, s), 1.36 (6H, s), 1.48 (3H, t, J = 7.0 Hz), 2.40 (2H, br s), 2.76 (2H, br s), 4.00 (3H, s), 4.21 (2H, q, J = 7.0 Hz), 6.63 (1H, s), 7.28-7.35 (1H, m), 7.50-7.60 (3H, m), 8.03 (2H, d, J = 8.4 Hz), 8.17 (1H, d, J = 8.4 Hz), 9.00 (1H, s), 12.07 (1H, s).

### Example 12

### 2-(Benzoylamino)-4-(6-ethoxy-3,4,8,9-tetrahydro-3,3,8,8-tetramethylfuro[2,3-h]isoquinolin-1-yl)benzoic acid

To a solution of methyl 2-(benzoylamino)-4-(6-ethoxy-3,4,8,9-tetrahydro-3,3,8,8-tetramethylfuro[2,3-h]isoquinolin-1-yl)benzoate (574 mg, 1.09 mmol) in methanol (5 ml) was added 5 M aqueous solution (2 ml) of sodium hydroxide, and the mixture was stirred for 1 hour at 60°C. After cooling, the residue was adjusted at pH 5.5 with 5 M hydrochloric acid, and methanol was distilled off under reduced pressure. The resultant crystals were washed with water and diethyl ether to give the title compound (517 mg, yield 93%).
¹H NMR (CDCl₃) δ 1.26 (6H, s), 1.50 (3H, t, J = 7.0 Hz), 1.67 (3H, s), 1.80 (3H, s), 2.19 (1H, d, J = 17.2 Hz), 2.44 (1H, d, J = 17.2 Hz), 3.00-3.20 (2H, m), 4.20 (2H, q, J = 7.0 Hz), 6.69 (1H, s), 6.8₂₋₆.87 (1H, m), 7.30-7.50 (3H, m), 7.70-7.82 (1H, m), 7.90-8.05 (2H, m), 8.79 (1H, s).

### Example 13

### Methyl 4-(6-ethoxy-3,4,8,9-tetrahydro-3,3,8,8-tetramethylfuro[2,3-h]isoquinolin-1-yl)-2-[(4-pyridinylcarbonyl)amino]benzoate

To a solution of methyl 2-amino-4-(6-ethoxy-3,4,8,9-tetrahydro-3,3,8,8-tetramethylfuro[2,3-h]isoquinolin-1-yl)benzoate hydrochloride (854 mg, 1.86 mmol) in N,N-dimethylformamide (8 ml) were added 4-(dimethylamino)pyridine (1.05 g, 8.60 mmol) and isonicotinic acid chloride hydrochloride (614 mg, 3.45 mmol), and the mixture was stirred at room temperature for 3 hours and at 50°C for 1 hour. Isonicotinic acid chloride hydrochloride (153 mg, 0.86 mmol) was further added thereto, and the mixture was stirred for 1 hour at 50°C. After cooling, the reaction mixture was combined with ice water, and extracted twice with ethyl acetate. The combined organic layer was washed with water and a saturated aqueous solution of sodium chloride, dried over sodium sulfate, filtered, and concentrated under reduced pressure. The resultant crystals were washed with diisopropyl ether to give the title compound (630 mg, yield 64%).
¹H NMR (CDCl₃) δ 1.32 (12H, s), 1.48 (3H, t, J = 7.0 Hz), 2.37 (2H, br s), 2.75 (2H, s), 4.02 (3H, s), 4.21 (2H, q, J = 7.0 Hz), 6.64 (1H, s), 7.30-7.35 (1H, m), 7.87-7.89 (2H, m), 8.18 (1H, d, J = 8.1 Hz), 8.85 (2H, d, J = 6.3 Hz), 8.95 (1H, d, J = 1.2 Hz), 12.27 (1H, s) .

### Example 14

### 4-(6-Ethoxy-3,4,8,9-tetrahydro-3,3,8,8-tetramethylfuro[2,3-h]isoquinolin-1-yl)-2-[(4-pyridinylcarbonyl)amino]benzoic acid

From methyl 4-(6-ethoxy-3,4,8,9-tetrahydro-3,3,8,8-tetramethylfuro[2,3-h]isoquinolin-1-yl)-2-[(4-pyridinylcarbonyl)amino]benzoate, the title compound was obtained by the method similar to that in Example 9. Yield 45%.
¹H NMR (CDCl₃+ DMSO-d₆ 4 drops) δ 1.28 (6H, s), 1.36 (6H, s), 1.47 (3H, t, J = 7.0 Hz), 2.32 (2H, s), 2.79 (2H, s), 4.21 (2H, q, J = 7.0 Hz), 6.65 (1H, s), 7.06 (1H, dd, J = 8.0, 1.2 Hz), 7.99 (2H, d, J = 6.0 Hz), 8.17 (1H, d, J = 8.0 Hz), 8.72 (2H, d, J = 6.0 Hz), 8.81 (1H, d, J = 1.2 Hz).

### Example 15

### Methyl 2-[[3-(acetylamino)benzoyl]amino]-4-(6-ethoxy-3,4,8,9-tetrahydro-3,3,8,8-tetramethylfuro[2,3-h]isoquinolin-1-yl)benzoate

To a solution of 3-(acetamido)benzoic acid (486 mg, 2.71 mmol) in toluene (2 ml) were added thionyl chloride (0.33 ml, 4.52 mmol) and N,N-dimethylformamide (1 drop), and the mixture was stirred for 1 hour at 80°C. After cooling, the solvent was distilled off under reduced pressure, and the residue was dissolved in N,N-dimethylformamide (6 ml). Methyl 2-amino-4-(6-ethoxy-3,4,8,9-tetrahydro-3,3,8,8-tetramethylfuro[2,3-h]isoquinolin-1-yl)benzoate hydrochloride (1.12 g, 2.44 mmol) and triethylamine (1.25 ml, 9.00 mmol) were added thereto, and the mixture was stirred at room temperature for 1 hour and at 60°C for 5 hours. To the obtained mixture were added triethylamine (0.47 ml, 3.39 mmol), and the acid chloride which was prepared with 3-(acetamido)benzoic acid (607 mg, 3.39 mmol), thionyl chloride (0.49 ml, 6.78 mmol) and toluene (2 ml) in the same method as described above, and the mixture was stirred at room temperature for 15 hours. The reaction mixture was combined with ice water, and extracted twice with ethyl acetate. The combined organic layer was washed with water and a saturated aqueous solution of sodium chloride, dried over sodium sulfate, filtered, and concentrated under reduced pressure. The residue was purified with a silica gel column chromatography (hexane/ethyl acetate, 3:1 followed by 1:1), and crystallized from ethyl acetate-diisopropyl ether to give the title compound (326 mg, yield 23%).
¹H NMR (CDCl₃) δ 1.26 (6H, s), 1.32 (6H, s), 1.47 (3H, t, J = 7.0 Hz), 2.20 (3H, s), 2.36 (2H, s), 2.68 (2H, s), 4.00 (3H, s), 4.19 (2H, q, J = 7.0 Hz), 6.61 (1H, s), 7.17 (1H, dd, J = 8.2, 1.4 Hz), 7.44-7.52 (2H, m), 7.74 (1H, d, J = 8.2 Hz), 7.90 (1H, s), 7.98-8.02 (1H, m), 8.10 (1H, d, J = 8.0 Hz), 8.96 (1H, s), 12.01 (1H, s).

### Example 16

### 2-[[3-(Acetylamino)benzoyl]amino]-4-(6-ethoxy-3,4,8,9-tetrahydro-3,3,8,8-tetramethylfuro[2,3-h]isoquinolin-1-yl)benzoic acid

To a suspension of methyl 2-[[3-(acetylamino)benzoyl]amino]-4-(6-ethoxy-3,4,8,9-tetrahydro-3,3,8,8-tetramethylfuro[2,3-h]isoquinolin-1-yl)benzoate (175 mg, 0.300 mmol) in methanol (1 ml) was added 5 M aqueous solution of sodium hydroxide (0.16 ml, 0.80 mmol), and the mixture was heated under reflux for 30 minutes. The reaction mixture was cooled, and diluted with water. 1 M hydrochloric acid (0.80 ml, 0.80 mmol) was added dropwise thereto, and the precipitated crystals were taken by filtration, and washed with water to give the title compound (159 mg, yield 93%).
¹H NMR (DMSO-d₆) δ 1.23 (6H, s), 1.25 (6H, br s), 1.35 (3H, t, J = 6.9 Hz), 2.08 (3H, s), 2.32-2.52 (2H, m), 2.80 (2H, br s), 4.15 (2H, q, J = 6.9 Hz), 6.90 (1H, s), 7.22 (1H, d, J = 8.1 Hz), 7.50 (1H, t, J = 8.0 Hz), 7.62 (1H, d, J = 7.8 Hz), 7.80 (1H, d, J = 8.1 Hz), 8.13 (1H, d, J = 8.1 Hz), 8.23 (1H, s), 8.76 (1H, s), 10.22 (1H, s).

### Example 17

### Methyl 2-(acetylamino)-4-(6-ethoxy-3,4,8,9-tetrahydro-3,3,8,8-tetramethylfuro[2,3-h]isoquinolin-1-yl)benzoate

To a solution of methyl 2-amino-4-(6-ethoxy-3,4,8,9-tetrahydro-3,3,8,8-tetramethylfuro[2,3-h]isoquinolin-1-yl)benzoate hydrochloride (1.20 g, 2.61 mmol) in N,N-dimethylformamide (8 ml) were added triethylamine (1.18 ml, 8.47 mmol) and acetyl chloride (0.19 ml, 2.66 mmol) under ice-cooling, and the mixture was stirred at room temperature for 1 hour and at 60°C for 3 hours. After cooling, triethylamine (0.91 ml, 6.53 mmol) and acetyl chloride (0.47 ml, 6.53 mmol) were added thereto, and the mixture was stirred at room temperature for 24 hours. The reaction mixture was combined with ice water, and extracted twice with ethyl acetate. The combined organic layer was washed with water and a saturated aqueous solution of sodium chloride, dried over sodium sulfate, filtered, and concentrated under reduced pressure. The residue was purified with a silica gel column chromatography (hexane/ethyl acetate, 3:1 followed by 1:1), and crystallized from diisopropyl ether-hexane to give the title compound (264 mg, yield 22%).
¹H NMR (CDCl₃) δ 1.24 (6H, s), 1.31 (6H, s), 1.46 (3H, t, J = 7.0 Hz), 2.22 (3H, s), 2.30 (2H, s), 2.66 (2H, s), 3.95 (3H, s), 4.18 (2H, q, J = 7.0 Hz), 6.59 (1H, s), 7.11 (1H, dd, J = 8.2, 1.4 Hz), 8.04 (1H, d, J = 8.2 Hz), 8.73 (1H, d, J = 1.4 Hz), 11.04 (1H, s).

### Example 18

### 2-(Acetylamino)-4-(6-ethoxy-3,4,8,9-tetrahydro-3,3,8,8-tetramethylfuro[2,3-h]isoquinolin-1-yl)benzoic acid

From methyl 2-(acetylamino)-4-(6-ethoxy-3,4,8,9-tetrahydro-3,3,8,8-tetramethylfuro[2,3-h]isoquinolin-1-yl)benzoate, the title compound was obtained by the method similar to that in Example 12. Yield 89%.
¹H NMR (CDCl₃) δ 1.27 (6H, s), 1.49 (3H, t, J = 7.0 Hz), 1. 62 (6H, s), 2.13 (3H, s), 2.14 (1H, d, J = 24.0 Hz), 2.38 (1H, d, J = 24.0 Hz), 2.99 (2H, s), 4.25 (2H, q, J = 7.0 Hz), 6.66 (1H, s), 6.80 (1H, d, J = 8.0 Hz), 7.67-7.90 (1H, br), 8.58 (1H, s), 9.70 (1H, br s).

### Example 19

### Methyl 4-(6-ethoxy-3,4,8,9-tetrahydro-3,3,8,8-tetramethylfuro[2,3-h]isoquinolin-1-yl)-2-[(methylsulfonyl)amino]benzoate

To a solution of methyl 2-amino-4-(6-ethoxy-3,4,8,9-tetrahydro-3,3,8,8-tetramethylfuro[2,3-h]isoquinolin-1-yl)benzoate hydrochloride (1.01 g, 2.20 mmol) in pyridine (4 ml) was added methanesulfonyl chloride (0.24 ml, 3.06 mmol), and the mixture was stirred at room temperature for 15 hours. After cooling, the reaction mixture was combined with ice water, and extracted twice with ethyl acetate. The combined organic layer was washed with water and a saturated aqueous solution of sodium chloride, dried over sodium sulfate, filtered, and concentrated under reduced pressure. The residue was purified with a basic silica gel column chromatography (hexane/ethyl acetate 5:1), and crystallized from diethyl ether-hexane to give the title compound (445 mg, yield 40%).
¹H NMR (CDCl₃) δ 1.24 (6H, s), 1.33 (6H, s), 1.46 (3H, t, J = 6.9 Hz), 2.26 (2H, s), 2.68 (2H, s), 3.09 (3H, s), 3.96 (3H, s), 4.18 (2H, q, J = 6.9 Hz), 6.60 (1H, s), 7.18 (1H, dd, J = 8.1, 1.2 Hz), 7.27 (1H, d, J = 1.2 Hz), 8.09 (1H, d, J = 8.1 Hz).

### Example 20

### 4-(6-Ethoxy-3,4,8,9-tetrahydro-3,3,8,8-tetramethylfuro[2,3-h]isoquinolin-1-yl)-2-[(methylsulfonyl)amino]benzoic acid

From methyl 4-(6-ethoxy-3,4,8,9-tetrahydro-3,3,8,8-tetramethylfuro[2,3-h]isoquinolin-1-yl)-2-[(methylsulfonyl)amino]benzoate, the title compound was obtained by the method similar to that in Example 12. Yield 93%.
¹H NMR (CDCl₃) δ 1.29 (3H, s), 1.32 (3H, s), 1.51 (3H, t, J = 7.0 Hz), 1.62 (3H, s), 1.74 (3H, s), 2.13 (1H, d, J = 17.0 Hz), 2.38 (1H, d, J = 17.0 Hz), 2.93 (3H, s), 2.99 (1H, d, J = 15.4 Hz), 3.18 (1H, d, J = 15.4 Hz), 4.27 (2H, q, J = 7.0 Hz), 6.70 (1H, s), 7.04 (1H, d, J = 7.7 Hz), 7.49 (1H, s), 7.87 (1H, d, J = 7.7 Hz).

### Example 21

### Methyl 4-(6-ethoxy-3,4,8,9-tetrahydro-3,3,8,8-tetramethylfuro[2,3-h]isoquinolin-1-yl)-2-[(2-pyridinylcarbonyl)amino]benzoate

To a solution of methyl 2-amino-4-(6-ethoxy-3,4,8,9-tetrahydro-3,3,8,8-tetramethylfuro[2,3-h]isoquinolin-1-yl)benzoate hydrochloride (813 mg, 1.77 mmol) in N,N-dimethylformamide (6 ml) were added 4-(dimethylamino)pyridine (1.10 g, 9.02 mmol) and picolinic acid chloride hydrochloride (730 mg, 4.10 mmol), and the mixture was stirred for 1 hour at room temperature. The reaction mixture was combined with ice water, and extracted twice with a mixed solution of ethyl acetate-tetrahydrofuran. The combined organic layer was washed with an aqueous solution of sodium chloride and a saturated aqueous solution of sodium chloride, dried over sodium sulfate, filtered, and concentrated under reduced pressure. The resultant crystals were washed with diisopropyl ether-hexane to give the title compound (738 mg, yield 79%).
¹H NMR (CDCl₃) δ 1.26 (6H, s), 1.30 (6H, s), 1.47 (3H, t, J = 7.0 Hz), 2.33 (2H, s), 2.69 (2H, s), 4.04 (3H, s), 4.19 (2H, q, J = 7.0 Hz), 6.60 (1H, s), 7.20 (1H, dd, J = 8.2, 1.6 Hz), 7.49 (1H, ddd, J = 7.8, 4.8, 1.2 Hz), 7.91 (1H, td, J = 7.8, 1.3 Hz), 8.13 (1H, d, J = 8.2 Hz), 8.26 (1H, dd, J = 7.8, 1.2 Hz), 8.79 (1H, dd, J = 4.8, 1.3 Hz), 9.05 (1H, d, J = 1.6 Hz).

### Example 22

### 4-(6-Ethoxy-3,4,8,9-tetrahydro-3,3,8,8-tetramethylfuro[2,3-h]isoquinolin-1-yl)-2-[(2-pyridinylcarbonyl)amino]benzoic acid hydrochloride

To a solution of methyl 4-(6-ethoxy-3,4,8,9-tetrahydro-3,3,8,8-tetramethylfuro[2,3-h]isoquinolin-1-yl)-2-[(2-pyridinylcarbonyl)amino]benzoate (444 mg, 0.842 mmol) in methanol (4 ml) was added 5 M aqueous solution (1 ml) of sodium hydroxide, and the mixture was stirred at room temperature for 2 hours. Methanol was distilled off under reduced pressure, and the residue was neutralized with 5 M hydrochloric acid, and extracted twice with ethyl acetate. The combined organic layer was washed with a saturated aqueous solution of sodium chloride, dried over sodium sulfate, filtered, and concentrated under reduced pressure. The residue was dissolved in ethyl acetate, and a solution of 4 M hydrogen chloride/ethyl acetate (0.64 ml) was added thereto. The mixture was concentrated under reduced pressure, and the residue was crystallized from ethanol-diisopropyl ether to give the title compound (432 mg, yield 93%).
¹H NMR (DMSO-d₆) δ 1.23 (6H, s), 1.38 (3H, t, J = 7.0 Hz), 1.44 (3H, s), 1.50 (3H, s), 2.30 (1H, d, J = 14.4 Hz), 2.44 (1H, d, J = 14.4 Hz), 3.13 (1H, d, J = 14.8 Hz), 3.27 (1H, d, J = 14.8 Hz), 4.26 (2H, q, J = 7.0 Hz), 7.11 (1H, s), 7.48 (1H, d, J = 8.2 Hz), 7.70-7.76 (1H, m), 8.07-8.20 (2H, m), 8.29 (1H, d, J = 8.2 Hz), 8.77 (1H, d, J = 4.4 Hz), 9.03 (1H, s).

### Example 23

### N-[2-(Aminocarbonyl)-5-(6-ethoxy-3,4,8,9-tetrahydro-3,3,8,8-tetramethylfuro[2,3-h]isoquinolin-1-yl)phenyl]-2-pyridinecarboxamide

To a solution of 4-(6-ethoxy-3,4,8,9-tetrahydro-3,3,8,8-tetramethylfuro[2,3-h]isoquinolin-1-yl)-2-[(2-pyridinylcarbonyl)amino]benzoic acid hydrochloride (228 mg, 0.415 mmol), 1-hydroxy-1H-benzotriazole·ammonium salt (70 mg, 0.539 mmol) and triethylamine (0.14 ml, 1.04 mmol) in N,N-dimethylformamide (2 ml) was added 1-ethyl-3-(3-dimethylaminopropyl)carbodiimide hydrochloride (103 mg, 0.539 mmol), and the mixture was stirred at room temperature for 2 hours and at 50°C for 4 hours. After cooling, the reaction mixture was combined with ice water, and extracted twice with ethyl acetate. The combined organic layer was washed with a saturated aqueous solution of sodium hydrogen carbonate, water and a saturated aqueous solution of sodium chloride, dried over sodium sulfate, filtered, and concentrated under reduced pressure. The residue was purified with a silica gel column chromatography (hexane/ethyl acetate 1:2 followed by ethyl acetate), and crystallized from ethyl acetate-hexane to give the title compound (141 mg, yield 66%).
¹H NMR (CDCl₃) δ 1.27 (6H, s), 1.29 (6H, s), 1.46 (3H, t, J = 7.0 Hz), 2.31 (2H, br s), 2.68 (2H, s), 4.18 (2H, q, J = 7.0 Hz), 6.59 (1H, s), 7.07 (1H, dd, J = 8.0, 1.8 Hz), 7.39 (1H, ddd, J = 7.5, 4.8, 1.2 Hz), 7.58 (1H, d, J = 8.0 Hz), 7.82 (1H, td, J = 7.5, 1.8 Hz), 8.19 (1H, ddd, J = 7.5, 1.2, 0.9 Hz), 8.65 (1H, ddd, J = 4.8, 1.8, 0.9 Hz), 8.96 (1H, d, J = 1.8 Hz).

### Example 24

### Methyl 4-(6-ethoxy-3,4,8,9-tetrahydro-3,3,8,8-tetramethylfuro[2,3-h]isoquinolin-1-yl)-2-[(3-pyridinylcarbonyl)amino]benzoate

With nicotinic acid chloride hydrochloride, the title compound was obtained by the method similar to that in Example 13. Yield 67%.
¹H NMR (CDCl₃) δ 1.26 (6H, s), 1.32 (6H, s), 1.47 (3H, t, J = 7.0 Hz), 2.35 (2H, s), 2.68 (2H, s), 4.00 (3H, s), 4.19 (2H, q, J = 7.0 Hz), 6.61 (1H, s), 7.20 (1H, dd, J = 8.2, 1.6 Hz), 7.47 (1H, dd, J = 8.0, 4.8 Hz), 8.12 (1H, d, J = 8.2 Hz), 8.32 (1H, dt, J = 8.0, 1.8 Hz), 8.80 (1H, dd, J = 4.8, 1.8 Hz), 8.96 (1H, d, J = 1.6 Hz), 9.29 (1H, d, J = 1.8 Hz), 12.21 (1H, s).

### Example 25

### 4-(6-Ethoxy-3,4,8,9-tetrahydro-3,3,8,8-tetramethylfuro[2,3-h]isoquinolin-1-yl)-2-[(3-pyridinylcarbonyl)amino]benzoic acid

From methyl 4-(6-ethoxy-3,4,8,9-tetrahydro-3,3,8,8-tetramethylfuro[2,3-h]isoquinolin-1-yl)-2-[(3-pyridinylcarbonyl)amino]benzoate, the title compound was obtained by the method similar to that in Example 9. Yield 90%.
¹H NMR (CDCl₃) δ 1.25 (6H, s), 1. 51 (3H, t, J = 6.9 Hz), 1.73 (3H, s), 1.80 (3H, s), 2.20 (1H, d, J = 16.6 Hz), 2.37 (1H, d, J = 16.6 Hz), 3.09 (1H, d, J = 16.6 Hz), 3.18 (1H, d, J = 16.6 Hz), 4.28 (2H, q, J = 6.9 Hz), 6.17 (1H, s), 6.87 (1H, d, J = 7.2 Hz), 7.30-7.38 (1H, m), 7.78-7.90 (1H, m), 8.10-8.25 (1H, m), 8.67-8.72 (2H, m), 9.05-9.13 (1H, m).

### Example 26

### Methyl 2-[(2-chloro-4-pyridinylcarbonyl)amino]-4-(6-ethoxy-3,4,8,9-tetrahydro-3,3,8,8-tetramethylfuro[2,3-h]isoquinolin-1-yl)benzoate

To a suspension of 2-chloroisonicotinic acid (678 mg, 4.30 mmol) in toluene (2 ml) were added thionyl chloride (0.38 ml, 5.16 mmol) and N,N-dimethylformamide (1 drop), and the mixture was stirred for 1 hour at 80°C. After cooling, the solvent was distilled off under reduced pressure, and the residue was dissolved in N,N-dimethylformamide (3 ml). Methyl 2-amino-4-(6-ethoxy-3,4,8,9-tetrahydro-3,3,8,8-tetramethylfuro[2,3-h]isoquinolin-1-yl)benzoate hydrochloride (852 mg, 1.86 mmol) and 4-(dimethylamino)pyridine (1.16 g, 9.46 mmol) were added thereto, and the mixture was stirred at room temperature for 2 hours. The reaction mixture was combined with ice water, and extracted twice with ethyl acetate. The combined organic layer was washed with water and a saturated aqueous solution of sodium chloride, dried over sodium sulfate, filtered, and concentrated under reduced pressure. The residue was purified with a silica gel column chromatography (hexane/ethyl acetate 5:1), and the obtained crystals were crystallized from diisopropyl ether-hexane to give the title compound (694 mg, yield 66%).
¹H NMR (CDCl₃) δ 1.26 (6H, s), 1.32 (6H, s), 1.47 (3H, t, J = 7.0 Hz), 2.33 (2H, s), 2.68 (2H, s), 4.00 (3H, s), 4.20 (2H, q, J = 7.0 Hz), 6.61 (1H, s), 7.24 (1H, d, J = 8.6 Hz), 7.78 (1H, d, J = 5.2 Hz), 7.92 (1H, s), 8.14 (1H, d, J = 8.6 Hz), 8.61 (1H, d, J = 5.2 Hz), 8.91 (1H, s), 12.28 (1H, s).

### Example 27

### 2-[(2-Chloro-4-pyridinylcarbonyl)amino]-4-(6-ethoxy-3,4,8,9-tetrahydro-3,3,8,8-tetramethylfuro[2,3-h]isoquinolin-1-yl)benzoic acid hydrochloride

To a solution of methyl 2-[(2-chloro-4-pyridinylcarbonyl)amino]-4-(6-ethoxy-3,4,8,9-tetrahydro-3,3,8,8-tetramethylfuro[2,3-h]isoquinolin-1-yl)benzoate (466 mg, 0.829 mmol) in methanol (4 ml) was added 5 M aqueous solution (1 ml) of sodium hydroxide, and the mixture was stirred at room temperature for 5 hours and at 50°C for 1 hour. After cooling, methanol was distilled off under reduced pressure, and the residue was neutralized with 5 M hydrochloric acid, and extracted twice with ethyl acetate. The combined organic layer was dried over sodium sulfate, and concentrated under reduced pressure. This was dissolved in ethyl acetate, and a solution of 4 M hydrogen chloride/ethyl acetate was added thereto. The mixture was concentrated under reduced pressure, and the residue was crystallized from ethyl acetate to give a mixture. To a suspension of 2-chloroisonicotinic acid (152 mg, 0.966 mmol) in toluene (1 ml) were added thionyl chloride (0.078 ml, 1.06 mmol) and N,N-dimethylformamide (1 drop), and the mixture was stirred for 1 hour at 80°C. After cooling, the solvent was distilled off under reduced pressure, and the residue was dissolved in N,N-dimethylformamide (1 ml). Previously obtained crystals and 4-(dimethylamino)pyridine (177 mg, 1.45 mmol) were added thereto under ice-cooling, and the mixture was stirred for 1 hour at room temperature. The reaction mixture was combined with ice water, neutralized with sodium hydrogen carbonate, and extracted twice with ethyl acetate. The combined organic layer was washed with water and a saturated aqueous solution of sodium chloride, dried over sodium sulfate, put through silica gel layer, and concentrated under reduced pressure. The residue was dissolved in methanol (1 ml), 5 M aqueous solution (0.5 ml) of sodium hydroxide was added thereto, and the mixture was stirred for 1 hour at room temperature. Methanol was distilled off under reduced pressure. The residue was combined with ethyl acetate, neutralized with 5 M hydrochloric acid, and concentrated under reduced pressure. To the residue was added ethanol, the insolubles were taken by filtration, and the filtrate was concentrated under reduced pressure. This procedure was repeated twice, and the residue was crystallized from ethanol-ethyl acetate to give the title compound (142 mg, yield 29%).
¹H NMR (DMSO-d₆) δ 1.24 (6H, s), 1.38 (3H, t, J = 7.0 Hz), 1.44 (6H, s), 2.30 (1H, d, J = 14.0 Hz), 2.45 (1H, d, J = 14.0 Hz), 3.14 (2H, s), 4.24 (2H, q, J = 7.0 Hz), 7.08 (1H, s), 7.51 (1H, d, J = 8.0 Hz), 7.86 (1H, d, J = 5.6 Hz), 7.94 (1H, s), 8.24 (1H, d, J = 8.0 Hz), 8.59 (1H, s), 8.70 (1H, d, J = 5.6 Hz), 12.36 (1H, s).

### Example 28

### Methyl 2-[(2,6-dichloro-4-pyridinylcarbonyl)amino]-4-(6-ethoxy-3,4,8,9-tetrahydro-3,3,8,8-tetramethylfuro[2,3-h]isoquinolin-1-yl)benzoate

With 2,6-dichloro-4-pyridine carboxylic acid, the title compound was obtained by the method similar to that in Example 26. Yield 46%.
¹H NMR (CDCl₃) δ 1.26 (6H, s), 1.32 (6H, s), 1.47 (3H, t, J = 7.0 Hz), 2.32 (2H, s), 2.68 (2H, s), 4.03 (3H, s), 4.19 (2H, q, J = 7.0 Hz), 6.61 (1H, s), 7.25 (1H, dd, J = 8.0, 1.6 Hz), 7.82 (2H, s), 8.14 (1H, d, J = 8.0 Hz), 8.86 (1H, d, J = 1.6 Hz), 12.29 (1H, s).

### Example 29

### Methyl 4-(6-ethoxy-3,4,8,9-tetrahydro-3,3,8,8-tetramethylfuro[2,3-h]isoquinolin-1-yl)-2-[(2-quinolinylcarbonyl)amino]benzoate

To a solution of 2-quinolinecarboxylic acid (656 mg, 3.79 mmol) in toluene (3 ml) were added thionyl chloride (0.41 ml, 5.67 mmol) and N,N-dimethylformamide (1 drop), and the mixture was stirred for 30 minutes at 80°C. After cooling, the solvent was distilled off under reduced pressure, and the residue was dissolved in N,N-dimethylformamide (6 ml). Methyl 2-amino-4-(6-ethoxy-3,4,8,9-tetrahydro-3,3,8,8-tetramethylfuro[2,3-h]isoquinolin-1-yl)benzoate (800 mg, 1.89 mmol) and 4-(dimethylamino)pyridine (924 mg, 7.56 mmol) were added thereto, and the mixture was stirred for 1.5 hours at room temperature.
The reaction mixture was combined with ice water, and extracted twice with ethyl acetate. The combined organic layer was washed with water and a saturated aqueous solution of sodium chloride, dried over sodium sulfate, filtered, and concentrated under reduced pressure. The residue was purified with a silica gel column chromatography (hexane/ethyl acetate, 3:1 followed by 2:1), and the obtained crystals were washed with hexane to give the title compound (831 mg, yield 76%).
¹H NMR (CDCl₃) δ 1.27 (6H, s), 1.30 (6H, s), 1.47 (3H, t, J = 7.0 Hz), 2.34 (2H, s), 2.69 (2H, s), 4.09 (3H, s), 4.20 (2H, q, J = 7.0 Hz), 6.61 (1H, s), 7.21 (1H, dd, J = 8.0, 1.4 Hz), 7.62-7.70 (1H, m), 7.80-7.93 (2H, m), 8.16 (1H, d, J = 8.0 Hz), 8.3₃₋₈.35 (3H, m), 9.09 (1H, d, J = 1.8 Hz).

### Example 30

### 4-(6-Ethoxy-3,4,8,9-tetrahydro-3,3,8,8-tetramethylfuro[2,3-h]isoquinolin-1-yl)-2-[(2-quinolinylcarbonyl)amino]benzoic acid

To a solution of methyl 4-(6-ethoxy-3,4,8,9-tetrahydro-3,3,8,8-tetramethylfuro[2,3-h]isoquinolin-1-yl)-2-[(2-quinolinylcarbonyl)amino]benzoate (563 mg, 0.975 mmol) in methanol (4 ml) was added 5 M aqueous solution (0.39 ml) of sodium hydroxide, and the mixture was stirred at room temperature for 1.5 hours and at 55°C for 3 hours. After cooling, methanol was distilled off under reduced pressure, and the residue was combined with water and acetone, and neutralized with 5 M hydrochloric acid. The precipitated crystals were washed with water and diethyl ether to give the title compound (481 mg, yield 88%).
¹H NMR (CDCl₃) δ 1.22 (6H, s), 1.50 (3H, t, J = 7.0 Hz), 1.75 (6H, s), 2.17 (1H, d, J = 17.7 Hz), 2.42 (1H, d, J = 17.7 Hz), 3.06 (2H, s), 4.26 (2H, q, J = 7.0 Hz), 6.70 (1H, s), 7.10-7.20 (1H, m), 7.55 (1H, t, J = 7.5 Hz), 7.67-7.79 (2H, m), 8.00-8.15 (3H, m), 8.34 (1H, d, J = 4.7 Hz), 8.93 (1H, s).

### Example 31

### Methyl 4-(6-ethoxy-3,4,8,9-tetrahydro-3,3,8,8-tetramethylfuro[2,3-h]isoquinolin-1-yl)-2-(phenylamino)benzoate

To a suspension of methyl 2-amino-4-(6-ethoxy-3,4,8,9-tetrahydro-3,3,8,8-tetramethylfuro[2,3-h]isoquinolin-1-yl)benzoate hydrochloride (815 mg, 1.78 mmol) in xylene (4 ml) were added bromobenzene (0.21 ml, 1.97 mmol), cesium carbonate (2.15 g, 6.60 mmol), palladium (II) acetate (11 mg, 0.0495 mmol) and 2,2'-bis(diphenylphosphino)-1,1'-binaphthyl (46 mg, 0.0743 mmol), and the mixture was stirred at 140°C for 7 hours. After cooling, the reaction mixture was combined with ice water, neutralized with sodium hydrogen carbonate, and extracted twice with ethyl acetate. The combined organic layer was washed with a saturated aqueous solution of sodium chloride, dried over magnesium sulfate, filtered, and concentrated under reduced pressure. The residue was purified with a silica gel column chromatography (hexane/ethyl acetate, 10:1 followed by 5:1), and crystallized from diisopropyl ether-hexane to give the title compound (369 mg, yield 42%).
¹H NMR (CDCl₃) δ 1.20 (6H, s), 1.33 (6H, s), 1.45 (3H, t, J = 7.0 Hz), 2.35 (2H, s), 2.63 (2H, s), 3.93 (3H, s), 4.16 (2H, q, J = 7.0 Hz), 6.56 (1H, s), 6.73 (1H, dd, J = 8.0, 1.4 Hz), 7.02-7.10 (1H, m), 7.21-7.35 (5H, m), 7.98 (1H, d, J = 8.0 Hz), 9.55 (1H, s).

### Example 32

### 4-(6-Ethoxy-3,4,8,9-tetrahydro-3,3,8,8-tetramethylfuro[2,3-h]isoquinolin-1-yl)-2-(phenylamino)benzoic acid

From methyl 4-(6-ethoxy-3,4,8,9-tetrahydro-3,3,8,8-tetramethylfuro[2,3-h]isoquinolin-1-yl)-2-(phenylamino)benzoate, the title compound was obtained by the method similar to that in Example 9. Yield 87%.
¹H NMR (CDCl₃) δ 1.30 (6H, s), 1.47 (3H, t, J = 7.0 Hz), 1.56 (6H, s), 2.36 (2H, s), 2.92 (2H, s), 4.20 (2H, q, J = 7.0 Hz), 6.52 (1H, dd, J = 7.6, 1.6 Hz), 6.93 (1H, br s), 7.12 (1H, d, J = 1.6 Hz), 7.21-7.27 (5H, m), 7.82 (1H, d, J = 7.6 Hz).

### Example 33

### Methyl [4-(6-ethoxy-3,4,8,9-tetrahydro-3,3,8,8-tetramethylfuro[2,3-h]isoquinolin-1-yl)phenoxy]acetate

A solution of 4-(6-ethoxy-3,4,8,9-tetrahydro-3,3,8,8-tetramethylfuro[2,3-h]isoquinolin-1-yl)phenol (0.500 g, 1.37 mmol) in N,N-dimethylformamide (5 ml) was cooled with ice, sodium hydride (66% dispersion in oil) (59.7 mg, 1.64 mmol) was added thereto, and the mixture was stirred at room temperature for 1 hour. To this was added methyl bromoacetate (0.155 ml, 1.64 mmol), and the mixture was further stirred at 80°C for 3 hours. The reaction solution was cooled to room temperature, combined with water, and extracted with ethyl acetate. The extracts were washed with water, and concentrated under reduced pressure. The residue was purified with a silica gel column chromatography (hexane/ethyl acetate, 1:1 followed by 1:2), and crystallized from diisopropyl ether to give the title compound (0.570 g, yield 55%).
¹H NMR (CDCl₃) δ 1.22 (6H, s), 1.32 (6H, s), 1.46 (3H, t, J = 7.0 Hz), 2.23 (2H, s), 2.65 (2H, s), 3.81 (3H, s), 4.18 (2H, q, J = 7.0 Hz), 4.67 (2H, s), 6.59 (1H, s), 6.92 (2H, dd, J = 6.8, 2.0 Hz), 7.35 (2H, dd, J = 6.8, 2.0 Hz).

### Example 34

### [4-(6-Ethoxy-3,4,8,9-tetrahydro-3,3,8,8-tetramethylfuro[2,3-h]isoquinolin-1-yl)phenoxy]acetic acid hydrochloride

From methyl [4-(6-ethoxy-3,4,8,9-tetrahydro-3,3,8,8-tetramethylfuro[2,3-h]isoquinolin-1-yl)phenoxy]acetate, the title compound was obtained as free base by the method similar to that in Example 9. This was dissolved in ethyl acetate, a solution of 4 M hydrogen chloride/ethyl acetate was added thereto, and the mixture was concentrated under reduced pressure to give the title compound. Yield 88%.
Amorphous.
¹H NMR (DMSO-d₆) δ 1.26 (6H, s), 1.37 (3H, t, J = 7.0 Hz), 1.41 (6H, s), 2.28 (2H, s), 3.11 (2H, s), 4.23 (2H, q, J = 7.0 Hz), 4.97 (2H, s), 7.06 (1H, s), 7.17 (1H, d, J = 8.4 Hz), 7.19 (1H, d, J = 8.4 Hz), 7.57 (2H, d, J = 8.4 Hz), 12.32 (1H, s).

### Example 35

### Methyl 2-[[[(2-ethoxy-2-oxoethyl)amino]carbonyl]amino]-4-(6-ethoxy-3,4,8,9-tetrahydro-3,3,8,8-tetramethylfuro[2,3-h]isoquinolin-1-yl)benzoate

To a suspension of methyl 2-amino-4-(6-ethoxy-3,4,8,9-tetrahydro-3,3,8,8-tetramethylfuro[2,3-h]isoquinolin-1-yl)benzoate hydrochloride (2.11 g, 4.60 mmol) in tetrahydrofuran (16 ml) were added triethylamine (1.25 ml, 8.95 mmol) and ethyl isocyanatoacetate (0.57 ml, 5.11 mmol) under ice-cooling, and the mixture was heated under reflux for 4 hours. Ethyl isocyanatoacetate (0.42 ml, 3.84 mmol) was further added in three portions, and the mixture was heated under reflux for 15 hours.
The reaction mixture was combined with water, and extracted twice with a mixed solution of ethyl acetate-tetrahydrofuran. The combined organic layer was washed with a saturated aqueous solution of sodium chloride, dried over sodium sulfate, filtered, and concentrated under reduced pressure. The resultant crystals were washed with diisopropyl ether to give the title compound (393 mg, yield 85%).
¹H NMR (CDCl₃) δ 1.23-1.32 (15H, s), 1.46 (3H, t, J = 7.0 Hz), 2.34 (2H, s), 2.64 (2H, s), 3.93 (3H, s), 3.98-4.28 (6H, m), 5.23 (1H, t, J = 5.2 Hz), 6.57 (1H, s), 7.00 (1H, dd, J = 8.0, 1.6 Hz), 7.99 (1H, d, J = 8.0 Hz), 8.56 (1H, d, J = 1.6 Hz), 10.51 (1H, s).

### Example 36

### Methyl 2-(2,5-dioxo-1-imidazolidinyl)-4-(6-ethoxy-3,4,8,9-tetrahydro-3,3,8,8-tetramethylfuro[2,3-h]isoquinolin-1-yl)benzoate

A solution of methyl 2-[[[(2-ethoxy-2-oxoethyl)amino]carbonyl]amino]-4-(6-ethoxy-3,4,8,9-tetrahydro-3,3,8,8-tetramethylfuro[2,3-h]isoquinolin-1-yl)benzoate (1.94 g, 3.52 mmol) in 5 M hydrochloric acid (5 ml) was stirred at 80°C for 7 hours. The solution was dissolved in methanol (10 ml), conc. sulfuric acid (0.56 ml, 10.6 mmol) was added thereto, and the mixture was stirred at 70°C for 15 hours. After cooling, methanol was distilled off under reduced pressure, the residue was neutralized with sodium hydrogen carbonate, and extracted twice with ethyl acetate. The combined organic layer was washed with a saturated aqueous solution of sodium chloride, dried over sodium sulfate, and concentrated under reduced pressure. The residue was purified with a silica gel column chromatography (hexane/ethyl acetate, 3:1 followed by 1:1), and crystallized from diethyl ether to give the title compound (812 mg, yield 46%).
¹H NMR (CDCl₃) δ 1.32 (6H, s), 1.35 (6H, s), 1.47 (3H, t, J = 7.0 Hz), 2.28 (2H, s), 2.75 (2H, s), 3.78 (3H, s), 4.20 (2H, q, J = 7.0 Hz), 4.78 (2H, s), 6.63 (1H, s), 7.16 (1H, dd, J = 8.2, 1.4 Hz), 7.21 (1H, d, J = 1.4 Hz), 8.05 (1H, d, J = 8.2 Hz), 9.62 (1H, s).

### Example 37

### 2-(2,5-Dioxo-1-imidazolidinyl)-4-(6-ethoxy-3,4,8,9-tetrahydro-3,3,8,8-tetramethylfuro[2,3-h]isoquinolin-1-yl)benzoic acid hydrochloride

To a solution of methyl 2-(2,5-dioxo-1-imidazolidinyl)-4-(6-ethoxy-3,4,8,9-tetrahydro-3,3,8,8-tetramethylfuro[2,3-h]isoquinolin-1-yl)benzoate (279 mg, 0.552 mmol) in methanol (2 ml) was added 1 M aqueous solution (1.16 ml) of sodium hydroxide, and the mixture was stirred at room temperature for 18 hours. The reaction solution was acidified with 5 M hydrochloric acid, and the solvent was distilled off under reduced pressure. The residue was combined with ethanol and acetone, and the insolubles were taken by filtration. The filtrate was concentrated under reduced pressure, and this procedure was repeated twice to give the title compound (271 mg, yield 93%).
Amorphous.
¹H NMR (DMSO-d₆) δ 1.25 (6H, s), 1.37 (3H, t, J = 7.0 Hz), 1.44 (6H, s), 2.30 (2H, s), 3.15 (2H, s), 4.24 (2H, q, J = 7.0 Hz), 4.61 (2H, s), 7.08 (1H, s), 7.40 (1H, d, J = 8.0 Hz), 7.43 (1H, s), 8.15 (1H, d, J = 8.0 Hz), 12.00 (1H, s).

### Example 38

### Methyl 4-(6-ethoxy-3,4,8,9-tetrahydro-3,3,8,8-tetramethylfuro[2,3-h]isoquinolin-1-yl)-2-(3-methyl-2,5-dioxo-1-imidazolidinyl)benzoate

From methyl 2-(2,5-dioxo-1-imidazolidinyl)-4-(6-ethoxy-3,4,8,9-tetrahydro-3,3,8,8-tetramethylfuro[2,3-h]isoquinolin-1-yl)benzoate and iodomethane, the title compound was obtained by the method similar to that in Example 33. Yield 86%.
¹H NMR (CDCl₃) δ 1.26 (6H, s), 1.35 (6H, s), 1.47 (3H, t, J = 7.0 Hz), 2.24 (2H, s), 2.70 (2H, s), 3.65 (3H, s), 3.80 (3H, s), 4.20 (2H, q, J = 7.0 Hz), 4.88 (2H, s), 6.63 (1H, s), 7.27 (1H, d, J = 8.0 Hz), 7.31 (1H, s), 8.22 (1H, d, J = 8.0 Hz).

### Example 39

### 4-(6-Ethoxy-3,4,8,9-tetrahydro-3,3,8,8-tetramethylfuro[2,3-h]isoquinolin-1-yl)-2-(3-methyl-2,5-dioxo-1-imidazolidinyl)benzoic acid

From methyl 4-(6-ethoxy-3,4,8,9-tetrahydro-3,3,8,8-tetramethylfuro[2,3-h]isoquinolin-1-yl)-2-(3-methyl-2,5-dioxo-1-imidazolidinyl)benzoate, the title compound was obtained by the method similar to that in Example 12. Yield 77%.
¹H NMR (DMSO-d₆) δ 1.33 (6H, s), 1.49 (6H, s), 1.49 (3H, t, J = 7.0 Hz), 2.08-2.15 (2H, m), 2.72-3.00 (2H, m), 3.61 (3H, s), 4.23 (2H, q, J = 7.0 Hz), 4.58-4.85 (2H, m), 6.67 (1H, s), 7.26 (1H, d, J = 8.0 Hz), 7.42 (1H, s), 8.32 (1H, d, J = 8.0 Hz).

### Example 40

### Methyl 2-[[(dimethylamino)carbonyl]amino]-4-(6-ethoxy-3,4,8,9-tetrahydro-3,3,8,8-tetramethylfuro[2,3-h]isoquinolin-1-yl)benzoate

To a solution of methyl 2-amino-4-(6-ethoxy-3,4,8,9-tetrahydro-3,3,8,8-tetramethylfuro[2,3-h]isoquinolin-1-yl)benzoate (869 mg, 2.06 mmol) in N,N-dimethylacetamide (5 ml) were added triethylamine (0.14 ml, 1.03 mmol) and 4-nitrophenyl chlorocarbonate (598 mg, 2.97 mmol), and the mixture was stirred for 1.5 hours at room temperature. A solution (2.6 ml, 5.15 mmol) of 2 M dimethylamine/tetrahydrofuran was added thereto under ice-cooling, and the mixture was stirred at room temperature for 15 hours. The reaction mixture was combined with ice water, neutralized with sodium hydrogen carbonate, and extracted twice with ethyl acetate. The combined organic layer was washed with a saturated aqueous solution of sodium hydrogen carbonate, water and a saturated aqueous solution of sodium chloride, dried over sodium sulfate, filtered, and concentrated under reduced pressure. The residue was purified with a silica gel column chromatography (hexane/ethyl acetate 1:1), and crystallized from diisopropyl ether-hexane to give the title compound (281 mg, yield 28%). The mother liquor was again purified with a silica gel column chromatography (hexane/ethyl acetate 1:1), and crystallized from diethyl ether-hexane to give the title compound (257 mg, yield 25%).
¹H NMR (CDCl₃) δ 1.22 (6H, s), 1.31 (6H, s), 1.45 (3H, t, J = 6.9 Hz), 2.35 (2H, br s), 2.64 (2H, s), 3.07 (6H, s), 3.93 (3H, s), 4.17 (2H, q, J = 6.9 Hz), 6.56 (1H, s), 6.97 (1H, dd, J = 8.1, 1.8 Hz), 7.99 (1H, d, J = 8.1 Hz), 8.63 (1H, d, J = 1.8 Hz), 10.65 (1H, s).

### Example 41

### 2-[[(Dimethylamino)carbonyl]amino]-4-(6-ethoxy-3,4,8,9-tetrahydro-3,3,8,8-tetramethylfuro[2,3-h]isoquinolin-1-yl)benzoic acid

From methyl 2-[[(dimethylamino)carbonyl]amino]-4-(6-ethoxy-3,4,8,9-tetrahydro-3,3,8,8-tetramethylfuro[2,3-h]isoquinolin-1-yl)benzoate, the title compound was obtained by the method similar to that in Example 12. Yield 60%.
¹H NMR (DMSO-d₆) δ 1.18 (6H, s), 1.22 (6H, s), 1.34 (3H, t, J = 6.9 Hz), 2.35 (2H, br s), 2.69 (2H, s), 2.95 (6H, s), 4.11 (2H, q, J = 6.9 Hz), 6.82 (1H, s), 6.99 (1H, d, J = 8.1 Hz), 7.99 (1H, d, J = 8.1 Hz), 8.44 (1H, s), 11.27 (1H, br s).

### Example 42

### Methyl 2-[[(phenylamino)carbonyl]amino]-4-(6-ethoxy-3,4,8,9-tetrahydro-3,3,8,8-tetramethylfuro[2,3-h]isoquinolin-1-yl)benzoate

With aniline, the title compound was obtained by the method similar to that in Example 40. Yield 69%.
¹H NMR (CDCl₃) δ 1.14 (6H, s), 1.23 (6H, s), 1.33 (3H, t, J = 6.9 Hz), 2.42 (2H, br s), 2.62 (2H, s), 3.92 (3H, s), 4.09 (2H, q, J = 6.9 Hz), 6.79 (1H, s), 6.94-6.99 (1H, m), 7.07 (1H, d, J = 8.2 Hz), 7.23-7.28 (2H, m), 7.47 (1H, d, J = 7.8 Hz), 7.98 (1H, d, J = 8.2 Hz), 8.33 (1H, s), 9.85 (1H, s), 10.06 (1H, s).

### Example 43

### 7-(6-Ethoxy-3,4,8,9-tetrahydro-3,3,8,8-tetramethylfuro[2,3-h]isoquinolin-1-yl)-3-phenyl-2,4(1H,3H)-quinazolinedione

From methyl 2-[[(phenylamino)carbonyl]amino]-4-(6-ethoxy-3,4,8,9-tetrahydro-3,3,8,8-tetramethylfuro[2,3-h]isoquinolin-1-yl)benzoate, the title compound was obtained by the method similar to that in Example 9. Yield 58%.
¹H NMR (CDCl₃) δ 1.35 (12H, s), 1.48 (3H, t, J = 7.0 Hz), 2.31 (2H, s), 2.75 (2H, br s), 4.20 (2H, q, J = 7.0 Hz), 6.63 (1H, s), 7.17 (1H, dd, J = 8.1, 1.5 Hz), 7.24-7.29 (3H, m), 7.42-7.54 (3H, m), 8.08 (1H, d, J = 8.1 Hz), 9.80 (1H, s).

### Example 44

### Methyl 2-[[2-(1,1-dimethylethoxy)-2-oxoethyl](trifluoroacetyl)amino]-4-(6-ethoxy-3,4,8,9-tetrahydro-3,3,8,8-tetramethylfuro[2,3-h]isoquinolin-1-yl)benzoate

From tert-butyl bromoacetate, the title compound was obtained by the method similar to that in Example 4. Yield 43%.
¹H NMR (CDCl₃) δ 1.16 (3H, s), 1.28 (3H, s), 1.31 (3H, s), 1.32 (3H, s), 1.46 (3H, t, J = 7.2 Hz), 1.49 (9H, s), 2.62 (1H, d, J = 15.8 Hz), 2.74 (1H, d, J = 15.8 Hz), 3.67 (1H, d, J = 16.8 Hz), 3.91 (3H, s), 4.19 (2H, q, J = 7.2 Hz), 4.85 (1H, d, J = 16.8 Hz), 6.61 (1H, s), 7.50 (1H, dd, J = 8.0, 1.4 Hz), 7.70 (1H, d, J = 1.4 Hz), 8.09 (1H, d, J = 8.0 Hz).

### Example 45

### N-[5-(6-Ethoxy-3,4,8,9-tetrahydro-3,3,8,8-tetramethylfuro[2,3-h]isoquinolin-1-yl)-2-(methoxycarbonyl)phenyl]glycine

To a solution of methyl 2-[[2-(1,1-dimethylethoxy)-2-oxoethyl](trifluoroacetyl)amino]-4-(6-ethoxy-3,4,8,9-tetrahydro-3,3,8,8-tetramethylfuro[2,3-h]isoquinolin-1-yl)benzoate (1.91 g, 3.02 mmol) in methanol (15 ml) was added potassium carbonate (835 mg, 6.04 mmol), and the mixture was stirred for 1 hour at 60°C. After cooling, methanol was distilled off under reduced pressure. The residue was combined with water, adjusted at pH 5 with 1 M hydrochloric acid, and extracted three times with ethyl acetate. The extract was concentrated under reduced pressure, and the obtained crystals were washed with diisopropyl ether to give the title compound (1.04 g, yield 72%).
¹H NMR (CDCl₃) δ 1.31 (6H, s), 1.49 (3H, t, J = 7.0 Hz), 1.54 (6H, s), 2.36 (2H, s), 2.95 (2H, s), 3.64 (2H, s), 3.83 (3H, s), 4.25 (2H, q, J = 7.0 Hz), 6.52 (1H, s), 6.54 (1H, d, J = 8.0 Hz), 6.67 (1H, s), 7.94 (1H, d, J = 8.0 Hz), 8.07 (1H, br s).

### Example 46

### Methyl 2-[(2-amino-2-oxoethyl)amino]-4-(6-ethoxy-3,4,8,9-tetrahydro-3,3,8,8-tetramethylfuro[2,3-h]isoquinolin-1-yl)benzoate

From N-[5-(6-ethoxy-3,4,8,9-tetrahydro-3,3,8,8-tetramethylfuro[2,3-h]isoquinolin-1-yl)-2-(methoxycarbonyl)phenyl]glycine, the title compound was obtained by the method similar to that in Example 10. Yield 78%.
¹H NMR (CDCl₃) δ 1.25 (6H, s), 1.32 (6H, s), 1.46 (3H, t, J = 7.0 Hz), 2.27 (2H, s), 2.68 (2H, s), 3.91 (3H, s), 3.97 (2H, d, J = 5.9 Hz), 4.19 (2H, q, J = 7.0 Hz), 5.37 (1H, br s), 6.40 (1H, br s), 6.60 (1H, s), 6.64 (1H, d, J = 1.4 Hz), 7.72 (1H, dd, J = 8.2, 1.4 Hz), 7.96 (1H, d, J = 8.2 Hz), 8.29 (1H, t, J = 5.9 Hz).

### Example 47

### 2-[(Carboxymethyl)amino]-4-(6-ethoxy-3,4,8,9-tetrahydro-3,3,8,8-tetramethylfuro[2,3-h]isoquinolin-1-yl)benzoic acid

From N-[5-(6-ethoxy-3,4,8,9-tetrahydro-3,3,8,8-tetramethylfuro[2,3-h]isoquinolin-1-yl)-2-(methoxycarbonyl)phenyl]glycine, the title compound was obtained by the method similar to that in Example 12. Yield 68%.
¹H NMR (DMSO-d₆) δ 1.15 (6H, s), 1.23 (6H, s), 1.33 (3H, t, J = 6.8 Hz), 2.32 (2H, s), 2.66 (2H, s), 3.99 (2H, s), 4.06 (2H, q, J = 6.8 Hz), 6.51 (1H, s), 6.56 (1H, d, J = 8.0 Hz), 6.79 (1H, s), 7.85 (1H, d, J = 8.0 Hz), 8.24 (1H, br s).

### Example 48

### Methyl 4-(6-ethoxy-3,4,8,9-tetrahydro-3,3,8,8-tetramethylfuro[2,3-h]isoquinolin-1-yl)-2-[ethyl(trifluoroacetyl)amino]benzoate

To a solution of methyl 4-(6-ethoxy-3,4,8,9-tetrahydro-3,3,8,8-tetramethylfuro[2,3-h]isoquinolin-1-yl)-2-[(trifluoroacetyl)amino]benzoate (3.04 g, 5.86 mmol) in N,N-dimethylformamide (20 ml) were added potassium tert-butoxide (724 mg, 6.45 mmol) and iodoethane (0.52 ml, 6.45 mmol), and the mixture was stirred at room temperature for 2 hours and at 50°C for 3 hours. Iodoethane (0.094 ml, 1.17 mmol) was further added thereto, and the mixture was stirred at 50°C for 5 hours. The reaction mixture was combined with ice water, and extracted twice with ethyl acetate. The combined organic layer was washed with water and a saturated aqueous solution of sodium chloride, dried over sodium sulfate, filtered, and concentrated under reduced pressure. The residue was purified with a silica gel column chromatography (hexane/ethyl acetate, 5:1 followed by 3:1) to give the title compound (1.76 g, yield 55%).
Amorphous.
¹H NMR (CDCl₃) δ 1.13-1.43 (15H, m), 1.46 (3H, t, J = 7.0 Hz), 2.11 (1H, d, J = 16.1 Hz), 2.22 (1H, d, J = 16.1 Hz), 2.65 (1H, d, J = 15.5 Hz), 2.73 (1H, d, J = 15.5 Hz), 3.35-3.55 (0.6 H, m), 3.88 (0.9H, s), 3.90 (2.1H, s), 3.96-4.16 (1.4H, m), 4.19 (2H, q, J = 7.0 Hz), 6.61 (1H, s), 7.25 (0.3H, d, J = 1.4 Hz), 7.30 (0.7H, d, J = 1.4 Hz), 7.52 (0.7H, dd, J = 7.8, 1.4 Hz), 7.63 (0.3H, dd, J = 7.8, 1.4 Hz), 8.12 (0.7H, d, J = 7.8 Hz), 8.19 (0.3H, d, J = 7.8 Hz).

### Example 49

### Methyl 4-(6-ethoxy-3,4,8,9-tetrahydro-3,3,8,8-tetramethylfuro[2,3-h]isoquinolin-1-yl)-2-(ethylamino)benzoate

### (Method 1)

To a solution of methyl 4-(6-ethoxy-3,4,8,9-tetrahydro-3,3,8,8-tetramethylfuro[2,3-h]isoquinolin-1-yl)-2-[ethyl(trifluoroacetyl)amino]benzoate (1.70 g, 3.11 mmol) in methanol (10 ml) was added potassium carbonate (645 mg, 4.67 mmol), and the mixture was stirred at 60°C for 10 hours. After cooling, methanol was distilled off under reduced pressure, and the residue was combined with water, and extracted twice with ethyl acetate. The combined organic layer was washed with a saturated aqueous solution of sodium chloride, dried over sodium sulfate, filtered, and concentrated under reduced pressure. The residue was purified with a basic silica gel column chromatography (hexane/ethyl acetate, 10:1), and crystallized from pentane to give the title compound (528 mg, 38%).
¹H NMR (CDCl₃) δ 1.24 (6H, s), 1.26-1.34 (9H, m), 1.46 (3H, t, J = 7.0 Hz), 2.36 (2H, s), 2.68 (2H, s), 3.17-3.30 (2H, m), 3.87 (3H, s), 4.18 (2H, q, J = 7.0 Hz), 6.57 (1H, dd, J = 8.0, 1.4 Hz), 6.59 (1H, d, J = 1.4 Hz), 6.67 (1H, s), 7.64 (1H, t, J = 5.5 Hz), 7.89 (1H, d, J = 8.0 Hz).

### (Method 2)

To a suspension of methyl 4-cyano-2-(ethylamino)benzoate (10 g) in methyl acetate (50 ml) was added dropwise trifluoromethanesulfonic acid (43.4 ml) at room temperature with stirring the suspension. Then, a solution of 7-ethoxy-2,3-dihydro-2,2-dimethyl-α-(1-methylethyl)-5-benzofuranmethanol(25.89 g) in methyl acetate (260 ml) was added dropwise thereto with keeping the internal temperature at 60 to 65°C. The mixture was stirred for 2 hours at 60°C, and the reaction solution was concentrated under reduced pressure. To the residue were added diisopropyl ether (10 ml) and ice water (50 ml), and added 25% ammonia water (50 ml) with ice-cooling. The layers were separated, and the aqueous layer was extracted with diisopropyl ether (50 ml). The organic layer was collected and washed twice with water. The organic layer was concentrated, and re-concentrated with adding ethyl acetate (30 ml). To the concentrated residue was added ethyl acetate (30 ml), and added dropwise a solution (12.3 ml) of 4M hydrogen chloride-ethyl acetate. Heptane (15 ml) was added thereto and the mixture was cooled with ice. The mixture was mixed with stirring for 2 hours at less than 10°C, and the crystals were taken by filtration to give the title compound as hydrochloride (15.3 g, yield 64%).
¹H NMR (CDCl₃) δ 1.25-1.36 (9H, m), 1.51 (3H, t, J = 7.0 Hz), 1.61 (3H, br s), 1.79(3H, br s), 2.30-2.60(2H, m), 2. 80-3.20 (2H, m), 3.30(1H, br s), 3.88(3H, s), 3.50(1H, br s), 4.27(2H, q, J = 7.0 Hz), 6.50-6.61(1H, m), 6.70(1H, s), 7.10(1H, m), 7.77(1H, s), 7.97(1H, d, J = 8.2 Hz), 14.5(1H, s).

### Example 50

### 4-(6-Ethoxy-3,4,8,9-tetrahydro-3,3,8,8-tetramethylfuro[2,3-h]isoquinolin-1-yl)-2-(ethylamino)benzoic acid

### (Method 1)

The aqueous layer extracted with ethyl acetate in Method 1 of Example 49 was neutralized with 5 M hydrochloric acid, and extracted three times with ethyl acetate. The combined organic layer was washed with a saturated aqueous solution of sodium chloride, dried over sodium sulfate, filtered, concentrated under reduced pressure, and crystallized from ethyl acetate-diisopropyl ether to give the title compound (327 mg, 24%).
¹H NMR (CDCl₃) δ 1.14 (3H, t, J = 7.0 Hz), 1.31 (6H, s), 1.47 (6H, s), 1.47 (3H, t, J = 7.0 Hz), 2.37 (2H, s), 2.83 (2H, s), 3.09 (2H, q, J = 7.0 Hz), 4,21 (2H, q, J = 7.0 Hz), 6.45 (1H, dd, J = 8.1, 1.5 Hz), 6.59 (1H, d, J = 1.5 Hz), 6.62 (1H, s), 7.84 (1H, d, J = 8.1 Hz), 8.06 (1H, br s).

### (Method 2)

To a solution of 7-ethoxy-2,3-dihydro-2,2-dimethyl-α-(1-methylethyl)-5-benzofuranmethanol (2.98 g, 11.3 mmol) and methyl 4-cyano-2-(ethylamino)benzoate (1.92 g, 9.40 mmol) in toluene (10 ml) and acetic acid (6 ml) was added conc. sulfuric acid (1.25 ml, 23.5 mmol) under ice-cooling, and the mixture was stirred for 2 hours at 80°C. Conc. sulfuric acid (0.25 ml, 4.7 mmol) was further added thereto, and the mixture was stirred for 1 hour at 80°C. Methanol (4.25 ml) was added dropwise thereto, and the mixture was stirred for 30 minutes at 60°C. After cooling, the reaction mixture was combined with ice water, and washed with ethyl acetate. The organic layer was extracted twice with 2 M hydrochloric acid, the combined aqueous layer was adjusted at pH 4.5 with sodium hydrogen carbonate, and extracted twice with ethyl acetate. The combined organic layer was washed with a saturated aqueous solution of sodium hydrogen carbonate, water and a saturated aqueous solution of sodium chloride, dried over sodium sulfate, filtered, and concentrated under reduced pressure. To the residue was added ethyl acetate, and the precipitated crystals were removed. The filtrate was concentrated under reduced pressure, the residue was purified with a basic silica gel column chromatography (hexane/ethyl acetate, 50:1 followed by 10:1), and crystallized from pentane to give methyl 4-(6-ethoxy-3,4,8,9-tetrahydro-3,3,8,8-tetramethylfuro[2,3-h]isoquinolin-1-yl)-2-(ethylamino)benzoate (1.54 g, yield 36%).

Methyl 4-(6-ethoxy-3,4,8,9-tetrahydro-3,3,8,8-tetramethylfuro[2,3-h]isoquinolin-1-yl)-2-(ethylamino)benzoate (1.54 g, 3.42 mmol) was suspended in methanol (7 ml) and 5 M aqueous solution (1.71 ml) of sodium hydroxide were added thereto, and the mixture was stirred at room temperature for 2 hours and at 50°C for 1.5 hours. After cooling, methanol was distilled off under reduced pressure, and the residue was combined with water and washed with diisopropyl ether and ethyl acetate. The aqueous layer was neutralized with 5 M hydrochloric acid, and extracted twice with ethyl acetate. The combined organic layer was washed with a saturated aqueous solution of sodium chloride, dried over sodium sulfate, filtered, concentrated under reduced pressure, and recrystallized from ethyl acetate-hexane to give the title compound (1.30 g, yield 87%).

### (Method 3)

To a solution of methyl 4-(6-ethoxy-3,4,8,9-tetrahydro-3,3,8,8-tetramethylfuro[2,3-h]isoquinolin-1-yl)-2-(ethylamino)benzoate hydrochloride (2 g) in methanol (4 ml) was added dropwise 5 M aqueous solution (2.87 ml) of sodium hydroxide and the mixture was stirred at the internal temperature of 45 to at 55°C for 2 hours. The mixture was cooled, methanol was distilled off, and the residue was adjusted at pH about 8 with 6 M hydrochloric acid. Ethyl acetate (8 ml) was added thereto, and the mixture was further adjusted at pH 6.1 with 6M hydrochloric acid. The mixture was mixed by stirring at room temperature for 1 hour and at less than 10°C for 2 hours. The precipitated crystals were taken by filtration, and washed with water (6 ml) and cooled ethyl acetate (6 ml) in this order to give the title compound (1.58 g, yield 88%).

### Example 51

### Methyl 4-(6-ethoxy-3,4,8,9-tetrahydro-3,3,8,8-tetramethylfuro[2,3-h]isoquinolin-1-yl)-2-[ethyl(2-pyridinylcarbonyl)amino]benzoate

With methyl 4-(6-ethoxy-3,4,8,9-tetrahydro-3,3,8,8-tetramethylfuro[2,3-h]isoquinolin-1-yl)-2-(ethylamino)benzoate and picolinic acid chloride hydrochloride, the title compound was obtained by the method similar to that in Example 13. Yield 37%.
¹H NMR (CDCl₃) δ 1.17 (3H, s), 1.21 (0.6H, t, J = 6.9 Hz), 1.29 (3H, s), 1.30 (3H, s), 1.31 (2.4H, t, J = 6.9 Hz), 1.36 (3H, s), 1.47 (3H, t, J = 6.9 Hz), 2.29 (1H, d, J = 16.2 Hz), 2.35-2.65 (1H, br), 2.62 (1H, d, J = 15.6 Hz), 2.70 (1H, d, J = 15.6 Hz), 3.40-3.56 (0.4H, m), 3.78 (2.4H, s), 3.90 (0.6H, s), 3.94-4.10 (1.6H, m), 4.18 (2H, q, J = 6.9 Hz), 6.91 (1H, s), 7.11 (0.8H, t, J = 6.3 Hz), 7.30-7.36 (1H, m), 7.48 (0.8H, s), 7.56-7.83 (3.2H, m), 8.06-8.16 (1H, m), 8.62 (0.2H, d, J = 5.1 Hz).

### Example 52

### 4-(6-Ethoxy-3,4,8,9-tetrahydro-3,3,8,8-tetramethylfuro[2,3-h]isoquinolin-1-yl)-2-[ethyl(2-pyridinylcarbonyl)amino]benzoic acid

From methyl 4-(6-ethoxy-3,4,8,9-tetrahydro-3,3,8,8-tetramethylfuro[2,3-h]isoquinolin-1-yl)-2-[ethyl(2-pyridinylcarbonyl)amino]benzoate, the title compound was obtained by the method similar to that in Example 9. Yield 60%.
¹H NMR (DMSO-d₆) δ 1.06-1.36 (18H, m), 2.20 (1H, t, J = 16.9 Hz), 2.31 (1H, t, J = 16.9 Hz), 2.62 (2H, s), 3.67-3.84 (1H, m), 3.87-4.04 (1H, m), 4.09 (2H, q, J = 6.8 Hz), 6.80 (1H, s), 7.24-7.30 (2.6H, m), 7.45-8.15 (4.2H, m), 8.64 (0.2H, d, J = 5.0 Hz).

### Example 53

### Methyl 2-(acetylethylamino)-4-(6-ethoxy-3,4,8,9-tetrahydro-3,3,8,8-tetramethylfuro[2,3-h]isoquinolin-1-yl)benzoate hydrochloride

From methyl 4-(6-ethoxy-3,4,8,9-tetrahydro-3,3,8,8-tetramethylfuro[2,3-h]isoquinolin-1-yl)-2-(ethylamino)benzoate, the title compound was obtained as free base by the method similar to that in Example 17. This was dissolved in ethyl acetate, and a solution of 4 M hydrogen chloride/ethyl acetate was added thereto. The mixture was concentrated under reduced pressure, and the residue was crystallized from ethyl acetate-diisopropyl ether to give the title compound. Yield 88%.
¹H NMR (DMSO-d₆) δ 0.92-1.05 (3H, m), 1.17 (6H, s), 1.37 (3H, t, J = 7.0 Hz), 1.48 (6H, s), 1.74 (2.25H, s), 2.12 (1H, d, J = 16.0 Hz), 2.16 (0.75H, s), 2.22 (1H, d, J = 16.0 Hz), 3.16 (2H, s), 3.30-3.55 (1.5H, br), 3.70-3.90 (0.5H, br), 3.82 (0.75H, s), 3.87 (2.25H, s), 4.24 (2H, q, J = 7.0 Hz), 7.09 (1H, s), 7.58 (0.25H, s), 7.51 (0.25H, d, J = 7.8 Hz), 7.74 (0.75H, s), 7.76 (0.75H, d, J = 7.8 Hz), 8.06 (0.25H, d, J = 7.8 Hz), 8.14 (0.75H, d, J = 7.8 Hz).

### Example 54

### 2-(Acetylethylamino)-4-(6-ethoxy-3,4,8,9-tetrahydro-3,3,8,8-tetramethylfuro[2,3-h]isoquinolin-1-yl)benzoic acid

From methyl 2-(acetylethylamino)-4-(6-ethoxy-3,4,8,9-tetrahydro-3,3,8,8-tetramethylfuro[2,3-h]isoquinolin-1-yl)benzoate, the title compound was obtained by the method similar to that in Example 12. Yield 71%.
¹H NMR (CDCl₃) δ 0.85-1.10 (3H, br), 1.31 (6H, s), 1.46-1.52 (12H, m), 2.19 (2H, s), 2.91 (2H, br s), 3.25-3.45 (1H, br), 3.65-4.10 (1H, br), 4.24 (2H, q, J = 7.2 Hz), 5.20-6.40 (1H, br), 6.68 (1H, s), 7.26 (1H, s), 7.56 (1H, d, J = 7.8 Hz), 7.98 (1H, d, J = 7.8 Hz).

### Example 55

### 2-Amino-4-(6-ethoxy-3,4,8,9-tetrahydro-3,3,8,8-tetramethylfuro[2,3-h]isoquinolin-1-yl)benzoic acid hydrochloride

From methyl 4-(6-ethoxy-3,4,8,9-tetrahydro-3,3,8,8-tetramethylfuro[2,3-h]isoquinolin-1-yl)-2-[(trifluoroacetyl)amino]benzoate, the title compound was obtained as a free base by the method similar to that in Example 9. This was dissolved in ethyl acetate, and a solution of 4 M hydrogen chloride/ethyl acetate was added thereto. The mixture was concentrated under reduced pressure to give the title compound. Yield 53%.
Amorphous.
¹H NMR (DMSO-d₆) δ 1.27 (6H, s), 1.37 (3H, t, J = 7.0 Hz), 1.43 (6H, s), 2.40 (2H, s), 3.15 (2H, s), 4.24 (2H, q, J = 7.0 Hz), 6.66 (1H, dd, J = 8.1, 1.0 Hz), 6.92 (1H, d, J = 1.0 Hz), 7.08 (1H, s), 7.89 (1H, d, J = 8.1 Hz).

### Example 56

### 2-Amino-4-(6-ethoxy-3,4,8,9-tetrahydro-3,3,8,8-tetramethylfuro[2,3-h]isoquinolin-1-yl)-N-methylbenzamide

To a suspension of 2-amino-4-(6-ethoxy-3,4,8,9-tetrahydro-3,3,8,8-tetramethylfuro[2,3-h]isoquinolin-1-yl)benzoic acid hydrochloride (691 mg, 1.55 mmol) in 1,2-dimethoxyethane (5 ml) were added methylamine hydrochloride (145 mg, 2.15 mmol), diethyl cyanophosphonate (0.24 ml, 1.58 mmol) and triethylamine (0.90 ml, 6.48 mmol), and the mixture was stirred at 80°C for 7 hours. After cooling, the reaction mixture was combined with water, and extracted twice with ethyl acetate. The combined organic layer was washed with a saturated aqueous solution of sodium chloride, dried over sodium sulfate, filtered, and concentrated under reduced pressure. The residue was purified with a basic silica gel column chromatography (hexane/ethyl acetate, 1:1 followed by 1:2) to give the title compound (254 mg, yield 39%).
Amorphous.
¹H NMR (CDCl₃) δ 1.23 (6H, s), 1.33 (6H, s), 1.46 (3H, t, J = 7.0 Hz), 2.35 (2H, s), 2.65 (2H, s), 2.97 (3H, d, J = 4.5 Hz), 4.17 (2H, q, J = 7.0 Hz), 5.56 (2H, br s), 6.28-6.38 (1H, br), 6.58 (1H, dd, J = 8.1, 1.0 Hz), 6.59 (1H, s), 6.72 (1H, d, J = 1.0 Hz), 7.28 (1H, d, J = 8.1 Hz).

### Example 57

### 2-(Benzoylamino)-4-(6-ethoxy-3,4,8,9-tetrahydro-3,3,8,8-tetramethylfuro[2,3-h]isoquinolin-1-yl)-N-methylbenzamide

To a suspension of 2-amino-4-(6-ethoxy-3,4,8,9-tetrahydro-3,3,8,8-tetramethylfuro[2,3-h]isoquinolin-1-yl)-N-methylbenzamide (122 mg, 0.289 mmol) in tetrahydrofuran (1 ml) were added triethylamine (0.044 ml, 0.318 mmol) and benzoyl chloride (0.037 ml, 0.318 mmol), and the mixture was stirred for 1 hour at room temperature. The reaction mixture was combined with ice water, neutralized with sodium hydrogen carbonate, and extracted twice with ethyl acetate. The combined organic layer was washed with a saturated aqueous solution of sodium chloride, dried over sodium sulfate, filtered, and concentrated under reduced pressure. The residue was purified with a basic silica gel column chromatography (hexane/ethyl acetate, 1:1 followed by 1:2), and the obtained crystals were washed with diisopropyl ether to give the title compound (77 mg, yield 51%).
¹H NMR (CDCl₃) δ 1.29 (12H, s), 1.46 (3H, t, J = 7.0 Hz), 2.28 (2H, s), 2.69 (2H, s), 3.12 (3H, d, J = 4.6 Hz), 4.18 (2H, q, J = 7.0 Hz), 6.59 (1H, s), 6.82 (1H, d, J = 7.8 Hz), 7.30-7.45 (4H, m), 7.70-7.85 (1H, br), 7.88-7.92 (2H, m), 8.92 (1H, s), 12.26 (1H, s).

### Example 58

### N-[5-(6-Ethoxy-3,4,8,9-tetrahydro-3,3,8,8-tetramethylfuro[2,3-h]isoquinolin-1-yl)-2-[(methylamino)carbonyl]phenyl]-2-pyridinecarboxamide

With 2-amino-4-(6-ethoxy-3,4,8,9-tetrahydro-3,3,8,8-tetramethylfuro[2,3-h]isoquinolin-1-yl)-N-methylbenzamide and picolinic acid chloride hydrochloride, the title compound was obtained by the method similar to that in Example 13. Yield 64%.
¹H NMR (CDCl₃) δ 1.28 (6H, s), 1.29 (6H, s), 1.46 (3H, t, J = 7.2 Hz), 2.29 (2H, br s), 2.68 (2H, s), 3.11 (3H, d, J = 4.8 Hz), 4.18 (2H, q, J = 7.2 Hz), 6.59 (1H, s), 6.85-6.95 (1H, br), 7.03 (1H, dd, J = 8.0 Hz), 7.38 (1H, dd, J = 7.7, 4.6 Hz), 7.49 (1H, d, J = 8.0 Hz), 7.78-7.87 (1H, m), 8.21 (1H, d, J = 7.7 Hz), 8.65 (1H, d, J = 4.6 Hz), 8.95 (1H, s).

### Example 59

### 4-(6-Ethoxy-3,4,8,9-tetrahydro-3,3,8,8-tetramethylfuro[2,3-h]isoquinolin-1-yl)-N-methyl-2-[(1-methylethylidene)amino]benzamide hydrochloride

2-Amino-4-(6-ethoxy-3,4,8,9-tetrahydro-3,3,8,8-tetramethylfuro[2,3-h]isoquinolin-1-yl)-N-methylbenzamide (352 mg, 0,835 mmol) was dissolved in ethyl acetate, a solution of 4 M hydrogen chloride/ethyl acetate was added thereto, and the mixture was concentrated under reduced pressure. The residue was combined with acetone, heated, and concentrated under reduced pressure to give the title compound (378 mg, yield 91%).
Amorphous.
¹H NMR (DMSO-d₆) δ 1.24 (3H, s), 1.27 (3H, s), 1.33-1.48 (15H, m), 2.34 (2H, s), 2.96 (3H, s), 3.09 (1H, d, J = 16.4 Hz), 3.21 (1H, d, J = 16.4 Hz), 4.23 (2H, q, J = 7.0 Hz), 6.82 (1H, s), 6.85 (1H, d, J = 7.8 Hz), 7.07 (1H, s), 7.31 (1H, s), 7.83 (1H, d, J = 7.8 Hz).

### Example 60

### N-[2-Amino-4-(6-ethoxy-3,4,8,9-tetrahydro-3,3,8,8-tetramethylfuro[2,3-h]isoquinolin-1-yl)benzoyl]glycine ethyl ester

With glycine ethyl ester hydrochloride, the title compound was obtained by the method similar to that in Example 56. Yield 59%.
¹H NMR (CDCl₃) δ 1.23-1.36 (15H, m), 1.46 (3H, t, J = 6.9 Hz), 2.37 (2H, s), 2.65 (2H, s), 4.12-4.26 (4H, m), 4.27 (2H, q, J = 6.9 Hz), 5.60 (2H, br s), 6.58 (1H, s), 6.58 (1H, dd, J = 8.2, 1.4 Hz), 6.76 (1H, d, J = 1.4 Hz), 6.86 (1H, t, J = 4.8 Hz), 7.37 (1H, d, J = 8.2 Hz).

### Example 61

### N-[4-(6-Ethoxy-3,4,8,9-tetrahydro-3,3,8,8-tetramethylfuro[2,3-h]isoquinolin-1-yl)-2-[(4-pyridinylcarbonyl)amino]benzoyl]glycine ethyl ester

With N-[2-amino-4-(6-ethoxy-3,4,8,9-tetrahydro-3,3,8,8-tetramethylfuro[2,3-h]isoquinolin-1-yl)benzoyl]glycine ethyl ester and isonicotinic acid chloride hydrochloride, the title compound was obtained by the method similar to that in Example 13. Yield 74%.
¹H NMR (CDCl₃) δ 1.28 (6H, s), 1.31 (6H, s), 1.35 (3H, t, J = 7.0 Hz), 1.46 (3H, t, J = 7.0 Hz), 2.32 (2H, br s), 2.69 (2H, s), 4.18 (2H, q, J = 7.0 Hz), 4.30 (2H, q, J = 7.0 Hz), 6.60 (1H, s), 7.04 (1H, d, J = 8.0 Hz), 7.58 (1H, d, J = 8.0 Hz), 7.74-7.76 (3H, m), 8.71-8.73 (2H, m), 8.84 (1H, d, J = 1.5 Hz).

### Example 62

### N-[4-(6-Ethoxy-3,4,8,9-tetrahydro-3,3,8,8-tetramethylfuro[2,3-h]isoquinolin-1-yl)-2-[(2-pyridinylcarbonyl)amino]benzoyl]glycine ethyl ester

With N-[2-amino-4-(6-ethoxy-3,4,8,9-tetrahydro-3,3,8,8-tetramethylfuro[2,3-h]isoquinolin-1-yl)benzoyl]glycine ethyl ester and picolinic acid chloride hydrochloride, the title compound was obtained by the method similar to that in Example 13. Yield 80%.
¹H NMR (CDCl₃) δ 1.26 (6H, s), 1.32 (6H, s), 1.33 (3H, t, J = 7.0 Hz), 1.47 (3H, t, J = 7.2 Hz), 2.36 (2H, s), 2.68 (2H, s), 4.19 (2H, q, J = 7.0 Hz), 4.23-4.34 (4H, m), 6.60 (1H, s), 6.93 (1H, t, J = 5.0 Hz), 7.18 (1H, dd, J = 8.1, 1.8 Hz), 7.45 (1H, ddd, J = 7.7, 4.8, 1.4 Hz), 7.65 (1H, d, J = 8.1), 7.87 (1H, td, J = 7.7, 1.8 Hz), 8.23 (1H, d, J = 7.7 Hz), 8.73 (1H, d, J = 4.8), 8.97 (1H, d, J = 1.4 Hz).

### Example 63

### N-[4-(6-Ethoxy-3,4,8,9-tetrahydro-3,3,8,8-tetramethylfuro[2,3-h]isoquinolin-1-yl)-2-[(2-pyridinylcarbonyl)amino]benzoyl]glycine

From N-[4-(6-ethoxy-3,4,8,9-tetrahydro-3,3,8,8-tetramethylfuro[2,3-h]isoquinolin-1-yl)-2-[(2-pyridinylcarbonyl)amino]benzoyl]glycine ethyl ester, the title compound was obtained by the method similar to that in Example 12. Yield 82%.
¹H NMR (CDCl₃) δ 1.30 (6H, s), 1.49 (6H, s), 1.49 (3H, t, J = 7.0 Hz), 2.14 (1H, d, J = 17.8 Hz), 2.52 (1H, d, J = 17.8 Hz), 2.75-3.10 (2H, br), 3.95 (2H, d, J = 4.8 Hz), 4.25 (2H, q, J = 7.0 Hz), 6.66 (1H, s), 7.34-7.41 (2H, m), 7.77-7.96 (3H, m), 8.14 (1H, d, J = 8.0 Hz), 8.66 (1H, d, J = 4.4 Hz), 8.95 (1H, s).

### Example 64

### N-[4-(6-Ethoxy-3,4,8,9-tetrahydro-3,3,8,8-tetramethylfuro[2,3-h]isoquinolin-1-yl)-2-[(3-pyridinylcarbonyl)amino]benzoyl]glycine ethyl ester

With N-[2-amino-4-(6-ethoxy-3,4,8,9-tetrahydro-3,3,8,8-tetramethylfuro[2,3-h]isoquinolin-1-yl)benzoyl]glycine ethyl ester and nicotinic acid chloride hydrochloride, the title compound was obtained by the method similar to that in Example 13. Yield 78%.
¹H NMR (CDCl₃) δ 1.28 (6H, s), 1.33 (6H, s), 1.34 (3H, t, J = 6.9 Hz), 1.47 (3H, t, J = 6.9 Hz), 2.35 (2H, br s), 2.69 (2H, s), 4.19 (2H, q, J = 6.9 Hz), 4.26-4.33 (4H, m), 6.60 (1H, s), 7.14 (1H, d, J = 7.8 Hz), 7.28-7.35 (1H, br), 7.38 (1H, dd, J = 8.1, 4.8 Hz), 7.63 (1H, d, J = 7.8 Hz), 8.21 (1H, dd, J = 8.1, 2.1 Hz), 8.73 (1H, d, J = 4.8 Hz), 8.87 (1H, s), 9.23 (1H, d, J = 2.1 Hz).

### Example 65

### N-[4-(6-Ethoxy-3,4,8,9-tetrahydro-3,3,8,8-tetramethylfuro[2,3-h]isoquinolin-1-yl)-2-[(3-pyridinylcarbonyl)amino]benzoyl]glycine

With N-[4-(6-ethoxy-3,4,8,9-tetrahydro-3,3,8,8-tetramethylfuro[2,3-h]isoquinolin-1-yl)-2-[(3-pyridinylcarbonyl)amino]benzoyl]glycine ethyl ester, the title compound was obtained by the method similar to that in Example 12. Yield 49%.
¹H NMR (CDCl₃) δ 1.31 (6H, s), 1.49 (6H, s), 1.49 (3H, t, J = 7.0 Hz), 2.22-2.53 (2H, br), 2.91 (2H, s), 3.78 (2H, s), 4.25 (2H, q, J = 7.0 Hz), 6.67 (1H, s), 6.95-7.35 (1H, br), 7.26-7.40 (2H, m), 7.88 (2H, d, J = 8.0 Hz), 8.21-8.26 (1H, m), 8.70 (1H, d, J = 4.4 Hz), 8.91 (1H, s), 9.24 (1H, s).

### Example 66

### N-[2-(Benzoylamino)-4-(6-ethoxy-3,4,8,9-tetrahydro-3,3,8,8-tetramethylfuro[2,3-h]isoquinolin-1-yl)benzoyl]glycine ethyl ester

With N-[2-amino-4-(6-ethoxy-3,4,8,9-tetrahydro-3,3,8,8-tetramethylfuro[2,3-h]isoquinolin-1-yl)benzoyl]glycine ethyl ester, the title compound was obtained by the method similar to that in Example 57. Yield 80%.
¹H NMR (CDCl₃) δ 1.29-1.35 (15H, m), 1.47 (3H, t, J = 6.9 Hz), 2.34 (2H, br s), 2.69 (2H, s), 4.19 (2H, q, J = 6.9 Hz), 4.28 (2H, q, J = 6.9 Hz), 6.59 (1H, s), 7.08 (1H, d, J = 7.8 Hz), 7.41-7.52 (4H, m), 7.63 (1H, d, J = 7.8 Hz), 7.95 (1H, d, J = 7.2 Hz), 8.91 (1H, s).

### Example 67

### N-[2-(Benzoylamino)-4-(6-ethoxy-3,4,8,9-tetrahydro-3,3,8,8-tetramethylfuro[2,3-h]isoquinolin-1-yl)benzoyl]glycine

With N-[2-(benzoylamino)-4-(6-ethoxy-3,4,8,9-tetrahydro-3,3,8,8-tetramethylfuro[2,3-h]isoquinolin-1-yl)benzoyl]glycine ethyl ester, the title compound was obtained by the method similar to that in Example 12. Yield 91%.
¹H NMR (DMSO-d₆) δ 1.16 (6H, s), 1.23 (6H, s), 1.34 (3H, t, J = 6.9 Hz), 2.42 (2H, br s), 2.65 (2H, s), 4.00 (2H, d, J = 4.0 Hz), 4.10 (2H, q, J = 6.9 Hz), 6.81 (1H, s), 7.24 (1H, d, J = 8.4 Hz), 7.56-7.64 (3H, m), 7.89-7.95 (3H, m), 8.70 (1H, s), 9.37 (1H, t, J = 4.0 Hz).

### Example 68

### N-[2-[[(E)-(Dimethylamino)methylidene]amino]-4-(6-ethoxy-3,4,8,9-tetrahydro-3,3,8,8-tetramethylfuro[2,3-h]isoquinolin-1-yl)benzoyl]glycine ethyl ester

With N-[2-amino-4-(6-ethoxy-3,4,8,9-tetrahydro-3,3,8,8-tetramethylfuro[2,3-h]isoquinolin-1-yl)benzoyl]glycine ethyl ester and 4-(acetamido)benzoic acid, the title compound was obtained by the method similar to that in Example 15. Yield 43%.
¹H NMR (CDCl₃) δ 1.21 (6H, s), 1.30 (6H, s), 1.30 (3H, t, J = 7.0 Hz), 1.46 (3H, t, J = 7.0 Hz), 2.25 (2H, s), 2.67 (2H, s), 3.11 (3H, s), 3.16 (3H, s), 4.18 (2H, q, J = 7.0 Hz), 4.24 (2H, q, J = 7.0 Hz), 4.25-4.36 (2H, br), 6.60 (1H, s), 6.90 (1H, d, J = 1.8 Hz), 7.08 (1H, dd, J = 8.1, 1.8 Hz), 7.69 (1H, s), 8.28 (1H, d, J = 8.1 Hz), 10.68 (1H, d, J = 4.6 Hz).

### Example 69

### 8-(6-Ethoxy-3,4,8,9-tetrahydro-3,3,8,8-tetramethylfuro[2,3-h]isoquinolin-1-yl)-4-methyl-3,4-dihydro-1H-1,4-benzodiazepine-2,5-dione

With sarcosine methyl ester hydrochloride, the title compound was obtained by the method similar to that in Example 56. Yield 76%.
¹H NMR (CDCl₃) δ 1.26 (6H, s), 1.34 (6H, s), 1.47 (3H, t, J = 7.0 Hz), 2.28 (2H, s), 2.68 (2H, s), 3.29 (3H, s), 3.87 (2H, s), 4.19 (2H, q, J = 7.0 Hz), 6.61 (1H, s), 7.12 (1H, s), 7.27 (1H, d, J = 8.1 Hz), 7.97 (1H, d, J = 8.1 Hz), 7.98 (1H, s).

### Example 70

### N-[4-(6-Ethoxy-3,4,8,9-tetrahydro-3,3,8,8-tetramethylfuro[2,3-h]isoquinolin-1-yl)-2-[(phenylmethyl)amino]benzoyl]glycine ethyl ester

To a solution of 4-(6-ethoxy-3,4,8,9-tetrahydro-3,3,8,8-tetramethylfuro[2,3-h]isoquinolin-1-yl)-2-[(phenylmethyl)amino]benzoic acid (810 mg, 1.62 mmol), glycine ethyl ester hydrochloride (249 mg, 1.79 mmol) and 1-hydroxy-1H-benzotriazole monohydrate (274 mg, 1.79 mmol) in N,N-dimethylformamide (6 ml) was added 1-ethyl-3-(3-dimethylaminopropyl)carbodiimide hydrochloride (404 mg, 2.11 mmol), and the mixture was stirred for 1 hour at room temperature. The reaction mixture was combined with ice water, and extracted three times with ethyl acetate. The combined organic layer was washed with a saturated aqueous solution of sodium chloride, dried over sodium sulfate, filtered, and concentrated under reduced pressure. The residue was purified with a basic silica gel column chromatography (hexane/ethyl acetate 2:1), and crystallized from diethyl ether-hexane to give the title compound (713 mg, yield 75%).
¹H NMR (CDCl₃) δ 1.22 (6H, s), 1.31 (6H, s), 1.32 (3H, t, J = 7.0 Hz), 1.46 (3H, t, J = 7.0 Hz), 2.26 (2H, s), 2.65 (2H, s), 4.17 (2H, q, J = 7.0 Hz), 4.26 (2H, q, J = 7.0 Hz), 4.38 (2H, d, J = 5.4 Hz), 6.57 (1H, s), 6.60 (1H, d, J = 8.0 Hz), 6.71 (1H, s), 7.19-7.35 (5H, m), 7.45 (1H, d, J = 8.0 Hz), 8.03 (1H, t, J = 5.4 Hz).

### Example 71

### N-[4-(6-Ethoxy-3,4,8,9-tetrahydro-3,3,8,8-tetramethylfuro[2,3-h]isoquinolin-1-yl)-2-[(phenylmethyl)amino]benzoyl]glycine

From N-[4-(6-ethoxy-3,4,8,9-tetrahydro-3,3,8,8-tetramethylfuro[2,3-h]isoquinolin-1-yl)-2-[(phenylmethyl)amino]benzoyl]glycine ethyl ester, the title compound was obtained by the method similar to that in Example 9. Yield 85%.
¹H NMR (CDCl₃) δ 1.29 (6H, s), 1.46-1.53 (9H, m), 2.16 (1H, d, J = 12.8 Hz), 2.31 (1H, d, J = 12.8 Hz), 2.91 (1H, d, J = 13.2 Hz), 3.01 (1H, d, J = 13.2 Hz), 3.77 (2H, s), 4.24 (2H, q, J = 7.0 Hz), 4.23 (2H, s), 6.59 (1H, d, J = 8.0 Hz), 6.66 (1H, s), 6.72 (1H, s), 7.17-7.37 (5H, m), 7.57 (1H, d, J = 8.0 Hz), 8.27 (1H, br s).

### Example 72

### N-(2-Amino-2-oxoethyl)-4-(6-ethoxy-3,4,8,9-tetrahydro-3,3,8,8-tetramethylfuro[2,3-h]isoquinolin-1-yl)-2-[(phenylmethyl)amino]benzamide hydrochloride

To a solution of 4-(6-ethoxy-3,4,8,9-tetrahydro-3,3,8,8-tetramethylfuro[2,3-h]isoquinolin-1-yl)-2-[(phenylmethyl)amino]benzoic acid (301 mg, 0.604 mmol), glycinamide hydrochloride (73 mg, 0.664 mmol), 1-hydroxy-1H-benzotriazole monohydrate (102 mg, 0.664 mmol) and triethylamine (0.093 ml, 0.664 mmol) in N,N-dimethylformamide (3 ml) was added 1-ethyl-3-(3-dimethylaminopropyl)carbodiimide hydrochloride (151 mg, 0.785 mmol) under ice-cooling, and the mixture was stirred at room temperature for 3 hours. To the reaction mixture was poured ice water, and the mixture was extracted three times with a mixed solution of ethyl acetate-tetrahydrofuran. The combined organic layer was washed with a saturated aqueous solution of sodium chloride, dried over sodium sulfate, filtered, and concentrated under reduced pressure. The residue was purified with a basic silica gel column chromatography (ethyl acetate followed by ethyl acetate/methanol 50:1) to give the title compound as free base. This was dissolved in ethyl acetate, a solution of 4 M hydrogen chloride/ethyl acetate was added thereto, and the mixture was concentrated under reduced pressure to give the title compound (237 mg, yield 66%).
Amorphous.
¹H NMR (DMSO-d₆) δ 1.21 (3H, s), 1.24 (3H, s), 1.37 (3H, t, J = 7.0 Hz), 1.43 (6H, s), 2.20 (1H, d, J = 17.0 Hz), 2.34 (1H, d, J = 17.0 Hz), 3.13 (2H, s), 3.80 (1H, d, J = 9.6 Hz), 3.82 (1H, d, J = 9.6 Hz), 4.23 (2H, q, J = 7.0 Hz), 4.43 (2H, d, J = 5.4 Hz), 6.75 (1H, dd, J = 8.0, 1.4 Hz), 6.91 (1H, d, J = 1.4 Hz), 7.06 (2H, s), 7.25-7.35 (5H, m), 7.48 (1H, s), 7.89 (1H, d, J = 8.0 Hz), 8.86 (1H, t, J = 5.4 Hz) .

### Example 73

### 4-(6-Ethoxy-3,4,8,9-tetrahydro-3,3,8,8-tetramethylfuro[2,3-h]isoquinolin-1-yl)-2-[(phenylmethyl)amino]benzamide

From 4-(6-ethoxy-3,4,8,9-tetrahydro-3,3,8,8-tetramethylfuro[2,3-h]isoquinolin-1-yl)-2-[(phenylmethyl)amino]benzoic acid, the title compound was obtained by the method similar to that in Example 10. Yield 74%.
¹H NMR (CDCl₃) δ 1.22 (6H, s), 1.30 (6H, s), 1.46 (3H, t, J = 6.9 Hz), 2.26 (2H, s), 2.65 (2H, s), 4.17 (2H, q, J = 6.9 Hz), 4.40 (2H, d, J = 5.4 Hz), 5.45-6.10 (2H, br), 6.56 (1H, dd, J = 8.1, 1.5 Hz), 6.57 (1H, s), 6.71 (1H, d, J = 1.5 Hz), 7.23-7.40 (6H, m), 8.29 (1H, t, J = 5.4 Hz).

### Example 74

### 4-(6-Ethoxy-3,4,8,9-tetrahydro-3,3,8,8-tetramethylfuro[2,3-h]isoquinolin-1-yl)-2-[(phenylmethyl)amino]benzonitrile

To a solution of 4-(6-ethoxy-3,4,8,9-tetrahydro-3,3,8,8-tetramethylfuro[2,3-h]isoquinolin-1-yl)-2-[(phenylmethyl)amino]benzamide (2.97 g, 5.96 mmol) in chloroform (20 ml) was added phosphorus oxychloride (2.78 ml, 29.8 mmol), and the mixture was stirred for 1 hour at 85°C. After cooling, the reaction mixture was poured into ice water, neutralized with sodium hydrogen carbonate, and extracted twice with ethyl acetate. The combined organic layer was washed with a saturated aqueous solution of sodium chloride, dried over sodium sulfate, filtered, and concentrated under reduced pressure. The resultant crystals were washed with hexane to give the title compound (2.57 g, yield 90%).
¹H NMR (CDCl₃) δ 1.23 (6H, s), 1.26 (6H, s), 1.46 (3H, d, J = 7.0 Hz), 2.21 (2H, s), 2.66 (2H, s), 4.17 (2H, q, J = 7.0 Hz), 4.43 (2H, d, J = 5.6 Hz), 4.97 (1H, t, J = 5.6 Hz), 6.58 (1H, s), 6.70 (1H, dd, J = 7.8, 1.2 Hz), 6.73 (1H, d, J = 1.2 Hz), 7.30-7.38 (5H, m), 7.43 (1H, d, J = 7.8 Hz).

### Example 75

### N-[2-Amino-4-(6-ethoxy-3,4,8,9-tetrahydro-3,3,8,8-tetramethylfuro[2,3-h]isoquinolin-1-yl)benzoyl]-2-methylalanine ethyl ester

With ethyl 2-aminoisobutyrate hydrochloride, the title compound was obtained by the method similar to that in Example 72. Yield 32%.
¹H NMR (CDCl₃) δ 1.22 (6H, s), 1.28 (3H, t, J = 7.0 Hz), 1.35 (6H, s), 1.46 (3H, t, J = 7.0 Hz), 1.70 (6H, s), 2.39 (2H, s), 2.65 (2H, s), 4.18 (2H, q, J = 7.0 Hz), 4.23 (2H, q, J = 7.0 Hz), 5.53 (2H, s), 6.59 (1H, s), 6.62 (1H, dd, J = 8.0, 1.8 Hz), 6.73 (1H, d, J = 1.8 Hz), 7.34 (1H, d, J = 8.0 Hz).

### Example 76

### N-[4-(6-Ethoxy-3,4,8,9-tetrahydro-3,3,8,8-tetramethylfuro[2,3-h]isoquinolin-1-yl)-2-[(phenylmethyl)amino]benzoyl]-2-methylalanine ethyl ester hydrochloride

The free base of the title compound which was obtained at the same time in Example 75, was dissolved in ethyl acetate, a solution of 4 M hydrogen chloride/ethyl acetate was added thereto, and the mixture was concentrated under reduced pressure to give the title compound. Yield 11%.
Amorphous.
¹H NMR (DMSO-d₆) δ 1.08 (3H, t, J = 7.0 Hz), 1.19 (3H, s), 1.25 (3H, s), 1.37 (3H, t, J = 7.0 Hz), 1.42 (6H, s), 1.47 (6H, s), 2.07 (1H, d, J = 14.0 Hz), 2.27 (1H, d, J = 14.0 Hz), 3.13 (2H, s), 4.03 (2H, q, J = 7.0 Hz), 4.23 (2H, q, J = 7.0 Hz), 4.43 (2H, s), 6.74 (1H, d, J = 7.8 Hz), 6.91 (1H, s), 7.06 (1H, s), 7.20-7.40 (5H, m), 7.87 (1H, d, J = 7.8 Hz), 8.24 (1H, br s), 8.76 (1H, s).

### Example 77

### N-[2-Amino-4-(6-ethoxy-3,4,8,9-tetrahydro-3,3,8,8-tetramethylfuro[2,3-h]isoquinolin-1-yl)benzoyl]-2-methylalanine

To a solution of N-[[2-amino-4-(6-ethoxy-3,4,8,9-tetrahydro-3,3,8,8-tetramethylfuro[2,3-h]isoquinolin-1-yl)benzoyl]-2-methylalanine ethyl ester (299 mg, 0.573 mmol) in methanol (2 ml) was added 5 M aqueous solution (0.5 ml) of sodium hydroxide, and the mixture was stirred at room temperature for 5 hours. The solvent was distilled off under reduced pressure, and the residue was combined with water, and neutralized with 5 M hydrochloric acid. The residue was combined with diethyl ether, and crystallized to give the title compound (73 mg, yield 26%).
¹H NMR (CDCl₃) δ 1.34 (6H, s), 1.48 (6H, s), 1.48 (3H, t, J = 7.0 Hz), 1.60 (6H, s), 2.37 (2H, s), 2.90 (2H, br s), 4.24 (2H, q, J = 7.0 Hz), 5.00-5.50 (2H, br s), 6.56 (1H, d, J = 8.0 Hz), 6.64 (1H, s), 6.71 (1H, s), 7.31 (1H, d, J = 8.0 Hz), 7.42 (1H, s).

### Example 78

### N-[4-(6-Ethoxy-3,4,8,9-tetrahydro-3,3,8,8-tetramethylfuro[2,3-h]isoquinolin-1-yl)-2-[(2-pyridinylcarbonyl)amino]benzoyl]-2-methylalanine ethyl ester

With N-[2-amino-4-(6-ethoxy-3,4,8,9-tetrahydro-3,3,8,8-tetramethylfuro[2,3-h]isoquinolin-1-yl)benzoyl]-2-methylalanine ethyl ester and picolinic acid chloride hydrochloride, the title compound was obtained by the method similar to that in Example 13. Yield 71%.
¹H NMR (CDCl₃) δ 1.22 (3H, t, J = 7.2 Hz), 1.25 (6H, s), 1.33 (6H, s), 1.47 (3H, t, J = 7.2 Hz), 1.74 (6H, s), 2.38 (2H, br s), 2.67 (2H, s), 4.16-4.26 (4H, m), 6.60 (1H, s), 6.81 (1H, s), 7.20 (1H, d, J = 7.8 Hz), 7.43-7.47 (1H, m), 7.58 (1H, d, J = 7.8 Hz), 7.83-7.89 (1H, m), 8.21 (1H, d, J = 7.8 Hz), 8.71 (1H, d, J = 4.8 Hz), 8.89 (1H, s).

### Example 79

### N-[4-(6-Ethoxy-3,4,8,9-tetrahydro-3,3,8,8-tetramethylfuro[2,3-h]isoquinolin-1-yl)-2-[(2-pyridinylcarbonyl)amino]benzoyl]-2-methylalanine hydrochloride

With N-[4-(6-ethoxy-3,4,8,9-tetrahydro-3,3,8,8-tetramethylfuro[2,3-h]isoquinolin-1-yl)-2-[(2-pyridinylcarbonyl)amino]benzoyl]-2-methylalanine ethyl ester, the title compound was obtained by the method similar to that in Example 12. Yield 49%.
¹H NMR (CDCl₃) δ 1.30 (3H, s), 1.36 (3H, s), 1.48-1.55 (6H, m), 1.68 (6H, s), 1.86 (3H, s), 2.26 (1H, d, J = 16.7 Hz), 2.71 (1H, d, J = 16.7 Hz), 2.82 (1H, d, J = 17.0 Hz), 3.27 (1H, d, J = 17.0 Hz), 4.29 (2H, q, J = 7.0 Hz), 6.71 (1H, s), 7.42-7.52 (1H, m), 7.75-8.02 (4H, m), 8.14 (1H, d, J = 7.6 Hz), 8.74 (1H, d, J = 4.4 Hz), 8.89 (1H, s).

### Example 80

### N-[4-(6-Ethoxy-3,4,8,9-tetrahydro-3,3,8,8-tetramethylfuro[2,3-h]isoquinolin-1-yl)-2-[(phenylmethyl)amino]benzoyl]-2-methylalanine hydrochloride

From N-[4-(6-ethoxy-3,4,8,9-tetrahydro-3,3,8,8-tetramethylfuro[2,3-h]isoquinolin-1-yl)-2-[(phenylmethyl)amino]benzoyl]-2-methylalanine ethyl ester hydrochloride, the title compound was obtained as free base by the method similar to that in Example 9. This was dissolved in ethyl acetate, a solution of 4 M hydrogen chloride/ethyl acetate was added thereto, and the mixture was concentrated under reduced pressure to give the title compound. Yield 73%.
Amorphous.
¹H NMR (DMSO-d₆) δ 1.21 (3H, s), 1.25 (3H, s), 1.37 (3H, t, J = 7.0 Hz), 1.43 (6H, s), 1.47 (6H, s), 2.21 (1H, d, J = 17.6 Hz), 2.36 (1H, d, J = 17.6 Hz), 3.13 (2H, s), 4.23 (2H, q, J = 7.0 Hz), 4.43 (2H, s), 6.74 (1H, d, J = 8.4 Hz), 6.91 (1H, s), 7.06 (1H, s), 7.25-7.40 (6H, m), 7.90 (1H, d, J = 8.4 Hz), 8.36 (1H, br s), 8.61 (1H, s).

### Example 81

### Methyl 4-(6-ethoxy-3,4,8,9-tetrahydro-3,3,8,8-tetramethylfuro[2,3-h]isoquinolin-1-yl)-2-hydroxybenzoate hydrochloride

From methyl 4-cyano-2-hydroxybenzoate methyl, the title compound was obtained by the method similar to that in Example 2. Yield 38%.
¹H NMR (DMSO-d₆) δ 1.25 (6H, s), 1.37 (3H, t, J = 7.0 Hz), 1.44 (6H, s), 2.30 (2H, s), 3.14 (2H, s), 3.92 (3H, s), 4.24 (2H, q, J = 7.0 Hz), 7.08 (1H, s), 7.11 (1H, dd, J = 8.0, 1.4 Hz), 7.30 (1H, d, J = 1.4 Hz), 7.95 (1H, d, J = 8.0 Hz), 10.86 (1H, s).

### Example 82

### Methyl 4-(6-ethoxy-3,4,8,9-tetrahydro-3,3,8,8-tetramethylfuro[2,3-h]isoquinolin-1-yl)-2-(phenylmethoxy)benzoate hydrochloride

To a solution of methyl 4-(6-ethoxy-3,4,8,9-tetrahydro-3,3,8,8-tetramethylfuro[2,3-h]isoquinolin-1-yl)-2-hydroxybenzoate hydrochloride (614 mg, 1.33 mmol) in N,N-dimethylformamide (5 ml) were added potassium tert-butoxide (313 mg, 2.79 mmol) and benzyl bromide (0.17 ml, 1.47 mmol), and the mixture was stirred at room temperature for 2 hours and at 50°C for 2 hours. Potassium tert-butoxide (149 mg, 1.33 mmol) and benzyl bromide (0.16 ml, 1.33 mmol) were further added thereto, and the mixture was stirred at room temperature for 2 hours. The reaction mixture was combined with ice water, and extracted twice with ethyl acetate. The combined organic layer was washed with 0.5 M aqueous solution of sodium hydroxide, water and a saturated aqueous solution of sodium chloride, dried over sodium sulfate, and concentrated under reduced pressure. The residue was purified with a silica gel column chromatography (hexane/ethyl acetate, 5:1 followed by 3:1) to give the title compound as free base. This was dissolved in ethyl acetate, a solution of 4 M hydrogen chloride/ethyl acetate was added thereto, and the mixture was concentrated under reduced pressure, and the residue was crystallized from ethyl acetate-diisopropyl ether to give the title compound (271 mg, yield 37%). ¹H NMR (DMSO-d₆) δ 1.23 (6H, s), 1.37 (3H, t, J = 7.0 Hz), 1.46 (6H, s), 2.24 (2H, s), 3.14 (2H, s), 3.87 (3H, s), 4.25 (2H, q, J = 7.0 Hz), 5.23 (2H, s), 7.09 (1H, s), 7.26 (1H, d, J = 7.8 Hz), 7.34-7.52 (5H, m), 7.60 (1H, s), 7.91 (1H, d, J = 7.8 Hz).

### Example 83

### 4-(6-Ethoxy-3,4,8,9-tetrahydro-3,3,8,8-tetramethylfuro[2,3-h]isoquinolin-1-yl)-2-(phenylmethoxy)benzoic acid hydrochloride

From methyl 4-(6-ethoxy-3,4,8,9-tetrahydro-3,3,8,8-tetramethylfuro[2,3-h]isoquinolin-1-yl)-2-(phenylmethoxy)benzoate hydrochloride, the title compound was obtained as free base by the method similar to that in Example 9. This was dissolved in ethyl acetate, a solution of 4 M hydrogen chloride/ethyl acetate was added thereto, and the mixture was concentrated under reduced pressure to give the title compound. Yield 85%.
Amorphous.
¹H NMR (DMSO-d₆) δ 1.24 (6H, s), 1.38 (3H, t, J = 7.0 Hz), 1.45 (6H, s), 2.26 (2H, s), 3.15 (2H, s), 3.87 (3H, s), 4.24 (2H, q, J = 7.0 Hz), 5.28 (2H, s), 7.09 (1H, s), 7.20-7.53 (7H, m), 7.86 (1H, d, J = 7.6 Hz).

### Example 84

### Methyl (E)-3-[4-(6-ethoxy-3,4,8,9-tetrahydro-3,3,8,8-tetramethylfuro[2,3-h]isoquinolin-1-yl)-2-methoxyphenyl]-2-propenoate

### (Method 1)

To a solution of 7-ethoxy-2,3-dihydro-2,2-dimethyl-α-(1-methylethyl)-5-benzofuranmethanol (799 mg, 3.02 mmol) and methyl (E)-3-(4-cyano-2-methoxyphenyl)-2-propenoate (547 mg, 2.52 mmol) in toluene (3.5 ml) and acetic acid (2 ml) was added conc. sulfuric acid (0.34 ml, 6.30 mmol), and the mixture was stirred for 2 hours at 70°C. After cooling, the reaction mixture was combined with ice water, washed with diisopropyl ether, neutralized with sodium hydrogen carbonate, and extracted twice with ethyl acetate. The combined organic layer was washed with a saturated aqueous solution of sodium chloride, dried over sodium sulfate, filtered, and concentrated under reduced pressure. The residue was purified with a basic silica gel column chromatography (hexane/ethyl acetate, 10:1 followed by 5:1), and crystallized from diisopropyl ether-hexane to give the title compound (376 mg, yield 32%).
¹H NMR (CDCl₃) δ 1.24 (6H, s), 1.33 (6H, s), 1.47 (3H, t, J = 7.0 Hz), 2.29 (2H, s), 2.67 (2H, s), 3.81 (3H, s), 3.91 (3H, s), 4.19 (2H, q, J = 7.0 Hz), 6.57 (1H, d, J = 16.2 Hz), 6.61 (1H, s), 6.96 (1H, s), 6.98 (1H, d, J = 7.8 Hz), 7.52 (1H, d, J = 7.8 Hz), 8.01 (1H, d, J = 16.2 Hz).

### (Method 2)

Methyl (E)-3-(4-cyano-2-methoxyphenyl)-2-propenoate (800 mg) was suspended in methyl acetate (8 ml), and trifluoromethanesulfonic acid(3.25 ml) was added dropwise thereto. Then, a solution of 7-ethoxy-2,3-dihydro-2,2-dimethyl-α-(1-methylethyl)-5-benzofuranmethanol (1.95 g) in methyl acetate (22 ml) was slowly added dropwise thereto at around 60°C. The mixture was stirred for 1 hour at around 60°C. Ice-cooled 25% ammonia water (6 ml) was added dropwise thereto. Toluene and water were further added thereto and the layers were separated. The organic layer was washed with water. The organic layer was extracted three times with 1 M hydrochloric acid. The organic layer was further combined with diisopropyl ether and further extracted with 1 M hydrochloric acid. The aqueous layer was combined, and washed with diisopropyl ether. The aqueous layer was alkalified by adding sodium hydrogen carbonate. The mixture was extracted with ethyl acetate. The extracts were washed with water, dried with anhydrous sodium sulfate, and concentrated under reduced pressure. The residue was subjected to a basic silica gel chromatography (20 g), and eluted with hexane/ethyl acetate=5/1 to give the title compound (1.45 g, yield 85%).

### Example 85

### (E)-3-[4-(6-Ethoxy-3,4,8,9-tetrahydro-3,3,8,8-tetramethylfuro[2,3-h]isoquinolin-1-yl)-2-methoxyphenyl]-2-propenoic acid

To a suspension of methyl (E)-3-[4-(6-ethoxy-3,4,8,9-tetrahydro-3,3,8,8-tetramethylfuro[2,3-h]isoquinolin-1-yl)-2-methoxyphenyl]-2-propenoate (277 mg, 0.598 mmol) in methanol (2 ml) was added 5 M aqueous solution (0.5 ml) of sodium hydroxide, and the mixture was stirred at room temperature for 1 hour and at 50°C for 30 minutes. After cooling, methanol was distilled off under reduced pressure, and the residue was combined with water and washed with diisopropyl ether. The aqueous layer was adjusted at pH 4.5 with 5 M hydrochloric acid, and extracted three times with a mixed solution of ethyl acetate-tetrahydrofuran. The combined organic layer was dried over sodium sulfate, filtered, concentrated under reduced pressure, and recrystallized from acetone-diisopropyl ether to give the title compound (240 mg, yield 89%).
¹H NMR (CDCl₃) δ 1.33 (6H, s), 1.38 (6H, s), 1.48 (3H, t, J = 7.0 Hz), 2.30 (2H, s), 2.78 (2H, s), 3.94 (3H, s), 4.21 (2H, q, J = 7.0 Hz), 6.48 (1H, d, J = 16.2 Hz), 6.64 (1H, s), 6.97 (1H, d, J = 7.9 Hz), 7.08 (1H, s), 7.50 (1H, d, J = 7.9 Hz), 7.91 (1H, d, J = 16.2 Hz).

### Example 86

### Methyl (E)-3-[4-(6-ethoxy-3,4,8,9-tetrahydro-3,3,8,8-tetramethylfuro[2,3-h]isoquinolin-1-yl)-2-hydroxyphenyl]-2-propenoate

From methyl (E)-3-[4-cyano-2-(phenylmethoxy)phenyl]-2-propenoate, the title compound was obtained by the method similar to that in Example 84. Yield 22%.
¹H NMR (CDCl₃) δ 1.28 (12H, s), 1.46 (3H, t, J = 7.0 Hz), 2.25 (2H, s), 2.78 (2H, br s), 3.77 (3H, s), 4.19 (2H, q, J = 7.0 Hz), 6.34 (1H, d, J = 16.1 Hz), 6.58 (1H, dd, J = 8.0, 1.4 Hz), 6.60 (1H, s), 6.82 (1H, d, J = 1.4 Hz), 7.16 (1H, d, J = 8.0 Hz), 7.82 (1H, d, J = 16.1 Hz).

### Example 87

### Methyl (E)-3-[4-(6-ethoxy-3,4,8,9-tetrahydro-3,3,8,8-tetramethylfuro[2,3-h]isoquinolin-1-yl)-2-(phenylmethoxy)phenyl]-2-propenoate hydrochloride

To a solution of methyl (E)-3-[4-(6-ethoxy-3,4,8,9-tetrahydro-3,3,8,8-tetramethylfuro[2,3-h]isoquinolin-1-yl)-2-hydroxy phenyl]-2-propenoate (283 mg, 0.630 mmol) in N,N-dimethylformamide (2.5 ml) were added potassium carbonate (96 mg, 0.692 mmol) and benzyl bromide (0.082 ml, 0.692 mmol), and the mixture was stirred at 60°C for 4 hours. After cooling, water was poured to the reaction mixture, and the mixture was extracted twice with ethyl acetate. The combined organic layer was washed with water and a saturated aqueous solution of sodium chloride, dried over sodium sulfate, and concentrated under reduced pressure. The residue was purified with a basic silica gel column chromatography (hexane/ethyl acetate, 20:1 followed by 7:1) to give the title compound as free base. This was dissolved in ethyl acetate, a solution of 4 M hydrogen chloride/ethyl acetate was added thereto, and the mixture was concentrated under reduced pressure to give the title compound (224 mg, yield 62%).
¹H NMR (DMSO-d₆) δ 1.24 (6H, s), 1.46 (3H, t, J = 6.8 Hz), 1.45 (6H, s), 2.28 (2H, s), 3.14 (2H, s), 3.72 (3H, s), 4.25 (2H, q, J = 6.8 Hz), 5,29 (2H, s), 6.84 (1H, d, J = 16.4 Hz), 7.09 (1H, s), 7.23 (1H, d, J = 8.2 Hz), 7.30-7.56 (6H, m), 7.95 (1H, d, J = 16.4 Hz), 8.04 (1H, d, J = 8.2 Hz).

### Example 88

### (E)-3-[4-(6-Ethoxy-3,4,8,9-tetrahydro-3,3,8,8-tetramethylfuro[2,3-h]isoquinolin-1-yl)-2-(phenylmethoxy)phenyl]-2-propenoic acid

From methyl (E)-3-[4-(6-ethoxy-3,4,8,9-tetrahydro-3,3,8,8-tetramethylfuro[2,3-h]isoquinolin-1-yl)-2-(phenylmethoxy)phenyl]-2-propenoate, the title compound was obtained by the method similar to that in Example 9. Yield 63%.
¹H NMR (CDCl₃) δ 1.30 (12H, s), 1.47 (3H, t, J = 7.0 Hz), 2.21 (2H, s), 2.72 (2H, s), 4.19 (2H, q, J = 7.0 Hz), 5,16 (2H, s), 6.50 (1H, d, J = 16.2 Hz), 6.61 (1H, s), 6.96 (1H, d, J = 8.0 Hz), 7.08 (1H, s), 7.29-7.43 (5H, m), 7.52 (1H, d, J = 8.0 Hz), 8.03 (1H, d, J = 16.2 Hz).

### Example 89

### 5-(6-Ethoxy-3,4,8,9-tetrahydro-3,3,8,8-tetramethylfuro[2,3-h]isoquinolin-1-yl)-2-(phenylmethyl)-1H-isoindol-1,3(2H)-dione

A suspension of a solution of 4-bromophthalic anhydride (854 mg, 1.72 mmol) in 28% sodium methoxide/methanol (8 ml) and tetrahydrofuran (8 ml) was heated under reflux for 10 minutes. After cooling, a 1:1 mixture of the precipitated 2-methyl 4-bromo-1,2-benzenedicarboxylate 1-sodium salt and 1-methyl 4-bromo-1,2-benzenedicarboxylate 2-sodium salt was taken by filtration, and washed with diethyl ether. The filtrate was concentrated under reduced pressure, the residue was combined with ice water, acidified with 2 M hydrochloric acid, and extracted twice with ethyl acetate. The combined organic layer was washed with water and a saturated aqueous solution of sodium chloride, dried over magnesium sulfate, filtered, concentrated under reduced pressure, and crystallized from diisopropyl ether-hexane to give 1:1 mixture of 4-bromo-1,2-benzenedicarboxylic acid 1-methyl ester and 4-bromo-1,2-benzenedicarboxylic acid 2-methyl ester (1.16 g, yield 20%). Furthermore, the mother liquor was concentrated to give the same mixture (1.63 g, yield 29%) as an oily matter. The mixture of the sodium salts was dissolved in water, acidified with 2 M hydrochloric acid, and extracted twice with ethyl acetate. The combined organic layer was washed with water and a saturated aqueous solution of sodium chloride, dried over magnesium sulfate, filtered, and concentrated under reduced pressure to give 1:1 mixture of 4-bromo-1,2-benzenedicarboxylic acid 1-methyl ester and 4-bromo-1,2-benzenedicarboxylic acid 2-methyl ester (1.41 g, yield 25%). A suspension of the resultant mixture (210 mg, 0.811 mmol) of bromo forms, zinc cyanate (52 mg, 0.446 mmol) and tetrakis(triphenylphosphine)palladium(0) (19 mg, 0.0162 mmol) in toluene (1.6 ml) and N-methyl-2-pyrrolidone (0.4 ml) was stirred at 100°C for 8 hours. After cooling, the reaction mixture was combined with water, and extracted twice with ethyl acetate. The combined organic layer was washed with a saturated aqueous solution of sodium chloride, dried over sodium sulfate, filtered, and concentrated under reduced pressure. The residue was dissolved in toluene (1.5 ml) and acetic acid (1 ml). 7-Ethoxy-2,3-dihydro-2,2-dimethyl-α-(1-methylethyl)-5-benzofuranmethanol (214 mg, 0.811 mmol) and conc. sulfuric acid (0.11 ml, 2.03 mmol) were added thereto, and the mixture was stirred for 2 hours at 80°C. After cooling, the reaction mixture was combined with ice water, washed with diisopropyl ether, neutralized with sodium hydrogen carbonate, and extracted twice with ethyl acetate. The combined organic layer was washed with a saturated aqueous solution of sodium chloride, dried over sodium sulfate, filtered, and concentrated under reduced pressure to give a crude product (130 mg) containing 1:1 mixture of 4-(6-ethoxy-3,4,8,9-tetrahydro-3,3,8,8-tetramethylfuro[2,3-h]isoquinolin-1-yl)-1,2-benzenedicarboxylic acid 1-methyl ester and 4-(6-ethoxy-3,4,8,9-tetrahydro-3,3,8,8-tetramethylfuro[2,3-h]isoquinolin-1-yl)-1,2-benzenedicarboxylic acid 2-methyl ester. This product (120 mg) without purification was dissolved in N,N-dimethylformamide (2 ml). Benzylamine (43 mg, 0.399 mmol), 1-hydroxy-1H-benzotriazole monohydrate (65 mg, 0.426 mmol) and 1-ethyl-3-(3-dimethylaminopropyl)carbodiimide hydrochloride (92 mg, 0.478 mmol) were added thereto, and the mixture was stirred at room temperature for 15 hours. The reaction mixture was combined with ice water, and extracted twice with ethyl acetate. The combined organic layer was washed with water and a saturated aqueous solution of sodium chloride, dried over sodium sulfate, filtered, and concentrated under reduced pressure. The residue was subjected to a silica gel column chromatography to give the title compound (26 mg, yield 1.8%).
¹H NMR (CDCl₃) δ 1.24 (6H, s), 1.31 (6H, s), 1.46 (3H, t, J = 7.0 Hz), 2.16 (2H, s), 2.68 (2H, s), 4.19 (2H, q, J = 7.0 Hz), 4.87 (2H, s), 6.62 (1H, s), 7.28-7.38 (3H, m), 7.46 (2H, dd, J = 7.6, 1.6 Hz), 7.72 (1H, d, J = 7.6, 1.3 Hz), 7.84 (1H, d, J = 7.6 Hz), 7.94 (1H, d, J = 1.3 Hz).

### Example 90

### 5-(6-Ethoxy-3,4,8,9-tetrahydro-3,3,8,8-tetramethylfuro[2,3-h]isoquinolin-1-yl)-1H-isoindol-1,3(2H)-dione

To a solution of 7-ethoxy-2,3-dihydro-2,2-dimethyl-α-(1-methylethyl)-5-benzofuranmethanol (17.4 g, 65.9 mmol) and 2,3-dihydro-1,3-dioxo-1H-isoindol-5-carbonitrile (6.30 g, 35.8 mmol) in toluene (40 ml) and acetic acid (25 ml) was added conc. sulfuric acid (9.46 ml, 55.0 mmol) under ice-cooling, and the mixture was stirred for 30 minutes at 80°C. Ethanol was added dropwise thereto, and the mixture was stirred for 30 minutes at the same temperature. After cooling, the reaction mixture was combined with ice water, washed with diisopropyl ether, neutralized with sodium hydrogen carbonate, and extracted three times with ethyl acetate. The combined organic layer was extracted three times with 1 M hydrochloric acid. The combined aqueous layer was neutralized with sodium hydrogen carbonate, and extracted twice with ethyl acetate. The combined organic layer was washed with a saturated aqueous solution of sodium chloride, dried over sodium sulfate, filtered, and concentrated under reduced pressure. To the residue was added ethyl acetate, and the precipitated crystals were taken and removed by filtration. The filtrate was concentrated under reduced pressure, the residue was purified with a silica gel column chromatography (hexane/ethyl acetate, 3:1 followed by 2:1), and the obtained crystals were washed with hexane to give the title compound (3.60 g, yield 16%). ¹H NMR (CDCl₃) δ 1.26 (6H, s), 1.32 (6H, s), 1.47 (3H, t, J = 6.9 Hz), 2.16 (2H, s), 2.70 (2H, s), 4.20 (2H, q, J = 6.9 Hz), 6.64 (1H, s), 7.79 (1H, dd, J = 7.8, 1.5 Hz), 7.88 (1H, dd, J = 7.8, 0.9 Hz), 7.95 (1H, dd, J = 1.5, 0.9 Hz), 7.98 (1H, s).

### Example 91

### Diethyl 4-(6-ethoxy-3,4,8,9-tetrahydro-3,3,8,8-tetramethylfuro[2,3-h]isoquinolin-1-yl)-1,2-benzenedicarboxylate hydrochloride

The washed solution of the title compound in Example 90 was concentrated under reduced pressure, and the residue was purified with a basic silica gel column chromatography (hexane/ethyl acetate, 10:1) to give the title compound as free base. This was dissolved in ethyl acetate, and a solution of 4 M hydrogen chloride/ethyl acetate was added thereto. The mixture was concentrated under reduced pressure, and the residue was crystallized from ethyl acetate-hexane to give a hydrochloride of the title compound (227 mg, yield 0.78%).
¹H NMR (DMSO-d₆) δ 1.25-1.44 (21H, m), 2.17 (1H, d, J = 17.0 Hz), 2.28 (1H, d, J = 17.0 Hz), 3.13 (2H, s), 4.19-4.40 (6H, m), 7.08 (1H, s), 7.88-8.01 (3H, m).

### Example 92

### 5-(6-Ethoxy-3,4,8,9-tetrahydro-3,3,8,8-tetramethylfuro[2,3-h]isoquinolin-1-yl)-2-methyl-1H-isoindol-1,3(2H)-dione

From 5-(6-ethoxy-3,4,8,9-tetrahydro-3,3,8,8-tetramethylfuro[2,3-h]isoquinolin-1-yl)-1H-isoindol-1,3(2H)-dione and iodomethane, the title compound was obtained by the method similar to that in Example 82. Yield 71%.
¹H NMR (CDCl₃) δ 1.25 (6H, s), 1.31 (6H, s), 1.47 (3H, t, J = 7.0 Hz), 2.16 (2H, s), 2.69 (2H, s), 3.21 (3H, s), 4.19 (2H, q, J = 7.0 Hz), 6.63 (1H, s), 7.75 (1H, dd, J = 7.8, 1.4 Hz), 7.86 (1H, dd, J = 7.8, 0.8 Hz), 7.92 (1H, dd, J = 1.4, 0.8 Hz).

### Example 93

### Methyl [[4-(6-ethoxy-3,4,8,9-tetrahydro-3,3,8,8-tetramethylfuro[2,3-h]isoquinolin-1-yl)phenyl]methoxy]acetate

To a solution of 7-ethoxy-2,3-dihydro-2,2-dimethyl-α-(1-methylethyl)-5-benzofuranmethanol (6.80 g, 25.7 mmol) and methyl [(4-cyanophenyl)methoxy]acetate (4.40 g, 21.4 mmol) in toluene (24 ml) and acetic acid (14 ml) was added conc. sulfuric acid (2.85 ml, 111 mmol), and the mixture was stirred for 1.5 hours at 80°C. Methanol (10.8 ml) was added dropwise thereto, and the mixture was stirred for 30 minutes at 70°C. After cooling, the reaction mixture was combined with ice water, washed with diisopropyl ether, neutralized with sodium hydrogen carbonate, and extracted twice with ethyl acetate. The combined organic layer was washed with a saturated aqueous solution of sodium chloride, dried over sodium sulfate, filtered, and concentrated under reduced pressure. The residue was purified with a basic silica gel column chromatography (hexane/ethyl acetate, 5:1 followed by 1:1) to give the title compound (1.18 g, yield 12%).
Amorphous.
¹H NMR (CDCl₃) δ 1.23 (6H, s), 1.31 (6H, s), 1.46 (3H, t, J = 7.0 Hz), 2.21 (2H, s), 2.66 (2H, s), 3.78 (3H, s), 4.10 (2H, s), 4.18 (2H, q, J = 7.0 Hz), 4.69 (2H, s), 6.60 (1H, s), 7.39 (4H, s).

### Alternative synthetic method

A solution of 4-(6-ethoxy-3,4,8,9-tetrahydro-3,3,8,8-tetramethylfuro[2,3-h]isoquinolin-1-yl)benzenemethanol (580 mg, 1.53 mmol) in N,N-dimethylformamide (4 ml) was cooled with ice, sodium hydride (66% dispersion in oil) (61 mg, 1.68 mmol) was added thereto, and the mixture was stirred at room temperature for 30 minutes. To this mixture was added methyl bromoacetate (0.16 ml, 1.68 mmol), and the mixture was stirred at room temperature for 5 hours. The reaction mixture was combined with ice water, and extracted three times with ethyl acetate. The combined organic layer was washed with water and a saturated aqueous solution of sodium chloride, dried over sodium sulfate, filtered, and concentrated under reduced pressure. The residue was purified with a silica gel column chromatography (hexane/ethyl acetate, 5:1 followed by 1:1) to give the title compound (179 mg, yield 26%).

### Example 94

### Methyl [[4-(6-ethoxy-3,4,8,9-tetrahydro-3,3,8,8-tetramethylfuro[2,3-h]isoquinolin-1-yl)phenyl]methoxy]acetate hydrochloride

Methyl [[4-(6-ethoxy-3,4,8,9-tetrahydro-3,3,8,8-tetramethylfuro[2,3-h]isoquinolin-1-yl)phenyl]methoxy]acetate (179 mg, 0.396 mmol) was dissolved in ethyl acetate, a solution of 4 M hydrogen chloride/ethyl acetate was added thereto, and the mixture was concentrated under reduced pressure to give the title compound (123 mg, yield 64%) as amorphous.
¹H NMR (DMSO-d₆) δ 1.23 (6H, s), 1.37 (3H, t, J = 7.0 Hz), 1.44 (6H, s), 2.21 (2H, s), 3.14 (2H, s), 3.69 (3H, s), 4.24 (2H, q, J = 7.0 Hz), 4.27 (2H, s), 4.71 (2H, s), 7.08 (1H, s), 7.58-7.65 (4H, m).

### Example 95

### [[4-(6-Ethoxy-3,4,8,9-tetrahydro-3,3,8,8-tetramethylfuro[2,3-h]isoquinolin-1-yl)phenyl]methoxy]acetic acid hydrochloride

To a solution of methyl [[4-(6-ethoxy-3,4,8,9-tetrahydro-3,3,8,8-tetramethylfuro[2,3-h]isoquinolin-1-yl)phenyl]methoxy]acetate (660 mg, 1.46 mmol) in methanol (8 ml) was added 5 M aqueous solution (1.5 ml) of sodium hydroxide, and the mixture was stirred for 30 minutes at room temperature. Methanol was distilled off under reduced pressure, the residue was combined with water washed with diisopropyl ether, and acidified with 5 M hydrochloric acid. Acetone was added thereto, the insolubles were removed by filtration, and the filtrate was concentrated under reduced pressure. The residue was crystallized from acetone-diisopropyl ether to give the title compound (594 mg, yield 86%).
¹H NMR (DMSO-d₆) δ 1.24 (6H, s), 1.37 (3H, t, J = 7.0 Hz), 1.43 (6H, s), 2.22 (2H, s), 3.14 (2H, s), 4.15 (2H, s), 4.23 (2H, q, J = 7.0 Hz), 4.70 (2H, s), 7.08 (1H, s), 7.61 (4H, s).

### Example 96

### N-[4-(6-Ethoxy-3,4,8,9-tetrahydro-3,3,8,8-tetramethylfuro[2,3-h]isoquinolin-1-yl)benzoyl]glycine ethyl ester

From 4-(6-ethoxy-3,4,8,9-tetrahydro-3,3,8,8-tetramethylfuro[2,3-h]isoquinolin-1-yl)benzoic acid hydrochloride and glycine ethyl ester hydrochloride, the title compound was obtained by the method similar to that in Example 72. Yield 68%.
¹H NMR (CDCl₃) δ 1.25 (6H, s), 1.31 (6H, s), 1.33 (3H, t, J = 7.2 Hz), 1.46 (3H, t, J = 7.0 Hz), 2.18 (2H, s), 2.68 (2H, s), 4.19 (2H, q, J = 7.0 Hz), 4.23-4.33 (4H, m), 6.61 (1H, s), 6.70 (1H, t, J = 5.0 Hz), 7.49 (2H, d, J = 8.5 Hz), 7.85 (2H, d, J = 8.5 Hz).

### Example 97

### N-[4-(6-Ethoxy-3,4,8,9-tetrahydro-3,3,8,8-tetramethylfuro[2,3-h]isoquinolin-1-yl)benzoyl]glycine hydrochloride

From N-[4-(6-ethoxy-3,4,8,9-tetrahydro-3,3,8,8-tetramethylfuro[2,3-h]isoquinolin-1-yl)benzoyl]glycine ethyl ester, the title compound was obtained by the method similar to that in Example 95. Yield 94%.
Amorphous.
¹H NMR (CDCl₃) δ 1.24 (6H, s), 1.37 (3H, t, J = 7.0 Hz), 1.45 (6H, s), 2.21 (2H, s), 3.16 (2H, s), 3.98 (2H, t, J = 5.8 Hz), 4.24 (2H, q, J = 7.0 Hz), 7.09 (1H, s), 7.76 (2H, d, J = 8.5 Hz), 8.13 (2H, d, J = 8.5 Hz), 9.24 (1H, t, J = 5.8 Hz).

### Example 98

### Methyl 5-(6-ethoxy-3,4,8,9-tetrahydro-3,3,8,8-tetramethylfuro[2,3-h]isoquinolin-1-yl)-2-((E)-3-methoxy-3-oxo-1-propenyl)benzoate

From methyl 5-cyano-2-((E)-3-methoxy-3-oxo-1-propenyl)benzoate, the title compound was obtained by the method similar to that in Example 90. Yield 4%.

### Example 99

### (E)-3-[4-(6-Ethoxy-3,4,8,9-tetrahydro-3,3,8,8-tetramethylfuro[2,3-h]isoquinolin-1-yl)-2-(methoxycarbonyl)phenyl]-2-propenoic acid

The title compound was obtained in Example 98 at the same time. Yield 14%.
¹H NMR (CDCl₃) δ 1.29 (6H, s), 1.33 (6H, s), 1.48 (3H, t, J = 7.0 Hz), 2.23 (2H, s), 2.70 (2H, s), 3.91 (3H, s), 4.20 (2H, q, J = 7.0 Hz), 6.35 (1H, d, J = 16.0 Hz), 6.63 (1H, s), 7.64 (1H, d, J = 8.0 Hz), 7.69 (1H, d, J = 8.0 Hz), 8.01 (1H, s), 8.49 (1H, d, J = 16.0 Hz).

### Example 100

### Methyl (E)-3-[4-(6-ethoxy-3,4,8,9-tetrahydro-3,3,8,8-tetramethylfuro[2,3-h]isoquinolin-1-yl)phenyl]-2-propenoate

To a solution of 7-ethoxy-2,3-dihydro-2,2-dimethyl-a-(1-methylethyl)-5-benzofuranmethanol (0.780 g, 2.94 mmol) and methyl (E)-3-(4-cyanophenyl)-2-propenoate (0.500 g, 2.67 mmol) in acetic acid (4.4 ml)-toluene (5 ml) was added dropwise conc. sulfuric acid (0.37 ml), and the mixture was stirred for 1 hour at 80°C. The reaction solution, which was cooled to room temperature, was combined with water, and washed with diisopropyl ether. The aqueous layer was cooled with ice, alkalified with conc. ammonia water, and extracted with ethyl acetate. The extracts were washed with water, and concentrated under reduced pressure. The residue was purified with a silica gel column chromatography (hexane/ethyl acetate 1:1) to give the title compound (0.480 g, yield 41%). Amorphous.
¹H NMR (CDCl₃) δ 1.24 (6H, s), 1.32 (6H, s), 1.46 (3H, t, J = 7.0 Hz), 2.23 (2H, s), 2.67 (2H, s), 3.82 (3H, s), 4.18 (2H, q, J = 7.0 Hz), 6.49 (1H, d, J = 15.8 Hz), 6.61 (1H, s), 7.43 (2H, d, J = 8.0 Hz), 7.56 (2H, d, J = 8.0 Hz), 7.73 (1H, d, J = 15.8 Hz).

### Example 101

### (E)-3-[4-(6-Ethoxy-3,4,8,9-tetrahydro-3,3,8,8-tetramethylfuro[2,3-h]isoquinolin-1-yl)phenyl]-2-propenoic acid

To a solution of methyl (E)-3-[4-(6-ethoxy-3,4,8,9-tetrahydro-3,3,8,8-tetramethylfuro[2,3-h]isoquinolin-1-yl)phenyl]-2-propenoate in methanol (5 ml) was added a 2 M aqueous solution (5 ml) of sodium hydroxide, and the mixture was stirred at room temperature for 4 hours. The reaction solution was combined with 2 M hydrochloric acid (5 ml), and the precipitated crystals were taken by filtration. This was washed with water and diisopropyl ether, dried, and recrystallized from methanol-ethyl acetate to give the title compound (0.610 g, yield 70%).
¹H NMR (DMSO-d₆) δ 1.13 (6H, s), 1.22 (6H, s), 1.33 (3H, t, J = 6.8 Hz), 2.25 (2H, s), 2.61 (2H, s), 4.09 (2H, q, J = 6.8 Hz), 6.54 (1H, d, J = 16.0 Hz), 6.78 (1H, s), 7.31-7.42 (3H, m), 7.61 (2H, d, J = 8.0 Hz).

### Example 102

### Methyl (E)-3-[4-(6-ethoxy-3,4,8,9-tetrahydro-3,3,8,8-tetramethylfuro[2,3-h]isoquinolin-1-yl)-2-methylphenyl]-2-propenoate

To a suspension of 7-ethoxy-2,3-dihydro-2,2-dimethyl-α-(1-methylethyl)-5-benzofuranmethanol (635 mg, 2.40 mmol) and methyl (E)-3-(4-cyano-2-methylphenyl)-2-propenoate (403 mg, 2.00 mmol) in toluene (3 ml) and acetic acid (1.5 ml) was added dropwise conc. sulfuric acid (0.29 ml, 5.4 mmol), and the mixture was stirred at 85°C for 1.5 hours. The reaction mixture was cooled to 65°C, methanol (1.2 ml) was added dropwise thereto, and the mixture was stirred for 1 hour at the same temperature. After cooling, the mixture was added dropwise to sodium hydrogen carbonate (1.4 g, 17 mmol), combined with water, and extracted twice with ethyl acetate. The combined organic layer was washed twice with water, and concentrated under reduced pressure. The residue was purified with a silica gel column chromatography (hexane/ethyl acetate 2:1) to give the title compound (385 mg, yield 43%).
Amorphous.
¹H NMR (CDCl₃) δ 1.24 (6H, s), 1.32 (6H, s), 1.47 (3H, t, J = 7.0 Hz), 2.25 (2H, s), 2.46 (3H, s), 2.66 (2H, s), 3.82 (3H, s), 4.19 (2H, q, J = 7.0 Hz), 6.41 (1H, d, J = 15.9 Hz), 6.61 (1H, s), 7.21-7.29 (2H, m), 7.57 (1H, d, J = 8.1 Hz), 8.00 (1H, d, J = 15.9 Hz).

### Example 103

### (E)-3-[4-(6-Ethoxy-3,4,8,9-tetrahydro-3,3,8,8-tetramethylfuro[2,3-h]isoquinolin-1-yl)-2-methylphenyl]-2-propenoic acid

To a solution of methyl (E)-3-[4-(6-ethoxy-3,4,8,9-tetrahydro-3,3,8,8-tetramethylfuro[2,3-h]isoquinolin-1-yl)-2-methylphenyl]-2-propenoate (279 mg, 0.623 mmol) in methanol (1 ml) was added a 5 M aqueous solution of sodium hydroxide (0.24 ml, 1.2 mmol), and the mixture was stirred at room temperature for 1.5 hours and at 50°C for 1 hour. The reaction mixture was combined with 1 M hydrochloric acid (1.2 ml, 1.2 mmol), and extracted twice with chloroform. The combined organic layer was washed with a saturated aqueous solution of sodium chloride, dried over sodium sulfate, filtered, concentrated under reduced pressure to give the title compound (257 mg, yield 95%).
Amorphous.
¹H NMR (DMSO-d₆) δ 1.14 (6H, s), 1.23 (6H, s), 1.33 (3H, t, J = 7.0 Hz), 2.27 (2H, s), 2.42 (3H, s), 2.62 (2H, s), 4.09 (2H, q, J = 7.0 Hz), 6.49 (1H, d, J = 15.9 Hz), 6.79 (1H, s), 7.21 (1H, d, J = 7.8 Hz), 7.26 (1H, s), 7.76 (1H, d, J = 7.8 Hz), 7.84 (1H, d, J = 15.9 Hz).

### Example 104

### Ethyl (E)-3-[4-(6-ethoxy-3,4,8,9-tetrahydro-3,3,8,8-tetramethylfuro[2,3-h]isoquinolin-1-yl)phenyl]-2-methyl-2-propenoate

To a solution of triethyl 2-phosphonopropionate (4.05 g, 17.0 mmol) in tetrahydrofuran (15 ml) was added sodium hydride (66% dispersion in oil) (0.62 g, 17 mmol) under ice-cooling, and the mixture was stirred at the same temperature for 15 minutes. To this mixture was added 4-cyanobenzaldehyde (1.86 g, 14.2 mmol), and the mixture was stirred at room temperature for 30 minutes. The obtained mixture was combined with a solution which had been separately prepared from triethyl 2-phosphonopropionate (0.81 g, 3.4 mmol), sodium hydride (66% dispersion in oil) (0.12 g, 3.3 mmol) and tetrahydrofuran (3 ml) in the same manner, and stirred at room temperature for 30 minutes. The reaction mixture was poured into a saturated aqueous solution of ammonium chloride, and extracted twice with ethyl acetate. The combined organic layer was washed twice with water, and concentrated under reduced pressure. The residue was purified with a silica gel column chromatography (hexane/ethyl acetate, 100:1 followed by 5:1) to give a semi-solid containing ethyl (E)-3-(4-cyanophenyl)-2-methyl-2-propenoate (3.09 g).
¹H NMR (CDCl₃) δ 1.36 (3H, t, J = 7.2 Hz), 2.10 (3H, d, J = 1.2 Hz), 4.29 (2H, q, J = 7.2 Hz), 7.47 (2H, d, J = 8.1 Hz), 7.64-7.66 (1H, m), 7.69 (2H, d, J = 8.1 Hz).

This product (972 mg) and 7-ethoxy-2,3-dihydro-2,2-dimethyl-α-(1-methylethyl)-5-benzofuranmethanol (1.43 g, 5.41 mmol) were suspended in toluene (7 ml) and acetic acid (3.5 ml), and conc. sulfuric acid (0.66 ml, 12 mmol) was added dropwise thereto. The resultant mixture was stirred at 85°C for 1.5 hours. To the reaction mixture was added dropwise ethanol (5 ml), and the mixture was stirred for 1 hour at the same temperature. After cooling, the mixture was added dropwise to sodium hydrogen carbonate (3.12 g, 37.1 mmol), combined with water, and extracted twice with ethyl acetate. The combined organic layer was washed with water and a saturated aqueous solution of sodium chloride, dried over sodium sulfate, filtered, and concentrated under reduced pressure. The residue was purified with a silica gel column chromatography (hexane/ethyl acetate, 20:1 followed by 5:1), and crystallized from hexane to give the title compound (734 mg, yield 35%).
¹H NMR (CDCl₃) δ 1.25 (6H, s), 1.32 (6H, s), 1.36 (3H, t, J = 7.1 Hz), 1.47 (3H, t, J = 6.9 Hz), 2.13 (3H, d, J = 1.5 Hz), 2.22 (2H, s), 2.67 (2H, s), 4.19 (2H, q, J = 7.0 Hz), 4.28 (2H, q, J = 7.1 Hz), 6.61 (1H, s), 7.43 (4H, s), 7.72 (1H, q, J = 1.5 Hz).

### Example 105

### (E)-3-[4-(6-Ethoxy-3,4,8,9-tetrahydro-3,3,8,8-tetramethylfuro[2,3-h]isoquinolin-1-yl)phenyl]-2-methyl-2-propenoic acid

To a suspension of ethyl (E)-3-[4-(6-ethoxy-3,4,8,9-tetrahydro-3,3,8,8-tetramethylfuro[2,3-h]isoquinolin-1-yl)phenyl]-2-methyl-2-propenoate (300 mg, 0.65 mmol) in ethanol (1.5 ml) was added a 5 M aqueous solution of sodium hydroxide (0.26 ml, 1.3 mmol), and the mixture was stirred at 50°C for 1.5 hours. The reaction mixture was combined with 1 M hydrochloric acid (1.3 ml, 1.3 mmol) under ice-cooling, and extracted twice with chloroform. The combined organic layer was washed with a saturated aqueous solution of sodium chloride, dried over sodium sulfate, filtered, and concentrated under reduced pressure. The residue was recrystallized from ethyl acetate-diethyl ether to give the title compound (190 mg, yield 67%).
¹H NMR (DMSO-d₆) δ 1.14 (6H, s), 1.23 (6H, s), 1.33 (3H, t, J = 6.8 Hz), 2.07 (3H, s), 2.24 (2H, s), 2.62 (2H, s), 4.09 (2H, q, J = 6.8 Hz), 6.79 (1H, s), 7.40 (2H, d, J = 7.4 Hz), 7.51 (2H, d, J = 7.4 Hz), 7.64 (1H, s), 12.57 (1H, br s).

### Example 106

### Ethyl (E)-3-[4-(6-ethoxy-3,4,8,9-tetrahydro-3,3,8,8-tetramethylfuro[2,3-h]isoquinolin-1-yl)phenyl]-2-butenoate

7-Ethoxy-2,3-dihydro-2,2-dimethyl-α-(1-methylethyl)-5-benzofuranmethanol (793 mg, 3.00 mmol) and ethyl (E)-3-(4-cyanophenyl)-2-butenoate (538 mg, 2.50 mmol) were suspended in toluene (4 ml) and acetic acid (2 ml), conc. sulfuric acid (0.37 ml, 6.9 mmol) was added dropwise thereto, and the mixture was stirred at 85°C for 1.5 hours. To the reaction mixture was added dropwise ethanol (3 ml), and the mixture was stirred for 2 hours at the same temperature. After cooling, the mixture was added dropwise to sodium hydrogen carbonate (1.75 g, 20.8 mmol), combined with water, and extracted twice with ethyl acetate. The combined organic layer was washed with water and a saturated aqueous solution of sodium chloride, and concentrated under reduced pressure. The residue was purified with a silica gel column chromatography (hexane/ethyl acetate, 5:1 followed by 2:1) to give the title compound (413 mg, yield 36%).
Amorphous.
¹H NMR (CDCl₃) δ 1.24 (6H, s), 1.32 (6H, s), 1.33 (3H, t, J = 7.2 Hz), 1.47 (3H, t, J = 7.1 Hz), 2.23 (2H, s), 2.59 (3H, d, J = 1.4 Hz), 2.67 (2H, s), 4.14-4.23 (2H, m), 4.23 (2H, q, J = 7.1 Hz), 6.19 (1H, q, J = 1.4 Hz), 6.61 (1H, s), 7.41 (2H, d, J = 8.6 Hz), 7.51 (2H, d, J = 8.6 Hz).

### Example 107

### (E)-3-[4-(6-Ethoxy-3,4,8,9-tetrahydro-3,3,8,8-tetramethylfuro[2,3-h]isoquinolin-1-yl)phenyl]-2-butenoic acid

To a solution of ethyl (E)-3-[4-(6-ethoxy-3,4,8,9-tetrahydro-3,3,8,8-tetramethylfuro[2,3-h]isoquinolin-1-yl)phenyl]-2-butenoate (206 mg, 0.446 mmol) in ethanol (1.5 ml) was added a 5 M aqueous solution of sodium hydroxide (0.18 ml, 0.90 mmol), and the mixture was stirred at room temperature for 4 hours and at 50°C for 4 hours. The reaction mixture was cooled, combined with 1 M hydrochloric acid (0.90 ml, 0.90 mmol), and extracted twice with chloroform. The combined organic layer was washed with a saturated aqueous solution of sodium chloride, dried over sodium sulfate, filtered, and concentrated under reduced pressure. The residue was crystallized from ethyl acetate-diethyl ether-hexane to give the title compound (40 mg, yield 21%).
¹H NMR (DMSO-d₆) δ 1.13 (6H, s), 1.23 (6H, s), 1.33 (3H, t, J = 7.1 Hz), 2.25 (2H, s), 2.50-2.53 (3H, m), 2.61 (2H, s), 4.09 (2H, q, J = 7.1 Hz), 6.18-6.21 (1H, m), 6.79 (1H, s), 7.38 (2H, d, J = 8.4 Hz), 7.61 (2H, d, J = 8.4 Hz), 12.20-12.40 (1H, br).

### Example 108

### Ethyl (Z)-3-[4-(6-ethoxy-3,4,8,9-tetrahydro-3,3,8,8-tetramethylfuro[2,3-h]isoquinolin-1-yl)phenyl]-2-butenoate

With ethyl (Z)-3-(4-cyanophenyl)-2-butenoate, the title compound was obtained by the method similar to that in Example 106. Yield 29%.
¹H NMR (CDCl₃) δ 1.18 (3H, t, J = 7.1 Hz), 1.24 (6H, s), 1.33 (6H, s), 1.46 (3H, t, J = 7.1 Hz), 2.16 (3H, d, J = 1.3 Hz), 2.28 (2H, s), 2.67 (2H, s), 4.02 (2H, q, J = 7.1 Hz), 4.18 (2H, q, J = 7.0 Hz), 5.92 (1H, q, J = 1.3 Hz), 6.59 (1H, s), 7.22 (2H, d, J = 8.4 Hz), 7.36 (2H, q, J = 8.4 Hz).

### Example 109

### Ethyl 4-(6-ethoxy-3,4,8,9-tetrahydro-3,3,8,8-tetramethylfuro[2,3-h]isoquinolin-1-yl)benzenepropanoate

To a solution of ethyl (E)-3-(4-cyanophenyl)-2-propenoate (8.04 g, 40.0 mmol) in ethyl acetate (40 ml) was added 10% palladium/carbon (50% water-containing product) (0.80 g), and the mixture was stirred under hydrogen atmosphere at room temperature for 80 minutes. The catalyst was filtered, and the filtrate was concentrated under reduced pressure to give oil matter (8.36 g) containing ethyl 4-cyanobenzenepropanoate.

This substance and 7-ethoxy-2,3-dihydro-2,2-dimethyl-α-(1-methylethyl)-5-benzofuranmethanol (10.6 g, 40.1 mmol) were dissolved in toluene (40 ml) and acetic acid (20 ml), and conc. sulfuric acid (5.9 ml, 0.11 mol) was added dropwise thereto. The resultant mixture was stirred at 85°C for 1.5 hours. To the reaction mixture was added dropwise ethanol (20 ml), and the mixture was stirred for 1 hour at the same temperature. After cooling, the mixture was added dropwise to sodium hydrogen carbonate (24.5 g, 0.292 mol), and ethyl acetate and water were added thereto. The organic layer was separated, and the aqueous layer was extracted with ethyl acetate. The combined organic layer was washed with water and a saturated aqueous solution of sodium chloride, dried through sodium sulfate-basic silica gel (eluting with ethyl acetate), and concentrated under reduced pressure. The residue was purified with a basic silica gel column chromatography (hexane/ethyl acetate, 10:1 followed by 3:1) to give the title compound (3.42 g, yield 19%).
Oily matter.
¹H NMR (CDCl₃) δ 1.23 (6H, s), 1.25 (3H, t, J = 7.2 Hz), 1.31 (6H, s), 1.46 (3H, t, J = 7.1 Hz), 2.19 (2H, s), 2.61 (2H, t, J = 7.7 Hz), 2.65 (2H, s), 2.98 (2H, t, J = 7.7 Hz), 4.13 (2H, q, J = 7.1 Hz), 4.18 (2H, q, J = 7.1 Hz), 6.59 (1H, s), 7.21 (2H, d, J = 8.4 Hz), 7.31 (2H, d, J = 8.4 Hz).

### Example 110

### 4-(6-Ethoxy-3,4,8,9-tetrahydro-3,3,8,8-tetramethylfuro[2,3-h]isoquinolin-1-yl)benzenepropanoic acid

To a solution of ethyl 4-(6-ethoxy-3,4,8,9-tetrahydro-3,3,8,8-tetramethylfuro[2,3-h]isoquinolin-1-yl)benzenepropanoate (450 mg, 1.00 mmol) in ethanol (1 ml) was added 5 M aqueous solution of sodium hydroxide (0.40 ml, 2.0 mmol), and the mixture was stirred at room temperature for 4.5 hours. The reaction mixture was combined with 1 M hydrochloric acid (2.0 ml, 2.0 mmol) under ice-cooling, and extracted twice with chloroform. The combined organic layer was washed with a saturated aqueous solution of sodium chloride, dried over sodium sulfate, filtered, concentrated under reduced pressure to give the title compound (418 mg, yield 99%).
Amorphous.
¹H NMR (DMSO-d₆) δ 1.12 (6H, s), 1.21 (6H, s), 1.32 (3H, t, J = 6.9 Hz), 2.18 (2H, s), 2.55 (2H, t, J = 7.5 Hz), 2.61 (2H, s), 2.87 (2H, t, J = 7.5 Hz), 4.08 (2H, q, J = 6.9 Hz), 6.78 (1H, s), 7.25 (4H, s), 12.15 (1H, br s).

### Example 111

### 6-(6-Ethoxy-3,4,8,9-tetrahydro-3,3,8,8-tetramethylfuro[2,3-h]isoquinolin-1-yl)-2H-1,4-benzoxazin-3(4H)-one

To a suspension of 7-ethoxy-2,3-dihydro-2,2-dimethyl-α-(1-methylethyl)-5-benzofuranmethanol (1.03 g, 3.90 mmol) and methyl (2-amino-4-cyanophenoxy)acetate (730 mg, 3.54 mmol) in toluene (4 ml), a solution of conc. sulfuric acid (0.71 ml, 13 mmol) in acetic acid (2 ml) was added dropwise under ice-cooling, and the mixture was stirred for 1 hour at 85°C. The reaction mixture was combined with water, washed with diisopropyl ether, neutralized with sodium hydrogen carbonate, and extracted twice with ethyl acetate. The combined organic layer was washed with water (twice) and a saturated aqueous solution of sodium chloride, dried through sodium sulfate-basic silica gel (eluting with ethyl acetate), and concentrated under reduced pressure. The residue was crystallized from ethyl acetate-diethyl ether to give the title compound (775 mg, yield 52%).
¹H NMR (CDCl₃) δ 1.23 (6H, br s), 1.35 (6H, s), 1.47 (3H, t, J = 6.9 Hz), 3.32 (2H, s), 2.66 (2H, s), 4.18 (2H, q, J = 6.9 Hz), 4.62 (2H, s), 6.60 (1H, s), 6.9₁₋₆.98 (3H, m), 8.46 (1H, br s) .

### Example 112

### 6-(6-Ethoxy-3,4,8,9-tetrahydro-3,3,8,8-tetramethylfuro[2,3-h]isoquinolin-1-yl)-4-(phenylmethyl)-2H-1,4-benzoxazin-3(4H)-one

To a suspension of 6-(6-ethoxy-3,4,8,9-tetrahydro-3,3,8,8-tetramethylfuro[2,3-h]isoquinolin-1-yl)-2H-1,4-benzoxazin-3(4H)-one (631 mg, 1.50 mmol) in N,N-dimethylformamide (3 ml) was added sodium hydride (66% dispersion in oil) (66 mg, 1.8 mmol), and the mixture was stirred at room temperature for 40 minutes. The obtained mixture was combined with benzyl bromide (0.23 ml, 1.9 mmol), and stirred at room temperature for 40 minutes. The reaction mixture was combined with water, and extracted twice with ethyl acetate. The combined organic layer was washed twice with water, and concentrated under reduced pressure. The residue was crystallized from ethyl acetate-diethyl ether to give the title compound (502 mg, yield 66%).
¹H NMR (CDCl₃) δ 1.19 (6H, br s), 1.22 (6H, s), 1.45 (3H, t, J = 7.1 Hz), 1.95 (2H, br s), 2.62 (2H, s), 4.17 (2H, q, J = 7.1 Hz), 4.72 (2H, s), 5.00-5.20 (2H, br), 6.57 (1H, s), 6.94 (1H, dd, J = 8.3, 1.4 Hz), 6.98 (1H, d, J = 8.3 Hz), 7.02 (1H, d, J = 1.4 Hz), 7.13-7.28 (5H, m).

### Example 113

### Sodium [4-(6-ethoxy-3,4,8,9-tetrahydro-3,3,8,8-tetramethylfuro[2,3-h]isoquinolin-1-yl)-2-[(phenylmethyl)amino]phenoxy]acetate

To a suspension of 6-(6-ethoxy-3,4,8,9-tetrahydro-3,3,8,8-tetramethylfuro[2,3-h]isoquinolin-1-yl)-4-(phenylmethyl)-2H-1,4-benzoxazin-3(4H)-one (409 mg, 0.801 mmol) in ethanol (1 ml) was added a 5 M aqueous solution of sodium hydroxide (160 µl, 0.800 mmol), and the mixture was heated under reflux for 20 hours. To the obtained mixture was further added 5 M aqueous solution of sodium hydroxide (160 µl, 0.800 mmol), and the mixture was heated under reflux for 7 hours. After cooling, the reaction mixture was combined with ethanol and diethyl ether, and crystallized to give the title compound.
¹H NMR (CDCl₃) δ 1.06 (6H, s), 1.19 (6H, s), 1.31 (3H, t, J = 6.7 Hz), 2.25 (2H, s), 2.53 (2H, s), 4.05 (2H, q, J = 6.7 Hz), 4.11 (2H, s), 4.26 (2H, d, J = 4.8 Hz), 6.1₂₋₆.24 (1H, m), 6.40 (1H, s), 6.45 (1H, d, J = 8.4 Hz), 6.66-6.72 (2H, m), 7.16-7.37 (5H, m).

### Example 114

### Methyl 4-(6-ethoxy-3,4,8,9-tetrahydro-3,3,8,8-tetramethylfuro[2,3-h]isoquinolin-1-yl)benzeneacetate

A solution of 7-ethoxy-2,3-dihydro-2,2-dimethyl-α-(1-methylethyl)-5-benzofuranmethanol (1067 mg, 4.04 mmol) and methyl 4-cyanobenzeneacetate (701 mg, 4.00 mmol) in toluene (4 ml) and acetic acid (2 ml) was cooled with ice, and conc. sulfuric acid (0.64 ml) was added dropwise thereto. The resultant mixture was stirred for 2 hours at 80°C. The reaction mixture was combined with methanol (2 ml), and the mixture was stirred at 60°C for 45 minutes. The resultant mixture was cooled, the aqueous layer was separated by adding water, and the organic layer was extracted with water. The combined aqueous layer was combined with an aqueous solution of 10% potassium carbonate, alkalified, and extracted with ethyl acetate. The organic layer was washed with a saturated aqueous solution of sodium chloride, dried, and concentrated under reduced pressure. The residue was subjected to a basic alumina column chromatography (hexane/ethyl acetate, 10:1 followed by 2:1), and crystallized from diisopropyl ether-hexane to give the title compound (285 mg, yield 17%).
¹H NMR (CDCl₃) δ 1.23 (6H, s), 1.31 (6H, s), 1.46 (3H, t), 2.21 (2H, s), 2.66 (2H, s), 3.66 (2H, s), 3.68 (3H, s), 4.18 (2H, q), 6.60 (1H, s), 7.29 (2H, d), 7.35 (2H, d).

### Example 115

### 4-(6-Ethoxy-3,4,8,9-tetrahydro-3,3,8,8-tetramethylfuro[2,3-h]isoquinolin-1-yl)benzeneacetic acid

To a solution of methyl 4-(6-ethoxy-3,4,8,9-tetrahydro-3,3,8,8-tetramethylfuro[2,3-h]isoquinolin-1-yl)benzeneacetate (285 mg, 0.676 mmol) in methanol (3 ml) was added a 1 M aqueous solution (2 ml) of sodium hydroxide, and the mixture was stirred at room temperature for 6 hours. The reaction solution was combined with 1 M hydrochloric acid, and further with 1 M hydrochloric acid until the pH become 4, and extracted with chloroform. The organic layer was washed with a saturated aqueous solution of sodium chloride, dried, and concentrated under reduced pressure. The resultant crude crystals were crystallized from ethyl acetate-hexane to give the title compound (251 mg, yield 91%).
¹H NMR (CDCl₃) δ 1.30 (6H, br s), 1.45 (6H, s), 1.47 (3H, t), 2.14 (2H, s), 2.81 (2H, s), 3.58 (2H, s), 4.21 (2H, q), 6.63 (1H, s), 7.26 (2H, d), 7.37 (2H, d).

### Example 116

### Methyl 4-(6-ethoxy-3,4,8,9-tetrahydro-3,3,8,8-tetramethylfuro[2,3-h]isoquinolin-1-yl)-α,α-dimethylbenzeneacetate

A solution of 7-ethoxy-2,3-dihydro-2,2-dimethyl-α-(1-methylethyl)-5-benzofuranmethanol (1462 mg, 5.52 mmol) and methyl 4-cyano-α,α-dimethylbenzeneacetate (1121 mg, 5.53 mmol) in toluene (4 ml) and acetic acid (2 ml) was cooled with ice, and conc. sulfuric acid (0.80 ml) was added dropwise thereto. The resultant mixture was stirred for 2 hours at 80°C. To the reaction mixture was added dropwise methanol (2 ml), and the mixture was stirred at 60°C for 45 minutes. The resultant mixture was cooled, the aqueous layer was separated by adding water, and the organic layer was extracted with water. The combined aqueous layer was combined with an aqueous solution of 10% potassium carbonate, alkalified, and extracted with ethyl acetate. The organic layer was washed with a saturated aqueous solution of sodium chloride, dried, and concentrated under reduced pressure. The residue was subjected to a basic alumina column chromatography (hexane/ethyl acetate, 10:1 followed by 2:1) and purified with a silica gel column chromatography (hexane/ethyl acetate 1:1), and recrystallized from ethyl acetate-hexane to give the title compound (799 mg, yield 32%).
¹H NMR (CDCl₃) δ 1.23 (6H, s), 1.30 (6H, s), 1.46 (3H, t), 1.59 (6H, s), 2.18 (2H, s), 2.66 (2H, s), 3.63 (3H, s), 4.18 (2H, q), 6.60 (1H, s), 7.33 (4H, s).

### Example 117

### 4-(6-Ethoxy-3,4,8,9-tetrahydro-3,3,8,8-tetramethylfuro[2,3-h]isoquinolin-1-yl)-α,α-dimethylbenzeneacetic acid

The separated fractions obtained by eluting with methanol/ethyl acetate (1:1) in basic alumina column chromatography in Example 116 were recrystallized from acetone-hexane to give the title compound (348 mg, yield 14%).
¹H NMR (DMSO-d₆) δ 1.13 (6H, br s), 1.21 (6H, s), 1.34 (3H, t), 1.51 (6H, s), 2.17 (2H, s), 2.59 (2H, s), 4.09 (2H, q), 6.75 (1H, s), 7.29 (2H, d), 7.37 (2H, d).

### Example 118

### 4-(6-Ethoxy-3,4,8,9-tetrahydro-3,3,8,8-tetramethylfuro[2,3-h]isoquinolin-1-yl)-α,α-dimethylbenzeneacetic acid hydrochloride hydrate

To a mixed solution of methyl 4-(6-ethoxy-3,4,8,9-tetrahydro-3,3,8,8-tetramethylfuro[2,3-h]isoquinolin-1-yl)-α,α-dimethylbenzeneacetate (507 mg, 1.13 mmol) in methanol (6 ml) and tetrahydrofuran (6 ml) was added a 1 M aqueous solution (4 ml) of sodium hydroxide, and the mixture was stirred at room temperature for 6 hours. The reaction solution was combined with 1 M hydrochloric acid, and further with 1 M hydrochloric acid until the pH 4, and extracted with ethyl acetate. The organic layer was washed with a saturated aqueous solution of sodium chloride, dried, and concentrated under reduced pressure. The resultant crude crystals were recrystallized from ethanol-diisopropyl ether to give the title compound (366 mg, yield 66%).
¹H NMR (DMSO-d₆) δ 1.23 (6H, br s), 1.40 (3H, t), 1.44 (6H, s), 1.56 (6H, s), 2.15 (2H, s), 3.13 (2H, s), 4.24 (2H, q), 7.05 (1H, s), 7.55 (2H, d), 7.61 (2H, d).

### Example 119

### 4-(6-Ethoxy-3,4,8,9-tetrahydro-3,3,8,8-tetramethylfuro[2,3-h]isoquinolin-1-yl)-N, α,α-trimethylbenzeneacetamide hydrochloride

To a solution of 4-(6-ethoxy-3,4,8,9-tetrahydro-3,3,8,8-tetramethylfuro[2,3-h]isoquinolin-1-yl)-α,α-dimethylbenzeneacetic acid hydrochloride hydrate (311 mg, 0.635 mmol), methylamine hydrochloride (65 mg, 0.963 mmol) and 1-hydroxy-1H-benzotriazole monohydrate (115 mg, 0.751 mmol) in N,N-dimethylformamide (3 ml) were added 1-ethyl-3-(3-dimethylaminopropyl)carbodiimide hydrochloride (150 mg, 0.782 mmol) and triethylamine (0.42 ml), and the mixture was stirred at room temperature for 24 hours. The reaction mixture was combined with a saturated aqueous solution of sodium hydrogen carbonate, and extracted with ethyl acetate. The organic layer was washed with a saturated aqueous solution of sodium chloride, dried, and concentrated under reduced pressure. The residue was subjected to a silica gel column chromatography (hexane/ethyl acetate 1:2) to prepare hydrochloride. The hydrochloride was recrystallized from ethanol-diisopropyl ether to give the title compound (290 mg, yield 94%).
¹H NMR (Free form, CDCl₃) δ 1.24 (6H, s), 1.31 (6H, s), 1.46 (3H, t), 1.60 (6H, s), 2.18 (2H, s), 2.66 (2H, s), 2.68 (3H, d), 4.18 (2H, q), 5.09 (1H, br s), 6.60 (1H, s), 7.37 (4H, s).

### Example 120

### 4-(6-Ethoxy-3,4,8,9-tetrahydro-3,3,8,8-tetramethylfuro[2,3-h]isoquinolin-1-yl)-α,α-dimethylbenzeneacetamide

To a solution of 4-(6-ethoxy-3,4,8,9-tetrahydro-3,3,8,8-tetramethylfuro[2,3-h]isoquinolin-1-yl)-α,α-dimethylbenzeneacetic acid hydrochloride hydrate (306 mg, 0.624 mmol) and 1-hydroxy-1H-benzotriazole ammonium salt (119 mg, 0.782 mmol) in acetonitrile (10 ml) were added 1-ethyl-3-(3-dimethylaminopropyl)carbodiimide hydrochloride (146 mg, 0.762 mmol) and triethylamine (0.22 ml), and the mixture was stirred at room temperature for 14 hours. The reaction mixture was combined with a saturated aqueous solution of sodium hydrogen carbonate, and extracted with ethyl acetate. The organic layer was washed with a saturated aqueous solution of sodium chloride, dried, and concentrated under reduced pressure. The residue was purified with a silica gel column chromatography (hexane/ethyl acetate 1:3). The resultant crude crystals were recrystallized from ethyl acetate-hexane to give the title compound (242 mg, yield 89%).
¹H NMR (CDCl₃) δ 1.24 (6H, s), 1.30 (6H, s), 1.46 (3H, t), 1.61 (6H, s), 2.18 (2H, s), 2.66 (2H, s), 4.17 (2H, q), 5.13 (1H, br s), 5.18 (1H, br s), 6.59 (1H, s), 7.36-7.43 (4H, m).

### Example 121

### Methyl 4-(6-ethoxy-3,4,8,9-tetrahydro-3,3,8,8-tetramethylfuro[2,3-h]isoquinolin-1-yl)-2-nitrobenzeneacetate

A solution of 7-ethoxy-2,3-dihydro-2,2-dimethyl-α-(1-methylethyl)-5-benzofuranmethanol (1463 mg, 5.53 mmol) and methyl 4-cyano-2-nitrobenzeneacetate (1103 mg, 5.00 mmol) in toluene (6 ml) and acetic acid (3 ml) was cooled with ice, and conc. sulfuric acid (0.8 ml) was added dropwise thereto. The resultant mixture was stirred for 2 hours at 80°C. To the reaction mixture was added methanol (3 ml), and the mixture was stirred for 1 hour at 60°C. The resultant mixture was cooled, the aqueous layer was separated by adding water, and the organic layer was extracted with water. The combined aqueous layer was combined with an aqueous solution of 10% potassium carbonate, alkalified, and extracted with ethyl acetate. The organic layer was washed with a saturated aqueous solution of sodium chloride, dried, and concentrated under reduced pressure. The residue was purified with a silica gel column chromatography (hexane/ethyl acetate 1:2). The resultant crude crystals were recrystallized from ethyl acetate-hexane to give the title compound (898 mg, yield 38%).
¹H NMR (CDCl₃) δ 1.25 (6H, s), 1.34 (6H, s), 1.47 (3H, t), 2.25 (2H, s), 2.68 (2H, s), 3.71 (3H, s), 4.07 (2H, s), 4.20 (2H, q), 6.63 (1H, s), 7.38 (1H, d), 7.66 (1H, dd), 8.19 (1H, d).

### Example 122

### 4-(6-Ethoxy-3,4,8,9-tetrahydro-3,3,8,8-tetramethylfuro[2,3-h]isoquinolin-1-yl)-2-nitrobenzeneacetic acid

To a solution of methyl 4-(6-ethoxy-3,4,8,9-tetrahydro-3,3,8,8-tetramethylfuro[2,3-h]isoquinolin-1-yl)-2-nitrobenzeneacetate (618 mg, 1.32 mmol) in methanol (6 ml) was added 1 M aqueous solution (3 ml) of sodium hydroxide, and the mixture was stirred at room temperature for 5 hours. The reaction solution was combined with 1 M hydrochloric acid (3 ml) and extracted with ethyl acetate. The organic layer was washed with a saturated aqueous solution of sodium chloride, dried, and concentrated under reduced pressure. The resultant crude crystals were recrystallized from ethanol-diisopropyl ether to give the title compound (570 mg, yield 95%).
¹H NMR (DMSO-d₆) δ 1.21 (6H, br s), 1.26 (6H, br s), 1.35 (3H, t), 2.28 (2H, s), 2.60 (2H, s), 3.35 (2H, s), 4.05-4.21 (2H, m), 6.86 (1H, br s), 7.48-7.80 (2H, m), 7.91-8.20 (1H, m).

### Example 123

### Methyl 4-(6-ethoxy-3,4,8,9-tetrahydro-3,3,8,8-tetramethylfuro[2,3-h]isoquinolin-1-yl)-2-[(2-pyridinylcarbonyl)amino]benzeneacetate

A solution of 7-ethoxy-2,3-dihydro-2,2-dimethyl-α-(1-methylethyl)-5-benzofuranmethanol (992 mg, 3.75 mmol), methyl 4-cyano-2-[(2-pyridinylcarbonyl)amino]benzeneacetate (1001 mg, 3.34 mmol) in toluene (4 ml) and acetic acid (2 ml) was cooled with ice, and conc. sulfuric acid (0.54 ml) was added dropwise thereto. The resultant mixture was stirred for 2 hours at 80°C. The reaction mixture was combined with methanol (2 ml), and stirred for 2 hours at 60°C. The resultant mixture was cooled, the aqueous layer was separated by adding water, and the organic layer was extracted with water. The combined aqueous layer was combined with an aqueous solution of 10% potassium carbonate, alkalified, and extracted with ethyl acetate. The organic layer was washed with a saturated aqueous solution of sodium chloride, dried, and concentrated under reduced pressure. The residue was purified with a basic silica gel column chromatography (hexane/ethyl acetate 1:1) to give the title compound (356 mg, yield 19%). Amorphous.
¹H NMR (CDCl₃) δ 1.23 (6H, s), 1.35 (6H, s), 1.46 (3H, t), 2.48 (2H, br s), 2.65 (2H, s), 3.68-3.81 (2H, m), 3.73 (3H, s), 4.18 (2H, q), 6.59 (1H, s), 7.28-7.34 (2H, m), 7.44-7.52 (1H, m), 7.89 (1H, dt), 8.07 (1H, d), 8.26 (1H, d), 8.62-8.69 (1H, m), 10.55 (1H, s).

### Example 124

### 4-(6-Ethoxy-3,4,8,9-tetrahydro-3,3,8,8-tetramethylfuro[2,3-h]isoquinolin-1-yl)-2-[(2-pyridinylcarbonyl)amino]benzeneacetic acid

To a solution of methyl 4-(6-ethoxy-3,4,8,9-tetrahydro-3,3,8,8-tetramethylfuro[2,3-h]isoquinolin-1-yl)-2-[(2-pyridinylcarbonyl)amino]benzeneacetate (135 mg, 0.25 mmol) in methanol (1 ml) was added 1 M aqueous solution (0.5 ml) of sodium hydroxide, and the mixture was stirred at room temperature for 14 hours. The reaction solution was combined with 1 M hydrochloric acid (0.5 ml) and extracted with ethyl acetate. The organic layer was washed with a saturated aqueous solution of sodium chloride, dried, and concentrated under reduced pressure. The resultant crude crystals were recrystallized from ethanol-diisopropyl ether to give the title compound (66 mg, yield 50%).
¹H NMR (DMSO-d₆) δ 1.14 (6H, br s), 1.25 (6H, br s), 1.34 (3H, t), 2.50 (2H, br s), 2.62 (2H, s), 3.73 (2H, s), 4.10 (2H, q), 6.75 (1H, s), 7.20 (1H, d), 7.37 (1H, d), 7.61-7.68 (1H, m), 7.76 (1H, s), 8.04 (1H, t), 8.12 (1H, d), 8.68 (1H, d), 10.54 (1H, s).

### Example 125

### 6-(6-Ethoxy-3,4,8,9-tetrahydro-3,3,8,8-tetramethylfuro[2,3-h]isoquinolin-1-yl)-1,3-dihydro-2H-indol-2-one

A solution of 7-ethoxy-2,3-dihydro-2,2-dimethyl-α-(1-methylethyl)-5-benzofuranmethanol (2114 mg, 8.00 mmol) and methyl 4-cyano-2-(trifluoroacetylamino)benzeneacetate (2065 mg, 7.21 mmol) in toluene (8 ml) and acetic acid (4 ml) was cooled with ice, and conc. sulfuric acid (1.2 ml) was added dropwise thereto. The resultant mixture was stirred for 1 hour at 80°C. The reaction mixture was combined with methanol (5 ml), and stirred for 1 hour at 60°C. The resultant mixture was cooled, the aqueous layer was separated by adding water, and the organic layer was extracted with water. The combined aqueous layer was combined with an aqueous solution of 10% potassium carbonate, alkalified, and extracted with ethyl acetate. The organic layer was washed with a saturated aqueous solution of sodium chloride, dried, and concentrated under reduced pressure. The residue was subjected to a basic alumina column chromatography (ethyl acetate/methanol 10:1) and purified with a silica gel column chromatography (ethyl acetate). The resultant crude crystals were recrystallized from ethyl acetate-hexane to give the title compound (542 mg, yield 19%).
¹H NMR (CDCl₃) δ 1.23 (6H, s), 1.34 (6H, s), 1.46 (3H, t), 2.30 (2H, s), 2.66 (2H, s), 3.56 (2H, s), 4.19 (2H, q), 6.60 (1H, s), 6.97-7.04 (2H, m), 7.20 (1H, d), 8.05 (1H, br s).

### Example 126

### 6-(6-Ethoxy-3,4,8,9-tetrahydro-3,3,8,8-tetramethylfuro[2,3-h]isoquinolin-1-yl)-1,3-dihydro-3,3-dimethyl-2H-indol-2-one

A solution of 7-ethoxy-2,3-dihydro-2,2-dimethyl-α-(1-methylethyl)-5-benzofuranmethanol (1500 mg, 5.67 mmol) and 6-cyano-1,3-dihydro-3,3-dimethyl-2H-indol-2-one (955 mg, 5.13 mmol) in toluene (5 ml) and acetic acid (2.5 ml) was cooled with ice, and conc. sulfuric acid (0.82 ml) was added dropwise thereto. The resultant mixture was stirred at 80°C for 85 minutes. The resultant mixture was cooled, the aqueous layer was separated by adding water, and the organic layer was extracted with water. The combined aqueous layer was combined with an aqueous solution of 10% potassium carbonate, alkalified, and extracted with ethyl acetate. The organic layer was washed with a saturated aqueous solution of sodium chloride, dried, and concentrated under reduced pressure. The residue was purified with a basic silica gel column chromatography (ethyl acetate). The resultant crude crystals were washed with diisopropyl ether to give the title compound (66 mg, yield 3%).
¹H NMR (CDCl₃) δ 1.24 (6H, br s), 1.33 (6H, s), 1.40 (6H, s), 1.47 (3H, t), 2.28 (2H, s), 2.66 (2H, s), 4.19 (2H, q), 6.60 (1H, s), 6.99-7.05 (2H, m), 7.16 (1H, d), 7.82 (1H, s).

### Example 127

### Methyl (E)-3-[2-amino-4-(6-ethoxy-3,4,8,9-tetrahydro-3,3,8,8-tetramethylfuro[2,3-h]isoquinolin-1-yl)phenyl]-2-propenoate

A solution of 7-ethoxy-2,3-dihydro-2,2-dimethyl-α-(1-methylethyl)-5-benzofuranmethanol (2550 mg, 9.65 mmol) and methyl 3-(2-amino-4-cyanophenyl)-2-propenoate (1771 mg, 8.76 mmol) in toluene (5 ml) and acetic acid (2.5 ml) was cooled with ice, and conc. sulfuric acid (0.82 ml) was added dropwise thereto. The resultant mixture was stirred at 80°C for 85 minutes. The resultant mixture was cooled, the aqueous layer was separated by adding water, and the organic layer was extracted with water. The combined aqueous layer was combined with an aqueous solution of 10% potassium carbonate, alkalified, and extracted with ethyl acetate. The organic layer was washed with a saturated aqueous solution of sodium chloride, dried, and concentrated under reduced pressure. The residue was purified with a silica gel column chromatography (ethyl acetate) and basic alumina column chromatography (hexane/ethyl acetate 4:1). The resultant crude crystals were washed with diisopropyl ether to give the title compound (895 mg, yield 23%).
¹H NMR (CDCl₃) δ 1.22 (6H, br s), 1.34 (6H, s), 1.46 (3H, t), 2.36 (2H, s), 2.65 (2H, s), 3.81 (3H, s), 4.00 (2H, s), 4.18 (2H, q), 6.39 (1H, d), 6.59 (1H, s), 6.7₂₋₆.82 (2H, m), 7.39 (1H, d), 7.84 (1H, d).

### Example 128

### 7-(6-Ethoxy-3,4,8,9-tetrahydro-3,3,8,8-tetramethylfuro[2,3-h]isoquinolin-1-yl)-2-quinolinol

The separated fractions obtained by eluting with methanol in basic alumina column chromatography in Example 127 were recrystallized from ethanol-diisopropyl ether to give the title compound (43 mg, yield 1%).
¹H NMR (CDCl₃) δ 1.26 (6H, br s), 1.30 (6H, s), 1.47 (3H, t), 2.22 (2H, s), 2.69 (2H, s), 4.19 (2H, q), 6.63 (1H, s), 6.71 (1H, d), 7.22 (1H, dd), 7.40 (1H, s), 7.57 (1H, d), 7.82 (1H, d), 11.13 (1H, br s).

### Example 129

### Methyl (E)-3-[4-(6-ethoxy-3,4,8,9-tetrahydro-3,3,8,8-tetramethylfuro[2,3-h]isoquinolin-1-yl)-2-[(2-pyridinylcarbonyl)amino]phenyl]-2-propenoate

To a solution of methyl (E)-3-[2-amino-4-(6-ethoxy-3,4,8,9-tetrahydro-3,3,8,8-tetramethylfuro[2,3-h]isoquinolin-1-yl)phenyl]-2-propenoate (711 mg, 1.59 mmol) and 4-(dimethylamino)pyridine (596 mg, 4.88 mmol) in N,N-dimethylformamide (5 ml) was added picolinic acid chloride hydrochloride (481 mg, 2.70 mmol), and the mixture was stirred at room temperature for 20 minutes. The obtained mixture was alkalified by adding an aqueous solution of 10% potassium carbonate, and extracted with ethyl acetate. The organic layer was washed with a saturated aqueous solution of sodium chloride, dried, and concentrated under reduced pressure. The residue was purified with a basic silica gel column chromatography (hexane/ethyl acetate 1:1). The resultant crude crystals were recrystallized from ethyl acetate-hexane to give the title compound (570 mg, yield 65%).
¹H NMR (CDCl₃) δ 1.24 (6H, s), 1.34 (6H, s), 1.46 (3H, t), 2.45 (2H, s), 2.66 (2H, s), 3.83 (3H, s), 4.19 (2H, q), 6.54 (1H, d), 6.60 (1H, d), 7.24-7.33 (1H, m), 7.46-7.55 (1H, m), 7.64 (1H, d), 7.86-7.95 (1H, m), 8.00 (1H, d), 8.18 (1H, d), 8.26 (1H, d), 8.66 (1H, d), 10.23 (1H, s).

### Example 130

### (E)-3-[4-(6-Ethoxy-3,4,8,9-tetrahydro-3,3,8,8-tetramethylfuro[2,3-h]isoquinolin-1-yl)-2-[(2-pyridinylcarbonyl)amino]phenyl]-2-propenoic acid

To a solution of methyl (E)-3-[4-(6-ethoxy-3,4,8,9-tetrahydro-3,3,8,8-tetramethylfuro[2,3-h]isoquinolin-1-yl)-2-[(2-pyridinylcarbonyl)amino]phenyl]-2-propenoate (417 mg, 0.753 mmol) in methanol (5 ml) was added 1 M aqueous solution (1.6 ml) of sodium hydroxide, and the mixture was stirred at room temperature for 5 hours. The reaction solution was combined with 1 M hydrochloric acid (1.6 ml) and extracted with ethyl acetate. The organic layer was washed with a saturated aqueous solution of sodium chloride, dried, and concentrated under reduced pressure. The resultant crude crystals were suspended in methanol to give the title compound (362 mg, yield 89%).
¹H NMR (CDCl₃) δ 1.29 (6H, br s), 1.31 (6H, br s), 1.46 (3H, t), 2.41 (2H, br s), 2.69 (2H, s), 4.19 (2H, q), 6.41 (1H, d), 6.60 (1H, s), 7.18-7.26 (1H, m), 7.32-7.41 (1H, m), 7.54 (1H, d), 7.70-7.82 (2H, m), 8.10-8.20 (2H, m), 8.55 (1H, d), 10.21 (1H, s).

### Example 131

### Methyl (E)-3-[4-(6-ethoxy-3,4,8,9-tetrahydro-3,3,8,8-tetramethylfuro[2,3-h]isoquinolin-1-yl)-2-[(1H-indol-2-ylcarbonyl)amino]phenyl]-2-propenoate

To a solution of indole-2-carboxylic acid (110 mg, 0.683 mmol) in tetrahydrofuran (2 ml) were added oxalyl chloride (0.07 ml) and N,N-dimethylformamide (1 drop) at 0°C, and the mixture was stirred at room temperature for 30 minutes. The solvent was distilled off under reduced pressure, and the residue was dissolved again in tetrahydrofuran (2 ml), and added to a solution of methyl (E)-3-[2-amino-4-(6-ethoxy-3,4,8,9-tetrahydro-3,3,8,8-tetramethylfuro[2,3-h]isoquinolin-1-yl)phenyl]-2-propenoate (254 mg, 0.549 mmol) and triethylamine (0.09 ml) in tetrahydrofuran (3 ml). The reaction mixture was stirred at room temperature for 1 hour. The obtained mixture was combined with an aqueous solution of 10% potassium carbonate, and extracted with ethyl acetate. The organic layer was washed with a saturated aqueous solution of sodium chloride, dried, and concentrated under reduced pressure. The residue was purified with a silica gel column chromatography (hexane/ethyl acetate 1:1) to give the title compound (305 mg, yield 94%).
Amorphous.
¹H NMR (CDCl₃) δ 1.33 (6H, s), 1.35 (6H, s), 1.48 (3H, t), 2.14 (2H, s), 2.75 (2H, s), 3.78 (3H, s), 4.20 (2H, q), 6.44 (1H, d), 6.61 (1H, s), 7.10-7.20 (3H, m), 7.26-7.35 (1H, m), 7.43 (1H, d), 7.59-7.68 (2H, m), 7.77 (1H, s), 7.84 (1H, d), 8.85 (1H, br s), 9.65 (1H, br s).

### Example 132

### (E)-3-[4-(6-Ethoxy-3,4,8,9-tetrahydro-3,3,8,8-tetramethylfuro[2,3-h]isoquinolin-1-yl)-2-[(1H-indol-2-ylcarbonyl)amino]phenyl]-2-propenoic acid

To a mixed solution of methyl (E)-3-[4-(6-ethoxy-3,4,8,9-tetrahydro-3,3,8,8-tetramethylfuro[2,3-h]isoquinolin-1-yl)-2-[(1H-indol-2-ylcarbonyl)amino]phenyl]-2-propenoate (300 mg, 0.507 mmol) in methanol (2 ml) and tetrahydrofuran (2 ml) was added in 1 M aqueous solution (2.5 ml) of sodium hydroxide, and the mixture was stirred at room temperature for 14 hours. The reaction solution was combined with 1 M hydrochloric acid (2.5 ml) and extracted with ethyl acetate. The organic layer was washed with a saturated aqueous solution of sodium chloride, dried, and concentrated under reduced pressure. The resultant crude crystals were recrystallized from ethanol-diisopropyl ether to give the title compound (171 mg, yield 58%).
¹H NMR (CD₃OD) δ 1.34 (6H, s), 1.45 (6H, s), 1.45 (3H, t), 2.2-2.8 (2H, m), 3.06 (2H, s), 4.26 (2H, q), 6.67 (1H, d), 6.93 (1H, s), 7.09 (1H, t), 7.26 (1H, t), 7.37 (1H, s), 7.47 (2H, d), 7.66 (1H, d), 7.74 (1H, d), 7.90 (1H, d), 8.02 (1H, d).

### Example 133

### Ethyl (E)-3-[2-amino-4-(6-ethoxy-3,4,8,9-tetrahydro-3,3,8,8-tetramethylfuro[2,3-h]isoquinolin-1-yl)phenyl]-2-propenoate

With ethyl (E)-3-(2-amino-4-cyanophenyl)-2-propenoate, the title compound was obtained by the method similar to that in Example 90. Yield 22%.
Oily matter.
¹H NMR (CDCl₃) δ 1.23 (6H, s), 1.34 (6H, s), 1.34 (3H, t, J = 7.0 Hz), 1.46 (3H, t, J = 7.0 Hz), 2.36 (2H, s), 2.65 (2H, s), 4.01 (2H, s), 4.22 (2H, q, J = 7.0 Hz), 4.27 (2H, q, J = 7.0 Hz), 6.39 (1H, d, J = 15.8 Hz), 6.59 (1H, s), 6.74-6.80 (2H, m), 7.39 (1H, d, J = 8.0 Hz), 7.83 (1H, d, J = 15.8 Hz).

### Example 134

### Ethyl (E)-3-[2-[[[(2-ethoxy-2-oxoethyl)amino]carbonyl]amino]-4-(6-ethoxy-3,4,8,9-tetrahydro-3,3,8,8-tetramethylfuro[2,3-h]isoquinolin-1-yl)phenyl]-2-propenoate

From ethyl (E)-3-[2-amino-4-(6-ethoxy-3,4,8,9-tetrahydro-3,3,8,8-tetramethylfuro[2,3-h]isoquinolin-1-yl)phenyl]-2-propenoate, the title compound was obtained by the method similar to that in Example 35. Yield 84%.
¹H NMR (CDCl₃) δ 1.26-1.34 (18H, m), 1.46 (3H, t, J = 6.9 Hz), 2.27 (2H, s), 2.69 (2H, br s), 3.99-4.06 (2H, m), 4.16-4.28 (6H, m), 5.92 (1H, br s), 6.29 (1H, d, J = 15.8 Hz), 6.57 (1H, s), 6.94 (1H, d, J = 8.0 Hz), 7.38 (1H, d, J = 8.0 Hz), 7.53 (1H, s), 7.76 (1H, br s), 7.79 (1H, d, J = 15.8 Hz).

### Example 135

### Methyl (E)-3-[2-(2,5-dioxo-1-imidazolidinyl)-4-(6-ethoxy-3,4,8,9-tetrahydro-3,3,8,8-tetramethylfuro[2,3-h]isoquinolin-1-yl)phenyl]-2-propenoate

From ethyl (E)-3-[2-[[[(2-ethoxy-2-oxoethyl)amino]carbonyl]amino]-4-(6-ethoxy-3,4,8,9-tetrahydro-3,3,8,8-tetramethylfuro[2,3-h]isoquinolin-1-yl)phenyl]-2-propenoate, the title compound was obtained by the method similar to that in Example 36. Yield 17%.
¹H NMR (CDCl₃) δ 1.20 (6H, s), 1.29 (6H, s), 1.39 (3H, t, J = 6.9 Hz), 2.40 (2H, br s), 2.56 (1H, d, J = 15.3 Hz), 2.61 (1H, d, J = 15.3 Hz), 3.72 (3H, s), 4.04 (1H, d, J = 21.3 Hz), 4.10-4.21 (3H, m), 6.44 (1H, d, J = 15.9 Hz), 6.52 (1H, s), 7.18 (1H, s), 7.28 (1H, br s), 7.49 (1H, d, J = 15.9 Hz), 7.51 (1H, d, J = 8.1 Hz), 7.72 (1H, d, J = 8.1 Hz).

### Example 136

### Dimethyl 7-(6-ethoxy-3,4,8,9-tetrahydro-3,3,8,8-tetramethylfuro[2,3-h]isoquinolin-1-yl)-1,4-dihydro-2-oxo-3,4(2H)-quinazolinediacetate hydrochloride

The free base of the title compound was obtained in Example 135 at the same time. This was dissolved in ethyl acetate, a solution of 4 M hydrogen chloride/ethyl acetate was added thereto, and the mixture was concentrated under reduced pressure to give the title compound. Yield 10%.
Amorphous.
¹H NMR (DMSO-d₆) δ 1.25 (3H, s), 1.28 (3H, s), 1.37 (3H, t, J = 7.0 Hz), 1.41 (3H, s), 1.45 (3H, s), 2.10-3.25 (6H, m), 3.35 (3H, s), 3.64 (3H, s), 4.05-4.42 (4H, m), 5.00-5.15 (1H, m), 6.94 (0.6H, s), 7.00 (0.4H, s), 7.08 (1H, s), 7.12 (1H, d, J = 8.1 Hz), 7.37 (1H, d, J = 8.1 Hz), 9.89 (1H, s).

### Example 137

### Methyl 2-[(3-chlorophenylmethyl)(trifluoroacetyl)amino]-4-(6-ethoxy-3,4,8,9-tetrahydro-3,3,8,8-tetramethylfuro[2,3-h]isoquinolin-1-yl)benzoate

Methyl 4-(6-ethoxy-3,4,8,9-tetrahydro-3,3,8,8-tetramethylfuro[2,3-h]isoquinolin-1-yl)-2-[(trifluoroacetyl)amino]benzoate (519 mg, 1 mmol), m-chlorobenzyl bromide (300 mg, 1.46 mmol), potassium carbonate (600 mg, 4.35 mmol) and sodium iodide (100 mg, 0.66 mmol) were stirred in N,N-dimethylformamide (4 ml) at the bath temperature of 50 to 55°C for 0.5 hour. The reaction solution was cooled to room temperature, and thioglycolic acid (0.1 ml) was added thereto, and the mixture was stirred for 15 minutes. The reaction solution was combined with water/a saturated aqueous solution of sodium chloride (2:1) and ethyl acetate/hexane (2:1), and mixed with stirring to separate the layers. The upper layer was washed with water and concentrated under reduced pressure. The residue was dissolved again in methanol and concentrated under reduced pressure to give the title compound (638 mg, yield 99%).
Amorphous.
¹H NMR (CDCl₃) δ 1.20 (3H, s), 1.25 (3H, s), 1.28 (3H, s), 1.30 (3H, s), 1.45 (3H, t), 2.14 (2H, dd), 2.65 (2H, dd), 3.75 (3H, s), 4.17 (2H, q), 4.56 (1H, d), 5.07 (1H, d), 6.59 (1H, d), 7.08-7.28 (5H, m), 7.50 (1H, dd), 8.06 (1H, dd).

### Example 138

### Methyl 2-[(3-chlorophenyl)methylamino]-4-(6-ethoxy-3,4,8,9-tetrahydro-3,3,8,8-tetramethylfuro[2,3-h]isoquinolin-1-yl)benzoate

Methyl 2-[(3-chlorophenylmethyl)(trifluoroacetyl)amino]-4-(6-ethoxy-3,4,8,9-tetrahydro-3,3,8,8-tetramethylfuro[2,3-h]isoquinolin-1-yl)benzoate (630 mg, 0.98 mmol) was dissolved in methanol (10 ml), potassium carbonate (500 mg, 3.62 mmol) was added thereto, and the mixture was stirred at room temperature for 17 hours. The mixture was further stirred at the bath temperature of 50-55°C for 0.5 hour and concentrated under reduced pressure. The residue was dissolved by adding water, adjusted at pH 4 to 5 with 2 M hydrochloric acid, combined with ethyl acetate, and mixed with stirring to separate the layers. The upper layer was washed with saline and concentrated under reduced pressure. The residue was purified with a silica gel column chromatography (hexane/ethyl acetate, 2:1, 1:1 followed by hexane/ethyl acetate/methanol 3:3:1) to give the title compound (400 mg, yield 75%).
Amorphous.
¹H NMR (CDCl₃) δ 1.22 (6H, s), 1.29 (6H, s), 1.45 (3H, t), 2.22 (2H, s), 2.65 (2H, s), 3.88 (3H, s), 4.17 (2H, q), 4.42 (2H, d), 6.57 (1H, s), 6.60 (1H, dd), 6.67 (1H, d), 7.22-7.23 (3H, m), 7.32 (1H, s), 7.92 (1H, d), 8.18 (1H, t).

### Example 139

### 2-[(3-Chlorophenyl)methylamino]-4-(6-ethoxy-3,4,8,9-tetrahydro-3,3,8,8-tetramethylfuro[2,3-h]isoquinolin-1-yl)benzoic acid

Methyl 2-[(3-chlorophenyl)methylamino]-4-(6-ethoxy-3,4,8,9-tetrahydro-3,3,8,8-tetramethylfuro[2,3-h]isoquinolin-1-yl)benzoate (217 mg, 0.397 mmol) was dissolved in a mixed solvent of methanol/tetrahydrofuran (1:1, 5 ml), 4 M aqueous solution (0.4 ml) of lithium hydroxide was added thereto, and the mixture was stirred at room temperature for 60 hours. The reaction solution was concentrated under reduced pressure, the residue was dissolved by adding water, and adjusted at pH 4 to 5 with 2 M hydrochloric acid. The produced precipitate was extracted with ethyl acetate, and the ethyl acetate layer was washed with water and concentrated under reduced pressure. The residue was washed with diisopropyl ether and dried to give the title compound (159 mg, yield 75%). Amorphous.
¹H NMR (CDCl₃) δ 1.25 (6H, s), 1.47 (3H, t), 1.48 (6H, br), 2.31 (2H, br), 2.86 (2H, s), 4.20 (2H, q), 4.26 (2H, br), 6.41 (1H, d), 6.49 (1H, s), 6.61 (1H, s), 6.90-7.17 (3H, m), 7.23 (1H, s), 7.79 (1H, d), 8.75 (1H, br).

### Example 140

### Methyl 4-(6-ethoxy-3,4,8,9-tetrahydro-3,3,8,8-tetramethylfuro[2,3-h]isoquinolin-1-yl)-2-[(2-quinolinylmethyl)(trifluoroacetyl)amino]benzoate

With stirring methyl 4-(6-ethoxy-3,4,8,9-tetrahydro-3,3,8,8-tetramethylfuro[2,3-h]isoquinolin-1-yl)-2-[(trifluoroacetyl)amino]benzoate (519 mg, 1 mmol), potassium carbonate (1.38g, 10 mmol) and sodium iodide (300 mg, 1.99 mmol) in N,N-dimethylformamide (4 ml), 2-chloromethylquinoline hydrochloride (278 mg, 1.3 mmol) was added thereto, and the mixture was stirred at room temperature for 15 hours. The reaction solution was combined with thioglycolic acid (0.1 ml), and the mixture was stirred for 15 minutes. The reaction solution was combined with water/a saturated aqueous solution of sodium chloride (2:1) and ethyl acetate/hexane (2:1), and the mixture was mixed with stirring to separate the layers. The upper layer was washed with water and concentrated under reduced pressure. The residue was washed with methanol and dried to give the title compound (639 mg, yield 97%).
Melting point: 185 to 186°C.
¹H NMR (CDCl₃) δ 1.12 (3H, s), 1.26 (9H, br), 1.47 (3H, t), 2.23 (2H, br), 2.57 (1H, d), 2.70 (1H, d), 3.71 (3H, s), 4.19 (2H, q), 4.76 (1H, d), 5.48 (1H, d), 6.60 (1H, s), 7.25-7.29 (1H, m), 7.48-7.54 (3H, m), 7.61 (1H, br), 7.77 (1H, d), 7.86 (1H, br), 8.08 (1H, d), 8.12 (1H, d).

### Example 141

### Methyl 4-(6-ethoxy-3,4,8,9-tetrahydro-3,3,8,8-tetramethylfuro[2,3-h]isoquinolin-1-yl)-2-[(2-quinolinylmethyl)amino]benzoate

Methyl 4-(6-ethoxy-3,4,8,9-tetrahydro-3,3,8,8-tetramethylfuro[2,3-h]isoquinolin-1-yl)-2-[(2-quinolinylmethyl)(trifluoroacetyl)amino]benzoate (550 mg, 0.863 mmol) was dissolved in methanol (20 ml), and potassium carbonate (480 mg, 3.48 mmol) was added thereto. The mixture was heated under reflux for 1 hour and concentrated under reduced pressure. The residue was dissolved by adding water, adjusted at pH 4 to 5 with 2 M hydrochloric acid, combined with ethyl acetate, and mixed with stirring to separate the layers. The upper layer was washed with a saturated aqueous solution of sodium chloride and concentrated under reduced pressure. The residue was purified with a silica gel column chromatography (hexane/ethyl acetate, 2:1, 1:1 followed by hexane/ethyl acetate/methanol 3:3:1) to give the title compound (422 mg, yield 90%).
Amorphous.
¹H NMR (CDCl₃) δ 1.21 (6H, s), 1.25 (6H, s), 1.45 (3H, t), 2.24 (2H, s), 2.65 (2H, s), 3.95 (3H, s), 4.17 (2H, q), 4.75 (2H, d), 6.57 (1H, s), 6.62 (1H, dd), 6.80 (1H, d), 7.44 (1H, dd), 7.52 (1H, t), 7.69-7.73 (1H, m), 7.79 (1H, d), 7.96 (1H, d), 8.10-8.13 (2H, m), 8.88 (1H, br).

### Example 142

### 4-(6-Ethoxy-3,4,8,9-tetrahydro-3,3,8,8-tetramethylfuro[2,3-h]isoquinolin-1-yl)-2-[(2-quinolinylmethyl)amino]benzoic acid

Methyl 4-(6-ethoxy-3,4,8,9-tetrahydro-3,3,8,8-tetramethylfuro[2,3-h]isoquinolin-1-yl)-2-[(2-quinolinylmethyl)amino]benzoate (250 mg, 0.444 mmol) was dissolved in methanol (4 ml), and 4 M aqueous solution (0.3 ml) of lithium hydroxide was added thereto. The mixture was heated under reflux for 4 hours, and concentrated under reduced pressure. The residue was dissolved by adding water, and adjusted at pH 4 to 5 with 2 M hydrochloric acid. The produced precipitate was extracted with ethyl acetate, and the ethyl acetate layer was washed with water and concentrated under reduced pressure. The residue was washed with diisopropyl ether and dried to give the title compound (190 mg, yield 79 %).
¹H NMR (CDCl₃) δ 1.15 (6H, br), 1.44 (6H, s), 1.46 (3H, t), 2.16 (2H, br), 2.81 (2H, s), 4.18 (2H, q), 4.66 (2H, s), 6.48 (1H, dd), 6.58 (1H, s), 6.67 (1H, s), 7.36 (1H, d), 7.43-7.47 (1H, m), 7.60-7.70 (1H, m), 7.69 (1H, d), 7.82 (1H, d), 7.94 (1H, d), 7.97 (1H, d).

### Example 143

### Methyl 2-[[(4-cyanophenyl)methyl]amino]-4-(6-ethoxy-3,4,8,9-tetrahydro-3,3,8,8-tetramethylfuro[2,3-h]isoquinolin-1-yl)benzoate

Methyl 4-(6-ethoxy-3,4,8,9-tetrahydro-3,3,8,8-tetramethylfuro[2,3-h]isoquinolin-1-yl)-2-[(trifluoroacetyl)amino]benzoate (520 mg, 1 mmol) and p-cyanobenzyl bromide (200 mg, 1.72 mmol) were dissolved in N,N-dimethylformamide (10 ml). To this mixture were added potassium iodide (100 mg) and potassium carbonate (500 mg, 3.61 mmol), and the mixture was stirred at room temperature overnight. The mixture was extracted with diisopropyl ether, washed with water, dried, and concentrated to give methyl 2-[[(4-cyanophenyl)methyl](trifluoroacetyl)amino]-4-(6-ethoxy-3,4,8,9-tetrahydro-3,3,8,8-tetramethylfuro[2,3-h]isoquinolin-1-yl)benzoate (730 mg) as a syrup.

The product was dissolved in methanol (10 ml), and potassium carbonate (300 mg) was added thereto. The mixture was stirred at room temperature overnight and concentrated. The residue was extracted with diisopropyl ether, washed with water, dried, and purified with a silica gel column chromatography (hexane/ethyl acetate, 5:1, 4:1, 1:1 followed by ethyl acetate) to give the title compound (410 mg, yield 76%).
Amorphous.
¹H NMR (CDCl₃) δ 1.20 (6H, s), 1.30 (6H, s), 1.45 (3H, t, J = 6.8 Hz), 2.35 (2H, s), 2.63 (2H, s), 3.89 (3H, s), 4.17 (2H, q, J = 6.8 Hz), 4.52 (2H, d, J = 5.6 Hz), 6.57 (1H, s), 6.6₁₋₆.66 (2H, m), 7.45 (2H, d, J = 8.4 Hz), 7.60 (2H, d, J = 8.4 Hz), 7.94 (1H, d, J = 8.0 Hz), 8.25 (1H, t, J = 5.6 Hz).

### Example 144

### 2-[[(4-Cyanophenyl)methyl]amino]-4-(6-ethoxy-3,4,8,9-tetrahydro-3,3,8,8-tetramethylfuro[2,3-h]isoquinolin-1-yl)benzoic acid

Methyl 2-[[(4-cyanophenyl)methyl]amino]-4-(6-ethoxy-3,4,8,9-tetrahydro-3,3,8,8-tetramethylfuro[2,3-h]isoquinolin-1-yl)benzoate (210 mg, 0.39 mmol) was dissolved in methanol (10 ml), and 2 M aqueous solution (6 ml) of sodium hydroxide was added thereto.
The mixture was stirred at room temperature for 4 hours, neutralized with hydrochloric acid, and concentrated. The residue was purified with a silica gel column chromatography (chloroform followed by chloroform/methanol 4:1) to give the title compound (177 mg, yield 87%).
Amorphous.
¹H NMR (CDCl₃) δ 1.15-1.35 (9H, br), 1.47 (3H, t, J = 6.8 Hz), 1.35-1.55 (3H, br), 2.00-2.40 (2H, br), 2.70-3.00 (2H, br), 4.21 (2H, q, J = 6.8 Hz), 4.28 (2H, s), 6.42 (1H, d, J = 6.8 Hz), 6.61 (2H, s), 7.21 (2H, d, J = 8.0 Hz), 7.49 (2H, d, J = 8.0 Hz), 7.77 (1H, d, J = 6.8 Hz), 8.67 (1H, br).

### Example 145

### Ethyl 4-(6-ethoxy-3,4,8,9-tetrahydro-3,3,8,8-tetramethylfuro[2,3-h]isoquinolin-1-yl)-2-[(2-pyridinylmethyl)amino]benzoate

Methyl 4-(6-ethoxy-3,4,8,9-tetrahydro-3,3,8,8-tetramethylfuro[2,3-h]isoquinolin-1-yl)-2-[(trifluoroacetyl)amino]benzoate (520 mg, 1.00 mmol) and 2-(chloromethyl)pyridine hydrochloride (215 mg, 1.31 mmol) were dissolved in N,N-dimethylformamide (5 ml). To this mixture were added potassium carbonate (550 mg, 3.97 mmol) and potassium iodide (100 mg), and the mixture was stirred at room temperature overnight. The mixture was neutralized with hydrochloric acid, extracted with diisopropyl ether, washed with water, dried, and purified with a silica gel column chromatography (hexane/ethyl acetate 5:1 followed by ethyl acetate) to give ethyl 4-(6-ethoxy-3,4,8,9-tetrahydro-3,3,8,8-tetramethylfuro[2,3-h]isoquinolin-1-yl)-2-[(2-pyridinylmethyl) (trifluoroacetyl)amino]benzoate as a syrup (726 mg).

The product was dissolved in methanol (10 ml), and potassium carbonate (300 mg) was added thereto. The mixture was stirred at room temperature overnight and concentrated. The residue was combined with ethyl acetate and stirred, and the ethyl acetate solution was purified with silica gel layer to give the title compound (510 mg, yield 96%).
Amorphous.
¹H NMR (CDCl₃) δ 1.22 (6H, s), 1.29 (6H, s), 1.42 (3H, t, J = 7.2 Hz), 1.45 (3H, t, J = 7.2 Hz), 2.27 (2H, s), 2.65 (2H, s), 4.17 (2H, q, J = 7.2 Hz), 4.37 (2H, q, J = 7.2 Hz), 4.57 (2H, d, J = 5.2 Hz), 6.57 (1H, s), 6.60 (1H, dd, J = 8.4, 1.2 Hz), 6.68 (1H, d, J = 1.2 Hz), 7.16-7.20 (1H, m), 7.31 (1H, d, J = 7.6 Hz), 7.63 (1H, td, J = 7.6, 2.0 Hz), 7.95 (1H, d, J = 8.4 Hz), 8.60 (2H, m).

### Example 146

### 4-(6-Ethoxy-3,4,8,9-tetrahydro-3,3,8,8-tetramethylfuro[2,3-h]isoquinolin-1-yl)-2-[(2-pyridinylmethyl)amino]benzoic acid

Ethyl 4-(6-ethoxy-3,4,8,9-tetrahydro-3,3,8,8-tetramethylfuro[2,3-h]isoquinolin-1-yl)-2-[(2-pyridinylmethyl)amino]benzoate (340 mg) was dissolved in methanol (6 ml), and 2 M aqueous solution (6 ml) of sodium hydroxide was added thereto. The mixture was stirred at room temperature for 2 hours, and methanol was distilled off. The residual aqueous solution was adjusted at pH 7 with hydrochloric acid, extracted with ethyl acetate, dried, and concentrated to give a crude product. The crude product was purified with a silica gel column chromatography (chloroform), and further purified with MCI CHP-20P resin column (water followed by methanol) to give the title compound (296 mg, yield 92%).
Amorphous.
¹H NMR (CD₃OD) δ 1.20 (6H, s), 1.26 (6H, s), 1.40 (3H, t, J = 6.8 Hz), 2.17 (2H, s), 2.82 (2H, s), 4.17 (2H, q, J = 6.8 Hz), 4.54 (2H, s), 6.52 (1H, s), 6.55 (1H, dd, J = 8.0, 1.2 Hz), 6.77 (1H, s), 7.25 (1H, m), 7.51 (1H, d, J = 8.0 Hz), 7.76 (1H, m), 8.00 (1H, d, J = 7.6 Hz), 8.41 (1H, m).

### Example 147

### Methyl 4-(6-ethoxy-3,4,8,9-tetrahydro-3,3,8,8-tetramethylfuro[2,3-h]isoquinolin-1-yl)-2-[[[4-(methoxycarbonyl)phenyl]methyl]amino]benzoate

Methyl 4-(6-ethoxy-3,4,8,9-tetrahydro-3,3,8,8-tetramethylfuro[2,3-h]isoquinolin-1-yl)-2-[(trifluoroacetyl)amino]benzoate (520 mg, 1.00 mmol) and methyl 4-(bromomethyl)benzoate (344 mg, 1.50 mmol) were dissolved in N,N-dimethylformamide (10 ml). To this mixture were added potassium iodide (200 mg, 1.20 mmol) and potassium carbonate (450 mg, 3.25 mmol), and the mixture was stirred at room temperature for 15 hours. The reaction solution was combined with diisopropyl ether and an aqueous solution of sodium chloride, and the layers were separated, dried with sodium sulfate and concentrated to give methyl 4-(6-ethoxy-3,4,8,9-tetrahydro-3,3,8,8-tetramethylfuro[2,3-h]isoquinolin-1-yl)-2-[[[4-(methoxycarbonyl)phenyl]methyl](trifluoroacetyl)amino]benzoate.

The product was dissolved in methanol (10 ml), potassium carbonate (300 mg) was added thereto, and the mixture was stirred at room temperature for 5 hours. The mixture was concentrated, and the residue was extracted with diisopropyl ether, dried, and concentrated to give a crude product. The crude product was purified with a silica gel column chromatography (hexane/ethyl acetate 4:1 followed by ethyl acetate) to give the title compound (393 mg, yield 68%).
Amorphous.
¹H NMR (CDCl₃) δ 1.21 (6H, s), 1.28 (6H, s), 1.44 (3H, t, J = 7.2 Hz), 2.20 (2H, s), 2.63 (2H, s), 3.88 (3H, s), 3.89 (3H, s), 4.16 (2H, q, J = 7.2 Hz), 4.51 (2H, d, J = 5.6 Hz), 6.56 (1H, s), 6.57 (1H, dd, J = 8.0, 1.6 Hz), 6.65 (1H, d, J = 1.6 Hz), 7.41 (2H, d, J = 8.4 Hz), 7.92 (1H, d, J = 8.0 Hz), 7.98 (2H, d, J = 8.4 Hz), 8.23 (1H, t, J = 5.6 Hz).

### Example 148

### 2-[[(4-Carboxylphenyl)methyl]amino]-4-(6-ethoxy-3,4,8,9-tetrahydro-3,3,8,8-tetramethylfuro[2,3-h]isoquinolin-1-yl)benzoic acid

Methyl 4-(6-ethoxy-3,4,8,9-tetrahydro-3,3,8,8-tetramethylfuro[2,3-h]isoquinolin-1-yl)-2-[[[4-(methoxycarbonyl)phenyl]methyl]amino]benzoate (233 mg) was dissolved in methanol(10 ml), and 2 M aqueous solution (6 ml) of sodium hydroxide was added thereto. The mixture was stirred at room temperature for 4 hours, and methanol was distilled off. The residual aqueous solution was adjusted at pH 4 with 1 M hydrochloric acid, and the precipitated crystals were taken by filtration, washed with water, and dried to give the title compound (174 mg, yield 78%).
¹H NMR (DMSO-d₆) δ 1.16 (6H, s), 1.20-1.45 (6H, m), 1.34 (3H, t, J = 6.9 Hz), 2.00-2.20 (2H, m), 2.60-3.30 (2H, br), 4.07-4.25 (2H, m), 4.60 (2H, s), 6.50-7.10 (3H, m), 7.43 (2H, d, J = 8.3 Hz), 7.8₃₋₈.02 (1H, m), 7.89 (2H, d, J = 8.3 Hz), 8.42-8.70 (1H, br), 12.50-13.30 (2H, m).

### Example 149

### Methyl 4-(6-ethoxy-3,4,8,9-tetrahydro-3,3,8,8-tetramethylfuro[2,3-h]isoquinolin-1-yl)-2-[[(2-methoxyphenyl)methyl]amino]benzoate

With 2-methoxybenzyl chloride, the title compound was obtained by the method similar to that in Example 147. Yield 90%. Amorphous.
¹H NMR (CDCl₃) δ 1.23 (6H, s), 1.27 (6H, s), 1.44 (3H, t, J = 6.8 Hz), 2.23 (2H, s), 2.65 (2H, s), 3.77 (3H, s), 3.85 (3H, s), 4.17 (2H, q, J = 6.8 Hz), 4.40 (2H, d, J = 5.2 Hz), 6.54 (1H, d, J = 7.6 Hz), 6.57 (1H, s), 6.74 (1H, s), 6.80-6.92 (2H, m), 7.21 (1H, t, J = 7.2 Hz), 7.27 (1H, s), 7.89 (1H, d, J = 8.0 Hz), 8.12 (1H, t, J = 5.2 Hz).

### Example 150

### 4-(6-Ethoxy-3,4,8,9-tetrahydro-3,3,8,8-tetramethylfuro[2,3-h]isoquinolin-1-yl)-2-[[(2-methoxyphenyl)methyl]amino]benzoic acid

From methyl 4-(6-ethoxy-3,4,8,9-tetrahydro-3,3,8,8-tetramethylfuro[2,3-h]isoquinolin-1-yl)-2-[[(2-methoxyphenyl)methyl]amino]benzoate, the title compound was obtained by the method similar to that in Example 148. Yield 99%.
¹H NMR (CDCl₃) δ 1.24 (6H, s), 1.36 (6H, s), 1.45 (3H, t, J = 7.2 Hz), 2.20 (2H, s), 2.75 (2H, s), 3.72 (3H, s), 4.18 (2H, q, J = 7.2 Hz), 4.36 (2H, s), 6.45 (1H, dd, J = 7.6, 1.2 Hz), 6.55 (1H, d, J = 1.2 Hz), 6.58 (1H, s), 6.77 (1H, d, J = 8.0 Hz), 6.81 (1H, t, J = 7.6 Hz), 7.12-7.18 (1H, m), 7.22-7.30 (1H, m), 7.83 (1H, d, J = 8.0 Hz).

### Example 151

### Methyl 2-[([1,1'-biphenyl]-4-ylmethyl)amino]-4-(6-ethoxy-3,4,8,9-tetrahydro-3,3,8,8-tetramethylfuro[2,3-h]isoquinolin-1-yl)benzoate

With 4-(chloromethyl)biphenyl, the title compound was obtained by the method similar to that in Example 147. Yield 76%. Amorphous.
¹H NMR (CDCl₃) δ 1.23 (6H, s), 1.29 (6H, s), 1.44 (3H, t, J = 6.8 Hz), 2.28 (2H, s), 2.65 (2H, s), 3.87 (3H, s), 4.16 (2H, q, J = 6.8 Hz), 4.47 (2H, d, J = 5.2 Hz), 6.57 (1H, s), 6.61 (1H, dd, J = 8.0, 1.2 Hz), 6.76 (1H, d, J = 1.2 Hz), 7.30-7.35 (1H, m), 7.38-7.45 (4H, m), 7.52-7.58 (4H, m), 7.93 (1H, d, J = 8.0 Hz), 8.15 (1H, t, J = 5.2 Hz).

### Example 152

### 2-[([1,1'-Biphenyl]-4-ylmethyl)amino]-4-(6-ethoxy-3,4,8,9-tetrahydro-3,3,8,8-tetramethylfuro[2,3-h]isoquinolin-1-yl)benzoic acid

From methyl 2-[([1,1'-biphenyl]-4-ylmethyl)amino]-4-(6-ethoxy-3,4,8,9-tetrahydro-3,3,8,8-tetramethylfuro[2,3-h]isoquinolin-1-yl)benzoate, the title compound was obtained by the method similar to that in Example 152. Yield 99%.
Amorphous.
¹H NMR (CDCl₃) δ 1.22 (6H, s), 1.42-1.48 (9H, m), 2.24 (2H, s), 2.81 (2H, s), 4.17 (2H, q, J = 6.8 Hz), 4.31 (2H, s), 6.42 (1H, d, J = 6.4 Hz), 6.58 (2H, s), 7.25-7.55 (9H, m), 7.83 (1H, d, J = 8.0 Hz).

### Example 153

### 2-[[(4-Chlorophenyl)methyl]amino]-4-(6-ethoxy-3,4,8,9-tetrahydro-3,3,8,8-tetramethylfuro[2,3-h]isoquinolin-1-yl)benzoic acid

Methyl 4-(6-ethoxy-3,4,8,9-tetrahydro-3,3,8,8-tetramethylfuro[2,3-h]isoquinolin-1-yl)-2-[(trifluoroacetyl)amino]benzoate (520 mg, 1.00 mmol) was dissolved in N,N-dimethylformamide (6 ml), and tert-butoxy potassium (150 mg, 1.33 mmol) was added thereto. Then, p-chlorobenzyl bromide (229 mg, 1.11 mmol) was added thereto, and the mixture was stirred at room temperature for 5 hours. The reaction solution was combined with diisopropyl ether and an aqueous solution of potassium dihydrogen phosphate, and stirred. The layers were separated, the diisopropyl ether layer was dried, and concentrated to give a crude product, which was purified with a silica gel column chromatography (hexane/ethyl acetate, 9:1 followed by 4:1) to give methyl 2-[[(4-chlorophenyl)methyl](trifluoroacetyl)amino]-4-(6-ethoxy-3,4,8,9-tetrahydro-3,3,8,8-tetramethylfuro[2,3-h]isoquinolin-1-yl)benzoate (250 mg) as a syrup.

The product was dissolved in methanol, hydrolyzed by adding 2 M aqueous solution of sodium hydroxide, neutralized with 2 M hydrochloric acid, and extracted with ethyl acetate to give a crude product, which was purified with a silica gel column chromatography (chloroform followed by chloroform/methanol 5:1) to give the title compound (yield 27%).
Amorphous.
¹H NMR (CDCl₃) δ 1.25 (6H, s), 1.46 (3H, t, J = 6.8 Hz), 1.47-1.53 (6H, m), 2.10-2.30 (2H, m), 2.85 (2H, s), 4.13-4.25 (2H, m), 4.20 (2H, q, J = 6.8 Hz), 6.39 (1H, d, J = 7.6 Hz), 6.52 (1H, s), 6.60 (1H, s), 7.11 (2H, d, J = 8.8 Hz), 7.17 (2H, d, J = 8.8 Hz), 7.78 (1H, d, J = 8.0 Hz), 8.61 (1H, br).

### Example 154

### 4-(6-Ethoxy-3,4,8,9-tetrahydro-3,3,8,8-tetramethylfuro[2,3-h]isoquinolin-1-yl)-2-[[(4-methoxyphenyl)methyl]amino]benzoic acid

Methyl 4-(6-ethoxy-3,4,8,9-tetrahydro-3,3,8,8-tetramethylfuro[2,3-h]isoquinolin-1-yl)-2-[(trifluoroacetyl)amino]benzoate (520 mg, 1.00 mmol) was dissolved in N,N-dimethylformamide (10 ml). To this mixture were added p-methoxybenzyl chloride (210 mg, 1.34 mmol), potassium iodide (230 mg, 1.38 mmol) and potassium carbonate (450 mg, 3.25 mmol), and the mixture was stirred at room temperature overnight. The reaction solution was extracted with diisopropyl ether, washed with water, dried, and concentrated to methyl give 4-(6-ethoxy-3,4,8,9-tetrahydro-3,3,8,8-tetramethylfuro[2,3-h]isoquinolin-1-yl)-2-[[(4-methoxyphenyl)methyl](trifluoroacetyl)amino]benzoate.

The product was dissolved in methanol (10 ml), 2 M aqueous solution (6 ml) of sodium hydroxide was added thereto, and the mixture was stirred at room temperature for 4 hours. The mixture was concentrated, and the residue was adjusted at pH 4 with hydrochloric acid. The precipitate was taken by filtration, washed with water, and dried to give the title compound (287 mg, yield 54%).
¹H NMR (CDCl₃) δ 1.29 (6H, s), 1.37 (6H, s), 1.45 (3H, t, J = 6.8 Hz), 2.19 (2H, s), 2.75 (2H, s), 3.70 (3H, s), 4.19 (2H, s), 4.19 (2H, q, J = 6.8 Hz), 6.40 (1H, d, J = 7.2 Hz), 6.53 (1H, s), 6.57 (1H, s), 6.71 (2H, d, J = 7.6 Hz), 7.12 (2H, d, J = 7.6 Hz), 7.78-7.90 (1H, br).

### Example 155

### 4-(6-Ethoxy-3,4,8,9-tetrahydro-3,3,8,8-tetramethylfuro[2,3-h]isoquinolin-1-yl)-2-[(3-phenoxypropyl)amino]benzoic acid

With 3-phenoxypropyl bromide, the title compound was obtained by the method similar to that in Example 154. Yield 75%. Amorphous.
¹H NMR (CDCl₃) δ 1.29 (6H, s), 1.45 (6H, s), 1.47 (3H, t, J = 7.2 Hz), 1.97 (2H, t, J = 6.4 Hz), 2.36 (2H, s), 2.80 (2H, s), 3.24 (1H, s), 3.91 (2H, t, J = 6.0 Hz), 4.20 (2H, q, J = 7.2 Hz), 6.44-6.52 (1H, m), 6.61 (2H, d, J = 6.4 Hz), 6.66 (1H, s), 6.80-6.90 (3H, m), 7.19 (2H, t, J = 8.0 Hz), 7.82-7.88 (1H, m).

### Example 156

### Methyl 4-(6-ethoxy-3,4,8,9-tetrahydro-3,3,8,8-tetramethylfuro[2,3-h]isoquinolin-1-yl)-2-[(2-methoxybenzoyl)amino]benzoate

Methyl 2-amino-4-(6-ethoxy-3,4,8,9-tetrahydro-3,3,8,8-tetramethylfuro[2,3-h]isoquinolin-1-yl)benzoate (211 mg, 0.5 mmol) was dissolved in N,N-dimethylacetamide (3 ml), 2-methoxybenzoyl chloride (0.1 ml, 0.74 mmol) was added thereto, and the mixture was stirred at room temperature for 1 hour. Then, the mixture was extracted with ethyl acetate, washed with water, dried, and concentrated to give a crude product. The crude product was treated with hexane, and the precipitated crystals were taken by filtration to give the title compound (259 mg, yield 93%).
¹H NMR (CDCl₃) δ 1.24 (6H, s), 1.32 (6H, s), 1.46 (3H, t, J = 6.8 Hz), 2.40 (2H, br), 2.66 (2H, s), 3.95 (3H, s), 4.09 (3H, s), 4.18 (2H, q, J = 6.8 Hz), 6.51 (1H, s), 7.00-7.10 (2H, m), 7.14-7.16 (1H, m), 7.48 (1H, m), 8.05 (1H, d, J = 8.0 Hz), 8.14 (1H, dd, J = 8.0, 1.6 Hz), 9.00 (1H, s), 12.10 (1H, s).

### Example 157

### 4-(6-Ethoxy-3,4,8,9-tetrahydro-3,3,8,8-tetramethylfuro[2,3-h]isoquinolin-1-yl)-2-[(2-methoxybenzoyl)amino]benzoic acid

From methyl 4-(6-ethoxy-3,4,8,9-tetrahydro-3,3,8,8-tetramethylfuro[2,3-h]isoquinolin-1-yl)-2-[(2-methoxybenzoyl)amino]benzoate, the title compound was obtained by the method similar to that in Example 9. Yield 89%.
¹H NMR (CDCl₃) δ 1.29 (6H, s), 1.50 (3H, t, J = 6.8 Hz), 1.63 (6H, s), 2.22 (1H, d, J = 16.0 Hz), 2.58 (1H, d, J = 16.0 Hz), 2.98 (2H, s), 3.96 (3H, s), 4.25 (2H, q, J = 6.8 Hz), 6.66 (1H, s), 6.8₃₋₆.90 (3H, m), 7.25-7.36 (1H, m), 7.72 (1H, d, J = 8.2 Hz), 7.87 (2H, d, J = 8.2 Hz), 8.30 (1H, br s), 8.37 (1H, s).

### Example 158

### Methyl 2-[(chloroacetyl)amino]-4-(6-ethoxy-3,4,8,9-tetrahydro-3,3,8,8-tetramethylfuro[2,3-h]isoquinolin-1-yl)benzoate

Methyl 2-amino-4-(6-ethoxy-3,4,8,9-tetrahydro-3,3,8,8-tetramethylfuro[2,3-h]isoquinolin-1-yl)benzoate (2.03 g, 4.80 mmol) were dissolved in N,N-dimethylacetamide (10 ml). To this mixture was added chloroacetyl chloride (0.5 ml, 6.28 mmol), and the mixture was stirred at room temperature for 3 hours. The reaction solution was concentrated, the residue was combined with ethyl acetate, and the precipitated crystals were taken by filtration to give the title compound (2.37 g, yield 99%).
¹H NMR (CDCl₃) δ 1.23 (6H, s), 1.31 (6H, s), 1.45 (3H, t, J = 7.2 Hz), 2.29 (2H, s), 2.66 (2H, s), 3.98 (3H, s), 4.17 (2H, q, J = 7.2 Hz), 4.18 (2H, s), 6.59 (1H, s), 7.19 (1H, dd, J = 8.0, 1.2 Hz), 8.08 (1H, d, J = 8.0 Hz), 8.73 (1H, s).

### Example 159

### Methyl 4-(6-ethoxy-3,4,8,9-tetrahydro-3,3,8,8-tetramethylfuro[2,3-h]isoquinolin-1-yl)-2-[[[(2-methoxy-2-oxoethyl)sulfanyl]acetyl]amino]benzoate

Methyl 2-[(chloroacetyl)amino]-4-(6-ethoxy-3,4,8,9-tetrahydro-3,3,8,8-tetramethylfuro[2,3-h]isoquinolin-1-yl)benzoate (500 mg, 1.0 mmol) was dissolved in methanol (20 ml). To this solution were added ethyl thioglycolate (0.13 ml, 1.18 mmol) and potassium carbonate (170 mg, 1.23 mmol), and the mixture was stirred for 1 hour at room temperature. The reaction solution was concentrated, and the residue was extracted with diisopropyl ether, washed with water, and dried, and the solvent was distilled off to give the title compound (524 mg, yield 92%).
¹H NMR (CDCl₃) δ 1.23 (6H, s), 1.31 (6H, s), 1.45 (3H, t, J = 7.2 Hz), 2.35 (2H, s), 2.65 (2H, s), 3.39 (2H, s), 3.52 (2H, s), 3.71 (3H, s), 3.97 (3H, s), 4.18 (2H, q, J = 7.2 Hz), 6.58 (1H, s), 7.16 (1H, dd, J = 8.0, 1.2 Hz), 8.06 (1H, d, J = 8.0 Hz), 8.74 (1H, s), 11.5 (1H, s).

### Example 160

### Methyl 4-(6-ethoxy-3,4,8,9-tetrahydro-3,3,8,8-tetramethylfuro[2,3-h]isoquinolin-1-yl)-2-[(2-thienylacetyl)amino]benzoate

Methyl 2-amino-4-(6-ethoxy-3,4,8,9-tetrahydro-3,3,8,8-tetramethylfuro[2,3-h]isoquinolin-1-yl)benzoate (211 mg, 0.5 mmol) was dissolved in N,N-dimethylacetamide (10 ml). To this solution was added thiophene-2-acetyl chloride (0.1 ml, 0.81 mmol) and the mixture was stirred for 3 hours at room temperature. The reaction solution was extracted with ethyl acetate, washed with water, and dried, concentrated, and the residue was treated with hexane to give the title compound (255 mg, yield 93%).
Amorphous.
¹H NMR (CDCl₃) δ 1.22 (6H, s), 1.29 (6H, s), 1.45 (3H, t, J = 7.2 Hz), 2.28 (2H, s), 2.64 (2H, s), 3.90 (3H, s), 3.95 (2H, s), 4.17 (2H, q, J = 7.2 Hz), 6.57 (1H, s), 6.99-7.05 (2H, m), 7.12 (1H, dd, J = 8.4, 1.6 Hz), 7.22-7.28 (1H, m), 8.01 (1H, d, J = 8.0 Hz), 8.75 (1H, d, J = 1.6 Hz), 11.1 (1H, s).

### Example 161

### Methyl 2-[[(E)-(dimethylamino)methylidene]amino]-4-(6-ethoxy-3,4,8,9-tetrahydro-3,3,8,8-tetramethylfuro[2,3-h]isoquinolin-1-yl)benzoate

To methyl 2-amino-4-(6-ethoxy-3,4,8,9-tetrahydro-3,3,8,8-tetramethylfuro[2,3-h]isoquinolin-1-yl)benzoate (250 mg, 0.6 mmol) was added N,N-dimethylformamide dimethylacetal (2 ml), and the mixture was stirred at 130°C for 3 hours, and concentrated. The residue was purified with a silica gel column chromatography (diisopropyl ether) to give the title compound (210 mg, yield 74%). ¹H NMR (CDCl₃) δ 1.21 (6H, s), 1.30 (6H, s), 1.45 (3H, t, J = 7.2 Hz), 2.29 (2H, s), 2.66 (2H, s), 3.01 (6H, s), 3.86 (3H, s), 4.17 (2H, q, J = 7.2 Hz), 6.58 (1H, s), 6.90 (1H, d, J = 1.6 Hz), 7.00 (1H, dd, J = 8.0, 1.6 Hz), 7.41 (1H, s), 7.79 (1H, d, J = 8.0 Hz).

### Example 162

### Methyl 2-amino-4-(3,4,8,9-tetrahydro-6-methoxy-3,3,8,8-tetramethylfuro[2,3-h]isoquinolin-1-yl)benzoate

To a mixture of methyl 2-amino-4-cyanobenzoate (2.62 g, 14.9 mmol) and 2,3-dihydro-7-methoxy-2,2-dimethyl-α-(1-methylethyl)-5-benzofuranmethanol (4.12 g, 16.8 mmol) in acetic acid (9.10 ml) and toluene (14.5 ml) was added conc. sulfuric acid (2.15 ml, 40.3 mmol) at room temperature, and the mixture was stirred at 80°C for 2 hours and at 65°C for 13 hours. To the reaction mixture was added methanol (26.0 ml), and the mixture was heated under reflux for 2 hours and 30 minutes. After cooling, the reaction solution was concentrated under reduced pressure. The resultant residue was distributed to ethyl acetate and a saturated aqueous solution of sodium hydrogen carbonate, and further the organic substances were extracted with ethyl acetate from the aqueous layer. The ethyl acetate solution was collected, washed with a saturated aqueous solution of sodium chloride, dried with sodium sulfate, and concentrated under reduced pressure. The resultant residue was purified with a silica gel column chromatography (chloroform/methanol, 50:1 followed by 20:1). The resultant crude product was purified with a basic silica gel column chromatography (hexane/ethyl acetate, 5:1 followed by 3:1), and crystallized from diisopropyl ether to give the title compound (1.23 g, yield 18%). Melting point: 161.0 to 162.0°C.
¹H NMR (CDCl₃) δ 1.23 (6H, br s), 1.34 (6H, s), 2.37 (2H, s), 2.67 (2H, s), 3.89 (3H, s), 3.92 (3H, s), 5.76 (2H, s), 6.60 (1H, s), 6.62 (1H, dd, J = 8.4, 1.2 Hz), 6.74 (1H, d, J = 1.2 Hz), 7.85 (1H, d, J = 8.4 Hz).

### Example 163

### Methyl 4-(3,4,8,9-tetrahydro-6-methoxy-3,3,8,8-tetramethylfuro[2,3-h]isoquinolin-1-yl)-2-[(trifluoroacetyl)amino]benzoate

To a solution of methyl 2-amino-4-(3,4,8,9-tetrahydro-6-methoxy-3,3,8,8-tetramethylfuro[2,3-h]isoquinolin-1-yl)benzoate (1.34 g, 3.28 mmol) and triethylamine (0.55 ml, 3.9 mmol) in tetrahydrofuran (13 ml) was added trifluoroacetic anhydride (0.51 ml, 3.6 mmol) at 0°C, and the mixture was stirred at the same temperature for 10 minutes and at room temperature for 2 hours and 10 minutes. The reaction solution was distributed to ethyl acetate and water, and the organic substances were extracted with ethyl acetate from the aqueous layer. The ethyl acetate solution was collected, washed with a saturated aqueous solution of sodium hydrogen carbonate and a saturated aqueous solution of sodium chloride, dried with sodium sulfate, and concentrated under reduced pressure. The resultant residue was washed with diisopropyl ether to give the title compound (0.435 g, yield 26%). Melting point: 146.0 to 147.0°C.
¹H NMR (CDCl₃) δ 1.25 (6H, s), 1.33 (6H, s), 2.29 (2H, s), 2.69 (2H, s), 3.82 (3H, s), 4.00 (3H, s), 6.62 (1H, s), 7.28 (1H, dd, J = 8.2, 1.6 Hz), 8.12 (1H, d, J = 8.0 Hz), 8.71 (1H, d, J = 1.6 Hz), 12.27 (1H, s).

### Example 164

### Methyl 2-[[3-(acetylamino)benzoyl]amino]-4-(3,4,8,9-tetrahydro-6-methoxy-3,3,8,8-tetramethylfuro[2,3-h]isoquinolin-1-yl)benzoate

To a suspension of 3-(acetamido)benzoic acid (215 mg, 1.20 mmol) in 1,2-dichloroethane (1.5 ml) were added thionyl chloride (0.18 ml, 2.5 mmol) and N,N-dimethylformamide (1 drop), and the mixture was stirred for 2 hours at 75°C. The reaction mixture was concentrated under reduced pressure, and the residue was dissolved in 1,2-dichloroethane (0.5 ml). This was added dropwise to a solution of methyl 2-amino-4-(3,4,8,9-tetrahydro-6-methoxy-3,3,8,8-tetramethylfuro[2,3-h]isoquinolin-1-yl)benzoate (337 mg, 0.825 mmol) and triethylamine (0.18 ml, 1.3 mmol) in 1,2-dichloroethane (1 ml), and the mixture was stirred at room temperature for 25 minutes. The reaction mixture was combined with water and ethyl acetate, and weakly alkalified with a saturated aqueous solution of sodium hydrogen carbonate. The organic layer was separated, and the aqueous layer was extracted with ethyl acetate. The combined organic layer was washed with water and a saturated aqueous solution of sodium chloride, dried through sodium sulfate-basic silica gel (eluting with ethyl acetate), and concentrated under reduced pressure. The residue was purified with a basic silica gel column chromatography (hexane/ethyl acetate 1:3), and crystallized from ethyl acetate-diethyl ether to give the title compound (160 mg, yield 34%).
¹H NMR (CDCl₃) δ 1.26 (6H, s), 1.32 (6H, s), 2.22 (3H, s), 2.37 (2H, br s), 2.70 (2H, s), 3.93 (3H, s), 4.01 (3H, s), 6.17 (1H, s), 7.17 (1H, dd, J = 8.2, 1.4 Hz), 7.40 (1H, br s), 7.49 (1H, t, J = 7.7 Hz), 7.75 (1H, d, J = 7.7 Hz), 7.89-7.92 (1H, m), 7.98-8.03 (1H, m), 8.11 (1H, d, J = 8.2 Hz), 8.96 (1H, d, J = 1.4 Hz), 12.04 (1H, s).

### Example 165

### 2-[[3-(Acetylamino)benzoyl]amino]-4-(3,4,8,9-tetrahydro-6-methoxy-3,3,8,8-tetramethylfuro[2,3-h]isoquinolin-1-yl)benzoic acid

To a suspension of methyl 2-[[3-(acetylamino)benzoyl]amino]-4-(3,4,8,9-tetrahydro-6-methoxy-3,3,8,8-tetramethylfuro[2,3-h]isoquinolin-1-yl)benzoate (85 mg, 0.15 mmol) in methanol (0.4 ml) was added 5 M aqueous solution (76 µl, 0.38 mmol) of sodium hydroxide, and the mixture was stirred at 75°C for 15 minutes.
The reaction mixture was combined with 1 M hydrochloric acid (0.38 ml, 0.38 mmol), and extracted twice with chloroform. The combined organic layer was washed with a saturated aqueous solution of sodium chloride, dried through sodium sulfate, concentrated under reduced pressure to give the title compound (81 mg, yield 97%). Amorphous.
¹H NMR (DMSO-d₆) δ 1.22 (6H, s), 1.25 (6H, br s), 2.08 (3H, s), 2.39 (2H, br s), 2.81 (2H, br s), 3.86 (3H, s), 6.92 (1H, s), 7.22 (1H, dd, J = 8.1, 1.5 Hz), 7.50 (1H, t, J = 7.8 Hz), 7.59-7.64 (1H, m), 7.77 (1H, m), 8.13 (1H, d, J = 8.1 Hz), 8.24 (1H, s), 8.75 (1H, d, J = 1.5 Hz), 10.21 (1H, s).

### Example 166

### Butyl 4-(6-ethoxy-3,4,8,9-tetrahydro-3,3,8,8-tetramethylfuro[2,3-h]isoquinolin-1-yl)-2-(1-pyrrolidinyl)benzeneacetate

To a solution of 7-ethoxy-2,3-dihydro-2,2-dimethyl-α-(1-methylethyl)-5-benzofuranmethanol (343 mg, 1.30 mmol) and methyl 4-cyano-2-pyrrolidinylbenzeneacetate (279 mg, 1.12 mmol) in butyl acetate (3 ml) was added methanesulfonic acid (0.5 ml). The resultant mixture was stirred at 90°C for 5 hours. The resultant mixture was cooled, the aqueous layer was separated by adding water, and the organic layer was extracted with water. The combined aqueous layer was combined with an aqueous solution of 10% potassium carbonate, alkalified, and extracted with ethyl acetate. The organic layer was washed with a saturated aqueous solution of sodium chloride, dried, and concentrated under reduced pressure. The residue was purified with a basic silica gel column chromatography (hexane/ethyl acetate 1:1) to give the title compound (173 mg, yield 28%).
Oily matter.
¹H NMR (CDCl₃) δ 0.91 (3H, t), 1.23 (6H, br s), 1.28-1.41 (2H, m), 1.31 (6H, s), 1.46 (3H, t), 1.52-1.63 (2H, m), 1.85-1.90 (4H, m), 2.27 (2H, s), 2.66 (2H, s), 3.08 (4H, br s), 3.71 (2H, s), 4.08 (2H, t), 4.18 (2H, q), 6.59 (1H, s), 6.97-7.02 (2H, m), 7.20 (1H, d).

### Example 167

### Butyl 4-(6-ethoxy-3,4,8,9-tetrahydro-3,3,8,8-tetramethylfuro[2,3-h]isoquinolin-1-yl)-2-[(2-pyridinylcarbonyl)amino]benzeneacetate

To a solution of 7-ethoxy-2,3-dihydro-2,2-dimethyl-α-(1-methylethyl)-5-benzofuranmethanol (668 mg, 2.53 mmol), methyl 4-cyano-2-[(2-pyridinylcarbonyl)amino]benzeneacetate (668 mg, 2.26 mmol) in butyl acetate (5 ml) was added methanesulfonic acid (0.85 ml). The resultant mixture was stirred for 2 hours at 90°C. The resultant mixture was cooled, the aqueous layer was separated by adding water, and the organic layer was extracted with water. The combined aqueous layer was combined with an aqueous solution of 10% potassium carbonate, alkalified, and extracted with ethyl acetate. The organic layer was washed with a saturated aqueous solution of sodium chloride, dried, and concentrated under reduced pressure. The residue was purified with a basic silica gel column chromatography (hexane/ethyl acetate 1:1) to give the title compound (465 mg, yield 35%).
Amorphous.
¹H NMR (CDCl₃) δ 0.89 (3H, t), 1.23 (6H, s), 1.30-1.40 (2H, m), 1.35 (6H, s), 1.46 (3H, t), 1.57-1.68 (2H, m), 2.47 (2H, br s), 2.65 (2H, s), 3.75 (2H, br s), 4.11 (2H, t), 4.18 (2H, q), 6.58 (1H, s), 7.21 (1H, dd), 7.31 (1H, d), 7.44-7.49 (1H, m), 7.88 (1H, dt), 8.07 (1H, d), 8.22-8.27 (1H, m), 8.60-8.64 (1H, m), 10.54 (1H, s).

### Example 168

### 4-(6-Ethoxy-3,4,8,9-tetrahydro-3,3,8,8-tetramethylfuro[2,3-h]isoquinolin-1-yl)-2-(1-pyrrolidinyl)benzeneacetic acid

To a solution of butyl 4-(6-ethoxy-3,4,8,9-tetrahydro-3,3,8,8-tetramethylfuro[2,3-h]isoquinolin-1-yl)-2-(1-pyrrolidinyl)benzeneacetate (140 mg, 0.263 mmol) in ethanol (2 ml) was added 1 M aqueous solution (0.5 ml) of sodium hydroxide, and the mixture was stirred for 2 hours at room temperature. The reaction solution was combined with 1 M hydrochloric acid (0.5 ml) and extracted with ethyl acetate. The organic layer was washed with a saturated aqueous solution of sodium chloride, dried, and concentrated under reduced pressure. The resultant residue was made as amorphous powders from ethyl acetate-hexane to give the title compound (101 mg, yield 81%).
¹H NMR (DMSO-d₆) δ 1.12 (6H, br s), 1.22 (6H, s), 1.34 (3H, t), 1.83 (4H, br s), 2.27 (2H, s), 2.59 (2H, s), 3.05 (4H, br s), 3.59 (2H, s), 4.08 (2H, q), 6.74 (1H, s), 6.84-6.93 (2H, m), 7.18 (1H, d).

In the following, the compounds prepared in Examples 1 to 168 are shown in Tables 1 to 10.

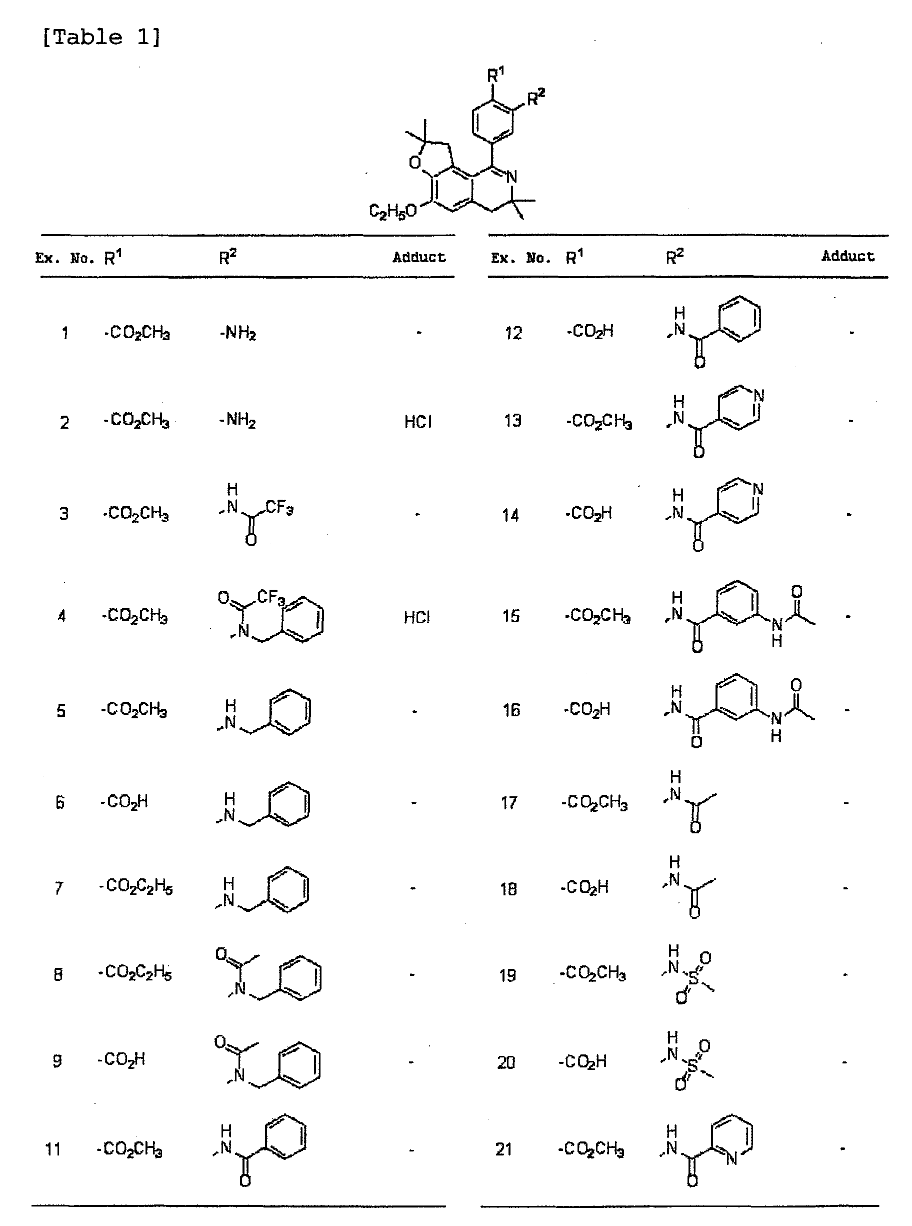

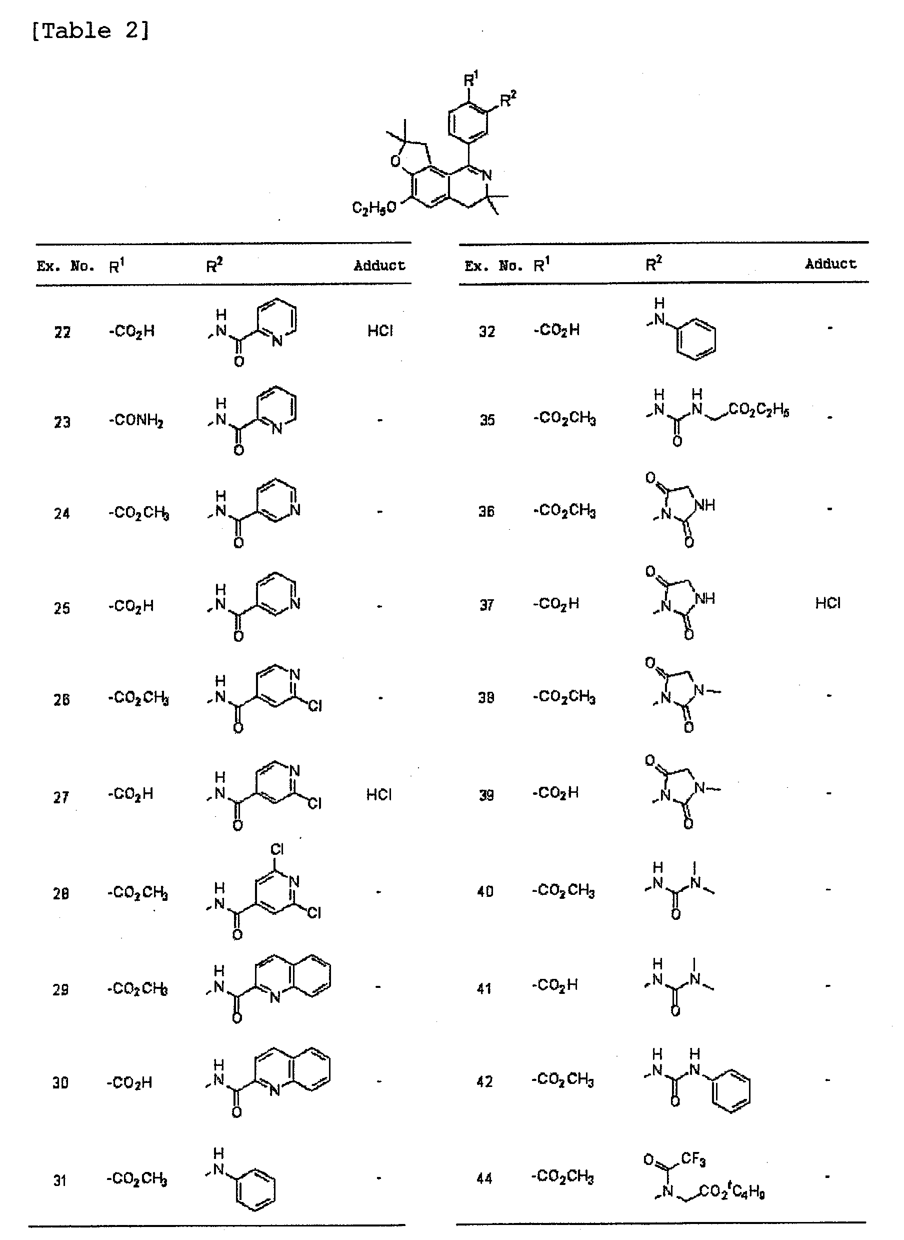

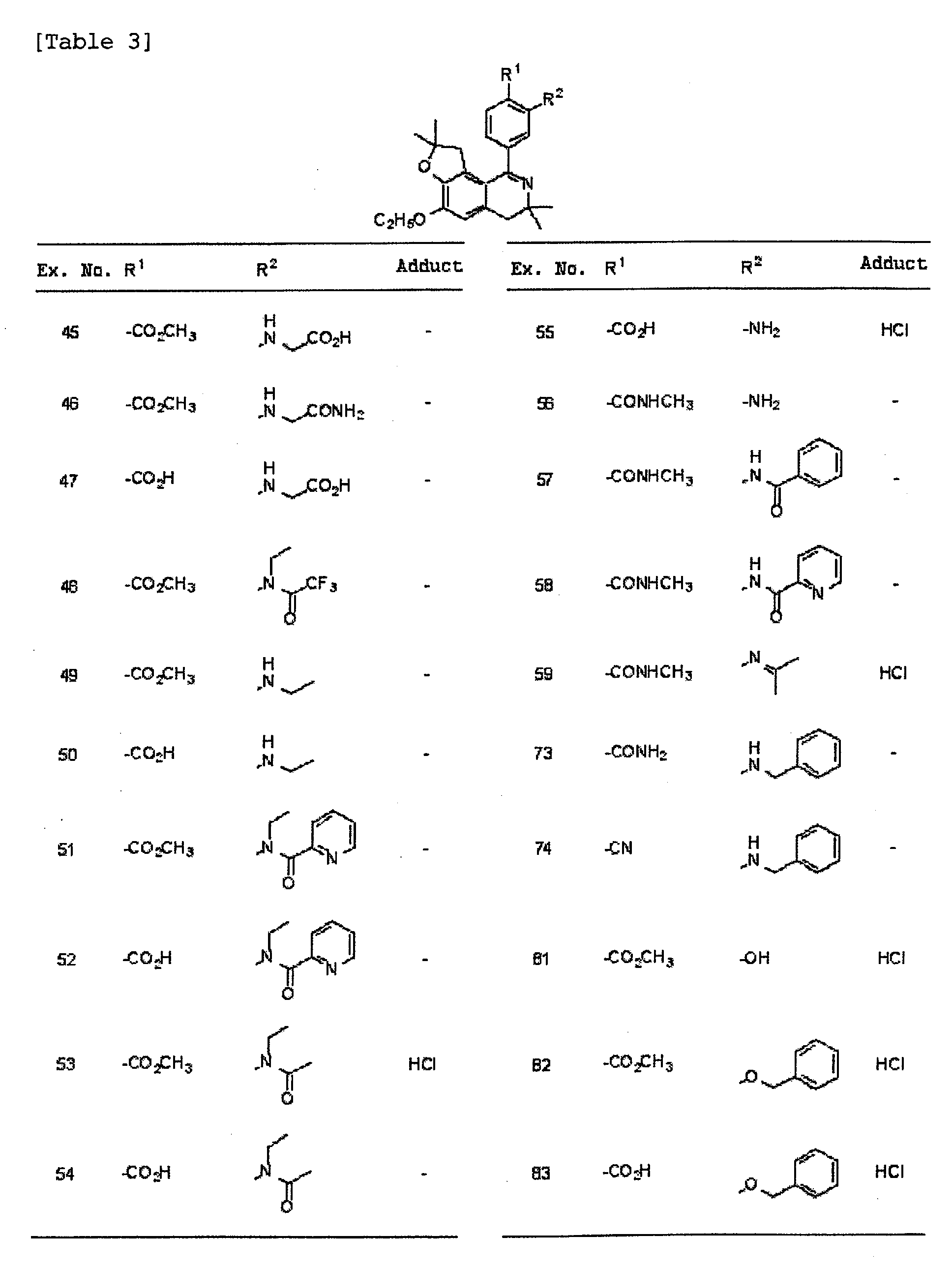

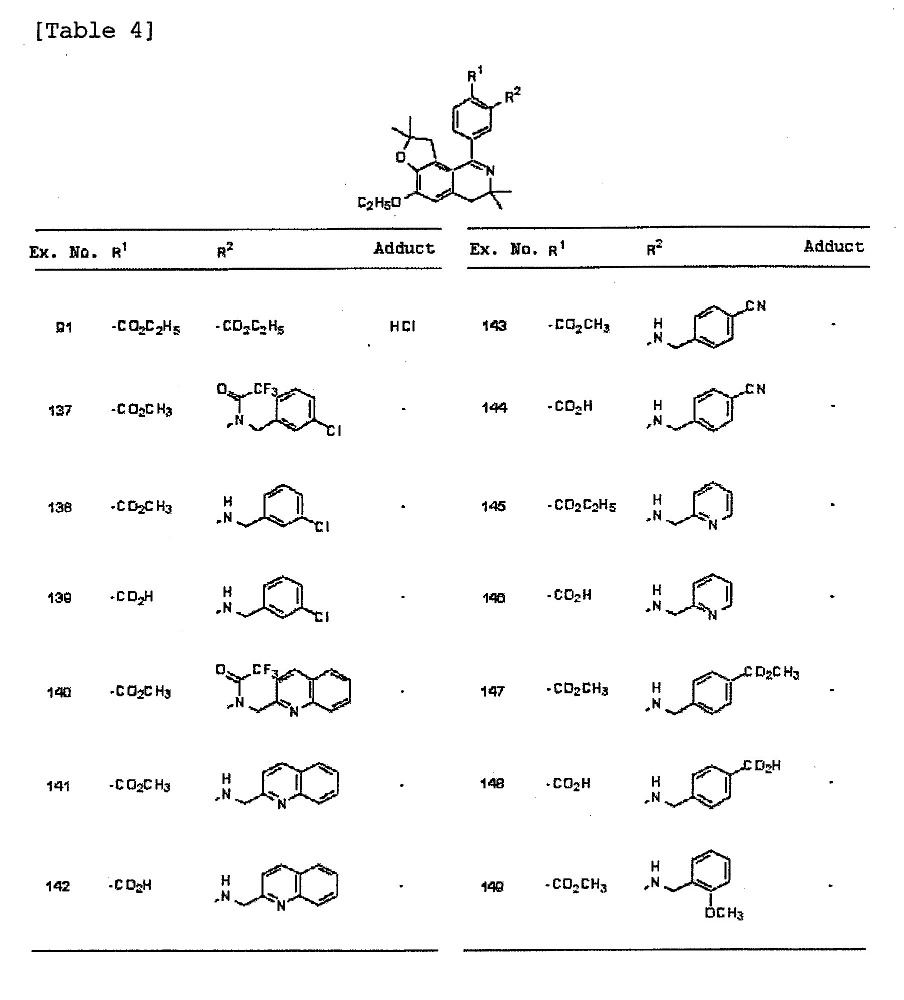

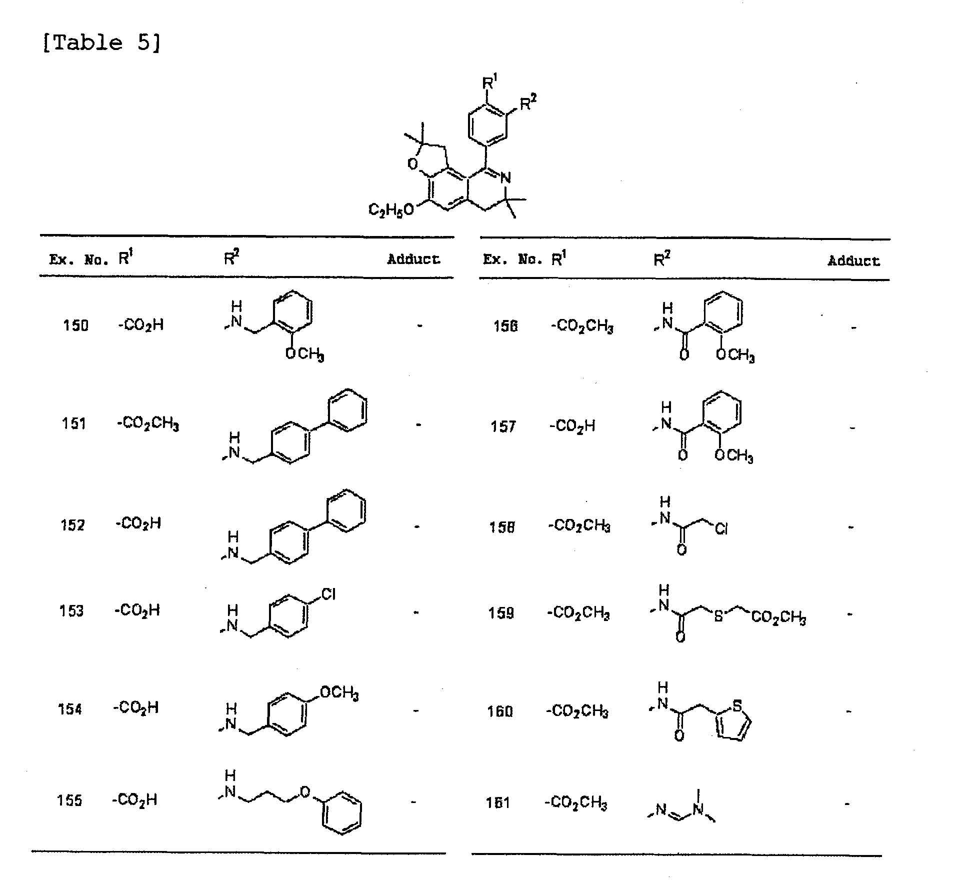

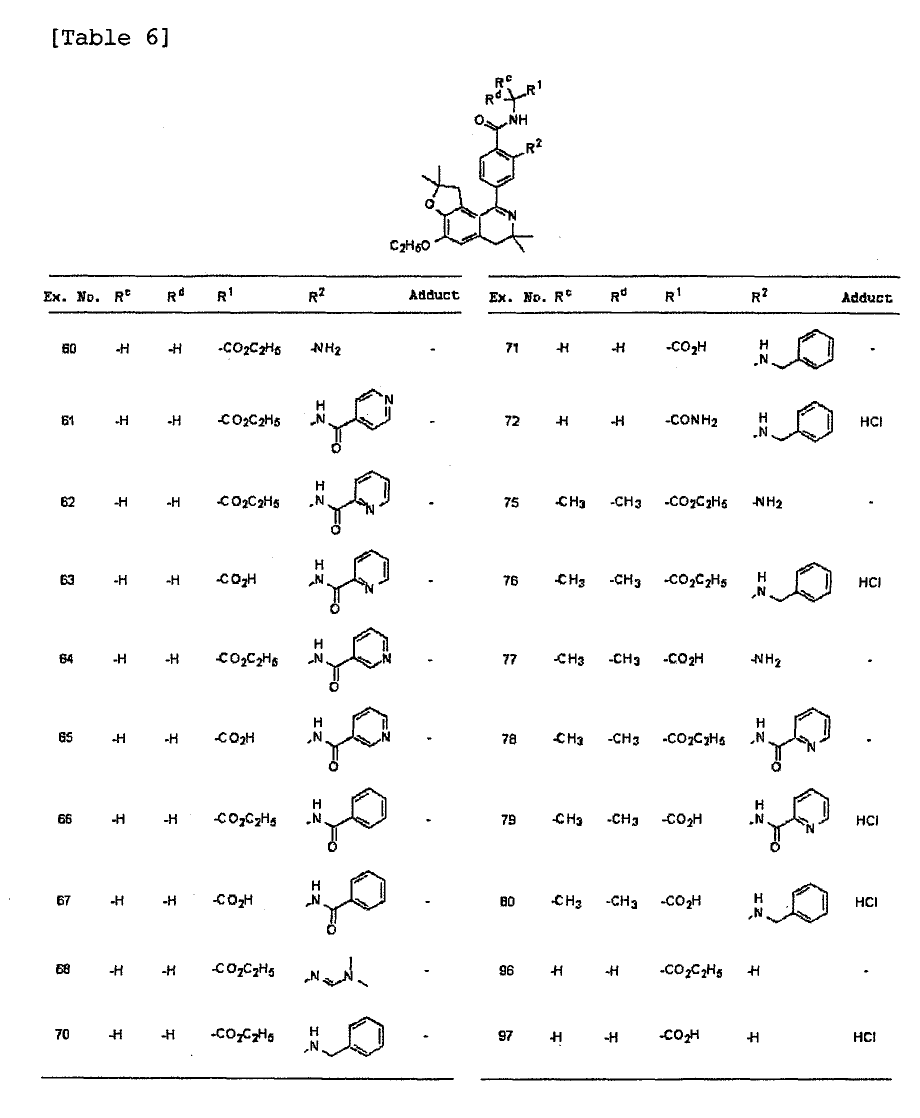

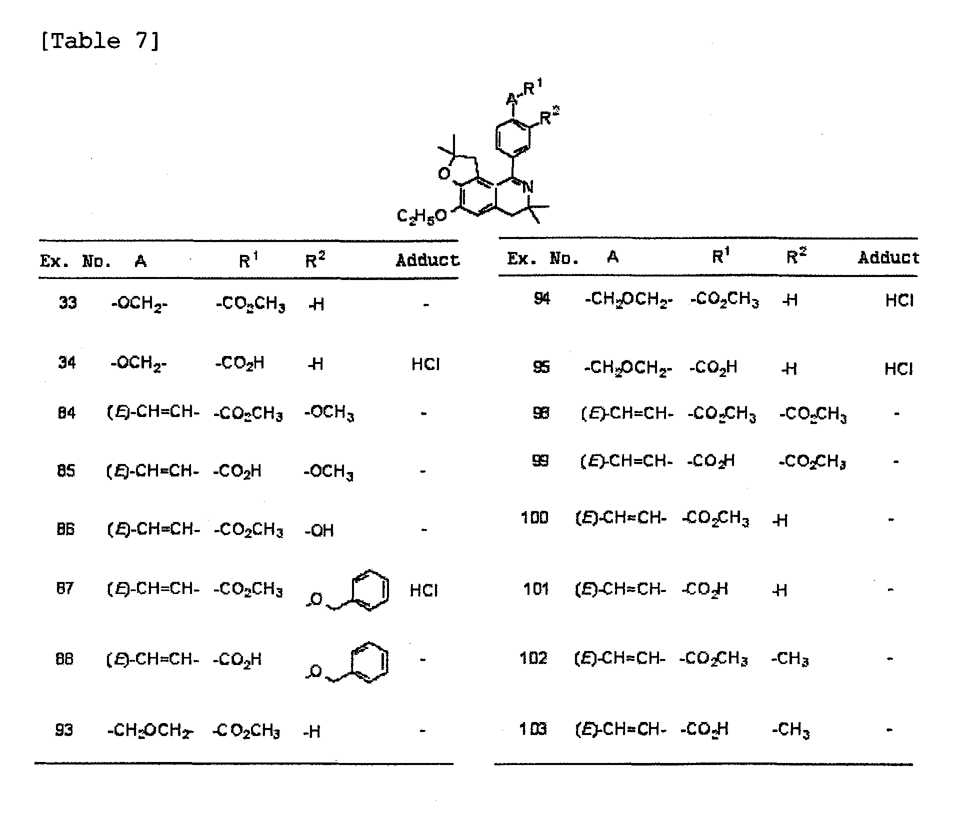

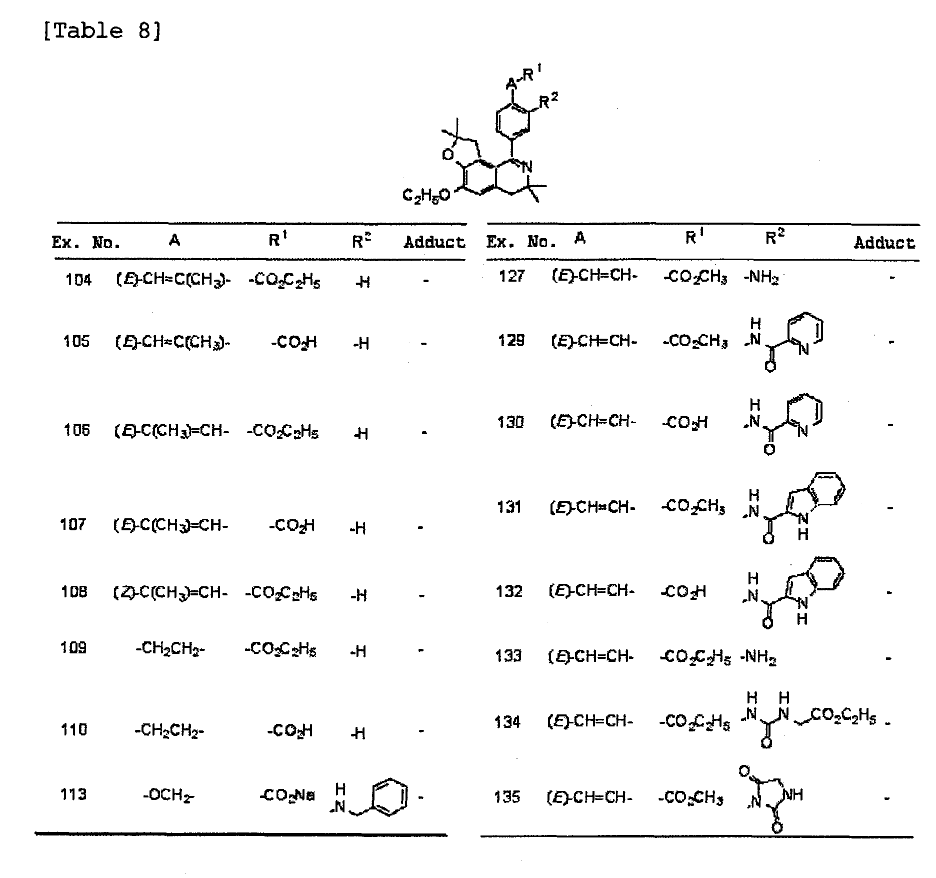

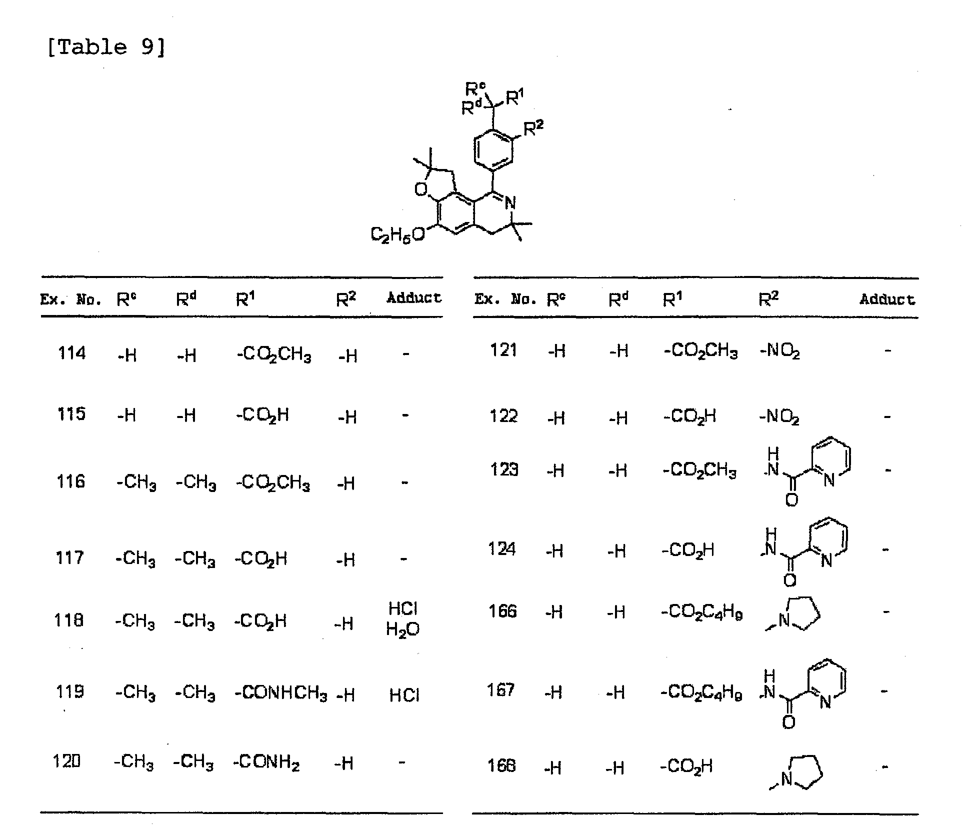

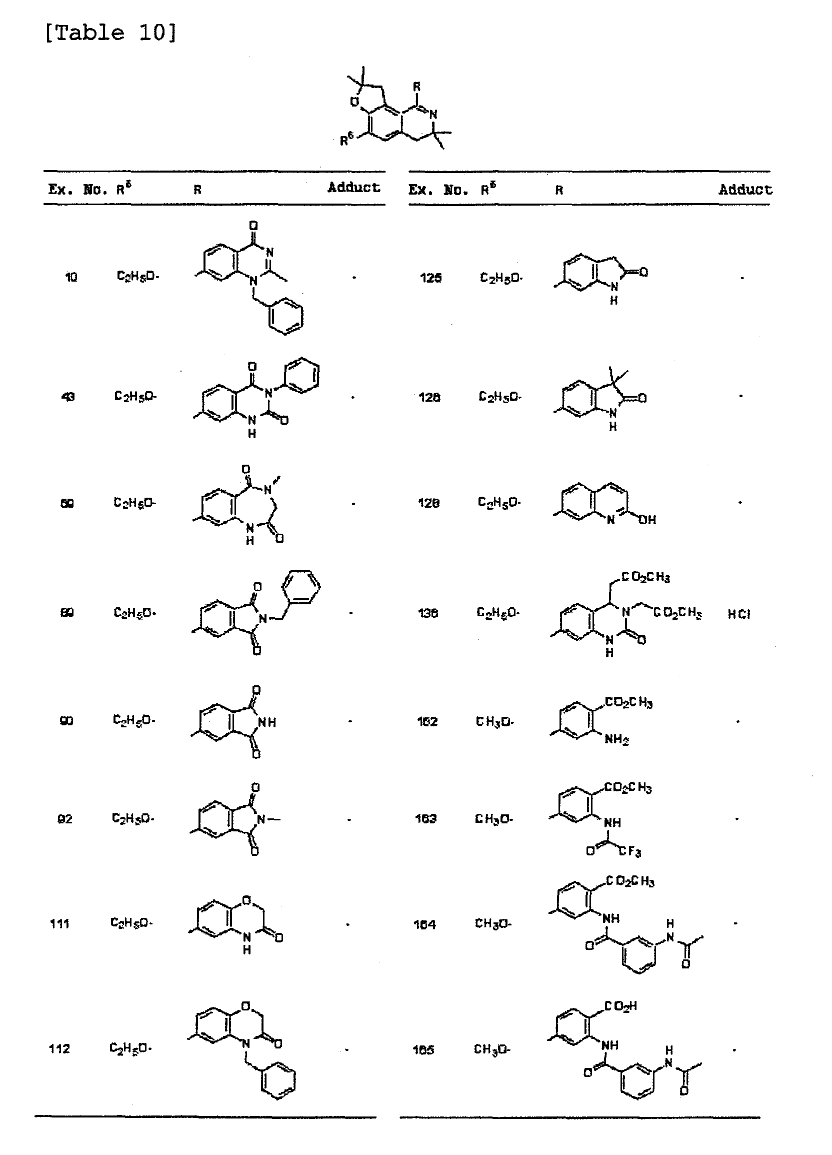

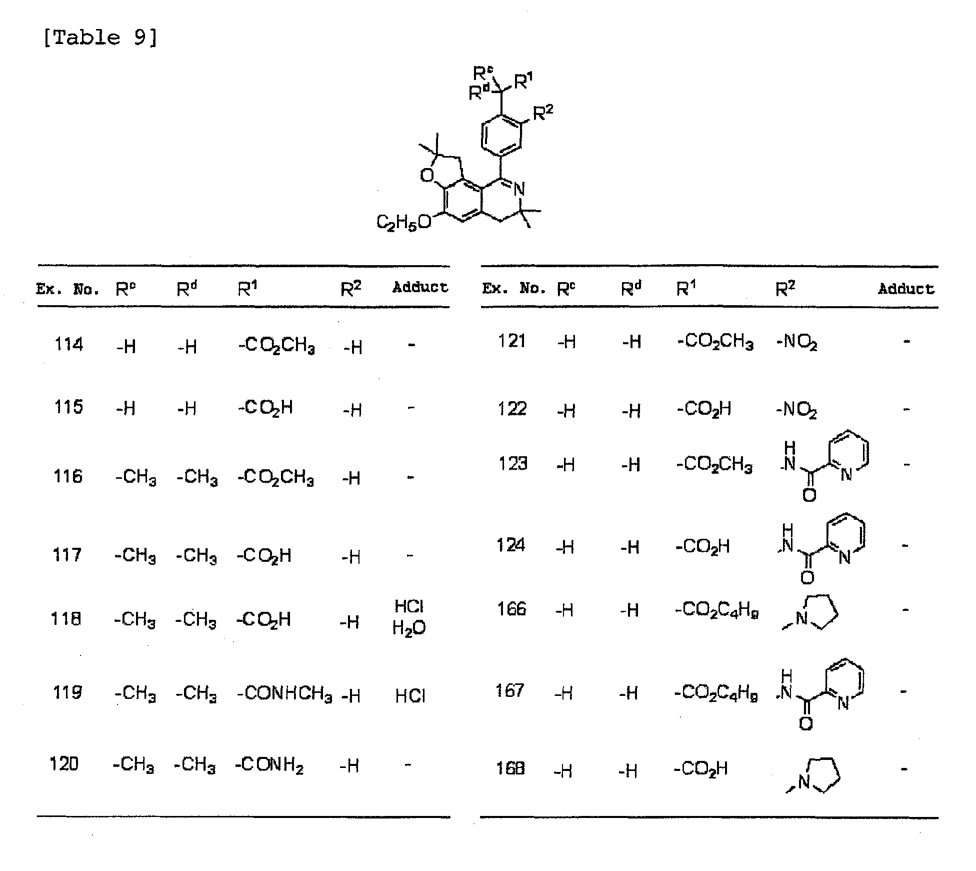

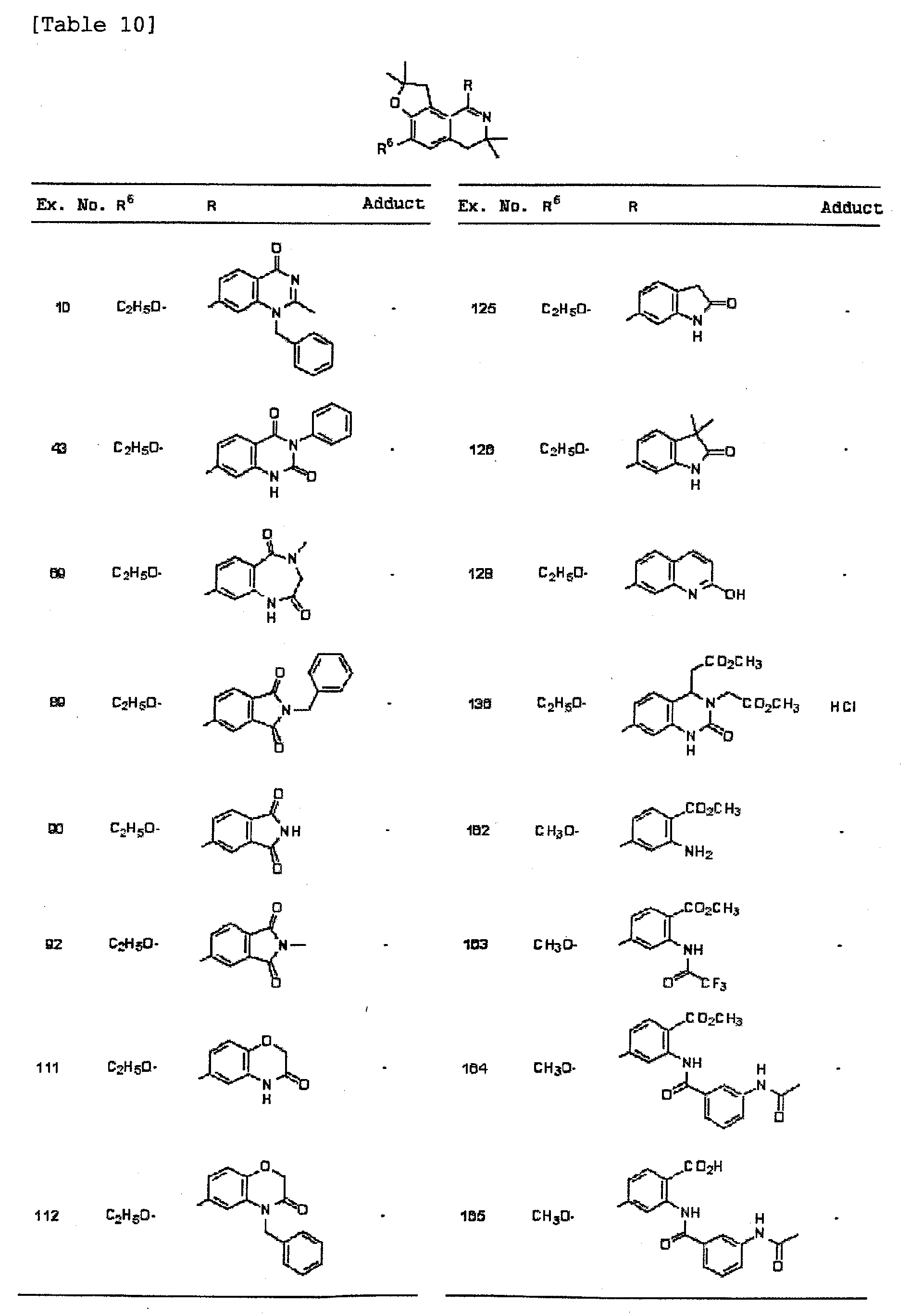

### Formulation Example 1

| | | |
|---|---|---|
| (1) | Compound of Example 6 | 10.0 mg |
| (2) | Lactose | 60.0 mg |
| (3) | Corn starch | 35.0 mg |
| (4) | Gelatin | 3.0 mg |
| (5) | Magnesium stearate | 2.0 mg |

A mixture of 10.0 mg of the compound obtained in Example 6, 60.0 mg of lactose and 35.0 mg of a corn starch was granulated using 0.03 ml of 10% aqueous solution of gelatin (3.0 mg as gelatin) through a 1 mm mesh sieve, dried at 40°C and then sieved again. The resultant granule was combined with 2.0 mg of magnesium stearate and then compressed. The resultant core was coated with a sugar coating comprising sucrose, titanium dioxide, talc and gum arabic in an aqueous suspension. The resultant coated tablet was imparted by polishing with beeswax to obtain a coated tablet.

### Formulation Example 2

| | | |
|---|---|---|
| (1) | Compound of Example 6 | 10.0 mg |
| (2) | Lactose | 70.0 mg |
| (3) | Corn starch | 50.0 mg |
| (4) | Soluble starch | 7.0 mg |
| (5) | Magnesium stearate | 3.0 mg |

10.0 mg of the compound obtained in Example 6 and 3.0 mg of magnesium stearate were granulated using 0.07 ml of an aqueous solution of a soluble starch (7.0 mg as soluble starch), dried, and then combined with 70.0 mg of lactose and 50.0 mg of a corn starch. The mixture was compressed into a tablet.

### Formulation Example 3

| | | |
|---|---|---|
| (1) | Compound of Example 1 | 5.0 mg |
| (2) | Sodium chloride | 20.0 mg |
| (3) | Distilled water | to 2 ml |

5.0 mg of the compound obtained in Example 6 and 20.0 mg of sodium chloride were dissolved in distilled water and water was then added to make the entire volume 2.0 ml. The solution was filtered, and filled aseptically in a 2 ml ampoule. The ampoule was sterilized, sealed, whereby obtaining an injection solution. Formulation Example 4

In a fluidized bed granulating drier (FD-5S, manufactured by POWREX Corporation), 1500 g of the compound obtained in Example 6, 2025 g of lactose and 556.5 g of corn starch were mixed homogeneously, and then an aqueous solution in which 126 g of hydroxypropylcellulose was dissolved was sprayed in the drier to conduct a granulation, after which the mixture was dried in the fluidized bed granulating drier. The resultant granule was ground using a power mill and sieved through a 1.5 mm punching screen to obtain sized granules. 3927 g of these sized granules were combined with 210 g of croscarmellose sodium and 63 g of magnesium stearate, and mixed in a tumbler mixer to obtain granules to be compacted into tablets. These granules were compacted into 300 mg core tablets using a 6.5 mm frame in a tablet-compacting machine. The resultant core tablets were coated with a solution containing hydroxypropylmethyl cellulose 2910 (TC-5) and macrogol 6000 dissolved therein in a Doria coater coating machine and titanium oxide and iron(III) oxide dispersed therein to obtain about 13500 film-coated tablets each containing 100 mg whose composition is shown below.

| Tablet formulation: | | |
|---|---|---|
| Composition | | Content (mg) |
| (1) | Compound of Example 6 | 100.0 |
| (2) | Lactose | 135.0 |
| (3) | Corn starch | 37.1 |
| (4) | Sodium croscarmellose | 15.0 |
| (5) | Hydroxypropylcellulose | 8.4 |
| (6) | Magnesium stearate | 4.5 |
| | Total (core tablet) | 300.0 |

| Film-coated tablet composition: | | |
|---|---|---|
| (1) | Core tablet | 300.0 |

| (Film component) | | |
|---|---|---|
| (2) | Hydroxypropylmethyl cellulose 2910 | 7.485 |
| (3) | Macrogol 6000 | 1.5 |
| (4) | Titanium oxide | 1.0 |
| (5) | Iron sesquioxide | 0.015 |
| | Total | 310.0 |

### Formulation Example 5

According to the method described in Formulation Example 4, about 13500 film-coated tablets having the formulation shown below each containing 25 mg of the compound obtained in Example 6 were obtained.

| Tablet formulation: | | |
|---|---|---|
| Composition | | Content (mg) |
| (1) | Compound of Example 6 | 25.0 |
| (2) | Lactose | 210.0 |
| (3) | Corn starch | 37.1 |
| (4) | Croscarmellose sodium | 15.0 |
| (5) | Hydroxypropylcellulose | 8.4 |
| (6) | Magnesium stearate | 4.5 |
| | Total (core tablet) | 300.0 |

| Film-coated formulation: | | |
|---|---|---|
| (1) | Core tablet | 300.0 |

| (Film ingredients) | | |
|---|---|---|
| (2) | Hydroxypropylcellulose 2910 | 7.485 |
| (3) | Macrogol 6000 | 1.5 |
| (4) | Titanium oxide | 1.0 |
| (5) | Iron sesquioxide | 0.015 |
| | Total | 310.0 |

### Formulation Example 6

According to the method described in Formulation Example 4, about 13500 film-coated tablets having the formulation shown below each containing 5 mg of the compound obtained in Example 6 were obtained.

| Tablet formulation: | | |
|---|---|---|
| Composition | | Content (mg) |
| (1) | Compound of Example 6 | 5.0 |
| (2) | Lactose | 230.0 |
| (3) | Corn starch | 37.1 |
| (4) | Croscarmellose sodium | 15.0 |
| (5) | Hydroxypropylcellulose | 8.4 |
| (6) | Magnesium stearate | 4.5 |
| | Total (core tablet) | 300.0 |

| Film-coated formulation: | | |
|---|---|---|
| (1) | Core tablet | 300.0 |

| (Film ingredients) | | |
|---|---|---|
| (2) | Hydroxypropylcellulose 2910 | 7.485 |
| (3) | Macrogol 6000 | 1.5 |
| (4) | Titanium oxide | 1.0 |
| (5) | Iron sesquioxide | 0.015 |
| | Total | 310.0 |

### Formulation Example 7

According to the method described in Formulation Example 4, about 13500 film-coated tablets having the formulation shown below each containing 1 mg of the compound obtained in Example 6 were obtained.

| Tablet formulation: | | |
|---|---|---|
| Composition | | Content (mg) |
| | Compound of Example 6 | 1.0 |
| | Lactose | 234.0 |
| | Corn starch | 37.1 |
| | Croscarmellose sodium | 15.0 |
| | Hydroxypropylcellulose | 8.4 |
| | Magnesium stearate | 4.5 |
| | Total (core tablet) | 300.0 |

| Film-coated formulation: | | |
|---|---|---|
| | Core tablet | 300.0 |

| (Film ingredients) | | |
|---|---|---|
| | Hydroxypropylcellulose 2910 | 7.485 |
| | Macrogol 6000 | 1.5 |
| | Titanium oxide | 1.0 |
| | Iron sesquioxide | 0.015 |
| | Total | 310.0 |

### Formulation Example 8

| | |
|---|---|
| White vaseline | 40 g |
| Cetanol | 10 g |
| Bleached beeswax | 5 g |
| Sorbitan sesquioleate | 5 g |
| Lauromocrogold | 0.5 g |
| Methyl p-oxybenzoate | 0.1 g |
| Propyl p-oxybenzoate | 0.1 g |
| Purified water | adequate amount |

An official absorption ointment (100 g) having the composition shown above was heated preliminarily at 70°C, and the solution was combined with a solution which was obtained by dissolving 1 g of the compound obtained in Example 6 in 20 ml of methanol with heating. At the same temperature, the mixture was stirred with heating for 10 minutes to remove residual methanol, and then cooled to room temperature to obtain a wettable ointment.

### Experimental Example 1

### Assay of phosphodiesterase IV-inhibiting action

### (1) Expression of recombinant human brain-derived phosphodiesterase 4D3 in Escherichia coli and purification thereof

Using the Escherichia coli BL21/pPDE4D3 (FERM BP-7075) producing recombinant human brain-derived phosphodiesterase 4D3, a recombinant human brain-derived phosphodiesterase 4D3 was obtained. The expression and purification of E. coli were in accordance with the protocol attached to GST Gene Fusion System (Pharmacia).

### (2) Assay of phosphodiesterase IV-inhibiting action

To a 96-well plate (OPTI plate, Packard), 10 µl of a buffer solution [0.5 M Tris-HCl (pH 7.5), 83 mM MgCl₂, 17 mM EGTA], 10 µl of the recombinant human brain-derived phosphodiesterase 4D3 (0.0034 mg/ml) obtained in Section (1) described above, 65 µl of Ultrapure water, 5 µl of an inhibitor sample and 10 µl of [³H] CAMP were added and reacted for 30 minutes at 30°C. After completing the reaction, 50 µl of SPA beads solution [18 mg/ml Yttrium silicate beads, 18 mM ZnSO₄] was added thereto, and the mixture was allowed to stand at room temperature for about 20 minutes. The radioactivity was counted using a scintillation counter (Topcount, Packard). Inhibiting activity of the compound against recombinant human brain-derived phosphodiesterase 4D3 was calculated, based on that enzymatic activity under absence of the compound was 100%. The inhibiting activities of the compounds of the present invention were shown in Table 11 as IC₅₀ value.

**[Table 11]**

| Example No. | Phosphodiesterase 4D inhibiting action (IC₅₀, nM) |
|---|---|
| 6 | 0.566 |
| 17 | 4.01 |
| 21 | 1.89 |
| 23 | 0.756 |
| 30 | 0.761 |
| 50 | 8.40 |
| 57 | 1.63 |
| 74 | 1.71 |
| 85 | 5.50 |
| 94 | 3.27 |
| 95 | 10.9 |
| 118 | 11.5 |
| 120 | 3.50 |
| 124 | 0.637 |

### INDUSTRIAL APPLICABILITY

The furoisoquinoline derivative of the present invention has an excellent phosphodiesterase IV-inhibiting action, and is useful as a prophylactic and/or therapeutic agent against inflammatory diseases, atopic dermatitis, allergic rhinitis, asthma, chronic obstructive pulmonary diseases, chronic rheumatoid arthritis, autoimmune diseases, depression, Alzheimer's dementia, osteoporosis, memory disorders, diabetes, atherosclerosis and the like.

## Claims

1. A compound represented by the formula 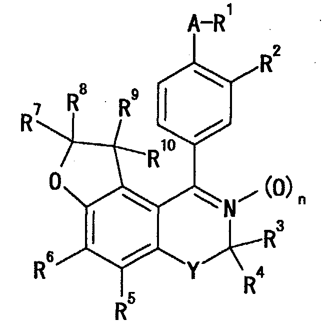 wherein A represents (1) a bond, (2) a group represented by the formula -CR^{a}=CR^{b}- (R^{a} and R^{b} each represent a hydrogen atom or a C₁₋₆ alkyl group), (3) a group represented by the formula-(CONH)ₚ-(C(R^{c}) (R^{d}))_{q}- (R^{c} and R^{d} each represent a hydrogen atom or a C₁₋₆ alkyl group, p represents 0 or 1 and q represents 1 or 2), (4) a group represented by the formula -CH₂OCH₂- or (5) a group represented by the formula -OCH₂-;
R¹ represents (1) a cyano group or (2) an optionally esterified or amidated carboxyl group;
R² represents (1) a hydrogen atom, (2) an optionally substituted hydroxy group, (3) an optionally substituted amino group, (4) an optionally substituted alkyl group, (5) an optionally esterified or amidated carboxyl group or (6) a nitro group, or R² and A or R¹ may be taken together with the adjacent carbon atom to form a ring;
R³ and R⁴ each represent (1) a hydrogen atom, (2) an optionally substituted hydrocarbon group or (3) an acyl group, or R³ and R⁴ may be taken together with the adjacent carbon atom to form an optionally substituted 3- to 8-membered ring;
R⁵ represents (1) a hydrogen atom, (2) a cyano group, (3) an optionally substituted hydrocarbon group, (4) an acyl group or (5) an optionally substituted hydroxy group;
R⁶ represents (1) a hydrogen atom, (2) an optionally substituted hydrocarbon group, (3) an acyl group, (4) an optionally substituted heterocyclic group, (5) a halogen atom, (6) an optionally substituted hydroxy group, (7) an optionally substituted thiol group, (8) a group represented by the formula-S(O)ᵣR¹¹ (R¹¹ represents an optionally substituted hydrocarbon group or an optionally substituted heterocyclic group and r is 1 or 2) or (9) an optionally substituted amino group;
R⁷ and R⁸ each represent (1) a hydrogen atom or (2) an optionally substituted hydrocarbon group, or R⁷ and R⁸ may be taken together with the adjacent carbon atom to form an optionally substituted 3- to 8-membered ring;
R⁹ and R¹⁰ each represent (1) a hydrogen atom or (2) an optionally substituted hydrocarbon group;
Y represents an optionally substituted methylene group; and
n represents 0 or 1,
provided that if A is a bond, R² is not a hydrogen atom, and if A is a group represented by the formula -(CONH)ₚ-(C(R^{c})(R^{d}))_{q}- (R^{c} and R^{d} each represent a hydrogen atom or a C₁₋₆ alkyl group, p represents 0 or 1 and q represents 1 or 2), R⁶ is not methoxy, or a salt thereof.

2. The compound according to claim 1, wherein R¹ is (i) a cyano group, (ii) a carboxyl group, (iii) a C₁₋₆ alkoxy-carbonyl group which may have 1 to 5 substituents selected from a group consisting of (1) a halogen atom, (2) a C₁₋₃ alkylenedioxy group, (3) a nitro group, (4) a cyano group, (5) an optionally halogenated C₁₋₆ alkyl group, (6) an optionally halogenated C₂₋₆ alkenyl group, (7) an optionally halogenated C₂₋₆ alkynyl group, (8) a C₃₋₈ cycloalkyl group, (9) a C₆₋₁₄ aryl group, (10) an optionally halogenated C₁₋₆ alkoxy group, (11) an optionally halogenated C₁₋₆ alkylthio group, (12) a hydroxy group, (13) an amino group, (14) a mono-C₁₋₆ alkylamino group, (15) a mono-C₆₋₁₄ arylamino group, (16) a di-C₁₋₆ alkylamino group, (17) a di-C₆₋₁₄ arylamino group, (18) an acyl group selected from formyl, carboxyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, sulfino, sulfo, C₁₋₆ alkoxysulfinyl, C₆₋₁₄ aryloxysulfinyl, C₁₋₆ alkoxysulfonyl and C₆₋₁₄ aryloxysulfonyl, (19) an acylamino group selected from formylamino, C₁₋₆ alkyl-carboxamide, C₆₋₁₄ aryl-carboxamide, C₁₋₆ alkoxy-carboxamide, C₁₋₆ alkylsulfonylamino and C₆₋₁₄ arylsulfonylamino, (20) an acyloxy group selected from C₁₋₆ alkyl-carbonyloxy, C₆₋₁₄ aryl-carbonyloxy, C₁₋₆ alkoxy-carbonyloxy, mono-C₁₋₆ alkyl-carbamoyloxy, di-C₁₋₆ alkyl-carbamoyloxy, mono-C₆₋₁₄ aryl-carbamoyloxy, di-C₆₋₁₄ aryl-carbamoyloxy and nicotinoyloxy, (21) a 5- to 14-membered heterocyclic group containing 1 to 4 hetero atoms selected from a nitrogen atom, a sulfur atom and an oxygen atom in addition to carbon atoms, (22) a phosphono group, (23) a C₆₋₁₄ aryloxy group, (24) a di-C₁₋₆ alkoxy-phosphoryl group, (25) a C₆₋₁₄ arylthio group, (26) a hydrazino group, (27) an imino group, (28) an oxo group, (29) an ureido group, (30) a C₁₋₆ alkyl-ureido group, (31) a di-C₁₋₆ alkyl-ureido group, (32) an oxide group and (33) a group formed by binding of 2 or 3 groups selected from (1) to (32) listed above and the like (hereinafter, abbreviated as Substituent group A), (iv) a C₃₋₈ cycloalkyloxy-carbonyl group which may have 1 to 5 substituents selected from Substituent group A described above, (v) a C₇₋₁₆ aralkyloxy-carbonyl group which may have 1 to 5 substituents selected from Substituent group A described above, (vi) a C₆₋₁₄ aryloxy-carbonyl group which may have 1 to 5 substituents selected from Substituent group A described above, (vii) a carbamoyl group, (viii) a mono-C₁₋₆ alkyl-carbamoyl group which may have 1 to 5 substituents selected from Substituent group A described above, (ix) a di-C₁₋₆ alkyl-carbamoyl group which may have 1 to 5 substituents selected from Substituent group A described above, (x) a mono-C₆₋₁₄ aryl-carbamoyl group which may have 1 to 5 substituents selected from Substituent group A described above or (xi) a di-C₆₋₁₄ aryl-carbamoyl group which may have 1 to 5 substituents selected from Substituent group A described above,
R² is (i) a hydrogen atom, (ii) a group represented by the formula -OR¹² (R¹² represents (a) a hydrogen atom, (b) a C₁₋₆ alkyl group, C₂₋₆ alkenyl group, C₂₋₆ alkynyl group, C₃₋₈ cycloalkyl group, C₃₋₈ cycloalkenyl group, C₆₋₁₄ aryl group or C₇₋₁₆ aralkyl group, each of which may have 1 to 5 substituents selected from Substituent group A described above, or (c) an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above), (iii) a group represented by the formula-NR¹³R¹⁴ (R¹³ and R¹⁴ are each (i') a hydrogen atom, (ii') a C₁₋₆ alkyl group, C₂₋₆ alkenyl group, C₂₋₆ alkynyl group, C₃₋₈ cycloalkyl group, C₃₋₈ cycloalkenyl group, C₆₋₁₄ aryl group or C₇₋₁₆ aralkyl group, each of which may have 1 to 5 substituents selected from Substituent group A described above, (iii') an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above or (iv') a 5- to 14-membered heterocycle containing 1 to 4 hetero atoms selected from a nitrogen atom, a sulfur atom and an oxygen atom in addition to carbon atoms, which may have 1 to 5 substituents selected from Substituent group A described above, or R¹³ and R¹⁴ may be taken together with the adjacent a nitrogen atom to form a 5- to 14-membered ring), (iv) a C₁₋₆ alkylideneamino group which may have 1 to 5 substituents selected from Substituent group A described above, (v) a C₁₋₆ alkyl group which may have 1 to 5 substituents selected from Substituent group A described above, (vi) a carboxyl group, (vii) a C₁₋₆ alkoxy-carbonyl group which may have 1 to 5 substituents selected from Substituent group A described above, (viii) a C₃₋₈ cycloalkyloxy-carbonyl group which may have 1 to 5 substituents selected from Substituent group A described above, (ix) a C₇₋₁₆ aralkyloxy-carbonyl group which may have 1 to 5 substituents selected from Substituent group A described above, (x) a C₆₋₁₄ aryloxy-carbonyl group which may have 1 to 5 substituents selected from Substituent group A described above, (xi) a carbamoyl group, (xii) a mono-C₁₋₆ alkyl-carbamoyl group which may have 1 to 5 substituents selected from Substituent group A described above, (xiii) a di-C₁₋₆ alkyl-carbamoyl group which may have 1 to 5 substituents selected from Substituent group A described above, (xiv) a mono-C₆₋₁₄ aryl-carbamoyl group which may have 1 to 5 substituents selected from Substituent group A described above, (xv) a di-C₆₋₁₄ aryl-carbamoyl group which may have 1 to 5 substituents selected from Substituent group A described above or (xvi) a nitro group, or R² and A or R¹ may be taken together to form a 5- to 14-membered ring containing 1 to 4 hetero atoms selected from a nitrogen atom and an oxygen atom in addition to carbon atoms, which may have 1 to 5 substituents selected from Substituent group A described above;
each of R³ and R⁴ is any of the following (i) to (iii):
(i) a hydrogen atom,
(ii) a C₁₋₆ alkyl group, C₂₋₆ alkenyl group, C₂₋₆ alkynyl group, C₃₋₈ cycloalkyl group, C₃₋₈ cycloalkenyl group, C₆₋₁₄ aryl group or C₇₋₁₆ aralkyl group, each of which may have 1 to 5 substituents selected from Substituent group A described above,
(iii) an acyl group selected from formyl, carboxyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, sulfino, sulfo, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above; or
R³ and R⁴ may be taken together with the adjacent carbon atom to form C₃₋₈ cycloalkane or a 3- to 8-membered heterocycle, which may have respectively 1 to 3 substituents selected from C₁₋₆ alkyl, C₆₋₁₄ aryl, C₇₋₁₆ aralkyl, amino, mono-C₁₋₆ alkylamino, mono-C₆₋₁₄ arylamino, di-C₁₋₆ alkylamino, di-C₆₋₁₄ arylamino and a 4- to 10-membered aromatic heterocyclic group,
R⁵ is (i) a hydrogen atom, (ii) a cyano group, (iii) a C₁₋₆ alkyl group, C₂₋₆ alkenyl group, C₂₋₆ alkynyl group, C₃₋₈ cycloalkyl group, C₃₋₈ cycloalkenyl group, C₆₋₁₄ aryl group or C₇₋₁₆ aralkyl group, each of which may have 1 to 5 substituents selected from Substituent group A described above, (iv) an acyl group selected from formyl, carboxyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, sulfino, sulfo, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above, or (v) a group represented by the formula -OR¹⁵ (R¹⁵ represents (a) a hydrogen atom, (b) a C₁₋₆ alkyl group, C₂₋₆ alkenyl group, C₂₋₆ alkynyl group, C₃₋₈ cycloalkyl group, C₃₋₈ cycloalkenyl group, C₆₋₁₄ aryl group or C₇₋₁₆ aralkyl group, each of which may have 1 to 5 substituents selected from Substituent group A described above, or (c) an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above),
R⁶ is any of the following (i) to (x):
(i) a hydrogen atom,
(ii) a C₁₋₆ alkyl group, C₂₋₆ alkenyl group, C₂₋₆ alkynyl group, C₃₋₈ cycloalkyl group, C₃₋₈ cycloalkenyl group, C₆₋₁₄ aryl group or C₇₋₁₆ aralkyl group, each of which may have 1 to 5 substituents selected from Substituent group A described above,
(iii) an acyl group selected from formyl, carboxyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, sulfino, sulfo, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above,
(iv) a 5- to 14-membered heterocycle containing 1 to 4 hetero atoms selected from a nitrogen atom, a sulfur atom and an oxygen atom in addition to carbon atoms, which may have 1 to 5 substituents selected from Substituent group A described above,
(v) a halogen atom,
(vi) a group represented by the formula -OR¹⁶ (R¹⁶ represents (i') a hydrogen atom, (ii') a C₁₋₆ alkyl group, C₂₋₆ alkenyl group, C₂₋₆ alkynyl group, C₃₋₈ cycloalkyl group, C₃₋₈ cycloalkenyl group, C₆₋₁₄ aryl group or C₇₋₁₆ aralkyl group, each of which may have 1 to 5 substituents selected from Substituent group A described above, (iii') an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above, or (iv') a 5- to 14-membered heterocycle containing 1 to 4 hetero atoms selected from a nitrogen atom, a sulfur atom and an oxygen atom in addition to carbon atoms, which may have 1 to 5 substituents selected from Substituent group A described above),
(vii) a group represented by the formula -SR¹⁷ (R¹⁷ represents (i') a hydrogen atom, (ii') a C₁₋₆ alkyl group, C₂₋₆ alkenyl group, C₂₋₆ alkynyl group, C₃₋₈ cycloalkyl group, C₃₋₈ cycloalkenyl group, C₆₋₁₄ aryl group or C₇₋₁₆ aralkyl group, each of which may have 1 to 5 substituents selected from Substituent group A described above, (iii') an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above or (iv') a 5- to 14-membered heterocycle containing 1 to 4 hetero atoms selected from a nitrogen atom, a sulfur atom and an oxygen atom in addition to carbon atoms, which may have 1 to 5 substituents selected from Substituent group A described above),
(viii) a group represented by the formula -S(O)ᵣR¹¹ (R¹¹ represents (i') a C₁₋₆ alkyl group, C₂₋₆ alkenyl group, C₂₋₆ alkynyl group, C₃₋₈ cycloalkyl group, C₃₋₈ cycloalkenyl group, C₆₋₁₄ aryl group or C₇₋₁₆ aralkyl group, each of which may have 1 to 5 substituents selected from Substituent group A described above or (ii') a 5- to 14-membered heterocycle containing 1 to 4 hetero atoms selected from a nitrogen atom, a sulfur atom and an oxygen atom in addition to carbon atoms, which may have 1 to 5 substituents selected from Substituent group A described above and r is 1 or 2) or
(ix) a group represented by the formula -NR¹⁸R¹⁹ (R¹⁸ and R¹⁹ each represent (i') a hydrogen atom, (ii') a C₁₋₆ alkyl group, C₂₋₆ alkenyl group, C₂₋₆ alkynyl group, C₃₋₈ cycloalkyl group, C₃₋₈ cycloalkenyl group, C₆₋₁₄ aryl group or C₇₋₁₆ aralkyl group, each of which may have 1 to 5 substituents selected from Substituent group A described above, (iii') an acyl group selected from formyl, carbamoyl, C₁₋₆ alkyl-carbonyl, C₃₋₈ cycloalkyl-carbonyl, C₁₋₆ alkoxy-carbonyl, C₆₋₁₄ aryl-carbonyl, C₇₋₁₆ aralkyl-carbonyl, C₆₋₁₄ aryloxy-carbonyl, C₇₋₁₆ aralkyloxy-carbonyl, 5- or 6-membered heterocyclic carbonyl containing 1 to 3 hetero atoms selected from a nitrogen atom, a sulfur atom and an oxygen atom in addition to carbon atoms, mono-C₁₋₆ alkyl-carbamoyl, di-C₁₋₆ alkyl-carbamoyl, mono-C₆₋₁₄ aryl-carbamoyl, di-C₆₋₁₄ aryl-carbamoyl, 5- or 6-membered heterocyclic carbamoyl containing 1 to 3 hetero atoms selected from a nitrogen atom, a sulfur atom and an oxygen atom in addition to carbon atoms, C₁₋₆ alkyl-thiocarbonyl, C₃₋₈ cycloalkyl-thiocarbonyl, C₁₋₆ alkoxy-thiocarbonyl, C₆₋₁₄ aryl-thiocarbonyl, C₇₋₁₆ aralkyl-thiocarbonyl, C₆₋₁₄ aryloxy-thiocarbonyl, C₇₋₁₆ aralkyloxy-thiocarbonyl, 5- or 6-membered heterocyclic thiocarbonyl containing 1 to 3 hetero atoms selected from a nitrogen atom, a sulfur atom and an oxygen atom in addition to carbon atoms, thiocarbamoyl, mono-C₁₋₆ alkyl-thiocarbamoyl, di-C₁₋₆ alkyl-thiocarbamoyl, mono-C₆₋₁₄ aryl-thiocarbamoyl, di-C₆₋₁₄ aryl-thiocarbamoyl, 5- or 6-membered heterocyclic thiocarbamoyl containing 1 to 3 hetero atoms selected from a nitrogen atom, a sulfur atom and an oxygen atom in addition to carbon atoms, sulfamoyl, mono-C₁₋₆ alkylsulfamoyl, di-C₁₋₆ alkylsulfamoyl, C₆₋₁₄ arylsulfamoyl, C₁₋₆ alkylsulfonyl, C₆₋₁₄ arylsulfonyl, C₁₋₆ alkylsulfinyl, C₆₋₁₄ arylsulfinyl, C₁₋₆ alkoxysulfinyl, C₆₋₁₄ aryloxysulfinyl, C₁₋₆ alkoxysulfonyl and C₆₋₁₄ aryloxysulfonyl, which may have 1 to 5 substituents selected from Substituent group A described above or (iv') a 5- to 14-membered heterocycle containing 1 to 4 hetero atoms selected from a nitrogen atom, a sulfur atom and an oxygen atom in addition to carbon atoms, which may have 1 to 5 substituents selected from Substituent group A described above),
R⁷ and R⁸ are each (i) a hydrogen atom or (ii) a C₁₋₆ alkyl group, C₂₋₆ alkenyl group, C₂₋₆ alkynyl group, C₃₋₈ cycloalkyl group, C₃₋₈ cycloalkenyl group, C₆₋₁₄ aryl group or C₇₋₁₆ aralkyl group, each of which may have 1 to 5 substituents selected from Substituent group A described above, or R⁷ and R⁸ may be taken together with the adjacent carbon atom to form C₃₋₈ cycloalkane or a 3- to 8-membered heterocycle, which may have respectively 1 to 3 substituents selected from C₁₋₆ alkyl, C₆₋₁₄ aryl, C₇₋₁₆ aralkyl, amino, mono-C₁₋₆ alkylamino, mono-C₆₋₁₄ arylamino, di-C₁₋₆ alkylamino, di-C₆₋₁₄ arylamino and a 4- to 10-membered aromatic heterocyclic group;
R⁹ and R¹⁰ are each (i) a hydrogen atom or (ii) a C₁₋₆ alkyl group, C₂₋₆ alkenyl group, C₂₋₆ alkynyl group, C₃₋₈ cycloalkyl group, C₃₋₈ cycloalkenyl group, C₆₋₁₄ aryl group or C₇₋₁₆ aralkyl group, each of which may have 1 to 5 substituents selected from Substituent group A described above, and
Y is a methylene group which may have 1 or 2 substituents selected from Substituent group A described above.

3. The compound according to claim 1, wherein A is (1) a bond, (2) a group represented by the formula -CR^{a}=CR^{b}- (R^{a} and R^{b} each represent a hydrogen atom or a C₁₋₆ alkyl group), (3) a group represented by the formula -(CONH)ₚ-(C(R^{c}) (R^{d}))_{q}- (R^{c} and R^{d} each represent a hydrogen atom or a C₁₋₆ alkyl group, p represents 0 or 1 and q represent s 1 or 2), (4) a group represented by the formula -CH₂OCH₂- or (5) a group represented by the formula -OCH₂-,
R¹ is (1) a cyano group, (2) a carboxyl group, (3) a C₁₋₆ alkoxycarbonyl group, (4) a carbamoyl group or (5) an N-mono-C₁₋₆ alkylcarbamoyl group,
R² is (1) a hydrogen atom, (2) a hydroxy group, (3) a C₁₋₆ alkoxy group, (4) a C₇₋₁₆ aralkyloxy group, (5) an amino group, (6) a mono-C₁₋₆ alkylamino group which may have one substituent selected from carboxyl, carbamoyl, quinolyl, phenoxy and pyridyl, (7) a mono-C₇₋₁₆ aralkylamino group which may have one substituent selected from a halogen atom, cyano, C₁₋₆ alkoxy, carboxyl and C₁₋₆ alkoxycarbonyl, (8) a mono-C₆₋₁₄ arylamino group, (9) a mono-C₁₋₆ alkylcarbonylamino group which may have 1 to 3 substituents selected from a halogen atom, thienyl and C₁₋₆ alkoxycarbonyl-C₁₋₆ alkylthio, (10) a mono-C₁₋₆ alkylsulfonylamino group, (11) a mono-C₆₋₁₄ arylcarbonylamino group which may have one substituent selected from C₁₋₆ alkoxy and C₁₋₆ alkylcarbonylamino, (12) a quinolylcarbonylamino group, (13) a pyridylcarbonylamino group which may have 1 or 2 halogen atoms, (14) an indolylcarbonylamino group, (15) a N-C₁₋₆ alkyl-N-C₁₋₆ alkylcarbonylamino group which may have 1 to 4 substituents selected from a halogen atom, C₁₋₆ alkoxycarbonyl and quinolyl, (16) a N-C₁₋₆ alkylcarbonyl-N-C₇₋₁₆ aralkylamino group which may have 1 to 3 halogens, (17) a N-C₁₋₆ alkyl-N-pyridylcarbonylamino group, (18) a C₁₋₆ alkylideneamino group which may have one di-C₁₋₆ alkylamino, (19) a mono-C₁₋₆ alkylureido group which may have one C₁₋₆ alkoxycarbonyl, (20) a di-C₁₋₆ alkylureido, (21) a mono-C₆₋₁₄ arylureido group, (22) a 1-imidazolidinyl group which may have 1 to 3 substituents selected from C₁₋₆ alkyl and oxo, (23) a C₁₋₆ alkyl group, (24) a C₁₋₆ alkoxycarbonyl group, (25) a nitro group or (26) a 1-pyrrolidinyl group, or
R² and A or R¹ may be taken together with the adjacent carbon atom to form a nitrogen-containing 5- to 7-membered ring which may have 1 to 3 substituents selected from (1) a hydroxy group, (2) C₁₋₆ alkyl which may have one C₁₋₆ alkoxy-carbonyl, (3) C₇₋₁₆ aralkyl, (4) C₆₋₁₄ aryl and (5) oxo,
R³ and R⁴ are each a C₁₋₆ alkyl group,
R⁵ is a hydrogen atom,
R⁶ is a C₁₋₆ alkoxy group,
R⁷ and R⁸ are each a C₁₋₆ alkyl group,
R⁹ and R¹⁰ are each a hydrogen atom,
Y is a methylene group and
n is 0.

4. A compound represented by the formula 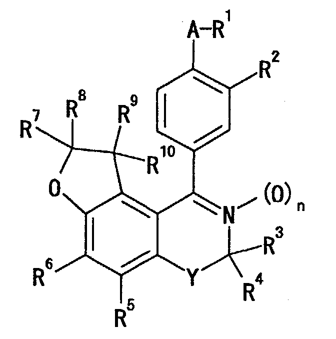 wherein A is (1) a bond, (2) a group represented by the formula -CH=CH-, (3) a group represented by the formula -CONH-C(R^{c}) (R^{d})- (R^{c} and R^{d} are each a hydrogen atom or a C₁₋₆ alkyl group), or (4) a group represented by the formula -OCH₂-,
R¹ is (1) a cyano group, (2) a carboxyl group, (3) a C₁₋₆ alkoxycarbonyl group, (4) a carbamoyl group or (5) an N-mono-C₁₋₆ alkylcarbamoyl group,
R² is (1) a hydroxy group, (2) a C₁₋₆ alkoxy group, (3) a C₇₋₁₆ aralkyloxy group, (4) an amino group, (5) a mono-C₁₋₆ alkylamino group which may have one substituent selected from carboxyl, carbamoyl, quinolyl, phenoxy and pyridyl, (6) a mono-C₇₋₁₆ aralkylamino group which may have one substituent selected from a halogen atom, cyano, C₁₋₆ alkoxy, carboxyl and C₁₋₆ alkoxycarbonyl, (7) a mono-C₆₋₁₄ arylamino group, (8) a mono-C₁₋₆ alkylcarbonylamino group which may have 1 to 3 substituents selected from a halogen atom, thienyl and C₁₋₆ alkoxycarbonyl-C₁₋₆ alkylthio, (9) a mono-C₁₋₆ alkylsulfonylamino group, (10) a mono-C₆₋₁₄ arylcarbonylamino group which may have one substituent selected from C₁₋₆ alkoxy and C₁₋₆ alkylcarbonylamino, (11) a quinolylcarbonylamino group, (12) a pyridylcarbonylamino group which may have 1 or 2 halogen atoms, (13) an indolylcarbonylamino group, (14) a N-C₁₋₆ alkyl-N-C₁₋₆ alkylcarbonylamino group which may have 1 to 4 substituents selected from a halogen atom, C₁₋₆ alkoxycarbonyl and quinolyl, (15) a N-C₁₋₆ alkylcarbonyl-N-C₇₋₁₆ aralkylamino group which may have 1 to 3 halogens, (16) a N-C₁₋₆ alkyl-N-pyridylcarbonylamino group, (17) a C₁₋₆ alkylideneamino group which may have one di-C₁₋₆ alkylamino, (18) a mono-C₁₋₆ alkylureido group which may have one C₁₋₆ alkoxycarbonyl, (19) a di-C₁₋₆ alkylureido group, (20) a mono-C₆₋₁₄ arylureido group, (21) a 1-imidazolidinyl group which may have 1 to 3 substituents selected from C₁₋₆ alkyl and oxo, (22) a C₁₋₆ alkyl group, (23) a C₁₋₆ alkoxycarbonyl group, (24) a nitro group or (25) a 1-pyrrolidinyl group, or R² and A or R¹ may be taken together with the adjacent carbon atom to form a nitrogen-containing 5- to 7-membered ring which may have 1 to 3 substituents selected from (1) a hydroxy group, (2) a C₁₋₆ alkyl group which may have one C₁₋₆ alkoxy-carbonyl, (3) a C₇₋₁₆ aralkyl group, (4) a C₆₋₁₄ aryl group and (5) an oxo group,
R³ and R⁴ are each a C₁₋₆ alkyl group,
R⁵ is a hydrogen atom,
R⁶ is a C₂₋₆ alkoxy group,
R⁷ and R⁸ are each a C₁₋₆ alkyl group,
R⁹ and R¹⁰ are each a hydrogen atom,
Y is a methylene group, and
n is 0, or a salt thereof.

5. A compound represented by the formula 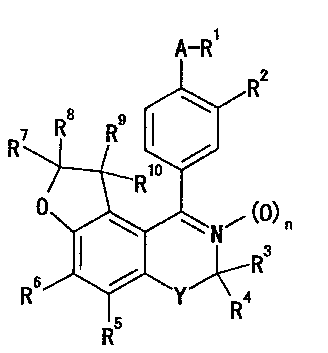 wherein A is (1) a group represented by the formula -CR^{a}=CR^{b}- (R^{a} and R^{b} are each a hydrogen atom or a C₁₋₆ alkyl group), (2) a group represented by the formula -(CONH)ₚ-(C(R^{c}) (R^{d}))_{q}- (R^{c} and R^{d} are each a hydrogen atom or a C₁₋₆ alkyl group, p is 0 or 1 and q is 1 or 2), (3) a group represented by the formula -CH₂OCH₂- or (4) a group represented by the formula -OCH₂-,
R¹ is (1) a carboxyl group, (2) a C₁₋₆ alkoxycarbonyl group, (3) an N-mono-C₁₋₆ alkylcarbamoyl group or (4) a carbamoyl group,
R² is a hydrogen atom,
R³ and R⁴ are each a C₁₋₆ alkyl group,
R⁵ is a hydrogen atom,
R⁶ is a C₂₋₆ alkoxy group,
R⁷ and R⁸ are each a C₁₋₆ alkyl group,
R⁹ and R¹⁰ are each a hydrogen atom,
Y is a methylene group, and
n is 0, or a salt thereof.

6. The compound according to claim 4, wherein A is (1) a bond or (2) a group represented by the formula -CH=CH-.

7. The compound according to claim 5, wherein A is (1) a group represented by the formula -CH=CH-, (2) a group represented by the formula -(C(R^{c}) (R^{d}))- (R^{c} and R^{d} each represent a hydrogen atom or a C₁₋₆ alkyl group) or (3) a group represented by the formula -CH₂OCH₂-.

8. The compound according to claim 4, wherein R¹ is a carboxyl group or a carbamoyl group.

9. The compound according to claim 5, wherein R¹ is a carboxyl group.

10. The compound according to claim 4, wherein R² is (1) a C₁₋₆ alkoxy group, (2) a mono-C₁₋₆ alkylamino group, (3) a mono-C₇₋₁₆ aralkylamino group, (4) a quinolylcarbonylamino group or (5) a pyridylcarbonylamino group.

11. The compound according to claim 4 or 5, wherein R³ and R⁴ are each methyl.

12. The compound according to claim 4 or 5, wherein R⁶ is ethoxy.

13. The compound according to claim 4 or 5, wherein R⁷ and R⁸ are each methyl.

14. The compound according to claim 4, which is 4-(6-ethoxy-3,4,8,9-tetrahydro-3,3,8,8-tetramethylfuro[2,3-h]isoquinolin-1-yl)-2-[(phenylmethyl)amino]benzoic acid, 4-(6-ethoxy-3,4,8,9-tetrahydro-3,3,8,8-tetramethylfuro[2,3-h]isoquinolin-1-yl)-2-(ethylamino)benzoic acid, (E)-3-[4-(6-ethoxy-3,4,8,9-tetrahydro-3,3,8,8-tetramethylfuro[2,3-h]isoquinolin-1-yl)-2-methoxyphenyl]-2-propenoic acid, 4-(6-ethoxy-3,4,8,9-tetrahydro-3,3,8,8-tetramethylfuro[2,3-h]isoquinolin-1-yl)-2-[(2-quinolinylcarbonyl)amino]benzoic acid, 4-(6-ethoxy-3,4,8,9-tetrahydro-3,3,8,8-tetramethylfuro[2,3-h]isoquinolin-1-yl)-2-[(2-pyridinylcarbonyl)amino]benzene acetic acid, N-[2-(aminocarbonyl)-5-(6-ethoxy-3,4,8,9-tetrahydro-3,3,8,8-tetramethylfuro[2,3-h]isoquinolin-1-yl)phenyl]-2-pyridinecarboxamide or a salt thereof.

15. The compound according to claim 5, which is [[4-(6-ethoxy-3,4,8,9-tetrahydro-3,3,8,8-tetramethylfuro[2,3-h]isoquinolin-1-yl)phenyl]methoxy]acetic acid, 4-(6-ethoxy-3,4,8,9-tetrahydro-3,3,8,8-tetramethylfuro[2,3-h]isoquinolin-1-yl)-α,α-dimethylbenzene acetic acid or a salt thereof.

16. The pharmaceutical composition comprising the compound according to claim 1 or a prodrug thereof.

17. The pharmaceutical composition according to claim 16, which is a phosphodiesterase IV inhibitor.

18. The pharmaceutical composition according to claim 16, which is a prophylactic and/or therapeutic agent against inflammatory diseases, atopic dermatitis, allergic rhinitis, asthma, chronic obstructive pulmonary diseases, chronic rheumatoid arthritis, autoimmune diseases, depression, Alzheimer's dementia, memory disorders, osteoporosis, diabetes or atherosclerosis.

19. A method of inhibiting phosphodiesterase IV, which comprises administering to a mammal an effective amount of the compound according to claim 1 or a prodrug thereof.

20. A method of preventing and/or treating inflammatory diseases, atopic dermatitis, allergic rhinitis, asthma, chronic obstructive pulmonary diseases, chronic rheumatoid arthritis, autoimmune diseases, depression, Alzheimer's dementia, memory disorders, osteoporosis, diabetes or atherosclerosis, which comprises administering to a mammal an effective amount of the compound according to claim 1 or a prodrug thereof.

21. Use of the compound according to claim 1 or a prodrug thereof for manufacturing a phosphodiesterase IV inhibitor.

22. Use of the compound according to claim 1 or a prodrug thereof for manufacturing a prophylactic and/or therapeutic agent against inflammatory diseases, atopic dermatitis, allergic rhinitis, asthma, chronic obstructive pulmonary diseases, chronic rheumatoid arthritis, autoimmune diseases, depression, Alzheimer's dementia, memory disorders, osteoporosis, diabetes or atherosclerosis.
